# EUROPEAN PATENT APPLICATION

(11) **EP 2 028 190 A1**
(43) Date of publication of application: **25.02.2009**
(21) Application number: 08159359.2
(22) Date of filing: 03.04.2000
(51) Int. Cl.: C07K 14/47, C12N 15/12, C12N 15/10, C12N 15/62, C07K 19/00, G01N 33/53, C12N 15/11, C12Q 1/68, A61K 39/395, A61K 38/17, A61P 35/00

(54) **Compounds and methods for therapy and diagnosis of lung cancer**

(30) Priority: 02.04.1999 US 285479; 17.12.1999 US 466396; 30.12.1999 US 476496; 10.01.2000 US 480884; 22.02.2000 US 510376
(62) Divisional of application: 00920102.1
(71) Applicant: CORIXA CORPORATION, Seattle, WA 98104 (US)
(72) Inventor: Wang, Tongtong, Indianapolis, IN 46032 (US); Fan, Liqun, Bellevue, WA 98006 (US)
(74) Representative: Walker, Ross Thomson

(57) **Abstract**

Compounds and methods for the treatment and diagnosis of lung cancer are provided. The inventive compounds include polypeptides containing at least a portion of a lung tumor protein. Vaccines and pharmaceutical compositions for immunotherapy of lung cancer comprising such polypeptides, or DNA molecules encoding such polypeptides, are also provided, together with DNA molecules for preparing the inventive polypeptides.

SEQUENCE LISTING

<110> Corixa Corporation et al.

<120> COMPOUNDS AND METHODS FOR THERAPY
AND DIAGNOSIS OF LUNG CANCER

<130> 210121.45501PC

<140> PCT
<141> 2000-04-03

<160> 350

<170> FastSEQ for Windows Version 3.0

<210> 1
<211> 315
<212> DNA
<213> Homo sapien

<220>
<221> misc_feature
<222> (1)...(315)
<223> n = A,T,C or G

<400> 1
gcagagacag actggtggtt gaacctggag gtgccaaaaa agccagctgc gggcccagga 60
cagctgccgt gagactcccg atgtcacagg cagtctgtgt ggttacagcg cccctcagtg 120
ttcatctcca gcagagacaa cggaggaggc tcccaccagg acggttctca ttatttatat 180
gttaatatgt ttgtaaactc atgtacagtt ttttttgggg gggaagcaat gggaanggta 240
naaattacaa atagaatcat ttgctgtaat ccttaaatgg caaacggtca ggccacgtga 300
aaaaaaaaaa aaaaa 315

<210> 2
<211> 380
<212> DNA
<213> Homo sapien

<400> 2
atttaggctt aagattttgt ttacccttgt tactaaggag caaattagta ttaaagtata 60
atatatataa acaaatacaa aaagttttga gtggttcagc ttttttattt tttttaatgg 120
cataactttt aacaacactg ctctgtaatg ggttgaactg tggtactcag actgagataa 180
ctgaaatgag tggatgtata gtgttattgc ataattatcc cactatgaag caaagggact 240
ggataaattc ccagtctaga ttattagcct ttgttaacca tcaagcacct agaagaagaa 300
ttattggaaa ttttgtcctc tgtaactggc actttggggt gtgacttatc ttttgccttt 360
gtaaaaaaaa aaaaaaaaaa 380

<210> 3
<211> 346
<212> DNA
<213> Homo sapien

<220>
<221> misc_feature

<220>
<221> misc_feature
<222> (1)...(346)
<223> n = A,T,C or G

<400> 3
ttgtaagtat acaattttag aaaggattaa atgttattga tcattttact gaatactgca 60
catcctcacc atacaccatc cactttccaa taacatttaa tcctttctaa aattgtaagt 120
atacaattgt actttctttg gattttcata acaaatatac catagactgt taattttatt 180
gaagtttcct taatggaatg agtcattttt gtcttgtgct tttgaggtta cctttgcttt 240
gacttccaac aatttgatca tatagtgttg agctgtggaa atctttaagt ttattctata 300
gcaataattt ctattnnnag annccnggnn naaaannann annaaa 346

<210> 4
<211> 372
<212> DNA
<213> Homo sapien

<220>
<221> misc_feature
<222> (1)...(372)
<223> n = A,T,C or G

<400> 4
actagtctca ttactccaga attatgctct tgtacctgtg tggctgggtt tcttagtcgt 60
tggtttggtt tggttttttg aactggtatg tagggtggtt cacagttcta atgtaagcac 120
tctcttctcc aagttgtgct ttgtggggac aatcattctt tgaacattag agaggaaggc 180
agttcaagct gttgaaaaga ctattgctta tttttgtttt taaagaccta cttgacgtca 240
tgtggacagt gcacgtgcct tacgctacat cttgttttct aggaagaagg ggatgcnggg 300
aaggantggg tgctttgtga tggataaaac gnctaaataa cacaccttta cattttgaaa 360
aaaacaaaac aa 372

<210> 5
<211> 698
<212> DNA
<213> Homo sapien

<220>
<221> misc_feature
<222> (1)...(698)
<223> n = A,T,C or G

<400> 5
actagtanga tagaaacact gtgtcccgag agtaaggaga gaagctacta ttgattagag 60
cctaacccag gttaactgca agaagaggcg ggatactttc agctttccat gtaactgtat 120
gcataaagcc aatgtagtcc agtttctaag atcatgttcc aagctaactg aatcccactt 180
caatacacac tcatgaactc ctgatggaac aataacaggc ccaagcctgt ggtatgatgt 240
gcacacttgc tagactcaga aaaaatacta ctctcataaa tgggtgggag tattttgggt 300
gacaacctac tttgcttggc tgagtgaagg aatgatattc atatnttcat ttattccatg 360
gacatttagt tagtgctttt tatataccag gcatgatgct gagtgacact cttgtgtata 420
tntccaaatn ttngtncngt cgctgcacat atctgaaatc ctatattaag antttcccaa 480
natgangtcc ctggtttttc cacgccactt gatcngtcaa ngatctcacc tctgtntgtc 540
ctaaaaccnt ctnctnnang gttagacngg acctctcttc tcccttcccg aanaatnaag 600
tgtgngaaga nanccncncn cccccctncn tncnncctng ccngctnnnc cncntgtngg 660

gggngccgcc cccgcggggg gacccccccn ttttcccc 698

<210> 6
<211> 740
<212> DNA
<213> Homo sapien

<220>
<221> misc_feature
<222> (1)...(740)
<223> n = A,T,C or G

<400> 6
actagtcaaa aatgctaaaa taatttggga gaaaatattt tttaagtagt gttatagttt 60
catgtttatc ttttattatg tnttgtgaag ttgtgtcttt tcactaatta cctatactat 120
gccaatattt ccttatatct atccataaca tttatactac atttgtaaga gaatatgcac 180
gtgaaactta acactttata aggtaaaaat gaggtttcca agatttaata atctgatcaa 240
gttcttgtta tttccaaata gaatggactt ggtctgttaa ggggctaagg gagaagaaga 300
agataaggtt aaaagttgtt aatgaccaaa cattctaaaa gaaatgcaaa aaaaaattta 360
ttttcaagcc ttcgaactat ttaaggaaag caaaatcatt tcctanatgc atatcatttg 420
tgagantttc tcantaatat cctgaatcat tcatttcagc tnaggcttca tgttgactcg 480
atatgtcatc tagggaaagt ctatttcatg gtccaaacct gttgccatag ttggtnaggc 540
tttcctttaa ntgtgaanta ttnacangaa attttctctt tnanagttct tnatagggtt 600
aggggtgtgg gaaaagcttc taacaatctg tagtgttncg tgttatctgt ncagaaccan 660
aatnacggat cgnangaagg actgggtcta tttacangaa cgaatnatct ngttnnntgt 720
gtnnncaact ccngggagcc 740

<210> 7
<211> 670
<212> DNA
<213> Homo sapien

<220>
<221> misc_feature
<222> (1)...(670)
<223> n = A,T,C or G

<400> 7
gctggggagc tcggcatggc ggtccccgct gcagccatgg ggccctcggc gttgggccag 60
agcggccccg gctcgatggc cccgtggtgc tcagtgagca gcggcccgtc gcgctacgtg 120
cttgggatgc aggagctgtt ccggggccac agcaagaccg cgagttcctg gcgcacagcg 180
ccaaggtgca ctcggtggcc tggagttgcg acgggcgtcg cctacctcgg ggtcttcgac 240
aagacgccac gtcttcttgc tgganaanga ccgttggtca aagaaaacaa ttatcgggga 300
catggggata gtgtggacca ctttgttggc atccaagtaa tcctgaccta tttgttacgg 360
cgtctggaga taaaaccatt cgcatctggg atgtgaggac tacaaaatgc attgccactg 420
tgaacactaa aggggagaac attaatatct gctggantcc tgatgggcan accattgctg 480
tagcnacaag gatgatgtgg tgactttatt gatgccaaga aaccccgttc caaagcaaaa 540
aaacanttcc aanttcgaag tcaccnaaat ctcctggaac aatgaacatn aatatnttct 600
tcctgacaat ggnccttggg tgtntcacat cctcagctnc cccaaaactg aancctgtnc 660
natccacccc 670

<210> 8
<211> 689
<212> DNA
<213> Homo sapien

<220>
<221> misc_feature
<222> (1)...(689)
<223> n = A,T,C or G

<400> 8
   actagtatct aggaatgaac agtaaaagag gagcagttgg ctacttgatt acaacagagt 60
   aaatgaagta ctggatttgg gaaaacctgg ttttattaga acatatggaa tgaaagccta 120
   cacctagcat tgcctactta gccccctgaa ttaacagagc ccaattgaga caaacccctg 180
   gcaacaggaa attcaaggga gaaaaagtaa gcaacttggg ctaggatgag ctgactccct 240
   tagagcaaag ganagacagc ccccattacc aaataccatt tttgcctggg gcttgtgcag 300
   ctggcagtgt tcctgcccca gcatggcacc ttatngtttt gatagcaact tcgttgaatt 360
   ttcaccaact tattacttga aattataata tagcctgtcc gtttgctgtn tccaggctgt 420
   gatatatntt cctagtggtt tgactttnaa aataaatnag gtttantttt ctccccccnn 480
   cnntnctncc nntcnctcnn cnntcccccc cnctcngtcc tccnnnnttn gggggggccn 540
   cccccncggn ggacccccct ttggtccctt agtggaggtt natggcccct ggnnttatcc 600
   nggccntann tttccccgtn nnaaatgntt ccccctccca ntcccnccac ctcaanccgg 660
   aagcctaagt ttntaccctg ggggtcccc 689

<210> 9
<211> 674
<212> DNA
<213> Homo sapien

<220>
<221> misc_feature
<222> (1)...(674)
<223> n = A,T,C or G

<400> 9
gtccactctc ctttgagtgt actgtcttac tgtgcactct gtttttcaac tttctagata 60
taaaaaatgc ttgttctata gtggagtaag agctcacaca cccaaggcag caagataact 120
gaaaaaagcg aggctttttt gccaccttgg taaaggccag ttcactgcta tagaactgct 180
ataagcctga agggaagtag ctatgagact ttccattttt cttagttctc ccaataggct 240
ccttcatgga aaaaggcttc ctgtaataat tttcacctaa tgaattagca gtgtgattat 300
ttctgaaata agagacaaat tgggccgcag agtcttcctg tgatttaaaa taaacaaccc 360
aaagttttgt ttggtcttca ccaaaggaca tactctaggg ggtatgttgt tgaagacatt 420
caaaaacatt agctgttctg tctttcaatt tcaagttatt ttggagactg cctccatgtg 480
agttaattac tttgctctgg aactagcatt attgtcatta tcatcacatt ctgtcatcat 540
catctgaata atattgtgga tttccccctc tgcttgcatc ttcttttgac tcctctggga 600
anaaatgtca aaaaaaaagg tcgatctact cngcaaggnc catctaatca ctgcgctgga 660
aggacccnct gccc 674

<210> 10
<211> 346
<212> DNA
<213> Homo sapien

<220>
<221> misc_feature
<222> (1)...(346)
<223> n = A,T,C or G

<400> 10

actagtctgc tgatagaaag cactatacat cctattgttt ctttctttcc aaaatcagcc 60
ttctgtctgt aacaaaaatg tactttatag agatggagga aaaggtctaa tactacatag 120
ccttaagtgt ttctgtcatt gttcaagtgt attttctgta acagaaacat atttggaatg 180
tttttctttt ccccttataa attgtaattc ctgaaatact gctgctttaa aaagtcccac 240
tgtcagatta tattatctaa caattgaata ttgtaaatat acttgtctta cctctcaata 300
aaagggtact tttctattan nnagnngnnn gnnnnataaa anaaaa 346

<210> 11
<211> 602
<212> DNA
<213> Homo sapien

<400> 11
actagtaaaa agcagcattg ccaaataatc cctaattttc cactaaaaat ataatgaaat 60
gatgttaagc tttttgaaaa gtttaggtta aacctactgt tgttagatta atgtatttgt 120
tgcttccctt tatctggaat gtggcattag cttttttatt ttaaccctct ttaattctta 180
ttcaattcca tgacttaagg ttggagagct aaacactggg atttttggat aacagactga 240
cagttttgca taattataat cggcattgta catagaaagg atatggctac cttttgttaa 300
atctgcactt tctaaatatc aaaaaaggga aatgaagtta taaatcaatt tttgtataat 360
ctgtttgaaa catgagtttt atttgcttaa tattagggct ttgccccttt tctgtaagtc 420
tcttgggatc ctgtgtagaa ctgttctcat taaacaccaa acagttaagt ccattctctg 480
gtactagcta caaattcggt ttcatattct acttaacaat ttaaataaac tgaaatattt 540
ctagatggtc tacttctgtt catataaaaa caaaacttga tttccaaaaa aaaaaaaaaa 600
aa 602

<210> 12
<211> 685
<212> DNA
<213> Homo sapien

<220>
<221> misc_feature
<222> (1)...(685)
<223> n = A,T,C or G

<400> 12
actagtcctg tgaaagtaca actgaaggca gaaagtgtta ggattttgca tctaatgttc 60
attatcatgg tattgatgga cctaagaaaa taaaaattag actaagcccc caaataagct 120
gcatgcattt gtaacatgat tagtagattt gaatatatag atgtagtatn ttgggtatct 180
aggtgtttta tcattatgta aaggaattaa agtaaaggac tttgtagttg tttttattaa 240
atatgcatat agtagagtgc aaaaatatag caaaaatana aactaaaggt agaaaagcat 300
tttagatatg ccttaatnta nnaactgtgc caggtggccc tcggaataga tgccaggcag 360
agaccagtgc ctgggtggtg cctccccttg tctgcccccc tgaagaactt ccctcacgtg 420
angtagtgcc ctcgtaggtg tcacgtggan tantggganc aggccgnncn gtnanaagaa 480
ancanngtga nagtttcncc gtngangcng aactgtccct gngccnnnac gctcccanaa 540
cntntccaat ngacaatcga gtttccnnnc tccngnaacc tngccgnnnn cnngcccnnc 600
cantntgnta accccgcgcc cggatcgctc tcnnntcgtt ctcncncnaa ngggntttcn 660
cnnccgccgt cncnnccccg cnncc 685

<210> 13
<211> 694
<212> DNA
<213> Homo sapien

<220>

<221> misc_feature
<222> (1)...(694)
<223> n = A,T,C or G

<400> 13
cactagtcac tcattagcgt tttcaatagg gctcttaagt ccagtagatt acgggtagtc 60
agttgacgaa gatctggttt acaagaacta attaaatgtt tcattgcatt tttgtaagaa 120
cagaataatt ttataaaatg tttgtagttt ataattgccg aaaataattt aaagacactt 180
tttctctgtg tgtgcaaatg tgtgtttgtg atccattttt tttttttttt taggacacct 240
gtttactagc tagctttaca atatgccaaa aaaggatttc tccctgaccc catccgtggt 300
tcaccctctt ttccccccat gctttttgcc ctagtttata acaaaggaat gatgatgatt 360
taaaaagtag ttctgtatct tcagtatctt ggtcttccag aaccctctgg ttgggaaggg 420
gatcattttt tactggtcat ttccctttgg agtgtactac tttaacagat ggaaagaact 480
cattggccat ggaaacagcc gangtgttgg gagccagcag tgcatggcac cgtccggcat 540
ctggcntgat tggtctggct gccgtcattg tcagcacagt gccatgggac atggggaana 600
ctgactgcac ngccaatggt tttcatgaag aatacngcat ncncngtgat cacgtnancc 660
angacgctat gggggncana gggccanttg cttc 694

<210> 14
<211> 679
<212> DNA
<213> Homo sapien

<220>
<221> misc_feature
<222> (1)...(679)
<223> n = A,T,C or G

<400> 14
cagccgcctg catctgtatc cagcgccang tcccgccagt cccagctgcg cgcgcccccc 60
agtcccgnac ccgttcggcc cangctnagt tagncctcac catnccggtc aaaggangca 120
ccaagtgcat caaatacctg cngtncggat ntaaattcat cttctggctt gccgggattg 180
ctgtccntgc cattggacta nggctccgat ncgactctca gaccanganc atcttcganc 240
naganactaa tnatnattnt tccagcttct acacaggagt ctatattctg atcggatccg 300
gcnccctcnt gatgctggtg ggcttcctga gctgctgcgg ggctgtgcaa gagtcccant 360
gcatgctggg actgttcttc ggcttcntct tggtgatatn cgccattgaa atacctgcgg 420
ccatctgggg atattccact ncgatnatgt gattaaggaa ntccacggag ttttacaagg 480
acacgtacaa cnacctgaaa accnnggatg anccccaccg ggaancnctg aangccatcc 540
actatgcgtt gaactgcaat ggtttggctg gggnccttga acaatttaat cncatacatc 600
tggccccann aaaggacntn ctcganncct tcnccgtgna attcngttct gatnccatca 660
cagaagtctc gaacaatcc 679

<210> 15
<211> 695
<212> DNA
<213> Homo sapien

<220>
<221> misc_feature
<222> (1)...(695)
<223> n = A,T,C or G

<400> 15
actagtggat aaaggccagg gatgctgctc aacctcctac catgtacagg gacgtctccc 60
cattacaact acccaatccg aagtgtcaac tgtgtcagga ctaanaaacc ctggttttga 120

ttaaaaaagg gcctgaaaaa aggggagcca caaatctgtc tgcttcctca cnttantcnt 180
tggcaaatna gcattctgtc tcnttggctg cngcctcanc ncaaaaaanc ngaactcnat 240
cnggcccagg aatacatctc ncaatnaacn aaattganca aggcnntggg aaatgccnga 300
tgggattatc ntccgcttgt tgancttcta agtttcnttc ccttcattcn accctgccag 360
ccnagttctg ttagaaaaat gccngaattc naacnccggt tttcntactc ngaatttaga 420
tctncanaaa cttcctggcc acnattcnaa ttnanggnca cgnacanatn ccttccatna 480
ancncacccc acntttgana gccangacaa tgactgcntn aantgaaggc ntgaaggaan 540
aactttgaaa ggaaaaaaaa ctttgtttcc ggccccttcc aacncttctg tgttnancac 600
tgccttctng naaccctgga agcccngnga cagtgttaca tgttgttcta nnaaacngac 660
ncttnaatnt cnatcttccc nanaacgatt ncncc 695

<210> 16
<211> 669
<212> DNA
<213> Homo sapien

<220>
<221> misc_feature
<222> (1)...(669)
<223> n = A,T,C or G

<400> 16
cgccgaagca gcagcgcagg ttgtccccgt ttcccctccc ccttcccttc tccggttgcc 60
ttcccgggcc ccttacactc cacagtcccg gtcccgccat gtcccagaaa caagaagaag 120
agaaccctgc ggaggagacc ggcgaggaga agcaggacac gcaggagaaa gaaggtattc 180
tgcctgagag agctgaagag gcaaagctaa aggccaaata cccaagccta ggacaaaagc 240
ctggaggctc cgacttcctc atgaagagac tccagaaagg gcaaaagtac tttgactcng 300
gagactacaa catggccaaa gccaacatga agaataagca gctgccaagt gcangaccag 360
acaagaacct ggtgactggt gatcacatcc ccaccccaca ggatctgccc agagaaagtc 420
ctcgctcgtc accagcaagc ttgcgggtgg ccaagttgaa tgatgctgcc ggggctctgc 480
canatctgag acgcttccct ccctgcccca cccgggtcct gtgctggctc ctgcccttcc 540
tgcttttgca gccangggtc aggaagtggc ncnggtngtg gctggaaagc aaaacccttt 600
cctgttggtg tcccacccat ggagcccctg gggcgagccc angaacttga ncctttttgt 660
tntcttncc 669

<210> 17
<211> 697
<212> DNA
<213> Homo sapien

<220>
<221> misc_feature
<222> (1)...(697)
<223> n = A,T,C or G

<400> 17
gcaagatatg gacaactaag tgagaaggta atnctctact gctctagntn ctccnggcnn 60
gacgcgctga ggagannnac gctggcccan ctgccggcca cacacgggga tcntggtnat 120
gcctgcccan gggancccca ncnctcggan cccatntcac acccgnnccn tncgcccacn 180
ncctggctcn cncngcccng nccagctcnc gnccccctcc gccnnnctcn ttnncntctc 240
cncnccctcc ncnacnacct cctacccncg gctccctccc cagccccccc ccgcaancct 300
ccacnacncc ntcnncncga ancnccnctc gcnctcngcc ccngccccct gccccccgcc 360
cncnacnncg cgntcccccg cgcncgcngc ctcnccccct cccacnacag ncncacccgc 420
agncacgcnc tccgcccnct gacgccccnn cccgccgcgc tcaccttcat ggnccnacng 480
ccccgctcnc nccnctgcnc gccgncnngg cgccccgccc cnnccgngtn ccncncgnng 540

ccccngcngn angcngtgcg cnncangncc gngccgnncn ncaccctccg nccnccgccc 600
cgcccgctgg gggctcccgc cncgcggntc antccccncc cntncgccca ctntccgntc 660
cnncnctcnc gctcngcgcn cgcccnccnc ccccccc 697

<210> 18
<211> 670
<212> DNA
<213> Homo sapien

<220>
<221> misc_feature
<222> (1)...(670)
<223> n = A,T,C or G

<400> 18
ctcgtgtgaa gggtgcagta cctaagccgg agcggggtag aggcgggccg gcaccccctt 60
ctgacctcca gtgccgccgg cctcaagatc agacatggcc cagaacttga acgacttggc 120
gggacggctg cccgccgggc cccggggcat gggcacggcc ctgaagctgt tgctgggggc 180
cggcgccgtg gcctacggtg tgcgcgaatc tgtgttcacc gtggaaggcg ggcncagagc 240
catcttcttc aatcggatcg gtggagtgca caggacacta tcctgggccg anggccttca 300
cttcaggatc cttggttcca gtaccccanc atctatgaca ttcgggccag acctcgaaaa 360
aatctcctcc ctacaggctc caaagaccta cagatggtga atatctccct gcgagtgttg 420
tctcgaccaa tgctcangaa cttcctaaca tgttccancg cctaagggct ggactacnaa 480
gaacgantgt tgccgtccat tgtcacgaag tgctcaagaa tttnggtggc caagttcaat 540
gncctcacnn ctgatcnccc agcggggcca agttanccct ggttgatccc cgggganctg 600
acnnaaaagg gccaaggact tcccctcatc ctggataatg tggccntcac aaagctcaac 660
tttanccacc 670

<210> 19
<211> 606
<212> DNA
<213> Homo sapien

<220>
<221> misc_feature
<222> (1)...(606)
<223> n = A,T,C or G

<400> 19
actagtgcca acctcagctc ccaggccagt tctctgaatg tcgaggagtt ccaggatctc 60
tggcctcagt tgtccttggt tattgatggg ggacaaattg gggatggcca gagccccgag 120
tgtcgccttg gctcaactgt ggttgatttg tctgtgcccg gaaagtttgg catcattcgt 180
ccaggctgtg ccctggaaag tactacagcc atcctccaac agaagtacgg actgctcccc 240
tcacatgcgt cctacctgtg aaactctggg aagcaggaag gcccaagacc tggtgctgga 300
tactatgtgt ctgtccactg acgactgtca aggcctcatt tgcagaggcc accggagcta 360
gggcactagc ctgactttta aggcagtgtg tctttctgag cactgtagac caagcccttg 420
gagctgctgg tttagccttg cacctgggga aaggatgtat ttatttgtat tttcatatat 480
cagccaaaag ctgaatggaa aagttnagaa cattcctagg tggccttatt ctaataagtt 540
tcttctgtct gttttgtttt tcaattgaaa agttattaaa taacagattt agaatctagt 600
gagacc 606

<210> 20
<211> 449
<212> DNA
<213> Homo sapien

<400> 20
actagtaaac aacagcagca gaaacatcag tatcagcagc gtcgccagca ggagaatatg 60
cagcgccaga gccgaggaga acccccgctc cctgaggagg acctgtccaa actcttcaaa 120
ccaccacagc cgcctgccag gatggactcg ctgctcattg caggccagat aaacacttac 180
tgccagaaca tcaaggagtt cactgcccaa aacttaggca agctcttcat ggcccaggct 240
cttcaagaat acaacaacta agaaaaggaa gtttccagaa aagaagttaa catgaactct 300
tgaagtcaca ccagggcaac tcttggaaga aatatatttg catattgaaa agcacagagg 360
atttctttag tgtcattgcc gattttggct ataacagtgt ctttctagcc ataataaaat 420
aaaacaaaat cttgactgct tgctcaaaa 449

<210> 21
<211> 409
<212> DNA
<213> Homo sapien

<400> 21
tatcaatcaa ctggtgaata attaaacaat gtgtggtgtg atcatacaaa gggtaccact 60
caatgataaa aggaacaagc tgcctatatg tggaacaaca tggatgcatt tcagaaactt 120
tatgttgagt gaaagaacaa acacggagaa catactatgt ggttctcttt atgtaacatt 180
acagaaataa aaacagaggc aaccaccttt gaggcagtat ggagtgagat agactggaaa 240
aaggaaggaa ggaaactcta cgctgatgga aatgtctgtg tcttcattgg gtggtagtta 300
tgtggggata tacatttgtc aaaatttatt gaactatata ctaaagaact ctgcatttta 360
ttgggatgta aataatacct caattaaaaa gacaaaaaaa aaaaaaaaa 409

<210> 22
<211> 649
<212> DNA
<213> Homo sapien

<220>
<221> misc_feature
<222> (1)...(649)
<223> n = A,T,C or G

<400> 22
acaattttca ttatcttaag cacattgtac atttctacag aacctgtgat tattctcgca 60
tgataaggat ggtacttgca tatggtgaat tactactgtt gacagtttcc gcagaaatcc 120
tatttcagtg gaccaacatt gtggcatggc agcaaatgcc aacattttgt ggaatagcag 180
caaatctaca agagaccctg gttggttttt cgttttgttt tctttgtttt ttcccccttc 240
tcctgaatca gcagggatgg aangagggta gggaagttat gaattactcc ttccagtagt 300
agctctgaag tgtcacattt aatatcagtt ttttttaaac atgattctag ttnaatgtag 360
aagagagaag aaagaggaag tgttcacttt tttaatacac tgatttagaa atttgatgtc 420
ttatatcagt agttctgagg tattgatagc ttgctttatt tctgccttta cgttgacagt 480
gttgaagcag ggtgaataac taggggcata tatatttttt ttttttgtaa gctgtttcat 540
gatgttttct ttggaatttc cggataagtt caggaaaaca tctgcatgtt gttatctagt 600
ctgaagttcn tatccatctc attacaacaa aaacncccag aacggnttg 649

<210> 23
<211> 669
<212> DNA
<213> Homo sapien

<220>
<221> misc_feature

<222> (1)...(669)
<223> n = A,T,C or G

<400> 23
actagtgccg tactggctga aatccctgca ggaccaggaa gagaaccagt tcagactttg 60
tactctcagt caccagctct ggaattagat aaattccttg aagatgtcag gaatgggatc 120
tatcctctga cagcctttgg gctgcctcgg ccccagcagc cacagcagga ggaggtgaca 180
tcacctgtcg tgccccctc tgtcaagact ccgacacctg aaccagctga ggtggagact 240
cgcaaggtgg tgctgatgca gtgcaacatt gagtcggtgg aggagggagt caaacaccac 300
ctgacacttc tgctgaagtt ggaggacaaa ctgaaccggc acctgagctg tgacctgatg 360
ccaaatgaga atatccccga gttggcggct gagctggtgc agctgggctt cattagtgag 420
gctgaccaga gccggttgac ttctctgcta gaagagactt gaacaagttc aattttgcca 480
ggaacagtac cctcaactca gccgctgtca ccgtctcctc ttagagctca ctcgggccag 540
gccctgatct gcgctgtggc tgtcctggac gtgctgcacc ctctgtcctt ccccccagtc 600
agtattacct gtgaagccct tccctccttt attattcagg anggctgggg gggctccttg 660
nttctaacc 669

<210> 24
<211> 442
<212> DNA
<213> Homo sapien

<400> 24
actagtacca tcttgacaga ggatacatgc tcccaaaacg tttgttacca cacttaaaaa 60
tcactgccat cattaagcat cagtttcaaa attatagcca ttcatgattt actttttcca 120
gatgactatc attattctag tcctttgaat ttgtaagggg aaaaaaaaca aaaacaaaaa 180
cttacgatgc acttttctcc agcacatcag atttcaaatt gaaaattaaa gacatgctat 240
ggtaatgcac ttgctagtac tacacacttt ggtacaacaa aaaacagagg caagaaacaa 300
cggaaagaga aaagccttcc tttgttggcc cttaaactga gtcaagatct gaaatgtaga 360
gatgatctct gacgatacct gtatgttctt attgtgtaaa taaaattgct ggtatgaaat 420
gacctaaaaa aaaaaaaaga aa 442

<210> 25
<211> 656
<212> DNA
<213> Homo sapien

<220>
<221> misc_feature
<222> (1)...(656)
<223> n = A,T,C or G

<400> 25
tgcaagtacc acacactgtt tgaattttgc acaaaaagtg actgtaggat caggtgatag 60
ccccggaatg tacagtgtct tggtgcacca agatgccttc taaaggctga cataccttgg 120
accctaatgg ggcagagagt atagccctag cccagtggtg acatgaccac tccctttggg 180
aggcctgagg tagaggggag tggtatgtgt tttctcagtg gaagcagcac atgagtgggt 240
gacaggatgt tagataaagg ctctagttag ggtgtcattg tcatttgaga gactgacaca 300
ctcctagcag ctggtaaagg ggtgctggan gccatggagg anctctagaa acattagcat 360
gggctgatct gattacttcc tggcatcccg ctcactttta tgggaagtct tattagangg 420
atgggacagt tttccatatc cttgctgtgg agctctggaa cactctctaa atttccctct 480
attaaaaatc actgccctaa ctacacttcc tccttgaagg aatagaaatg gaactttctc 540
tgacatantt cttggcatgg ggagccagcc acaaatgana atctgaacgt gtccaggttt 600
ctcctganac tcatctacat agaattggtt aaaccctccc ttggaataag gaaaaa 656

<210> 26
<211> 434
<212> DNA
<213> Homo sapien

<220>
<221> misc_feature
<222> (1)...(434)
<223> n = A,T,C or G

<400> 26
actagttcag actgccacgc caaccccaga aaatacccca catgccagaa aagtgaagtc 60
ctaggtgttt ccatctatgt ttcaatctgt ccatctacca ggcctcgcga taaaaacaaa 120
acaaaaaaac gctgccaggt tttagaagca gttctggtct caaaaccatc aggatcctgc 180
caccagggtt cttttgaaat agtaccacat gtaaaaggga atttggcttt cacttcatct 240
aataactgaa ttgtcaggct ttgattgata attgtagaaa taagtagcct tctgttgtgg 300
gaataagtta taatcagtat tcatctcttt gttttttgtc actcttttct ctctaattgt 360
gtcatttgta ctgtttgaaa aatatttctt ctatnaaatt aaactaacct gccttaaaaa 420
aaaaaaaaaa aaaa 434

<210> 27
<211> 654
<212> DNA
<213> Homo sapien

<220>
<221> misc_feature
<222> (1)...(654)
<223> n = A,T,C or G

<400> 27
actagtccaa cacagtcaga aacattgttt tgaatcctct gtaaaccaag gcattaatct 60
taataaacca ggatccattt aggtaccact tgatataaaa aggatatcca taatgaatat 120
tttatactgc atcctttaca ttagccacta aatacgttat tgcttgatga agacctttca 180
cagaatccta tggattgcag catttcactt ggctacttca tacccatgcc ttaaagaggg 240
gcagtttctc aaaagcagaa acatgccgcc agttctcaag ttttcctcct aactccattt 300
gaatgtaagg gcagctggcc cccaatgtgg ggaggtccga acattttctg aattcccatt 360
ttcttgttcg cggctaaatg acagtttctg tcattactta gattccgatc tttcccaaag 420
gtgttgattt acaaagaggc cagctaatag cagaaatcat gaccctgaaa gagagatgaa 480
attcaagctg tgagccaggc agganctcag tatggcaaag gtcttgagaa tcngccattt 540
ggtacaaaaa aaattttaaa gcntttatgt tataccatgg aaccatagaa anggcaaggg 600
aattgttaag aanaatttta agtgtccaga cccanaanga aaaaaaaaaa aaaa 654

<210> 28
<211> 670
<212> DNA
<213> Homo sapien

<220>
<221> misc_feature
<222> (1)...(670)
<223> n = A,T,C or G

<400> 28
cgtgtgcaca tactgggagg atttccacag ctgcacggtc acagccctta cggattgcca 60

ggaaggggcg aaagatatgt gggataaact gagaaaagaa nccaaaaacc tcaacatcca 120
aggcagctta ttcgaactct gcggcagcgg caacggggcg gcggggtccc tgctcccggc 180
gttcccggtg ctcctggtgt ctctctcggc agctttagcg acctgncttt ccttctgagc 240
gtggggccag ctccccccgc ggcgcccacc cacnctcact ccatgctccc ggaaatcgag 300
aggaagatca ttagttcttt ggggacgttn gtgattctct gtgatgctga aaaacactca 360
tatagggaat gtgggaaatc ctganctctt tnttatntcg tntgatttct tgtgttttat 420
ttgccaaaat gttaccaatc agtgaccaac cnagcacagc caaaaatcgg acntcngctt 480
tagtccgtct tcacacacag aataagaaaa cggcaaaccc accccacttt tnantttnat 540
tattactaan ttttttctgt tgggcaaaag aatctcagga acngccctgg ggccnccgta 600
ctanagttaa ccnagctagt tncatgaaaa atgatgggct ccncctcaat gggaaagcca 660
agaaaaagnc 670

<210> 29
<211> 551
<212> DNA
<213> Homo sapien

<220>
<221> misc_feature
<222> (1)...(551)
<223> n = A,T,C or G

<400> 29
actagtcctc cacagcctgt gaatccccct agacctttca agcatagtga gcggagaaga 60
agatctcagc gtttagccac cttacccatg cctgatgatt ctgtagaaaa ggtttcttct 120
ccctctccag ccactgatgg gaaagtattc tccatcagtt ctcaaaatca gcaagaatct 180
tcagtaccag aggtgcctga tgttgcacat ttgccacttg agaagctggg accctgtctc 240
cctcttgact taagtcgtgg ttcagaagtt acagcaccgg tagcctcaga ttcctcttac 300
cgtaatgaat gtcccagggc agaaaaagag gatacncaga tgcttccaaa tccttcttcc 360
aaagcaatag ctgatgggaa gaggagctcc agcagcagca ggaatatcga aaacagaaaa 420
aaaagtgaaa ttgggaagac aaaagctcaa cagcatttgg taaggagaaa aganaagatg 480
aggaaggaag agagaagaga gacnaagatc nctacggacc gnnncggaag aagaagaagn 540
aaaaaanaaa a 551

<210> 30
<211> 684
<212> DNA
<213> Homo sapien

<220>
<221> misc_feature
<222> (1)...(684)
<223> n = A,T,C or G

<400> 30
actagttcta tctggaaaaa gcccgggttg gaagaagctg tggagagtgc gtgtgcaatg 60
cgagactcat ttcttggaag catccctggc aaaaatgcag ctgagtacaa ggttatcact 120
gtgatagaac ctggactgct ttttgagata atagagatgc tgcagtctga agagacttcc 180
agcacctctc agttgaatga attaatgatg gcttctgagt caactttact ggctcaggaa 240
ccacgagaga tgactgcaga tgtaatcgag cttaaaggga aattcctcat caacttagaa 300
ggtggtgata ttcgtgaaga gtcttcctat aaagtaattg tcatgccgac tacgaaagaa 360
aaatgccccc gttgttggaa gtatacagcg ggagtcttca gatacactgt gtcctcgatg 420
tgcagaagtt gtcagtggga aaatagtatt aacagctcac tcgagcaaga accctcctga 480
cagtactggg ctagaagttt ggatggatta tttacaatat aggaaagaaa gccaagaatt 540
aggtnatgag tggatgagta aatggtggan gatggggaat tcaaatcaga attatggaag 600

aagttnttcc tgttactata gaaaggaatt atgtttattt acatgcagaa aatatanatg 660
tgtggtgtgt accgtggatg gaan 684

<210> 31
<211> 654
<212> DNA
<213> Homo sapien

<220>
<221> misc_feature
<222> (1)...(654)
<223> n = A,T,C or G

<400> 31
gcgcagaaaa ggaaccaata tttcagaaac aagcttaata ggaacagctg cctgtacatc 60
aacatcttct cagaatgacc cagaagttat catcgtggga gctggcgtgc ttggctctgc 120
tttggcagct gtgctttcca gagatggaag aaaggtgaca gtcattgaga gagacttaaa 180
agagcctgac agaatagttg gagaattcct gcagccgggt ggttatcatg ttctcaaaga 240
ccttggtctt ggagatacag tggaaggtct tgatgcccag gttgtaaatg gttacatgat 300
tcatgatcag ggaaagcaaa tcagangttc agattcctta ccctctgtca gaaaacaatc 360
aagtgcagag tggaagagct ttccatcacg gaagattcat catgagtctc cggaaagcag 420
ctatggcaga gcccaatgca aagtttattg aaggtgttgt gttacagtta ttagaggaag 480
atgatgttgt gatgggagtt cagtacaagg ataaagagac tgggagatat caaggaactc 540
catgctccac tgactgttgt tgcagatggg cttttctcca anttcaggaa aagcctggtc 600
tcaataaagt ttctgtatca ctcatttggt tggcttctta tgaagaatgc nccc 654

<210> 32
<211> 673
<212> DNA
<213> Homo sapien

<220>
<221> misc_feature
<222> (1)...(673)
<223> n = A,T,C or G

<400> 32
actagtgaag aaaaagaaat tctgatacgg gacaaaaatg ctcttcaaaa catcattctt 60
tatcacctga caccaggagt tttcattgga aaaggatttg aacctggtgt tactaacatt 120
ttaaagacca cacaaggaag caaaatcttt ctgaaagaag taaatgatac acttctggtg 180
aatgaattga aatcaaaaga atctgacatc atgacaacaa atggtgtaat tcatgttgta 240
gataaactcc tctatccagc agacacacct gttggaaatg atcaactgct ggaaatactt 300
aataaattaa tcaaatacat ccaaattaag tttgttcgtg gtagcacctt caaagaaatc 360
cccgtgactg tctatnagcc aattattaaa aaatacacca aaatcattga tgggagtgcc 420
tgtgggaaat aactgaaaaa gagaccgaga agaacgaatc attacaggtc ctgaaataaa 480
atacctagga tttctactgg aggtggagaa acagaagaac tctgaagaaa ttgttacaag 540
aagangtccc aaggtcacca aattcattga aggtggtgat ggtctttatt tgaagatgaa 600
gaaattaaaa gacgcttcag ggagacnccc catgaaggaa ttgccagcca caaaaaaatt 660
cagggattag aaa 673

<210> 33
<211> 673
<212> DNA
<213> Homo sapien

<220>
<221> misc_feature
<222> (1)...(673)
<223> n = A,T,C or G

<400> 33
actagttatt tactttcctc cgcttcagaa ggtttttcag actgagagcc taagcatact 60
ggatctgttg tttcttttgg gtctcacctc atcagtgtgc atagtggcag aaattataaa 120
gaaggttgaa aggagcaggg aaaagatcca gaagcatgtt agttcgacat catcatcttt 180
tcttgaagta tgatgcatat tgcattattt tatttgcaaa ctaggaattg cagtctgagg 240
atcatttaga agggcaagtt caagaggata tgaagatttg agaacttttt aactattcat 300
tgactaaaaa tgaacattaa tgttnaagac ttaagacttt aacctgctgg cagtcccaaa 360
tgaaattatg caactttgat atcatattcc ttgatttaaa ttgggctttt gtgattgant 420
gaaactttat aaagcatatg gtcagttatt tnattaaaaa ggcaaaacct gaaccacctt 480
ctgcacttaa agaagtctaa cagtacaaat acctatctat cttagatgga tntatttntt 540
tntattttta aatattgtac tatttatggt nggtggggct ttcttactaa tacacaaatn 600
aatttatcat ttcaanggca ttctatttgg gtttagaagt tgattccaag nantgcatat 660
ttcgctactg tnt 673

<210> 34
<211> 684
<212> DNA
<213> Homo sapien

<220>
<221> misc_feature
<222> (1)...(684)
<223> n = A,T,C or G

<400> 34
actagtttat tcaagaaaag aacttactga ttcctctgtt cctaaagcaa gagtggcagg 60
tgatcagggc tggtgtagca tccggttcct ttagtgcagc taactgcatt tgtcactgat 120
gaccaaggag gaaatcacta agacatttga gaagcagtgg tatgaacgtt cttggacaag 180
ccacagttct gagccttaac cctgtagttt gcacacaaga acgagctcca cctccccttc 240
ttcaggagga atctgtgcgg atagattggc tggacttttc aatggttctg ggttgcaagt 300
gggcactgtt atggctgggt atggagcgga cagccccagg aatcagagcc tcagcccggc 360
tgcctggttg gaaggtacag gtgttcagca ccttcggaaa aagggcataa agtngtgggg 420
gacaattctc agtccaagaa gaatgcattg accattgctg gctatttgct tncctagtan 480
gaattggatn catttttgac cangatnntt ctnctatgct ttnttgcaat gaaatcaaat 540
cccgcattat ctacaagtgg tatgaagtcc tgcnnccccc agagaggctg ttcaggcnat 600
gtcttccaag ggcagggtgg gttacaccat tttacctccc ctctcccccc agattatgna 660
cncagaagga atttntttcc tccc 684

<210> 35
<211> 614
<212> DNA
<213> Homo sapien

<220>
<221> misc_feature
<222> (1)...(614)
<223> n = A,T,C or G

<400> 35
actagtccaa cgcgttngcn aatattcccc tggtagccta cttccttacc cccgaatatt 60

ggtaagatcg agcaatggct tcaggacatg ggttctcttc tcctgtgatc attcaagtgc 120
tcactgcatg aagactggct tgtctcagtg tntcaacctc accagggctg tctcttggtc 180
cacacctcgc tccctgttag tgccgtatga cagcccccat canatgacct tggccaagtc 240
acggtttctc tgtggtcaat gttggtnggc tgattggtgg aaagtanggt ggaccaaagg 300
aagncncgtg agcagncanc nccagttctg caccagcagc gcctccgtcc tactngggtg 360
ttccngtttc tcctggccct gngtgggcta nggcctgatt cgggaanatg cctttgcang 420
gaaggganga taantgggat ctaccaattg attctggcaa aacnatntct aagattnttn 480
tgctttatgt ggganacana tctanctctc atttnntgct gnanatnaca ccctactcgt 540
gntcgancnc gtcttcgatt ttcgganaca cnccantnaa tactggcgtt ctgttgttaa 600
aaaaaaaaaa aaaa 614

<210> 36
<211> 686
<212> DNA
<213> Homo sapien

<220>
<221> misc_feature
<222> (1)...(686)
<223> n = A,T,C or G

<400> 36
gtggctggcc cggttctccg cttctcccca tcccctactt tcctccctcc ctccctttcc 60
ctccctcgtc gactgttgct tgctggtcgc agactccctg acccctccct cacccctccc 120
taacctcggt gccaccggat tgcccttctt ttcctgttgc ccagcccagc cctagtgtca 180
gggcgggggc ctggagcagc ccgaggcact gcagcagaag ananaaaaga cacgacnaac 240
ctcagctcgc cagtccggtc gctngcttcc cgccgcatgg caatnagaca gacgccgctc 300
acctgctctg ggcacacgcg acccgtggtt gatttggcct tcagtggcat cacccttatg 360
ggtatttctt aatcagcgct tgcaaagatg gttaacctat gctacgccag ggagatacag 420
gagactggat tggaacattt ttggggtcta aaggtctgtt tggggtgcaa cactgaataa 480
ggatgccacc aaagcagcta cagcagctgc agatttcaca gcccaagtgt gggatgctgt 540
ctcagganat naattgataa cctggctcat aacacattgt caagaatgtg gatttcccca 600
ggatattatt atttgtttac cggggganag gataactgtt tcncntattt taattgaaca 660
aactnaaaca aaanctaagg aaatcc 686

<210> 37
<211> 681
<212> DNA
<213> Homo sapien

<220>
<221> misc_feature
<222> (1)...(681)
<223> n = A,T,C or G

<400> 37
gagacanacn naacgtcang agaanaaaag angcatggaa cacaanccag gcncgatggc 60
caccttccca ccagcancca gcgcccccca gcngccccca ngnccggang accangactc 120
cancctgnat caatctganc tctattcctg gcccatncct acctcggagg tggangccgn 180
aaaggtcgca cnnncagaga agctgctgcc ancaccancc gccccnnccc tgncgggctn 240
nataggaaac tggtgaccnn gctgcanaat tcatacagga gcacgcgang ggcacnnnct 300
cacactgagt tnnngatgan gcctnaccan ggacctnccc cagcnnattg annacnggac 360
tgcggaggaa ggaagacccc gnacnggatc ctggccggcn tgccaccccc ccacccctag 420
gattatnccc cttgactgag tctctgaggg gctacccgaa cccgcctcca ttccctacca 480
natnntgctc natcgggact gacangctgg ggatnggagg ggctatcccc cancatcccc 540

tnanaccaac agcnacngan natnggggct ccccngggtc ggngcaacnc tcctncaccc 600
cggcgcnggc cttcggtgnt gtcctccntc aacnaattcc naaanggcgg gccccccngt 660
ggactcctcn ttgttccctc c 681

<210> 38
<211> 687
<212> DNA
<213> Homo sapien

<220>
<221> misc_feature
<222> (1)...(687)
<223> n = A,T,C or G

<400> 38
canaaaaaaa aaaacatggc cgaaaccagn aagctgcgcg atggcgccac ggcccctctt 60
ctcccggcct gtgtccggaa ggtttccctc cgaggcgccc cggctcccgc aagcggagga 120
gagggcggga cntgccgggg ccggagctca naggccctgg ggccgctctg ctctcccgcc 180
atcgcaaggg cggcgctaac ctnaggcctc cccgcaaagg tccccnangc ggnggcggcg 240
gggggctgtg anaaccgcaa aaanaacgct gggcgcgcng cgaacccgtc cacccccgcg 300
aaggananac ttccacagan gcagcgtttc cacagcccan agccacnttt ctagggtgat 360
gcaccccagt aagttcctgn cggggaagct caccgctgtc aaaaaanctc ttcgctccac 420
cggcgcacna aggggangan ggcangangc tgccgcccgc acaggtcatc tgatcacgtc 480
gcccgcccta ntctgctttt gtgaatctcc actttgttca accccacccg ccgttctctc 540
ctccttgcgc cttcctctna ccttaanaac cagcttcctc tacccnatng tanttnctct 600
gcncnngtng aaattaattc ggtccnccgg aacctcttnc ctgtggcaac tgctnaaaga 660
aactgctgtt ctgnttactg cngtccc 687

<210> 39
<211> 695
<212> DNA
<213> Homo sapien

<220>
<221> misc_feature
<222> (1)...(695)
<223> n = A,T,C or G

<400> 39
actagtctgg cctacaatag tgtgattcat gtaggacttc tttcatcaat tcaaaacccc 60
tagaaaaacg tatacagatt atataagtag ggataagatt tctaacattt ctgggctctc 120
tgacccctgc gctagactgt ggaaagggag tattattata gtatacaaca ctgctgttgc 180
cttattagtt ataacatgat aggtgctgaa ttgtgattca caatttaaaa acactgtaat 240
ccaaactttt ttttttaact gtagatcatg catgtgaatg ttaatgttaa tttgttcaan 300
gttgttatgg gtagaaaaaa ccacatgcct taaaatttta aaaagcaggg cccaaactta 360
ttagtttaaa attaggggta tgtttccagt ttgttattaa ntggttatag ctctgtttag 420
aanaaatcna ngaacangat ttngaaantt aagntgacat tatttnccag tgacttgtta 480
atttgaaatc anacacggca ccttccgttt tggtnctatt ggnntttgaa tccaancngg 540
ntccaaatct tnttggaaac ngtccnttta acttttttac nanatcttat ttttttattt 600
tggaatggcc ctatttaang ttaaaagggg ggggnnccac naccattcnt gaataaaact 660
naatatatat ccttggtccc ccaaaattta aggng 695

<210> 40
<211> 674
<212> DNA

<213> Homo sapien

<220>
<221> misc_feature
<222> (1)...(674)
<223> n = A,T,C or G

<400> 40
actagtagtc agttgggagt ggttgctata ccttgacttc atttatatga atttccactt 60
tattaaataa tagaaaagaa aatcccggtg cttgcagtag agttatagga cattctatgc 120
ttacagaaaa tatagccatg attgaaatca aatagtaaag gctgttctgg ctttttatct 180
tcttagctca tcttaaataa gtagtacact tgggatgcag tgcgtctgaa gtgctaatca 240
gttgtaacaa tagcacaaat cgaacttagg atgtgtttct tctcttctgt gtttcgattt 300
tgatcaattc tttaattttg ggaacctata atacagtttt cctattcttg gagataaaaa 360
ttaaatggat cactgatatt taagtcattc tgcttctcat ctnaatattc catattctgt 420
attagganaa antacctccc agcacagccc cctctcaaac cccacccaaa accaagcatt 480
tggaatgagt ctcctttatt tccgaantgt ggatggtata acccatatcn ctccaatttc 540
tgnttgggtt gggtattaat ttgaactgtg catgaaaagn ggnaatcttt nctttgggtc 600
aaantttncc ggttaatttg nctngncaaa tccaatttnc tttaagggtg tctttataaa 660
atttgctatt cngg 674

<210> 41
<211> 657
<212> DNA
<213> Homo sapien

<220>
<221> misc_feature
<222> (1)...(657)
<223> n = A,T,C or G

<400> 41
   gaaacatgca agtaccacac actgtttgaa ttttgcacaa aaagtgactg tagggatcag 60
   gtgatagccc cggaatgtac agtgtcttgg tgcaccaaga tgccttctaa aggctgacat 120
   accttgggac cctaatgggg cagagagtat agccctagcc cagtggtgac atgaccactc 180
   cctttgggag gctgaagtta aagggaatgg tatgtgtttt ctcatggaag cagcacatga 240
   atnggtnaca ngatgttaaa ntaaggntct antttgggtg tcttgtcatt tgaaaaantg 300
   acacactcct ancanctggt aaaggggtgc tggaagccat ggaagaactc taaaaacatt 360
   agcatgggct gatctgatta cttcctggca tcccgctcac ttttatggga agtcttatta 420
   naaggatggg ananttttcc atatccttgc tgttggaact ctggaacact ctctaaattt 480
   ccctctatta aaaatcactg nccttactac acttcctcct tganggaata gaaatggacc 540
   tttctctgac ttagttcttg gcatggganc cagcccaaat taaaatctga cttntccggt 600
   ttctccngaa ctcacctact tgaattggta aaacctcctt tggaattagn aaaaacc 657

<210> 42
<211> 389
<212> DNA
<213> Homo sapien

<220>
<221> misc_feature
<222> (1)...(389)
<223> n = A,T,C or G

<400> 42
   actagtgctg aggaatgtaa acaagtttgc tgggccttgc gagacttcac caggttgttt 60
   cgatagctca cactcctgca ctgtgcctgt cacccaggaa tgtctttttt aattagaaga 120
   caggaagaaa acaaaaacca gactgtgtcc cacaatcaga aacctccgtt gtggcagang 180
   ggccttcacc gccaccaggg tgtcccgcca gacagggaga gactccagcc ttctgaggcc 240
   atcctgaaga attcctgttt gggggttgtg aaggaaaatc acccggattt aaaaagatgc 300
   tgttgcctgc ccgcgtngtn gggaagggac tggtttcctg gtgaatttct taaaagaaaa 360
   atattttaag ttaagaaaaa aaaaaaaaa 389

<210> 43
<211> 279
<212> DNA
<213> Homo sapien

<400> 43
actagtgaca agctcctggt cttgagatgt cttctcgtta aggagatggg ccttttggag 60
gtaaaggata aaatgaatga gttctgtcat gattcactat tctagaactt gcatgacctt 120
tactgtgtta gctctttgaa tgttcttgaa attttagact ttctttgtaa acaaataata 180
tgtccttatc attgtataaa agctgttatg tgcaacagtg tggagatcct tgtctgattt 240
aataaaatac ttaaacactg aaaaaaaaaa aaaaaaaaa 279

<210> 44
<211> 449
<212> DNA
<213> Homo sapien

<220>
<221> misc_feature
<222> (1)...(449)
<223> n = A,T,C or G

<400> 44
actagtagca tcttttctac aacgttaaaa ttgcagaagt agcttatcat taaaaaacaa 60
caacaacaac aataacaata aatcctaagt gtaaatcagt tattctaccc cctaccaagg 120
atatcagcct gttttttccc ttttttctcc tgggaataat tgtgggcttc ttcccaaatt 180
tctacagcct ctttcctctt ctcatgcttg agcttccctg tttgcacgca tgcgttgtgc 240
aagantgggc tgtttngctt ggantncggt ccnagtggaa ncatgctttc ccttgttact 300
gttggaagaa actcaaacct tcnancccta ggtgttncca ttttgtcaag tcatcactgt 360
atttttgtac tggcattaac aaaaaaagaa atnaaatatt gttccattaa actttaataa 420
aactttaaaa gggaaaaaaa aaaaaaaaa 449

<210> 45
<211> 559
<212> DNA
<213> Homo sapien

<220>
<221> misc_feature
<222> (1)...(559)
<223> n = A,T,C or G

<400> 45
actagtgtgg gggaatcacg gacacttaaa gtcaatctgc gaaataattc ttttattaca 60
cactcactga agtttttgag tcccagagag ccattctatg tcaaacattc caagtactct 120
ttgagagccc agcattacat caacatgccc gtgcagttca aaccgaagtc cgcaggcaaa 180
tttgaagctt tgcttgtcat tcaaacagat gaaggcaaga gtattgctat tcgactaatt 240

ggtgaagctc ttggaaaaaa ttnactagaa tactttttgt gttaagttaa ttacataagt 300
tgtattttgt taactttatc tttctacact acaattatgc ttttgtatat atattttgta 360
tgatggatat ctataattgt agattttgtt tttacaagct aatactgaag actcgactga 420
aatattatgt atctagccca tagtattgta cttaactttt acagggtgaa aaaaaaattc 480
tgtgtttgca ttgattatga tattctgaat aaatatggga atatatttta atgtgggtaa 540
aaaaaaaaaa aaaaaggaa 559

<210> 46
<211> 731
<212> DNA
<213> Homo sapien

<220>
<221> misc_feature
<222> (1)...(731)
<223> n = A,T,C or G

<400> 46
actagttcta gtaccatggc tgtcatagat gcaaccatta tattccattt agtttcttcc 60
tcaggttccc taacaattgt ttgaaactga atatatatgt ttatgtatgt gtgtgtgttc 120
actgtcatgt atatggtgta tatgggatgt gtgcagtttt cagttatata tatattcata 180
tatacatatg catatatatg tataatatac atatatacat gcatacactt gtataatata 240
catatatata cacatatatg cacacatatn atcactgagt tccaaagtga gtctttattt 300
ggggcaattg tattctctcc ctctgtctgc tcactgggcc tttgcaagac atagcaattg 360
cttgatttcc tttggataag agtcttatct tcggcactct tgactctagc cttaacttta 420
gatttctatt ccagaatacc tctcatatct atcttaaaac ctaaganggg taaagangtc 480
ataagattgt agtatgaaag antttgctta gttaaattat atctcaggaa actcattcat 540
ctacaaatta aattgtaaaa tgatggtttg ttgtatctga aaaaatgttt agaacaagaa 600
atgtaactgg gtacctgtta tatcaaagaa cctcnattta ttaagtctcc tcatagccan 660
atccttatat ngccctctct gacctgantt aatananact tgaataatga atagttaatt 720
taggnttggg c 731

<210> 47
<211> 640
<212> DNA
<213> Homo sapien

<220>
<221> misc_feature
<222> (1)...(640)
<223> n = A,T,C or G

<400> 47
tgcgngccgg tttggccctt ctttgtanga cactttcatc cgccctgaaa tcttcccgat 60
cgttaataac tcctcaggtc cctgcctgca caggtttttt tcttantttg ttgcctaaca 120
gtacaccaaa tgtgacatcc tttcaccaat atngattnct tcataccaca tcntcnatgg 180
anacgactnc aacaattttt tgatnacccn aaanactggg ggctnnaana agtacantct 240
ggagcagcat ggacctgtcn gcnactaang gaacaanagt nntgaacatt tacacaacct 300
ttggtatgtc ttactgaaag anagaaacat gcttctnncc ctagaccacg aggncaaccg 360
caganattgc caatgccaag tccgagcggt tagatcaggt aatacattcc atggatgcat 420
tacatacntt gtccccgaaa nanaagatgc cctaanggct tcttcanact ggtccngaaa 480
acanctacac ctggtgcttg ganaacanac tctttggaag atcatctggc acaagttccc 540
cccagtgggt tttnccttgg cacctanctt accanatcna ttcggaancc attctttgcc 600
ntggcnttnt nttgggacca ntcttctcac aactgnaccc 640

<210> 48
<211> 257
<212> DNA
<213> Homo sapien

<400> 48
actagtatat gaaaatgtaa atatcacttg tgtactcaaa caaaagttgg tcttaagctt 60
ccaccttgag cagccttgga aacctaacct gcctctttta gcataatcac attttctaaa 120
tgattttctt tgttcctgaa aaagtgattt gtattagttt tacatttgtt ttttggaaga 180
ttatatttgt atatgtatca tcataaaata tttaaataaa aagtatcttt agagtgaaaa 240
aaaaaaaaaa aaaaaaa 257

<210> 49
<211> 652
<212> DNA
<213> Homo sapien

<220>
<221> misc_feature
<222> (1)...(652)
<223> n = A,T,C or G

<400> 49
actagttcag atgagtggct gctgaagggg cccccttgtc attttcatta taacccaatt 60
tccacttatt tgaactctta agtcataaat gtataatgac ttatgaatta gcacagttaa 120
gttgacacta gaaactgccc atttctgtat tacactatca aataggaaac attggaaaga 180
tggggaaaaa aatcttattt taaaatggct tagaaagttt tcagattact ttgaaaattc 240
taaacttctt tctgtttcca aaacttgaaa atatgtagat ggactcatgc attaagactg 300
ttttcaaagc tttcctcaca tttttaaagt gtgattttcc ttttaatata catatttatt 360
ttctttaaag cagctatatc ccaacccatg actttggaga tatacctatn aaaccaatat 420
aacagcangg ttattgaagc agctttctca aatgttgctt cagatgtgca agttgcaaat 480
tttattgtat ttgtanaata caatttttgt tttaaactgt atttcaatct atttctccaa 540
gatgcttttc atatagagtg aaatatccca ngataactgc ttctgtgtcg tcgcatttga 600
cgcataactg cacaaatgaa cagtgtatac ctcttggttg tgcattnacc cc 652

<210> 50
<211> 650
<212> DNA
<213> Homo sapien

<220>
<221> misc_feature
<222> (1)...(650)
<223> n = A,T,C or G

<400> 50
ttgcgctttg atttttttag ggcttgtgcc ctgtttcact tatagggtct agaatgcttg 60
tgttgagtaa aaaggagatg cccaatattc aaagctgcta aatgttctct ttgccataaa 120
gactccgtgt aactgtgtga acacttggga tttttctcct ctgtcccgag gtcgtcgtct 180
gctttctttt ttgggttctt tctagaagat tgagaaatgc atatgacagg ctgagancac 240
ctccccaaac acacaagctc tcagccacan gcagcttctc cacagcccca gcttcgcaca 300
ggctcctgga nggctgcctg ggggaggcag acatgggagt gccaaggtgg ccagatggtt 360
ccaggactac aatgtcttta tttttaactg tttgccactg ctgccctcac ccctgcccgg 420
ctctggagta ccgtctgccc canacaagtg ggantgaaat gggggtgggg gggaacactg 480
attcccantt agggggtgcc taactgaaca gtagggatan aaggtgtgaa cctgngaant 540

gcttttataa attatnttcc ttgttanatt tattttttaa tttaatctct gttnaactgc 600
ccngggaaaa ggggaaaaaa aaaaaaaaat tctntttaaa cacatgaaca 650

<210> 51
<211> 545
<212> DNA
<213> Homo sapien

<220>
<221> misc_feature
<222> (1)...(545)
<223> n = A,T,C or G

<400> 51
tggcgtgcaa ccagggtagc tgaagtttgg gtctgggact ggagattggc cattaggcct 60
cctganattc cagctccctt ccaccaagcc cagtcttgct acgtggcaca gggcaaacct 120
gactcccttt gggcctcagt ttcccctccc cttcatgana tgaaaagaat actacttttt 180
cttgttggtc taacnttgct ggacncaaag tgtngtcatt attgttgtat tgggtgatgt 240
gtncaaaact gcagaagctc actgcctatg agaggaanta agagagatag tggatganag 300
ggacanaagg agtcattatt tggtatagat ccacccntcc caacctttct ctcctcagtc 360
cctgcncctc atgtntctgg tntggtgagt cctttgtgcc accanccatc atgctttgca 420
ttgctgccat cctgggaagg gggtgnatcg tctcacaact tgttgtcatc gtttganatg 480
catgctttct tnatnaaaca aanaaannaa tgtttgacag ngtttaaaat aaaaaanaaa 540
caaaa 545

<210> 52
<211> 678
<212> DNA
<213> Homo sapien

<220>
<221> misc_feature
<222> (1)...(678)
<223> n = A,T,C or G

<400> 52
   actagtagaa gaactttgcc gcttttgtgc ctctcacagg cgcctaaagt cattgccatg 60
   ggaggaagac gatttggggg gggagggggg gggggcangg tccgtggggc tttccctant 120
   ntatctccat ntccantgnn cnntgtcgcc tcttccctcg tcncattnga anttantccc 180
   tggnccccnn nccctctccn ncctncncct cccccctccg ncncctccnn ctttttntan 240
   ncttccccat ctccntcccc cctnanngtc ccaacnccgn cagcaatnnc ncacttnctc 300
   nctccncncc tccnnccgtt cttctnttct cnacntntnc ncnnntnccn tgccnntnaa 360
   annctctccc cnctgcaanc gattctctcc ctccncnnan ctntccactc cntncttctc 420
   ncncgctcct nttcntcnnc ccacctctcn ccttcgnccc cantacnctc nccncccttn 480
   cgnntcnttn nnntcctcnn accncccncc tcccttcncc cctcttctcc ccggtntntc 540
   tctctcccnc nncncnncct cnncccntcc nngcgnccnt ttccgccccn cnccnccntt 600
   ccttcntcnc cantccatcn cntntnccat nctncctncc nctcacnccc gctncccccn 660
   ntctctttca cacngtcc 678

<210> 53
<211> 502
<212> DNA
<213> Homo sapien

<220>

<221> misc_feature
<222> (1)...(502)
<223> n = A,T,C or G

<400> 53
tgaagatcct ggtgtcgcca tgggccgccg ccccgcccgt tgttaccggt attgtaagaa 60
caagccgtac ccaaagtctc gcttctgccg aggtgtccct gatgccaaaa ttcgcatttt 120
tgacctgggg cggaaaaang caaaantgga tgagtctccg ctttgtggcc acatggtgtc 180
agatcaatat gagcagctgt cctctgaagc cctgnangct gcccgaattt gtgccaataa 240
gtacatggta aaaagtngtg gcnaagatgc ttccatatcc gggtgcggnt ccaccccttc 300
cacgtcatcc gcatcaacaa gatgttgtcc tgtgctgggg ctgacaggct cccaacaggc 360
atgcgaagtg cctttggaaa acccanggca ctgtggccag ggttcacatt gggccaattn 420
atcatgttca tccgcaccaa ctgcagaaca angaacntgt naattnaagc cctgcccagg 480
gncaanttca aatttcccgg cc 502

<210> 54
<211> 494
<212> DNA
<213> Homo sapien

<220>
<221> misc_feature
<222> (1)...(494)
<223> n = A,T,C or G

<400> 54
actagtccaa gaaaaatatg cttaatgtat attacaaagg ctttgtatat gttaacctgt 60
tttaatgcca aaagtttgct ttgtccacaa tttccttaag acctcttcag aaagggattt 120
gtttgcctta atgaatactg ttgggaaaaa acacagtata atgagtgaaa agggcagaag 180
caagaaattt ctacatctta gcgactccaa gaagaatgag tatccacatt tagatggcac 240
attatgagga ctttaatctt tccttaaaca caataatgtt ttcttttttc ttttattcac 300
atgatttcta agtatatttt tcatgcagga cagtttttca accttgatgt acagtgactg 360
tgttaaattt ttctttcagt ggcaacctct ataatcttta aaatatggtg agcatcttgt 420
ctgttttgaa ngggatatga cnatnaatct atcagatggg aaatcctgtt tccaagttag 480
aaaaaaaaaa aaaa 494

<210> 55
<211> 606
<212> DNA
<213> Homo sapien

<220>
<221> misc_feature
<222> (1)...(606)
<223> n = A,T,C or G

<400> 55
actagtaaaa agcagcattg ccaaataatc cctaattttc cactaaaaat ataatgaaat 60
gatgttaagc tttttgaaaa gtttaggtta aacctactgt tgttagatta atgtatttgt 120
tgcttccctt tatctggaat gtggcattag cttttttatt ttaaccctct ttaattctta 180
ttcaattcca tgacttaagg ttggagagct aaacactggg atttttggat aacagactga 240
cagttttgca taattataat cggcattgta catagaaagg atatggctac cttttgttaa 300
atctgcactt tctaaatatc aaaaaaggga aatgaagtat aaatcaattt ttgtataatc 360
tgtttgaaac atgantttta tttgcttaat attanggctt tgcccttttc tgttagtctc 420
ttgggatcct gtgtaaaact gttctcatta aacaccaaac agttaagtcc attctctggt 480

actagctaca aattccgttt catattctac ntaacaattt aaattaactg aaatatttct 540
anatggtcta cttctgtcnt ataaaaacna aacttgantt nccaaaaaaa aaaaaaaaaa 600
aaaaaa 606

<210> 56
<211> 183
<212> DNA
<213> Homo sapien

<400> 56
actagtatat ttaaacttac aggcttattt gtaatgtaaa ccaccatttt aatgtactgt 60
aattaacatg gttataatac gtacaatcct tccctcatcc catcacacaa ctttttttgt 120
gtgtgataaa ctgattttgg tttgcaataa aaccttgaaa aataaaaaaa aaaaaaaaaa 180
aaa 183

<210> 57
<211> 622
<212> DNA
<213> Homo sapien

<220>
<221> misc_feature
<222> (1)...(622)
<223> n = A,T,C or G

<400> 57
actagtcact actgtcttct ccttgtagct aatcaatcaa tattcttccc ttgcctgtgg 60
gcagtggaga gtgctgctgg gtgtacgctg cacctgccca ctgagttggg gaaagaggat 120
aatcagtgag cactgttctg ctcagagctc ctgatctacc ccacccccta ggatccagga 180
ctgggtcaaa gctgcatgaa accaggccct ggcagcaacc tgggaatggc tggaggtggg 240
agagaacctg acttctcttt ccctctccct cctccaacat tactggaact ctatcctgtt 300
agggatcttc tgagcttgtt tccctgctgg gtgggacaga agacaaagga gaagggangg 360
tctacaanaa gcagcccttc tttgtcctct ggggttaatg agcttgacct ananttcatg 420
gaganaccan aagcctctga tttttaattt ccntnaaatg tttgaagtnt atatntacat 480
atatatattt ctttnaatnt ttgagtcttt gatatgtctt aaaatccant ccctctgccn 540
gaaacctgaa ttaaaaccat gaanaaaaat gtttncctta aagatgttan taattaattg 600
aaacttgaaa aaaaaaaaaa aa 622

<210> 58
<211> 433
<212> DNA
<213> Homo sapien

<400> 58
gaacaaattc tgattggtta tgtaccgtca aaagacttga agaaatttca tgattttgca 60
gtgtggaagc gttgaaaatt gaaagttact gcttttccac ttgctcatat agtaaaggga 120
tcctttcagc tgccagtgtt gaataatgta tcatccagag tgatgttatc tgtgacagtc 180
accagcttta agctgaacca ttttatgaat accaaataaa tagacctctt gtactgaaaa 240
catatttgtg actttaatcg tgctgcttgg atagaaatat ttttactggt tcttctgaat 300
tgacagtaaa cctgtccatt atgaatggcc tactgttcta ttatttgttt tgacttgaat 360
ttatccacca aagacttcat ttgtgtatca tcaataaagt tgtatgtttc aactgaaaaa 420
aaaaaaaaaa aaa 433

<210> 59
<211> 649

<212> DNA
<213> Homo sapien

<220>
<221> misc_feature
<222> (1)...(649)
<223> n = A,T,C or G

<400> 59
actagttatt atctgacttt cnggttataa tcattctaat gagtgtgaag tagcctctgg 60
tgtcatttgg atttgcattt ctctgatgag tgatgctatc aagcaccttt gctggtgctg 120
ttggccatat gtgtatgttc cctggagaag tgtctgtgct gagccttggc ccacttttta 180
attaggcgtn tgtcttttta ttactgagtt gtaaganttc tttatatatt ctggattcta 240
gacccttatc agatacatgg tttgcaaata ttttctccca ttctgtgggt tgtgttttca 300
ctttatcgat aatgtcctta gacatataat aaatttgtat tttaaaagtg acttgatttg 360
ggctgtgcaa ggtgggctca cgcttgtaat cccagcactt tgggagactg aggtgggtgg 420
atcatatgan gangctagga gttcgaggtc agcctggcca gcatagcgaa aacttgtctc 480
tacnaaaaat acaaaaatta gtcaggcatg gtggtgcacg tctgtaatac cagcttctca 540
ggangctgan gcacaaggat cacttgaacc ccagaangaa gangttgcag tganctgaag 600
atcatgccag ggcaacaaaa atgagaactt gtttaaaaaa aaaaaaaaa 649

<210> 60
<211> 423
<212> DNA
<213> Homo sapien

<220>
<221> misc_feature
<222> (1)...(423)
<223> n = A,T,C or G

<400> 60
actagttcag gccttccagt tcactgacaa acatggggaa gtgtgcccag ctggctggaa 60
acctggcagt gataccatca agcctgatgt ccaaaagagc aaagaatatt tctccaagca 120
gaagtgagcg ctgggctgtt ttagtgccag gctgcggtgg gcagccatga gaacaaaacc 180
tcttctgtat tttttttttc cattagtana acacaagact cngattcagc cgaattgtgg 240
tgtcttacaa ggcagggctt tcctacaggg ggtgganaaa acagcctttc ttcctttggt 300
aggaatggcc tgagttggcg ttgtgggcag gctactggtt tgtatgatgt attagtagag 360
caacccatta atcttttgta gtttgtatna aacttganct gagaccttaa acaaaaaaaa 420
aaa 423

<210> 61
<211> 423
<212> DNA
<213> Homo sapien

<220>
<221> misc_feature
<222> (1)...(423)
<223> n = A,T,C or G

<400> 61
cgggactgga atgtaaagtg aagttcggag ctctgagcac gggctcttcc cgccgggtcc 60
tccctcccca gaccccagag ggagaggccc accccgccca gccccgcccc agcccctgct 120
caggtctgag tatggctggg agtcgggggc cacaggcctc tagctgtgct gctcaagaag 180

actggatcag ggtanctaca agtggccggg ccttgccttt gggattctac cctgttccta 240
atttggtgtt ggggtgcggg gtccctggcc cccttttcca cactncctcc ctccngacag 300
caacctccct tggggcaatt gggcctggnt ctccncccgn tgttgcnacc ctttgttggt 360
ttaaggnctt taaaaatgtt annttttccc ntgccngggt taaaaaagga aaaaactnaa 420
aaa 423

<210> 62
<211> 683
<212> DNA
<213> Homo sapien

<220>
<221> misc_feature
<222> (1)...(683)
<223> n = A,T,C or G

<400> 62
gctggagagg ggtacggact ttcttggagt tgtcccaggt tggaatgaga ctgaactcaa 60
gaagagaccc taagagactg gggaatggtt cctgccttca ggaaagtgaa agacgcttag 120
gctgtcaaca cttaaaggaa gtccccttga agcccagagt ggacagacta gacccattga 180
tggggccact ggccatggtc cgtggacaag acattccngt gggccatggc acaccggggg 240
ggatcaaaat gtgtacttgt ggggtctcgc cccttgccaa aaccaaacca ntcccactcc 300
tgtcnttgga ctttcttccc attccctcct ccccaaatgc acttcccctc ctccctctgc 360
ccctcctgtg tttttggaat tctgtttccc tcaaaattgt taatttttta nttttngacc 420
atgaacttat gtttggggtc nangttcccc ttnccaatgc atactaatat attaatggtt 480
atttattttt gaaatatttt ttaatgaact tggaaaaaat tnntggaatt tccttncttc 540
cnttttnttt ggggggggtg gggggntggg ttaaaatttt tttggaancc cnatnggaaa 600
ttnttacttg gggcccccct naaaaaantn anttccaatt cttnnatngc ccctnttccn 660
ctaaaaaaaa ananannaaa aan 683

<210> 63
<211> 731
<212> DNA
<213> Homo sapien

<220>
<221> misc_feature
<222> (1)...(731)
<223> n = A,T,C or G

<400> 63
   actagtcata aagggtgtgc gcgtcttcga cgtggcggtc ttggcgccac tgctgcgaga 60
   cccggccctg gacctcaagg tcatccactt ggtgcgtgat ccccgcgcgg tggcgagttc 120
   acggatccgc tcgcgccacg gcctcatccg tgagagccta caggtggtgc gcagccgaga 180
   ccgcgagctc accgcatgcc cttcttggag gccgcgggcc acaagcttgg cgcccanaaa 240
   gaaggcgtng ggggcccgca aantaccacg ctctgggcgc tatggaangt cctcttgcaa 300
   taatattggt tnaaaanctg canaanagcc cctgcanccc cctgaactgg gntgcagggc 360
   cncttacctn gtttggntgc ggttacaaag aacctgtttn ggaaaaccct nccnaaaacc 420
   ttccgggaaa attntncaaa tttttnttgg ggaattnttg ggtaaacccc ccnaaaatgg 480
   gaaacntttt tgccctnnaa antaaaccat tnggttccgg gggccccccc ncaaaaccct 540
   tttttntttt tttntgcccc cantnncccc ccggggcccc tttttttngg ggaaaanccc 600
   cccccctncc nanantttta aaagggnggg anaatttttn nttncccccc gggncccccn 660
   ggngntaaaa nggtttcncc cccccgaggg gnggggnnnc ctcnnaaacc cntntcnnna 720
   ccncnttttn n 731

<210> 64
<211> 313
<212> DNA
<213> Homo sapien

<220>
<221> misc_feature
<222> (1)...(313)
<223> n = A,T,C or G

<400> 64
actagttgtg caaaccacga ctgaagaaag acgaaaagtg ggaaataact tgcaacgtct 60
gttagagatg gttgctacac atgttgggtc tgtagagaaa catcttgagg agcagattgc 120
taaagttgat agagaatatg aagaatgcat gtcagaagat ctctcggaaa atattaaaga 180
gattagagat aagtatgaga agaaagctac tctaattaag tcttctgaag aatgaagatn 240
aaatgttgat catgtatata tatccatagt gaataaaatt gtctcagtaa agttgtaaaa 300
aaaaaaaaaa aaa 313

<210> 65
<211> 420
<212> DNA
<213> Homo sapien

<220>
<221> misc_feature
<222> (1)...(420)
<223> n = A,T,C or G

<400> 65
actagttccc tggcaggcaa gggcttccaa ctgaggcagt gcatgtgtgg cagagagagg 60
caggaagctg gcagtggcag cttctgtgtc tagggagggg tgtggctccc tccttccctg 120
tctgggaggt tggagggaag aatctaggcc ttagcttgcc ctcctgccac ccttcccctt 180
gtagatactg ccttaacact ccctcctctc tcagctgtgg ctgccaccca agccaggttt 240
ctccgtgctc actaatttat ttccaggaaa ggtgtgtgga agacatgagc cgtgtataat 300
atttgtttta acattttcat tgcaagtatt gaccatcatc cttggttgtg tatcgttgta 360
acacaaatta atgatattaa aaagcatcca aacaaagccn annnnnaana nnannngaaa 420

<210> 66
<211> 676
<212> DNA
<213> Homo sapien

<220>
<221> misc_feature
<222> (1)...(676)
<223> n = A,T,C or G

<400> 66
actagtttcc tatgatcatt aaactcattc tcagggttaa gaaaggaatg taaatttctg 60
cctcaatttg tacttcatca ataagttttt gaagagtgca gatttttagt caggtcttaa 120
aaataaactc acaaatctgg atgcatttct aaattctgca aatgtttcct ggggtgactt 180
aacaaggaat aatcccacaa tatacctagc tacctaatac atggagctgg ggctcaaccc 240
actgttttta aggatttgcg cttacttgtg gctgaggaaa aataagtagt tccgagggaa 300
gtagttttta aatgtgagct tatagatngg aaacagaata tcaacttaat tatggaaatt 360
gttagaaacc tgttctcttg ttatctgaat cttgattgca attactattg tactggatag 420

actccagccc attgcaaagt ctcagatatc ttanctgtgt agttgaattc cttggaaatt 480
ctttttaaga aaaaattgga gtttnaaaga aataaacccc tttgttaaat gaagcttggc 540
tttttggtga aaaanaatca tcccgcaggg cttattgttt aaaaanggaa ttttaagcct 600
ccctggaaaa anttgttaat taaatgggga aaatgntggg naaaaattat ccgttagggt 660
ttaaagggaa aactta 676

<210> 67
<211> 620
<212> DNA
<213> Homo sapien

<220>
<221> misc_feature
<222> (1)...(620)
<223> n = A,T,C or G

<400> 67
   caccattaaa gctgcttacc aagaacttcc ccagcatttt gacttccttg tttgatagct 60
   gaattgtgag caggtgatag aagagccttt ctagttgaac atacagataa tttgctgaat 120
   acattccatt taatgaaggg gttacatctg ttacgaagct actaagaagg agcaagagca 180
   taggggaaaa aaatctgatc agaacgcatc aaactcacat gtgccccctc tactacaaac 240
   agattgtagt gctgtggtgg tttattccgt tgtgcagaac ttgcaagctg agtcactaaa 300
   cccaaagaga ggaaattata ggttagttaa acattgtaat cccaggaact aagtttaatt 360
   cacttttgaa gtgttttgtt ttttattttt ggtttgtctg atttactttg ggggaaaang 420
   ctaaaaaaaa agggatatca atctctaatt cagtgcccac taaaagttgt ccctaaaaag 480
   tctttactgg aanttatggg actttttaag ctccaggtnt tttggtcctc caaattaacc 540
   ttgcatgggc cccttaaaat tgttgaangg cattcctgcc tctaagtttg ggggaaattc 600
   ccccnttttn aaaatttgga 620

<210> 68
<211> 551
<212> DNA
<213> Homo sapien

<220>
<221> misc_feature
<222> (1)...(551)
<223> n = A,T,C or G

<400> 68
actagtagct ggtacataat cactgaggag ctatttctta acatgctttt atagaccatg 60
ctaatgctag accagtattt aagggctaat ctcacacctc cttagctgta agagtctggc 120
ttagaacaga cctctctgtg caataacttg tggccactgg aaatccctgg gccggcattt 180
gtattggggt tgcaatgact cccaagggcc aaaagagtta aaggcacgac tgggatttct 240
tctgagactg tggtgaaact ccttccaagg ctgagggggt cagtangtgc tctgggaggg 300
actcggcacc actttgatat tcaacaagcc acttgaagcc caattataaa attgttattt 360
tacagctgat ggaactcaat ttgaaccttc aaaactttgt tagtttatcc tattatattg 420
ttaaacctaa ttacatttgt ctagcattgg atttggttcc tgtngcatat gtttttttcn 480
cctatgtgct cccctccccc nnatcttaat ttaaaccnca attttgcnat tcnccnnnnn 540
nannnannna a 551

<210> 69
<211> 396
<212> DNA
<213> Homo sapien

<220>
<221> misc_feature
<222> (1)...(396)
<223> n = A,T,C or G

<400> 69
cagaaatgga aagcagagtt ttcatttctg tttataaacg tctccaaaca aaaatggaaa 60
gcagagtttt cattaaatcc ttttaccttt ttttttcttt ggtaatcccc tcaaataaca 120
gtatgtggga tattgaatgt taaagggata tttttttcta ttatttttat aattgtacaa 180
aattaagcaa atgttaaaag ttttatatgc tttattaatg ttttcaaaag gtatnataca 240
tgtgatacat tttttaagct tcagttgctt gtcttctggt actttctgtt atgggctttt 300
ggggagccan aaaccaatct acnatctctt tttgtttgcc aggacatgca ataaaattta 360
aaaaataaat aaaaactatt nagaaattga aaaaaa 396

<210> 70
<211> 536
<212> DNA
<213> Homo sapien

<220>
<221> misc_feature
<222> (1)...(536)
<223> n = A,T,C or G

<400> 70
actagtgcaa aagcaaatat aaacatcgaa aaggcgttcc tcacgttagc tgaagatatc 60
cttcgaaaga cccctgtaaa agagcccaac agtgaaaatg tagatatcag cagtggagga 120
ggcgtgacag gctggaagag caaatgctgc tgagcattct cctgttccat cagttgccat 180
ccactacccc gttttctctt cttgctgcaa aataaaccac tctgtccatt tttaactcta 240
aacagatatt tttgtttctc atcttaacta tccaagccac ctattttatt tgttctttca 300
tctgtgactg cttgctgact ttatcataat tttcttcaaa caaaaaaatg tatagaaaaa 360
tcatgtctgt gacttcattt ttaaatgnta cttgctcagc tcaactgcat ttcagttgtt 420
ttatagtcca gttcttatca acattnaaac ctatngcaat catttcaaat ctattctgca 480
aattgtataa gaataaaagt tagaatttaa caattaaaaa aaaaaaaaaa aaaaaa 536

<210> 71
<211> 865
<212> DNA
<213> Homo sapien

<220>
<221> misc_feature
<222> (1)...(865)
<223> n = A,T,C or G

<400> 71
gacaaagcgt taggagaaga anagaggcag ggaanactnc ccaggcacga tggccncctt 60
cccaccagca accagcgccc cccaccagcc cccaggcccg gacgacgaag actccatcct 120
ggattaatct nacctctntc gcctgnccca ttcctacctc ggaggtggag gccggaaagg 180
tcncaccaag aganaanctg ctgccaacac caaccgcccc agccctggcg ggcacganag 240
gaaactggtg accaatctgc agaattctna gaggaanaag cnaggggccc cgcgctnaga 300
cagagctgga tatgangcca gaccatggac nctacncccn ncaatncana cgggactgcg 360
gaagatggan gacccncgac nngatcaggc cngctnncca nccccccacc cctatgaatt 420
attcccgctg aangaatctc tganngcttt ccannaaagc gcctccccnc cnaacgnaan 480

tncaacatng ggattanang ctgggaactg naaggggcaa ancctnnaat atccccagaa 540
acaanctctc ccnaanaaac tggggcncct catnggtggn accaactatt aactaaaccg 600
cacgccaagn aantataaaa gggggccccc tccncggnng accccctttt gtcccttaat 660
ganggttatc cnccttgcgt accatggtnc ccnnttctgt ntgnatgttt ccnctcccct 720
ccncctatnt cnagccgaac tcnnatttnc ccgggggtgc natcnantng tncncctttn 780
ttngttgncc cngccctttc cgncggaacn cgtttccccg ttantaacgg cacccggggn 840
aagggtgntt ggccccctcc ctccc 865

<210> 72
<211> 560
<212> DNA
<213> Homo sapien

<220>
<221> misc_feature
<222> (1)...(560)
<223> n = A,T,C or G

<400> 72
cctggacttg tcttggttcc agaacctgac gacccggcga cggcgacgtc tcttttgact 60
aaaagacagt gtccagtgct ccngcctagg agtctacggg gaccgcctcc cgcgccgcca 120
ccatgcccaa cttctctggc aactggaaaa tcatccgatc ggaaaacttc gangaattgc 180
tcnaantgct gggggtgaat gtgatgctna ngaanattgc tgtggctgca gcgtccaagc 240
cagcagtgga gatcnaacag gagggagaca ctttctacat caaaacctcc accaccgtgc 300
gcaccacaaa gattaacttc nnngttgggg aggantttga ggancaaact gtggatngga 360
ngcctgtnaa aacctggtga aatgggagaa tganaataaa atggtctgtg ancanaaact 420
cctgaaagga gaaggccccc anaactcctg gaccngaaaa actgacccnc cnatngggga 480
actgatnctt gaaccctgaa cgggcgggat ganccttttt tnttgccncc naangggttc 540
tttccntttc cccaaaaaaa 560

<210> 73
<211> 379
<212> DNA
<213> Homo sapien

<220>
<221> misc_feature
<222> (1)...(379)
<223> n = A,T,C or G

<400> 73
ctggggancc ggcggtnngc nccatntcnn gncgcgaagg tggcaataaa aanccnctga 60
aaccgcncaa naaacatgcc naagatatgg acgaggaaga tngngctttc nngnacaanc 120
gnanngagga acanaacaaa ctcnangagc tctcaagcta atgccgcggg gaaggggccc 180
ttggccacnn gtggaattaa gaaatctggc aaanngtann tgttccttgt gcctnangag 240
ataagngacc ctttatttca tctgtattta aacctctctn ttccctgnca taacttcttt 300
tnccacgtan agntggaant anttgttgtc ttggactgtt gtncatttta gannaaactt 360
ttgttcaaaa aaaaaataa 379

<210> 74
<211> 437
<212> DNA
<213> Homo sapien

<220>

<221> misc_feature
<222> (1)...(437)
<223> n = A,T,C or G

<400> 74
actagttcag actgccacgc caaccccaga aaatacccca catgccagaa aagtgaagtc 60
ctaggtgttt ccatctatgt ttcaatctgt ccatctacca ggcctcgcga taaaaacaaa 120
acaaaaaaac gctgccaggt tttanaagca gttctggtct caaaaccatc aggatcctgc 180
caccagggtt cttttgaaat agtaccacat gtaaaaggga atttggcttt cacttcatct 240
aatcactgaa ttgtcaggct ttgattgata attgtagaaa taagtagcct tctgttgtgg 300
gaataagtta taatcagtat tcatctcttt gttttttgtc actcttttct ctctnattgt 360
gtcatttgta ctgtttgaaa aatatttctt ctataaaatt aaactaacct gccttaaaaa 420
aaaaaaaaaa aaaaaaa 437

<210> 75
<211> 579
<212> DNA
<213> Homo sapien

<220>
<221> misc_feature
<222> (1)...(579)
<223> n = A,T,C or G

<400> 75
ctccgtcgcc gccaagatga tgtgcggggc gccctccgcc acgcagccgg ccaccgccga 60
gacccagcac atcgccgacc aggtgaggtc ccagcttgaa gagaaagaaa acaagaagtt 120
ccctgtgttt aaggccgtgt cattcaagag ccaggtggtc gcggggacaa actacttcat 180
caaggtgcac gtcggcgacg aggacttcgt acacctgcga gtgttccaat ctctccctca 240
tgaaaacaag cccttgacct tatctaacta ccagaccaac aaagccaagc atgatgagct 300
gacctatttc tgatcctgac tttggacaag gcccttcagc cagaagactg acaaagtcat 360
cctccgtcta ccagagcgtg cacttgtgat cctaaaataa gcttcatctc cgggctgtgc 420
ccttggggtg gaaggggcan gatctgcact gcttttgcat ttctcttcct aaatttcatt 480
gtgttgattc tttccttcca ataggtgatc ttnattactt tcagaatatt ttccaaatna 540
gatatatttt naaaatcctt aaaaaaaaaa aaaaaaaaa 579

<210> 76
<211> 666
<212> DNA
<213> Homo sapien

<220>
<221> misc_feature
<222> (1)...(666)
<223> n = A,T,C or G

<400> 76
   gtttatccta tctctccaac cagattgtca gctccttgag ggcaagagcc acagtatatt 60
   tccctgtttc ttccacagtg cctaataata ctgtggaact aggttttaat aattttttaa 120
   ttgatgttgt tatgggcagg atggcaacca gaccattgtc tcagagcagg tgctggctct 180
   ttcctggcta ctccatgttg gctagcctct ggtaacctct tacttattat cttcaggaca 240
   ctcactacag ggaccaggga tgatgcaaca tccttgtctt tttatgacag gatgtttgct 300
   cagcttctcc aacaataaaa agcacgtggt aaaacacttg cggatattct ggactgtttt 360
   taaaaaatat acagtttacc gaaaatcata ttatcttaca atgaaaagga ntttatagat 420
   cagccagtga acaacctttt cccaccatac aaaaattcct tttcccgaan gaaaanggct 480
   ttctcaataa ncctcacttt cttaanatct tacaagatag ccccganatc ttatcgaaac 540
   tcattttagg caaatatgan ttttattgtn cgttacttgt ttcaaaattt ggtattgtga 600
   atatcaatta ccacccccat ctcccatgaa anaaanggga aanggtgaan ttcntaancg 660
   cttaaa 666

<210> 77
<211> 396
<212> DNA
<213> Homo sapien

<220>
<221> misc_feature
<222> (1)...(396)
<223> n = A,T,C or G

<400> 77
ctgcagcccg ggggatccac taatctacca nggttatttg gcagctaatt ctanatttgg 60
atcattgccc aaagttgcac ttgctggtct cttgggattt ggccttggaa aggtatcata 120
catanganta tgccanaata aattccattt ttttgaaaat canctccntg gggctggttt 180
tggtccacag cataacangc actgcctcct tacctgtgag gaatgcaaaa taaagcatgg 240
attaagtgag aagggagact ctcagccttc agcttcctaa attctgtgtc tgtgactttc 300
gaagtttttt aaacctctga atttgtacac atttaaaatt tcaagtgtac tttaaaataa 360
aatacttcta atgggaacaa aaaaaaaaaa aaaaaa 396

<210> 78
<211> 793
<212> DNA
<213> Homo sapien

<220>
<221> misc_feature
<222> (1)...(793)
<223> n = A,T,C or G

<400> 78
gcatcctagc cgccgactca cacaaggcag gtgggtgagg aaatccagag ttgccatgga 60
gaaaattcca gtgtcagcat tcttgctcct tgtggccctc tcctacactc tggccagaga 120
taccacagtc aaacctggag ccaaaaagga cacaaaggac tctcgaccca aactgcccca 180
gaccctctcc agaggttggg gtgaccaact catctggact cagacatatg aagaagctct 240
atataaatcc aagacaagca acaaaccctt gatgattatt catcacttgg atgagtgccc 300
acacagtcna gctttaaaga aagtgtttgc tgaaaataaa gaaatccaga aattggcaga 360
gcagtttgtc ctcctcaatc tggtttatga aacaactgac aaacaccttt ctcctgatgg 420
ccagtatgtc ccaggattat gtttgttgac ccatctctga cagttgaagc cgatatcctg 480
ggaagatatt cnaaccgtct ctatgcttac aaactgcaga tacgctctgt tgcttgacac 540
atgaaaaagc tctcaagttg ctnaaaatga attgtaagaa aaaaaatctc cagccttctg 600
tctgtcggct tgaaaattga aaccagaaaa atgtgaaaaa tggctattgt ggaacanatn 660
gacacctgat taggttttgg ttatgttcac cactattttt aanaaaanan nttttaaaat 720
ttggttcaat tntctttttn aaacaatntg tttctacntt gnganctgat ttctaaaaaa 780
aataatnttt ggc 793

<210> 79
<211> 456
<212> DNA
<213> Homo sapien

<220>
<221> misc_feature
<222> (1)...(456)
<223> n = A,T,C or G

<400> 79
actagtatgg ggtgggaggc cccacccttc tcccctaggc gctgttcttg ctccaaaggg 60
ctccgtggag agggactggc agagctgang ccacctgggg ctggggatcc cactcttctt 120
gcagctgttg agcgcaccta accactggtc atgcccccac ccctgctctc cgcacccgct 180
tcctcccgac cccangacca ggctacttct cccctcctct tgcctccctc ctgcccctgc 240
tgcctctgat cgtangaatt gangantgtc ccgccttgtg gctganaatg gacagtggca 300
ggggctggaa atgggtgtgt gtgtgtgtgt gtgtgtgtgt gtgtgtgtgt gcnccccccc 360
tgcaagaccg agattgaggg aaancatgtc tgctgggtgt gaccatgttt cctctccata 420
aantncccct gtgacnctca naaaaaaaaa aaaaaa 456

<210> 80
<211> 284
<212> DNA
<213> Homo sapien

<220>
<221> misc_feature
<222> (1)...(284)
<223> n = A,T,C or G

<400> 80
ctttgtacct ctagaaaaga taggtattgt gtcatgaaac ttgagtttaa attttatata 60
taaaactaaa agtaatgctc actttagcaa cacatactaa aattggaacc atactgagaa 120
gaatagcatg acctccgtgc aaacaggaca agcaaatttg tgatgtgttg attaaaaaga 180
aataaataaa tgtgtatatg tgtaacttgt atgtttatgt ggaatacaga ttgggaaata 240
aaatgtattt cttactgtga aaaaaaaaaa aaaaaaaaaa aana 284

<210> 81
<211> 671
<212> DNA
<213> Homo sapien

<220>
<221> misc_feature
<222> (1)...(671)
<223> n = A,T,C or G

<400> 81
gccaccaaca ttccaagcta ccctgggtac ctttgtgcag tagaagctag tgagcatgtg 60
agcaagcggt gtgcacacgg agactcatcg ttataattta ctatctgcca agagtagaaa 120
gaaaggctgg ggatatttgg gttggcttgg ttttgatttt ttgcttgttt gtttgttttg 180
tactaaaaca gtattatctt ttgaatatcg tagggacata agtatataca tgttatccaa 240
tcaagatggc tagaatggtg cctttctgag tgtctaaaac ttgacacccc tggtaaatct 300
ttcaacacac ttccactgcc tgcgtaatga agttttgatt catttttaac cactggaatt 360
tttcaatgcc gtcattttca gttagatnat tttgcacttt gagattaaaa tgccatgtct 420
atttgattag tcttattttt ttatttttac aggcttatca gtctcactgt tggctgtcat 480
tgtgacaaag tcaaataaac ccccnaggac aacacacagt atgggatcac atattgtttg 540
acattaagct ttggccaaaa aatgttgcat gtgttttacc tcgacttgct aaatcaatan 600
canaaaggct ggctnataat gttggtggtg aaataattaa tnantaacca aaaaaaaaan 660
aaaaaaaaaa a 671

<210> 82
<211> 217
<212> DNA
<213> Homo sapien

<220>
<221> misc_feature
<222> (1)...(217)
<223> n = A,T,C or G

<400> 82
   ctgcagatgt ttcttgaatg ctttgtcaaa ttaanaaagt taaagtgcaa taatgtttga 60
   agacaataag tggtggtgta tcttgtttct aataagataa acttttttgt ctttgcttta 120
   tcttattagg gagttgtatg tcagtgtata aaacatactg tgtggtataa caggcttaat 180
   aaattcttta aaaggaaaaa aaaaaaaaaa aaaaaaa 217

<210> 83
<211> 460
<212> DNA
<213> Homo sapien

<220>
<221> misc_feature
<222> (1)...(460)
<223> n = A,T,C or G

<400> 83
cgcgagtggg agcaccagga tctcgggctc ggaacgagac tgcacggatt gttttaagaa 60
aatggcagac aaaccagaca tgggggaaat cgccagcttc gatnaggcca agctgaanaa 120
aacggagacg caggagaaga acaccctgcc gaccaaagag accattgagc angagaagcg 180
gagtgaaatt tcctaagatc ctggaggatt tcctaccccc gtcctcttcg agaccccagt 240
cgtgatgtgg aggaagagcc acctgcaaga tggacacgag ccacaagctg cactgtgaac 300
ctgggcactc cgcgccgatg ccaccggcct gtgggtctct gaagggaccc cccccaatcg 360
gactgccaaa ttctccggtt tgccccggga tattatacaa nattatttgt atgaataatg 420
annataaaac acacctcgtg gcancaaana aaaaaaaaaa 460

<210> 84
<211> 323
<212> DNA
<213> Homo sapien

<220>
<221> misc_feature
<222> (1)...(323)
<223> n = A,T,C or G

<400> 84
tggtggatct tggctctgtg gagctgctgg gacgggatct aaaagactat tctggaagct 60
gtggtccaan gcattttgct ggcttaacgg gtcccggaac aaaggacacc agctctctaa 120
aattgaagtt tacccganat aacaatcttt tgggcagaga tgcctatttt aacaaacncc 180
gtccctgcgc aacaacnaac aatctctggg aaataccggc catgaacntg ctgtctcaat 240
cnancatctc tctagctgac cgatcatatc gtcccagatt actacanatc ataataattg 300
atttcctgta naaaaaaaaa aaa 323

<210> 85
<211> 771
<212> DNA
<213> Homo sapien

<220>
<221> misc_feature
<222> (1)...(771)
<223> n = A,T,C or G

<400> 85
aaactgggta ctcaacactg agcagatctg ttctttgagc taaaaaccat gtgctgtacc 60
aanagtttgc tcctggctgc tttgatgtca gtgctgctac tccacctctg cggcgaatca 120
gaagcaagca actttgactg ctgtcttgga tacacagacc gtattcttca tcctaaattt 180
attgtgggct tcacacggca gctggccaat gaaggctgtg acatcaatgc tatcatcttt 240
cacacaaaga aaaagttgtc tgtgtgcgca aatccaaaac agacttgggt gaaatatatt 300
gtgcgtctcc tcagtaaaaa agtcaagaac atgtaaaaac tgtggctttt ctggaatgga 360
attggacata gcccaagaac agaaagaact tgctggggtt ggaggtttca cttgcacatc 420
atgganggtt tagtgcttat cttatttgtg cctcctggac ttgtccaatt natgaagtta 480
atcatattgc atcatanttt gctttgttta acatcacatt naaattaaac tgtattttat 540
gttatttata gctntaggtt ttctgtgttt aactttttat acnaantttc ctaaactatt 600
ttggtntant gcaanttaaa aattatattt gggggggaa taaatattgg antttctgca 660
gccacaagct ttttttaaaa aaccantaca nccnngttaa atggtnggtc ccnaatggtt 720
tttgcttttn antagaaaat ttnttagaac natttgaaaa aaaaaaaaaa a 771

<210> 86
<211> 628
<212> DNA
<213> Homo sapien

<220>
<221> misc_feature
<222> (1)...(628)
<223> n = A,T,C or G

<400> 86
actagtttgc tttacatttt tgaaaagtat tatttttgtc caagtgctta tcaactaaac 60
cttgtgttag gtaagaatgg aatttattaa gtgaatcagt gtgacccttc ttgtcataag 120
attatcttaa agctgaagcc aaaatatgct tcaaaagaaa angactttat tgttcattgt 180
agttcataca ttcaaagcat ctgaactgta gtttctatag caagccaatt acatccataa 240
gtggagaang aaatagatta atgtcnaagt atgattggtg gagggagcaa ggttgaagat 300
aatctggggt tgaaattttc tagttttcat tctgtacatt tttagttnga catcagattt 360
gaaatattaa tgtttacctt tcaatgtgtg gtatcagctg gactcantaa cacccctttc 420
ttccctnggg gatggggaat ggattattgg aaaatggaaa gaaaaaagta cttaaagcct 480
tcctttcnca gtttctggct cctaccctac tgatttancc agaataagaa aacattttat 540
catcntctgc tttattccca ttaatnaant tttgatgaat aaatctgctt ttatgcnnac 600
ccaaggaatt nagtggnttc ntcnttgt 628

<210> 87
<211> 518
<212> DNA
<213> Homo sapien

<220>
<221> misc_feature

<222> (1)...(518)
<223> n = A,T,C or G

<400> 87
tttttatttt tttttagaga gtagttcagc ttttatttat aaatttattg cctgttttat 60
tataacaaca ttatactgtt tatggtttaa tacatatggt tcaaaatgta taatacatca 120
agtagtacag ttttaaaatt ttatgcttaa aacaagtttt gtgtaaaaaa tgcagataca 180
ttttacatgg caaatcaatt tttaagtcat cctaaaaatt gatttttttt tgaaatttaa 240
aaacacattt aatttcaatt tctctcttat ataaccttta ttactatagc atggtttcca 300
ctacagttta acaatgcagc aaaattccca tttcacggta aattgggttt taagcggcaa 360
ggttaaaatg ctttgaggat cctnaatacc ctttgaactt caaatgaagg ttatggttgt 420
naatttaacc ctcatgccat aagcagaagc acaagtttag ctgcattttg ctctaaactg 480
taaaancgag ccccccgttg aaaaagcaaa agggaccc 518

<210> 88
<211> 1844
<212> DNA
<213> Homo sapien

<400> 88
   gagacagtga atcctagtat caaaggattt ttggcctcag aaaaagttgt tgattatttt 60
   tattttattt tatttttcga gactccgtct caaaaaaaaa aaaaaaaaaa agaatcacaa 120
   ggtatttgct aaagcatttt gagctgcttg gaaaaaggga agtagttgca gtagagtttc 180
   ttccatcttc ttggtgctgg gaagccatat atgtgtcttt tactcaagct aaggggtata 240
   agcttatgtg ttgaatttgc tacatctata tttcacatat tctcacaata agagaatttt 300
   gaaatagaaa tatcatagaa catttaagaa agtttagtat aaataatatt ttgtgtgttt 360
   taatcccttt gaaggatcct atccaaagaa aatattttac actgagctcc ttcctacacg 420
   tctcagtaac agatcctgtg ttagtctttg aaaatagctc attttttaaa tgtcagtgag 480
   tagatgtagc atacatatga tgtataatga cgtgtattat gttaacaatg tctgcagatt 540
   ttgtaggaat acaaaacatg gcctttttta taagcaaaac gggccaatga ctagaataac 600
   acataggcca atctgtgaat atgtattata agcagcattc cagaaaagta gttggtgaaa 660
   taattttcaa gtcaaaaagg gatatggaaa gggaattatg agtaacctct attttttaag 720
   ccttgctttt aaattaaacg ctacagccat ttaagccttg aggataataa agcttgagag 780
   taataatgtt aggttagcaa aggtttagat gtatcacttc atgcatgcta ccatgatagt 840
   aatgcagctc ttcgagtcat ttctggtcat tcaagatatt cacccttttg cccatagaaa 900
   gcaccctacc tcacctgctt actgacattg tcttagctga tcacaagatc attatcagcc 960
   tccattattc cttactgtat ataaaataca gagttttata ttttcctttc ttcgtttttc 1020
   accatattca aaacctaaat ttgtttttgc agatggaatg caaagtaatc aagtgttcgt 1080
   gctttcacct agaagggtgt ggtcctgaag gaaagaggtc cctaaatatc ccccaccctg 1140
   ggtgctcctc cttccctggt accctgacta ccagaagtca ggtgctagag cagctggaga 1200
   agtgcagcag cctgtgcttc cacagatggg ggtgctgctg caacaaggct ttcaatgtgc 1260
   ccatcttagg gggagaagct agatcctgtg cagcagcctg gtaagtcctg aggaggttcc 1320
   attgctcttc ctgctgctgt cctttgcttc tcaacggggc tcgctctaca gtctagagca 1380
   catgcagcta acttgtgcct ctgcttatgc atgagggtta aattaacaac cataaccttc 1440
   atttgaagtt caaaggtgta ttcaggatcc tcaaagcatt ttaaccttgc cgcttaaaac 1500
   ccaatttacc gtgaaatggg aattttgctg cattgttaaa ctgtagtgga aaccatgcta 1560
   tagtaataaa ggttatataa gagagaaatt gaaattaaat gtgtttttaa atttcaaaaa 1620
   aaaatcaatc tttaggatga cttaaaaatt gatttgccat gtaaaatgta tctgcatttt 1680
   ttacacaaaa cttgttttaa gcataaaatt ttaaaactgt actacttgat gtattataca 1740
   ttttgaacca tatgtattaa accataaaca gtataatgtt gttataataa aacaggcaat 1800
   aaatttataa ataaaagctg aaaaaaaaaa aaaaaaaaaa aaaa 1844

<210> 89
<211> 523
<212> DNA

<213> Homo sapien

<220>
<221> misc_feature
<222> (1)...(523)
<223> n = A,T,C or G

<400> 89
tttttttttt tttttttagt caatccacat ttattgatca cttattatgt accaggcact 60
gggataaaga tgactgttag tcactcacag taaggaagaa aactagcaaa taagacgatt 120
acaatatgat gtagaaaatg ctaagccaga gatatagaaa ggtcctattg ggtccttctg 180
tcaccttgtc tttccacatc cctacccttc acaggccttc cctccagctt cctgcccccg 240
ctccccactg cagatcccct gggattttgc ctagagctaa acgagganat gggccccctg 300
gccctggcat gacttgaacc caaccacaga ctgggaaagg gagcctttcg anagtggatc 360
actttgatna gaaaacacat agggaattga agagaaantc cccaaatggc cacccgtgct 420
ggtgctcaag aaaagtttgc agaatggata aatgaaggat caagggaatt aatanatgaa 480
taattgaatg gtggctcaat aagaatgact ncnttgaatg acc 523

<210> 90
<211> 604
<212> DNA
<213> Homo sapien

<220>
<221> misc_feature
<222> (1)...(604)
<223> n = A,T,C or G

<400> 90
ccagtgtggt ggaatgcaaa gattaccccg gaagctttcg agaagctggg attccctgca 60
gcaaaggaaa tagccaatat gtgtcgtttc tatgaaatga agccagaccg agatgtcaat 120
ctcacccacc aactaaatcc caaagtcaaa agcttcagcc agtttatctc agagaaccag 180
gggagccttc aagggcatgt agaaaatcag ctgttcagat aggcctctgc accacacagc 240
ctctttcctc tctgatcctt ttcctcttta cggcacaaca ttcatgtttg acagaacatg 300
ctggaatgca attgtttgca acaccgaagg atttcctgcg gtcgcctctt cagtaggaag 360
cactgcattg gtgataggac acggtaattt gattcacatt taacttgcta gttagtgata 420
aggggtggta cacctgtttg gtaaaatgag aagcctcgga aacttgggag cttctctcct 480
accactaatg gggagggcag attattactg ggatttctcc tggggtgaat taatttcaag 540
ccctaattgc tgaaattccc ctnggcaggc tccagttttc tcaactgcat tgcaaaattc 600
cccc 604

<210> 91
<211> 858
<212> DNA
<213> Homo sapien

<220>
<221> misc_feature
<222> (1)...(858)
<223> n = A,T,C or G

<400> 91
tttttttttt ttttttttta tgattattat tttttttatt gatctttaca tcctcagtgt 60
tggcagagtt tctgatgctt aataaacatt tgttctgatc agataagtgg aaaaaattgt 120
catttcctta ttcaagccat gcttttctgt gatattctga tcctagttga acatacagaa 180

ataaatgtct aaaacagcac ctcgattctc gtctataaca ggactaagtt cactgtgatc 240
ttaaataagc ttggctaaaa tgggacatga gtggaggtag tcacacttca gcgaagaaag 300
agaatctcct gtataatctc accaggagat tcaacgaatt ccaccacact ggactagtgg 360
atcccccggg ctgcaggaat tcgatatcaa gcttatcgat accgtcgacc tcgagggggg 420
gcccggtacc caattcgccc tatagtgagt cgtattacgc gcgctcactg gccgtcgttt 480
tacaacgtcg tgactgggaa aaccctggcg ttacccaact taatcgcctt gcagcacatc 540
cccctttcgc cagctggcgt aatagcgaan agcccgcacc gatcgccctt ncaacagttg 600
cgcagcctga atggcgaatg ggacgcgccc tgtagcggcg cattaagcgc cggcngggtg 660
tggnggntcc cccacgtgac cgntacactt ggcagcgcct tacgccggtc nttcgctttc 720
ttcccttcct ttctcgcacc gttcgccggg tttccccgnn agctnttaat cgggggnctc 780
cctttanggg tncnaattaa nggnttacng gaccttngan cccaaaaact ttgattaggg 840
ggaaggtccc cgaagggg 858

<210> 92
<211> 585
<212> DNA
<213> Homo sapien

<220>
<221> misc_feature
<222> (1)...(585)
<223> n = A,T,C or G

<400> 92
gttgaatctc ctggtgagat tatacaggag attctctttc ttcgctgaag tgtgactacc 60
tccactcatg tcccatttta gccaagctta tttaagatca cagtgaactt agtcctgtta 120
tagacgagaa tcgaggtgct gttttagaca tttatttctg tatgttcaac taggatcaga 180
atatcacaga aaagcatggc ttgaataagg aaatgacaat tttttccact tatctgatca 240
gaacaaatgt ttattaagca tcagaaactc tgccaacact gaggatgtaa agatcaataa 300
aaaaaataat aatcatnann naaanannan nngaagggcg gccgccaccg cggtggagct 360
ccagcttttg ttccctttag tgagggttaa ttgcgcgctt ggcgttaatc atggtcatag 420
ctgtttcctg tgtgaaattg ttatccggct cacaattccn cncaacatac gagccgggaa 480
gcntnangtg taaaagcctg gggtgcctaa attgagtgag ctnactcaca ttaattgngt 540
tgcgctccac ttgcccgctt ttccantccg ggaaacctgt tcgnc 585

<210> 93
<211> 567
<212> DNA
<213> Homo sapien

<220>
<221> misc_feature
<222> (1)...(567)
<223> n = A,T,C or G

<400> 93
   cggcagtgtt gctgtctgcg tgtccacctt ggaatctggc tgaactggct gggaggacca 60
   agactgcggc tggggtgggc anggaaggga accgggggct gctgtgaagg atcttggaac 120
   ttccctgtac ccaccttccc cttgcttcat gtttgtanag gaaccttgtg ccggccaagc 180
   ccagtttcct tgtgtgatac actaatgtat ttgctttttt tgggaaatan anaaaaatca 240
   attaaattgc tantgtttct ttgaannnnn nnnnnnnnnn nnnnnnnggg ggggncgccc 300
   ccncggngga aacnccccct tttgttccct ttaattgaaa ggttaattng cncncntggc 360
   gttaanccnt gggccaaanc tngttncccg tgntgaaatt gttnatcccc tcccaaattc 420
   ccccccnncc ttccaaaccc ggaaancctn annntgttna ancccggggg gttgcctaan 480
   ngnaattnaa ccnaaccccc ntttaaatng nntttgcncn ccacnngccc cnctttccca 540
   nttcggggaa aaccctntcc gtgccca 567

<210> 94
<211> 620
<212> DNA
<213> Homo sapien

<220>
<221> misc_feature
<222> (1)...(620)
<223> n = A,T,C or G

<400> 94
actagtcaaa aatgctaaaa taatttggga gaaaatattt tttaagtagt gttatagttt 60
catgtttatc ttttattatg ttttgtgaag ttgtgtcttt tcactaatta cctatactat 120
gccaatattt ccttatatct atccataaca tttatactac atttgtaana naatatgcac 180
gtgaaactta acactttata aggtaaaaat gaggtttcca anatttaata atctgatcaa 240
gttcttgtta tttccaaata gaatggactt ggtctgttaa gggctaagga gaagaggaag 300
ataaggttaa aagttgttaa tgaccaaaca ttctaaaaga aatgcaaaaa aaaagtttat 360
tttcaagcct tcgaactatt taaggaaagc aaaatcattt cctaaatgca tatcatttgt 420
gagaatttct cattaatatc ctgaatcatt catttcacta aggctcatgt tnactccgat 480
atgtctctaa gaaagtacta tttcatggtc caaacctggt tgccatantt gggtaaaggc 540
tttcccttaa gtgtgaaant atttaaaatg aaattttcct ctttttaaaa attctttana 600
agggttaagg gtgttgggga 620

<210> 95
<211> 470
<212> DNA
<213> Homo sapien

<220>
<221> misc_feature
<222> (1)...(470)
<223> n = A,T,C or G

<400> 95
ctcgaccttc tctgcacagc ggatgaaccc tgagcagctg aagaccagaa aagccactat 60
nactttntgc ttaattcang agcttacang attcttcaaa gagtgngtcc agcatccttt 120
gaaacatgag ttcttaccag cagaagcaga cctttacccc accacctcag cttcaacagc 180
agcaggtgaa acaacccatc cagcctccac ctnaggaaat atttgttccc acaaccaagg 240
agccatgcca ctcaaaggtt ccacaacctg naaacacaaa nattccagag ccaggctgta 300
ccaaggtccc tgagccaggg ctgtaccaan gtccctgagc caggttgtac caangtccct 360
gagccaggat gtaccaaggt ccctgancca ggttgtccaa ggtccctgag ccaggctaca 420
ccaagggcct gngccaggca gcatcaangt ccctgaccaa ggcttatcaa 470

<210> 96
<211> 660
<212> DNA
<213> Homo sapien

<220>
<221> misc_feature
<222> (1)...(660)
<223> n = A,T,C or G

<400> 96
tttttttttt tttttttttt ggaattaaaa gcaatttaat gagggcagag caggaaacat 60
gcatttcttt tcattcgaat cttcagatga accctgagca gccgaagacc agaaaagcca 120
tgaagacttt ctgcttaatt caggggctta caggattctt cagagtgtgt gtgaacaaaa 180
gctttatagt acgtattttt aggatacaaa taagagagag actatggctt ggggtgagaa 240
tgtactgatt acaaggtcta cagacaatta agacacagaa acagatggga agagggtgnc 300
cagcatctgg nggttggctt ctcaagggct tgtctgtgca ccaaattact tctgcttggn 360
cttctgctga gctgggcctg gagtgaccgt tgaaggacat ggctctggta cctttgtgta 420
gcctgncaca ggaactttgg tgtatccttg ctcaggaact ttgatggcac ctggctcagg 480
aaacttgatg aagccttggt caagggacct tgatgcttgc tggctcaggg accttggngn 540
ancctgggct canggacctt tgncncaacc ttggcttcaa gggacccttg gnacatcctg 600
gcnnagggac ccttgggncc aaccctgggc ttnagggacc ctttggntnc nanccttggc 660

<210> 97
<211> 441
<212> DNA
<213> Homo sapien

<220>
<221> misc_feature
<222> (1)...(441)
<223> n = A,T,C or G

<400> 97
gggaccatac anagtattcc tctcttcaca ccaggaccag ccactgttgc agcatgagtt 60
cccagcagca gaagcagccc tgcatcccac cccctcagct tcagcagcag caggtgaaac 120
agccttgcca gcctccacct caggaaccat gcatccccaa aaccaaggag ccctgccacc 180
ccaaggtgcc tgagccctgc caccccaaag tgcctgagcc ctgccagccc aaggttccag 240
agccatgcca ccccaaggtg cctgagccct gcccttcaat agtcactcca gcaccagccc 300
agcagaanac caagcagaag taatgtggtc cacagccatg cccttgagga gccggccacc 360
agatgctgaa tcccctatcc cattctgtgt atgagtccca tttgccttgc aattagcatt 420
ctgtctcccc caaaaaaaaa a 441

<210> 98
<211> 600
<212> DNA
<213> Homo sapien

<220>
<221> misc_feature
<222> (1)...(600)
<223> n = A,T,C or G

<400> 98
gtattcctct cttcacacca ggaccagcca ctgttgcagc atgagttccc agcagcagaa 60
gcagccctgc atcccacccc ctcagcttca gcagcagcag gtgaaacagc cttgccagcc 120
tccacctcag gaaccatgca tccccaaaac caaggagccc tgccacccca aggtgcctga 180
gccctgccac cccaaagtgc ctgagccctg ccagcccaag gttccagagc catgccaccc 240
caaggtgcct gagccctgcc cttcaatagt cactccagca ccagcccagc agaanaccaa 300
gcagaagtaa tgtggtccac agccatgccc ttgaggagcc ggccaccana tgctgaatcc 360
cctatcccat tctgtgtatg agtcccattt gccttgcaat tagcattctg tctcccccaa 420
aaaagaatgt gctatgaagc tttctttcct acacactctg agtctctgaa tgaagctgaa 480
ggtcttaant acaganctag ttttcagctg ctcagaattc tctgaagaaa agatttaaga 540
tgaaaggcaa atgattcagc tccttattac cccattaaat tcnctttcaa ttccaaaaaa 600

<210> 99
<211> 667
<212> DNA
<213> Homo sapien

<220>
<221> misc_feature
<222> (1)...(667)
<223> n = A,T,C or G

<400> 99
actagtgact gagttcctgg caaagaaatt tgacctggac cagttgataa ctcatgtttt 60
accatttaaa aaaatcagtg aaggatttga gctgctcaat tcaggacaaa gcattcgaac 120
ggtcctgacg ttttgagatc caaagtggca ggaggtctgt gttgtcatgg tgaactggag 180
tttctcttgt gagagttccc tcatctgaaa tcatgtatct gtctcacaaa tacaagcata 240
agtagaagat ttgttgaaga catagaaccc ttataaagaa ttattaacct ttataaacat 300
ttaaagtctt gtgagcacct gggaattagt ataataacaa tgttnatatt tttgatttac 360
attttgtaag gctataattg tatcttttaa gaaaacatac cttggatttc tatgttgaaa 420
tggagatttt taagagtttt aaccagctgc tgcagatata ttactcaaaa cagatatagc 480
gtataaagat atagtaaatg catctcctag agtaatattc acttaacaca ttggaaacta 540
ttatttttta gatttgaata tnaatgttat tttttaaaca cttgttatga gttacttggg 600
attacatttt gaaatcagtt cattccatga tgcanattac tgggattaga ttaagaaaga 660
cggaaaa 667

<210> 100
<211> 583
<212> DNA
<213> Homo sapien

<220>
<221> misc_feature
<222> (1)...(583)
<223> n = A,T,C or G

<400> 100
gttttgtttg taagatgatc acagtcatgt tacactgatc taaaggacat atatataacc 60
ctttaaaaaa aaaatcactg cctcattctt atttcaagat gaatttctat acagactaga 120
tgtttttctg aagatcaatt agacattttg aaaatgattt aaagtgtttt ccttaatgtt 180
ctctgaaaac aagtttcttt tgtagtttta accaaaaaag tgcccttttt gtcactggat 240
tctcctagca ttcatgattt ttttttcata caatgaaatt aaaattgcta aaatcatgga 300
ctggctttct ggttggattt caggtaagat gtgtttaagg ccagagcttt tctcagtatt 360
tgattttttt ccccaatatt tgatttttta aaaatataca catnggtgct gcatttatat 420
ctgctggttt aaaattctgt catatttcac ttctagcctt ttagttatgg caaatcatat 480
tttactttta cttaaagcat ttggtnattt ggantatctg gttctannct aaaaaaanta 540
attctatnaa ttgaantttt ggtactcnnc catatttgga tcc 583

<210> 101
<211> 592
<212> DNA
<213> Homo sapien

<220>
<221> misc_feature
<222> (1)...(592)
<223> n = A,T,C or G

<400> 101
gtggagacgt acaaagagca gccgctcaag acacctggga agaaaaagaa aggcaagccc 60
gggaaacgca aggagcagga aaagaaaaaa cggcgaactc gctctgcctg gttagactct 120
ggagtgactg ggagtgggct agaaggggac cacctgtctg acacctccac aacgtcgctg 180
gagctcgatt cacggaggca ttgaaatttt cagcaganac cttccaagga catattgcag 240
gattctgtaa tagtgaacat atggaaagta ttagaaatat ttattgtctg taaatactgt 300
aaatgcattg gaataaaact gtctccccca ttgctctatg aaactgcaca ttggtcattg 360
tgaatatttt tttttttgcc aaggctaatc caattattat tatcacattt accataattt 420
attttgtcca ttgatgtatt tattttgtaa atgtatcttg gtgctgctga atttctatat 480
tttttgtaca taatgcnttt anatatacct atcaagtttg ttgataaatg acncaatgaa 540
gtgncncnan ttggnggttg aatttaatga atgcctaatt ttattatccc aa 592

<210> 102
<211> 587
<212> DNA
<213> Homo sapien

<220>
<221> misc_feature
<222> (1)...(587)
<223> n = A,T,C or G

<400> 102
cgtcctaagc acttagacta catcagggaa gaacacagac cacatccctg tcctcatgcg 60
gcttatgttt tctggaagaa agtggagacc nagtccttgg ctttagggct ccccggctgg 120
gggctgtgca ntccggtcag ggcgggaagg gaaatgcacc gctgcatgtg aacttacagc 180
ccaggcggat gccccttccc ttagcactac ctggcctcct gcatcccctc gcctcatgtt 240
cctcccacct tcaaanaatg aanaacccca tgggcccagc cccttgccct ggggaaccaa 300
ggcagccttc caaaactcag gggctgaagc anactattag ggcaggggct gactttgggt 360
gacactgccc attccctctc agggcagctc angtcacccn ggnctcttga acccagcctg 420
ttcctttgaa aaagggcaaa actgaaaagg gcttttccta naaaaagaaa aaccagggaa 480
ctttgccagg gcttcnntnt taccaaaacn ncttctcnng gatttttaat tccccattng 540
gcctccactt accnggggcn atgccccaaa attaanaatt tcccatc 587

<210> 103
<211> 496
<212> DNA
<213> Homo sapien

<220>
<221> misc_feature
<222> (1)...(496)
<223> n = A,T,C or G

<400> 103
anaggactgg ccctacntgc tctctctcgt cctacctatc aatgcccaac atggcagaac 60
ctgcanccct tggncactgc anatggaaac ctctcagtgt cttgacatca ccctacccnt 120
gcggtgggtc tccaccacaa ccactttgac tctgtggtcc ctgnanggtg gnttctcctg 180
actggcagga tggaccttan ccnacatatc cctctgttcc ctctgctnag anaaagaatt 240
cccttaacat gatataatcc acccatgcaa ntngctactg gcccagctac catttaccat 300
ttgcctacag aatttcattc agtctacact ttggcattct ctctggcgat agagtgtggc 360
tgggctgacc gcaaaaggtg ccttacacac tggcccccac cctcaaccgt tgacncatca 420
gangcttgcc tcctccttct gattnncccc catgttggat atcagggtgc tcnagggatt 480
ggaaaagaaa caaaac 496

<210> 104
<211> 575
<212> DNA
<213> Homo sapien

<220>
<221> misc_feature
<222> (1)...(575)
<223> n = A,T,C or G

<400> 104
gcacctgctc tcaatccnnc tctcaccatg atcctccgcc tgcanaaact cctctgccaa 60
ctatggangt ggtttcnggg gtggctcttg ccaactggga agaagccgtg gtgtctctac 120
ctgttcaact cngtttgtgt ctgggggatc aactnggggc tatggaagcg gctnaactgt 180
tgttttggtg gaagggctgg taattggctt tgggaagtng cttatngaag ttggcctngg 240
gaagttgcta ttgaaagtng ccntggaagt ngntttggtg gggggttttg ctggtggcct 300
ttgttnaatt tgggtgcttt gtnaatggcg gccccctcnc ctgggcaatg aaaaaaatca 360
ccnatgcngn aaacctcnac nnaacagcct gggcttccct cacctcgaaa aaagttgctc 420
cccccccaaa aaaggncaan cccctcaann tggaangttg aaaaaatcct cgaatgggga 480
ncccnaaaac aaaaancccc ccntttcccn gnaanggggg aaataccncc cccccactta 540
cnaaaaccct tntaaaaaac cccccgggaa aaaaa 575

<210> 105
<211> 619
<212> DNA
<213> Homo sapien

<220>
<221> misc_feature
<222> (1)...(619)
<223> n = A,T,C or G

<400> 105
cactagtagg atagaaacac tgtgtcccga gagtaaggag agaagctact attgattaga 60
gcctaaccca ggttaactgc aagaagaggc gggatacttt cagctttcca tgtaactgta 120
tgcataaagc caatgtagtc cagtttctaa gatcatgttc caagctaact gaatcccact 180
tcaatacaca ctcatgaact cctgatggaa caataacagg cccaagcctg tggtatgatg 240
tgcacacttg ctagactcan aaaaaatact actctcataa atgggtggga gtattttggt 300
gacaacctac tttgcttggc tgagtgaagg aatgatattc atatattcat ttattccatg 360
gacatttagt tagtgctttt tatataccag gcatgatgct gagtgacact cttgtgtata 420
tttccaaatt tttgtacagt cgctgcacat atttgaaatc atatattaag acttccaaaa 480
aatgaagtcc ctggtttttc atggcaactt gatcagtaaa ggattcncct ctgtttggta 540
cttaaaacat ctactatatn gttnanatga aattcctttt ccccncctcc cgaaaaaana 600
aagtggtggg gaaaaaaaa 619

<210> 106
<211> 506
<212> DNA
<213> Homo sapien

<220>
<221> misc_feature
<222> (1)...(506)
<223> n = A,T,C or G

<400> 106
cattggtnct ttcatttgct ntggaagtgt nnatctctaa cagtggacaa agttcccngt 60
gccttaaact ctgtnacact tttgggaant gaaaanttng tantatgata ggttattctg 120
angtanagat gttctggata ccattanatn tgcccccngt gtcagaggct catattgtgt 180
tatgtaaatg gtatntcatt cgctactatn antcaattng aaatanggtc tttgggttat 240
gaatantnng cagcncanct nanangctgt ctgtngtatt cattgtggtc atagcacctc 300
acancattgt aacctcnatc nagtgagaca nactagnaan ttcctagtga tggctcanga 360
ttccaaatgg nctcatntcn aatgtttaaa agttanttaa gtgtaagaaa tacagactgg 420
atgttccacc aactagtacc tgtaatgacn ggcctgtccc aacacatctc ccttttccat 480
gactgtggta ncccgcatcg gaaaaa 506

<210> 107
<211> 452
<212> DNA
<213> Homo sapien

<220>
<221> misc_feature
<222> (1)...(452)
<223> n = A,T,C or G

<400> 107
gttgagtctg tactaaacag taagatatct caatgaacca taaattcaac tttgtaaaaa 60
tcttttgaag catagataat attgtttggt aaatgtttct tttgtttggt aaatgtttct 120
tttaaagacc ctcctattct ataaaactct gcatgtagag gcttgtttac ctttctctct 180
ctaaggttta caataggagt ggtgatttga aaaatataaa attatgagat tggttttcct 240
gtggcataaa ttgcatcact gtatcatttt cttttttaac cggtaagant ttcagtttgt 300
tggaaagtaa ctgtganaac ccagtttccc gtccatctcc cttagggact acccatagaa 360
catgaaaagg tccccacnga agcaagaaga taagtctttc atggctgctg gttgcttaaa 420
ccactttaaa accaaaaaat tccccttgga aa 452

<210> 108
<211> 502
<212> DNA
<213> Homo sapien

<220>
<221> misc_feature
<222> (1)...(502)
<223> n = A,T,C or G

<400> 108
atcttcttcc cttaattagt tnttatttat ntattaaatt ttattgcatg tcctggcaaa 60
caaaaagaga ttgtagattg gcttctggct ccccaaaagc ccataacaga aagtaccaca 120
agaccncaac tgaagcttaa aaaatctatc acatgtataa tacctttnga agaacattaa 180
tanagcatat aaaactttta acatntgctt aatgttgtnc aattataaaa ntaatngaaa 240
aaaatgtccc tttaacatnc aatatcccac atagtgttat ttnaggggat taccnngnaa 300
naaaaaaagg gtagaaggga tttaatgaaa actctgcttn ccatttctgt ttanaaacgt 360
ctccagaaca aaaacttntc aantctttca gctaaccgca tttgagctna ggccactcaa 420
aaactccatt agncccactt tctaanggtc tctanagctt actaancctt ttgacccctt 480
accctggnta ctcctgccct ca 502

<210> 109
<211> 1308

<212> DNA
<213> Homo sapien

<400> 109
acccgaggtc tcgctaaaat catcatggat tcacttggcg ccgtcagcac tcgacttggg 60
tttgatcttt tcaaagagct gaagaaaaca aatgatggca acatcttctt ttcccctgtg 120
ggcatcttga ctgcaattgg catggtcctc ctggggaccc gaggagccac cgcttcccag 180
ttggaggagg tgtttcactc tgaaaaagag acgaagagct caagaataaa ggctgaagaa 240
aaagaggtga ttgagaacac agaagcagta catcaacaat tccaaaagtt tttgactgaa 300
ataagcaaac tcactaatga ttatgaactg aacataacca acaggctgtt tggagaaaaa 360
acatacctct tccttcaaaa atacttagat tatgttgaaa aatattatca tgcatctctg 420
gaacctgttg attttgtaaa tgcagccgat gaaagtcgaa agaagattaa ttcctgggtt 480
gaaagcaaaa caaatgaaaa aatcaaggac ttgttcccag atggctctat tagtagctct 540
accaagctgg tgctggtgaa catggtttat tttaaagggc aatgggacag ggagtttaag 600
aaagaaaata ctaaggaaga gaaattttgg atgaataaga gcacaagtaa atctgtacag 660
atgatgacac agagccattc ctttagcttc actttcctgg aggacttgca ggccaaaatt 720
ctagggattc catataaaaa caacgaccta agcatgtttg tgcttctgcc caacgacatc 780
gatggcctgg agaagataat agataaaata agtcctgaga aattggtaga gtggactagt 840
ccagggcata tggaagaaag aaaggtgaat ctgcacttgc cccggtttga ggtggaggac 900
agttacgatc tagaggcggt cctggctgcc atggggatgg gcgatgcctt cagtgagcac 960
aaagccgact actcgggaat gtcgtcaggc tccgggttgt acgcccagaa gttcctgcac 1020
agttcctttg tggcagtaac tgaggaaggc accgaggctg cagctgccac tggcataggc 1080
tttactgtca catccgcccc aggtcatgaa aatgttcact gcaatcatcc cttcctgttc 1140
ttcatcaggc acaatgaatc caacagcatc ctcttcttcg gcagattttc ttctccttaa 1200
gatgatcgtt gccatggcat tgctgctttt agcaaaaaac aactaccagt gttactcata 1260
tgattatgaa aatcgtccat tcttttaaat ggtggctcac ttgcattt 1308

<210> 110
<211> 391
<212> PRT
<213> Homo sapien

<400> 110

<210> 111
<211> 1419
<212> DNA
<213> Homo sapien

<400> 111
ggagaactat aaattaagga tcccagctac ttaattgact tatgcttcct agttcgttgc      60
ccagccacca ccgtctctcc aaaaacccga ggtctcgcta aatcatcatg ggattcactt      120
ggcgccgtca gcactcgact tgggtttgat cttttcaaag agctgaagaa aacaaatgat      180
ggcaacatct tcttttcccc tgtgggcatc ttgactgcaa ttggcatggt cctcctgggg      240
acccgaggag ccaccgcttc ccagttggag gaggtgtttc actctgaaaa agagacgaag      300
agctcaagaa taaaggctga agaaaaagag gtggtaagaa taaaggctga aggaaaagag      360
attgagaaca cagaagcagt acatcaacaa ttccaaaagt ttttgactga aataagcaaa      420
ctcactaatg attatgaact gaacataacc aacaggctgt ttggagaaaa aacatacctc      480
ttccttcaaa aatacttaga ttatgttgaa aaatattatc atgcatctct ggaacctgtt      540
gattttgtaa atgcagccga tgaaagtcga aagaagatta attcctgggt tgaaagcaaa      600
acaaatgaaa aaatcaagga cttgttccca gatggctcta ttagtagctc taccaagctg      660
gtgctggtga acatggttta ttttaaaggg caatgggaca gggagtttaa gaaagaaaat      720
actaaggaag agaaattttg gatgaataag agcacaagta aatctgtaca gatgatgaca      780
cagagccatt cctttagctt cactttcctg gaggacttgc aggccaaaat tctagggatt      840
ccatataaaa acaacgacct aagcatgttt gtgcttctgc ccaacgacat cgatggcctg      900
gagaagataa tagataaaat aagtcctgag aaattggtag agtggactag tccagggcat      960
atggaagaaa gaaaggtgaa tctgcacttg ccccggtttg aggtggagga cagttacgat     1020
ctagaggcgg tcctggctgc catggggatg ggcgatgcct tcagtgagca caaagccgac     1080
tactcgggaa tgtcgtcagg ctccgggttg tacgcccaga agttcctgca cagttccttt     1140
gtggcagtaa ctgaggaagg caccgaggct gcagctgcca ctggcatagg ctttactgtc     1200

acatccgccc caggtcatga aaatgttcac tgcaatcatc ccttcctgtt cttcatcagg 1260
cacaatgaat ccaacagcat cctcttcttc ggcagatttt cttctcctta agatgatcgt 1320
tgccatggca ttgctgcttt tagcaaaaaa caactaccag tgttactcat atgattatga 1380
aaatcgtcca ttcttttaaa tggtggctca cttgcattt 1419

<210> 112
<211> 400
<212> PRT
<213> Homo sapien

<400> 112

<210> 113
<211> 957
<212> DNA
<213> Homo sapien

<400> 113
ctcgaccttc tctgcacagc ggatgaaccc tgagcagctg aagaccagaa aagccactat 60
gactttctgc ttaattcagg agcttacagg attcttcaaa gagtgtgtcc agcatccttt 120
gaaacatgag ttcttaccag cagaagcaga cctttacccc accacctcag cttcaacagc 180
agcaggtgaa acaacccagc cagcctccac ctcaggaaat atttgttccc acaaccaagg 240
agccatgcca ctcaaaggtt ccacaacctg gaaacacaaa gattccagag ccaggctgta 300
ccaaggtccc tgagccaggc tgtaccaagg tccctgagcc aggttgtacc aaggtccctg 360
agccaggatg taccaaggtc cctgagccag gttgtaccaa ggtccctgag ccaggctaca 420
ccaaggtccc tgagccaggc agcatcaagg tccctgacca aggcttcatc aagtttcctg 480
agccaggtgc catcaaagtt cctgagcaag gatacaccaa agttcctgtg ccaggctaca 540
caaaggtacc agagccatgt ccttcaacgg tcactccagg cccagctcag cagaagacca 600
agcagaagta atttggtgca cagacaagcc cttgagaagc caaccaccag atgctggaca 660
ccctcttccc atctgtttct gtgtcttaat tgtctgtaga ccttgtaatc agtacattct 720
caccccaagc catagtctct ctcttatttg tatcctaaaa atacggtact ataaagcttt 780
tgttcacaca cactctgaag aatcctgtaa gcccctgaat taagcagaaa gtcttcatgg 840
cttttctggt cttcggctgc tcagggttca tctgaagatt cgaatgaaaa gaaatgcatg 900
tttcctgctc tgccctcatt aaattgcttt taattccaaa aaaaaaaaaa aaaaaaa 957

<210> 114
<211> 161
<212> PRT
<213> Homo sapien

<400> 114
Met Ser Ser Tyr Gln Gln Lys Gln Thr Phe Thr Pro Pro Pro Gln Leu
1               5                   10                  15
Gln Gln Gln Gln Val Lys Gln Pro Ser Gln Pro Pro Pro Gln Glu Ile
            20                  25                  30
Phe Val Pro Thr Thr Lys Glu Pro Cys His Ser Lys Val Pro Gln Pro
        35                  40                  45
Gly Asn Thr Lys Ile Pro Glu Pro Gly Cys Thr Lys Val Pro Glu Pro
    50                  55                  60
Gly Cys Thr Lys Val Pro Glu Pro Gly Cys Thr Lys Val Pro Glu Pro
65                  70                  75                  80
Gly Cys Thr Lys Val Pro Glu Pro Gly Cys Thr Lys Val Pro Glu Pro
                85                  90                  95
Gly Tyr Thr Lys Val Pro Glu Pro Gly Ser Ile Lys Val Pro Asp Gln
            100                 105                 110
Gly Phe Ile Lys Phe Pro Glu Pro Gly Ala Ile Lys Val Pro Glu Gln
        115                 120                 125
Gly Tyr Thr Lys Val Pro Val Pro Gly Tyr Thr Lys Val Pro Glu Pro
    130                 135                 140
Cys Pro Ser Thr Val Thr Pro Gly Pro Ala Gln Gln Lys Thr Lys Gln

145                 150                 155                 160
Lys

<210> 115
<211> 506
<212> DNA
<213> Homo sapien

<220>
<221> misc_feature
<222> (1)...(506)
<223> n = A,T,C or G

<400> 115
cattggtnct ttcatttgct ntggaagtgt nnatctctaa cagtggacaa agttcccngt      60
gccttaaact ctgtnacact tttgggaant gaaaanttng tantatgata ggttattctg     120
angtanagat gttctggata ccattanatn tgcccccngt gtcagaggct catattgtgt     180
tatgtaaatg gtatntcatt cgctactatn antcaattng aaatanggtc tttgggttat     240
gaatantnng cagcncanct nanangctgt ctgtngtatt cattgtggtc atagcacctc     300
acancattgt aacctcnatc nagtgagaca nactagnaan ttcctagtga tggctcanga     360
ttccaaatgg nctcatntcn aatgtttaaa agttanttaa gtgtaagaaa tacagactgg     420
atgttccacc aactagtacc tgtaatgacn ggcctgtccc aacacatctc ccttttccat     480
gactgtggta ncccgcatcg gaaaaa                                          506

<210> 116
<211> 3079
<212> DNA
<213> Homo sapien

<400> 116
ggatccccgg gtttcctaaa ccccccacag agtcctgccc aggccaaaga gcaaggaaaa      60
ggtcaaaggg cagaaaaaat gctgagttag gaggagctat ggaaggataa acctggcctt     120
aaagaggtca aagtggttta tagggggcgc tgagggcttc ccacattctc tggcctaaac     180
cttgcaggca gatctgccca gtgggctctg ggatagctgt gccttcccta acaaaaaaat     240
tgtgcacaaa aggatgaaac tctattttcc ctctagcaca taaccaagaa tataaggcta     300
cagattgcct ttcccagagg gaaaaccctg cagcaacctg ctgcctggaa aagtgtaaga     360
gcagatcact ggggaatcgt ttgccccccg ctgatggaca gcttccccaa gctccaaggg     420
caggtgctca gcatgtaccg tactgggatg gttgtcaata ctcctggtcc tgtaagagtc     480
ccaggacact gccatgccaa tgccccctca gttcctggca tcctttttgg gctgctcaca     540
gccccagcct ctatggtgaa gacatacttg ctagcagcgt caccaacttg ttgccaagag     600
atcagtgctc gaaggcaagg ttatttctaa ctgagcagag cctgccagga agaaagcgtt     660
tgcaccccac accactgtgc aggtgtgacc ggtgagctca cagctgcccc ccaggcatgc     720
ccagcccact taatcatcac agctcgacag ctctctcgcc cagcccagtt ctggaaggga     780
taaaaagggg catcaccgtt cctgggtaac agagccacct tctgcgtcct gctgagctct     840
gttctctcca gcacctccca acccactagt gcctggttct cttgctccac caggaacaag     900
ccaccatgtc tcgccagtca agtgtgtctt ccggagcggg gggcagtcgt agcttcagca     960
ccgcctctgc catcaccccg tctgtctccc gcaccagctt cacctccgtg tcccggtccg    1020
ggggtggcgg tggtggtggc ttcggcaggg tcagccttgc gggtgcttgt ggagtgggtg    1080
gctatggcag ccggagcctc tacaacctgg ggggctccaa gaggatatcc atcagcacta    1140
gtggtggcag cttcaggaac cggtttggtg ctggtgctgg aggcggctat ggctttggag    1200
gtggtgccgg tagtggattt ggtttcggcg gtggagctgg tggtggcttt gggctcggtg    1260
gcggagctgg ctttggaggt ggcttcggtg gccctggctt tcctgtctgc cctcctggag    1320
gtatccaaga ggtcactgtc aaccagagtc tcctgactcc cctcaacctg caaatcgacc    1380
ccagcatcca gagggtgagg accgaggagc gcgagcagat caagaccctc aacaataagt    1440

ttgcctcctt catcgacaag gtgcggttcc tggagcagca gaacaaggtt ctggaaacaa 1500
agtggaccct gctgcaggag cagggcacca agactgtgag gcagaacctg gagccgttgt 1560
tcgagcagta catcaacaac ctcaggaggc agctggacag catcgtgggg gaacggggcc 1620
gcctggactc agagctgaga aacatgcagg acctggtgga agacttcaag aacaagtatg 1680
aggatgaaat caacaagcgt accactgctg agaatgagtt tgtgatgctg aagaaggatg 1740
tagatgctgc ctacatgaac aaggtggagc tggaggccaa ggttgatgca ctgatggatg 1800
agattaactt catgaagatg ttctttgatg cggagctgtc ccagatgcag acgcatgtct 1860
ctgacacctc agtggtcctc tccatggaca acaaccgcaa cctggacctg gatagcatca 1920
tcgctgaggt caaggcccag tatgaggaga ttgccaaccg cagccggaca gaagccgagt 1980
cctggtatca gaccaagtat gaggagctgc agcagacagc tggccggcat ggcgatgacc 2040
tccgcaacac caagcatgag atctctgaga tgaaccggat gatccagagg ctgagagccg 2100
agattgacaa tgtcaagaaa cagtgcgcca atctgcagaa cgccattgcg gatgccgagc 2160
agcgtgggga gctggccctc aaggatgcca ggaacaagct ggccgagctg gaggaggccc 2220
tgcagaaggc caagcaggac atggcccggc tgctgcgtga gtaccaggag ctcatgaaca 2280
ccaagctggc cctggacgtg gagatcgcca cttaccgcaa gctgctggag ggcgaggaat 2340
gcagactcag tggagaagga gttggaccag tcaacatctc tgttgtcaca agcagtgttt 2400
cctctggata tggcagtggc agtggctatg gcggtggcct cggtggaggt cttggcggcg 2460
gcctcggtgg aggtcttgcc ggaggtagca gtggaagcta ctactccagc agcagtgggg 2520
gtgtcggcct aggtggtggg ctcagtgtgg ggggctctgg cttcagtgca agcagtagcc 2580
gagggctggg ggtgggcttt ggcagtggcg ggggtagcag ctccagcgtc aaatttgtct 2640
ccaccacctc ctcctcccgg aagagcttca agagctaaga acctgctgca agtcactgcc 2700
ttccaagtgc agcaacccag cccatggaga ttgcctcttc taggcagttg ctcaagccat 2760
gttttatcct tttctggaga gtagtctaga ccaagccaat tgcagaacca cattctttgg 2820
ttcccaggag agccccattc ccagcccctg gtctcccgtg ccgcagttct atattctgct 2880
tcaaatcagc cttcaggttt cccacagcat ggcccctgct gacacgagaa cccaaagttt 2940
tcccaaatct aaatcatcaa aacagaatcc ccaccccaat cccaaatttt gttttggttc 3000
taactacctc cagaatgtgt tcaataaaat gttttataat ataagctggt gtgcagaatt 3060
gttttttttt tctacccaa 3079

<210> 117
<211> 6921
<212> DNA
<213> Homo sapien

<400> 117
   gaattctgac tgtccactca aaacttctat tccgatcaaa gctatctgtg actacagaca 60
   aattgagata accatttaca aagacgatga atgtgttttg gcgaataact ctcatcgtgc 120
   taaatggaag gtcattagtc ctactgggaa tgaggctatg gtcccatctg tgtgcttcac 180
   cgttcctcca ccaaacaaag aagcggtgga ccttgccaac agaattgagc aacagtatca 240
   gaatgtcctg actctttggc atgagtctca cataaacatg aagagtgtag tatcctggca 300
   ttatctcatc aatgaaattg atagaattcg agctagcaat gtggcttcaa taaagacaat 360
   gctacctggt gaacatcagc aagttctaag taatctacaa tctcgttttg aagattttct 420
   ggaagatagc caggaatccc aagtcttttc aggctcagat ataacacaac tggaaaagga 480
   ggttaatgta tgtaagcagt attatcaaga acttcttaaa tctgcagaaa gagaggagca 540
   agaggaatca gtttataatc tctacatctc tgaagttcga aacattagac ttcggttaga 600
   gaactgtgaa gatcggctga ttagacagat tcgaactccc ctggaaagag atgatttgca 660
   tgaaagtgtg ttcagaatca cagaacagga gaaactaaag aaagagctgg aacgacttaa 720
   agatgatttg ggaacaatca caaataagtg tgaggagttt ttcagtcaag cagcagcctc 780
   ttcatcagtc cctaccctac gatcagagct taatgtggtc cttcagaaca tgaaccaagt 840
   ctattctatg tcttccactt acatagataa gttgaaaact gttaacttgg tgttaaaaaa 900
   cactcaagct gcagaagccc tcgtaaaact ctatgaaact aaactgtgtg aagaagaagc 960
   agttatagct gacaagaata atattgagaa tctaataagt actttaaagc aatggagatc 1020
   tgaagtagat gaaaagagac aggtattcca tgccttagag gatgagttgc agaaagctaa 1080
   agccatcagt gatgaaatgt ttaaaacgta taaagaacgg gaccttgatt ttgactggca 1140
   caaagaaaaa gcagatcaat tagttgaaag gtggcaaaat gttcatgtgc agattgacaa 1200
   cagguuacgg gacuuagagg gcauuggcaa aucacugaag uacuacagag acacuuacca 1260
   uccuuuagau gauuggaucc agcagguuga aacuacucag agaaagauuc aggaaaauca 1320
   gccugaaaau aguaaaaccc uagccacaca guugaaucaa cagaagaugc ugguguccga 1380
   aauagaaaug aaacagagca aauggacgga gugucaaaaa uaugcagaac aguacucagc 1440
   uacagugaag gacuaugaau uacaaacaau gaccuaccgg gccaugguag auucacaaca 1500
   aaaaucucca gugaaacgcc gaagaaugca gaguucagca gaucucauua uucaagaguu 1560
   cauggaccua aggacucgau auacugcccu ggucacucuc augacacaau auauuaaauu 1620
   ugcuggugau ucauugaaga ggcuggaaga ggaggagauu aaaaggugua aggagacuuc 1680
   ugaacauggg gcauauucag aucugcuuca gcgucagaag gcaacagugc uugagaauag 1740
   caaacuuaca ggaaagauaa gugaguugga aagaauggua gcugaacuaa agaaacaaaa 1800
   gucccgagua gaggaagaac uuccgaaggu cagggaggcu gcagaaaaug aauugagaaa 1860
   gcagcagaga aauguagaag auaucucucu gcagaagaua agggcugaaa gugaagccaa 1920
   gcaguaccgc agggaacuug aaaccauugu gagagagaag gaagccgcug aaagagaacu 1980
   ggagcgggug aggcagcuca ccauagaggc cgaggcuaaa agagcugccg uggaagagaa 2040
   ccuccugaau uuucgcaauc aguuggagga aaacaccuuu accagacgaa cacuggaaga 2100
   ucaucuuaaa agaaaagauu uaagucucaa ugauuuggag caacaaaaaa auaaauuaau 2160
   ggaagaauua agaagaaaga gagacaauga ggaagaacuc uugaagcuga uaaagcagau 2220
   ggaaaaagac cuugcauuuc agaaacaggu agcagagaaa caguugaaag aaaagcagaa 2280
   aauugaauug gaagcaagaa gaaaaauaac ugaaauucag uauacaugua gagaaaaugc 2340
   auugccagug uguccgauca cacaggcuac aucaugcagg gcaguaacgg gucuccagca 2400
   agaacaugac aagcagaaag cagaagaacu caaacagcag guagaugaac uaacagcugc 2460
   caauagaaag gcugaacaag acaugagaga gcugacauau gaacuuaaug cccuccagcu 2520
   ugaaaaaacg ucaucugagg aaaaggcucg uuugcuaaaa gauaaacuag augaaacaaa 2580
   uaauacacuc agaugccuua aguuggagcu ggaaaggaag gaucaggcgg agaaagggua 2640
   uucucaacaa cucagagagc uugguaggca auugaaucaa accacaggua aagcugaaga 2700
   agccaugcaa gaagcuagug aucucaagaa aauaaagcgc aauuaucagu uagaauuaga 2760
   aucucuuaau caugaaaaag ggaaacuaca aagagaagua gacagaauca caagggcaca 2820
   ugcuguagcu gagaagaaua uucagcauuu aaauucacaa auucauucuu uucgagauga 2880
   gaaagaauua gaaagacuac aaaucugcca gagaaaauca gaucaucuaa aagaacaauu 2940
   ugagaaaagc caugagcagu ugcuucaaaa uaucaaagcu gaaaaagaaa auaaugauaa 3000
   aauccaaagg cucaaugaag aauuggagaa aaguaaugag ugugcagaga ugcuaaaaca 3060
   aaaaguagag gagcuuacua ggcagaauaa ugaaaccaaa uuaaugaugc agagaauuca 3120
   ggcagaauca gagaauauag uuuuagagaa acaaacuauc cagcaaagau gugaagcacu 3180
   gaaaauucag gcagaugguu uuaaagauca gcuacgcagc acaaaugaac acuugcauaa 3240
   acagacaaaa acagagcagg auuuucaaag aaaaauuaaa ugccuagaag aagaccuggc 3300
   gaaaagucaa aauuugguaa gugaauuuaa gcaaaagugu gaccaacaga acauuaucau 3360
   ccagaauacc aagaaagaag uuagaaaucu gaaugcggaa cugaaugcuu ccaaagaaga 3420
   gaagcgacgc ggggagcaga aaguucagcu acaacaagcu caggugcaag aguuaaauaa 3480
   cagguugaaa aaaguacaag acgaauuaca cuuaaagacc auagaggagc agaugaccca 3540
   cagaaagaug guucuguuuc aggaagaauc ugguaaauuc aaacaaucag cagaggaguu 3600
   ucggaagaag auggaaaaau uaauggaguc caaagucauc acugaaaaug auauuucagg 3660
   cauuaggcuu gacuuugugu cucuucaaca agaaaacucu agagcccaag aaaaugcuaa 3720
   gcuuugugaa acaaacauua aagaacuuga aagacagcuu caacaguauc gugaacaaau 3780
   gcagcaaggg cagcacaugg aagcaaauca uuaccaaaaa ugucagaaac uugaggauga 3840
   gcugauagcc cagaagcgug agguugaaaa ccugaagcaa aaaauggacc aacagaucaa 3900
   agagcaugaa caucaauuag uuuugcucca gugugaaauu caaaaaaaga gcacagccaa 3960
   agacuguacc uucaaaccag auuuugagau gacagugaag gagugccagc acucuggaga 4020
   gcuguccucu agaaacacug gacaccuuca cccaacaccc agauccccuc uguugagaug 4080
   gacucaagaa ccacagccau uggaagagaa guggcagcau cggguuguug aacagauacc 4140
   caaagaaguc caauuccagc caccaggggc uccacucgag aaagagaaaa gccagcagug 4200
   uuacucugag uacuuuucuc agacaagcac cgaguuacag auaacuuuug augagacaaa 4260
   ccccauuaca agacugucug aaauugagaa gauaagagac caagcccuga acaauucuag 4320
   accaccuguu agguaucaag auaacgcaug ugaaauggaa cuggugaagg uuuugacacc 4380
   cuuagagaua gcuaagaaca agcaguauga uaugcauaca gaagucacaa cauuaaaaca 4440
   agaaaagaac ccaguuccca gugcugaaga auggaugcuu gaagggugca gagcaucugg 4500
   tggactcaag aaaggggatt tccttaagaa gggcttagaa ccagagacct tccagaactt 4560
   tgatggtgat catgcatgtt cagtcaggga tgatgaattt aaattccaag ggcttaggca 4620
   cactgtgact gccaggcagt tggtggaagc taagcttctg gacatgagaa caattgagca 4680
   gctgcgactc ggtcttaaga ctgttgaaga agttcagaaa actcttaaca agtttctgac 4740
   gaaagccacc tcaattgcag ggctttacct agaatctaca aaagaaaaga tttcatttgc 4800
   ctcagcggcc gagagaatca taatagacaa aatggtggct ttggcatttt tagaagctca 4860
   ggctgcaaca ggttttataa ttgatcccat ttcaggtcag acatattctg ttgaagatgc 4920
   agttcttaaa ggagttgttg accccgaatt cagaattagg cttcttgagg cagagaaggc 4980
   agctgtggga tattcttatt cttctaagac attgtcagtg tttcaagcta tggaaaatag 5040
   aatgcttgac agacaaaaag gtaaacatat cttggaagcc cagattgcca gtgggggtgt 5100
   cattgaccct gtgagaggca ttcgtgttcc tccagaaatt gctctgcagc aggggttgtt 5160
   gaataatgcc atcttacagt ttttacatga gccatccagc aacacaagag ttttccctaa 5220
   tcccaataac aagcaagctc tgtattactc agaattactg cgaatgtgtg tatttgatgt 5280
   agagtcccaa tgctttctgt ttccatttgg ggagaggaac atttccaatc tcaatgtcaa 5340
   gaaaacacat agaatttctg tagtagatac taaaacagga tcagaattga ccgtgtatga 5400
   ggctttccag agaaacctga ttgagaaaag tatatatctt gaactttcag ggcagcaata 5460
   tcagtggaag gaagctatgt tttttgaatc ctatgggcat tcttctcata tgctgactga 5520
   tactaaaaca ggattacact tcaatattaa tgaggctata gagcagggaa caattgacaa 5580
   agccttggtc aaaaagtatc aggaaggcct catcacactt acagaacttg ctgattcttt 5640
   gctgagccgg ttagtcccca agaaagattt gcacagtcct gttgcagggt attggctgac 5700
   tgctagtggg gaaaggatct ctgtactaaa agcctcccgt agaaatttgg ttgatcggat 5760
   tactgccctc cgatgccttg aagcccaagt cagtacaggg ggcataattg atcctcttac 5820
   tggcaaaaag taccgggtgg ccgaagcttt gcatagaggc ctggttgatg aggggtttgc 5880
   ccagcagctg cgacagtgtg aattagtaat cacagggatt ggccatccca tcactaacaa 5940
   aatgatgtca gtggtggaag ctgtgaatgc aaatattata aataaggaaa tgggaatccg 6000
   atgtttggaa tttcagtact tgacaggagg gttgatagag ccacaggttc actctcggtt 6060
   atcaatagaa gaggctctcc aagtaggtat tatagatgtc ctcattgcca caaaactcaa 6120
   agatcaaaag tcatatgtca gaaatataat atgccctcag acaaaaagaa agttgacata 6180
   taaagaagcc ttagaaaaag ctgattttga tttccacaca ggacttaaac tgttagaagt 6240
   atctgagccc ctgatgacag gaatttctag cctctactat tcttcctaat gggacatgtt 6300
   taaataactg tgcaaggggt gatgcaggct ggttcatgcc actttttcag agtatgatga 6360
   tatcggctac atatgcagtc tgtgaattat gtaacatact ctatttcttg agggctgcaa 6420
   attgctaagt gctcaaaata gagtaagttt taaattgaaa attacataag atttaatgcc 6480
   cttcaaatgg tttcatttag ccttgagaat ggtttttgga aacttggcca cactaaaatg 6540
   tttttttttt tttacgtaga atgtgggata aacttgatga actccaagtt cacagtgtca 6600
   tttcttcaga actccccttc attgaatagt gatcatttat taaatgataa attgcactcg 6660
   ctgaaagagc acgtcatgaa gcaccatgga atcaaagaga aagatataaa ttcgttccca 6720
   cagccttcaa gctgcagtgt tttagattgc ttcaaaaaat gaaaaagttt tgcctttttc 6780
   gatatagtga ccttctttgc atattaaaat gtttaccaca atgtcccatt tctagttaag 6840
   tcttcgcact tgaaagctaa cattatgaat attatgtgtt ggaggagggg aaggattttc 6900
   ttcattctgt gtattttccg g 6921

<210> 118
<211> 946
<212> DNA
<213> Homo sapien

<400> 118
cttctgactg ggctcaggct gacaggtaga gctcaccatg gcttcttgtg tccttgtccc 60
ctccccatca cagctgtggt gcagtccacc gtctccagtg gctatggcgg tgccagtggt 120
gtcggcagtg gcttaggcct gggtggagga agcagctact cctatggcag tggtcttggc 180
gttggaggtg gcttcagttc cagcagtggc agagccattg ggggtggcct cagctctgtt 240
ggaggcggca gttccaccat caagtacacc accacctcct cctccagcag gaagagctat 300
aagcactaaa gtgcgtctgc tagctctcgg tcccacagtc ctcaggcccc tctctggctg 360
cagagccctc tcctcaggtt gcctgtcctc tcctggcctc cagtctcccc tgctgtccca 420

ggtagagctg gggatgaatg cttagtgccc tcacttcttc tctctctctc tataccatct 480
gagcacccat tgctcaccat cagatcaacc tctgatttta catcatgatg taatcaccac 540
tggagcttca ctgttactaa attattaatt tcttgcctcc agtgttctat ctctgaggct 600
gagcattata agaaaatgac ctctgctcct tttcattgca gaaaattgcc aggggcttat 660
ttcagaacaa cttccactta ctttccactg gctctcaaac tctctaactt ataagtgttg 720
tgaaccccca cccaggcagt atccatgaaa gcacaagtga ctagtcctat gatgtacaaa 780
gcctgtatct ctgtgatgat ttctgtgctc ttcactgttt gcaattgcta aataaagcag 840
atttataata catatattct tttactttgc cttgctttgg ggccaaagtt ttgggcttaa 900
acttttttat ctgataagtg aatagttgtt tttaaaagat aatcta 946

<210> 119
<211> 8948
<212> DNA
<213> Homo sapien

<400> 119
tcaacagccc ctgctccttg ggcccctcca tgccatgccg taatctctcc cacccgacca 60
acaccaacac ccagctccga cgcagctcct ctgcgccctt gccgccctcc gagccacagc 120
tttcctcccg ctcctgcccc cggcccgtcg ccgtctccgc gctcgcagcg gcctcgggag 180
ggcccaggta gcgagcagcg acctcgcgag ccttccgcac tcccgcccgg ttccccggcc 240
gtccgcctat ccttggcccc ctccgctttc tccgcgccgg cccgcctcgc ttatgcctcg 300
gcgctgagcc gctctcccga ttgcccgccg acatgagctg caacggaggc tcccacccgc 360
ggatcaacac tctgggccgc atgatccgcg ccgagtctgg cccggacctg cgctacgagg 420
tgaccagcgg cggcgggggc accagcagga tgtactattc tcggcgcggc gtgatcaccg 480
accagaactc ggacggctac tgtcaaaccg gcacgatgtc caggcaccag aaccagaaca 540
ccatccagga gctgctgcag aactgctccg actgcttgat gcgagcagag ctcatcgtgc 600
agcctgaatt gaagtatgga gatggaatac aactgactcg gagtcgagaa ttggatgagt 660
gttttgccca ggccaatgac caaatggaaa tcctcgacag cttgatcaga gagatgcggc 720
agatgggcca gccctgtgat gcttaccaga aaaggcttct tcagctccaa gagcaaatgc 780
gagcccttta taaagccatc agtgtccctc gagtccgcag ggccagctcc aagggtggtg 840
gaggctacac ttgtcagagt ggctctggct gggatgagtt caccaaacat gtcaccagtg 900
aatgtttggg gtggatgagg cagcaaaggg cggagatgga catggtggcc tggggtgtgg 960
acctggcctc agtggagcag cacattaaca gccaccgggg catccacaac tccatcggcg 1020
actatcgctg gcagctggac aaaatcaaag ccgacctgcg cgagaaatct gcgatctacc 1080
agttggagga ggagtatgaa aacctgctga aagcgtcctt tgagaggatg gatcacctgc 1140
gacagctgca gaacatcatt caggccacgt ccagggagat catgtggatc aatgactgcg 1200
aggaggagga gctgctgtac gactggagcg acaagaacac caacatcgct cagaaacagg 1260
aggccttctc catacgcatg agtcaactgg aagttaaaga aaaagagctc aataagctga 1320
aacaagaaag tgaccaactt gtcctcaatc agcatccagc ttcagacaaa attgaggcct 1380
atatggacac tctgcagacg cagtggagtt ggattcttca gatcaccaag tgcattgatg 1440
ttcatctgaa agaaaatgct gcctactttc agttttttga agaggcgcag tctactgaag 1500
catacctgaa ggggctccag gactccatca ggaagaagta cccctgcgac aagaacatgc 1560
ccctgcagca cctgctggaa cagatcaagg agctggagaa agaacgagag aaaatccttg 1620
aatacaagcg tcaggtgcag aacttggtaa acaagtctaa gaagattgta cagctgaagc 1680
ctcgtaaccc agactacaga agcaataaac ccattattct cagagctctc tgtgactaca 1740
aacaagatca gaaaatcgtg cataaggggg atgagtgtat cctgaaggac aacaacgagc 1800
gcagcaagtg gtacgtgacg ggcccgggag gcgttgacat gcttgttccc tctgtggggc 1860
tgatcatccc tcctccgaac ccactggccg tggacctctc ttgcaagatt gagcagtact 1920
acgaagccat cttggctctg tggaaccagc tctacatcaa catgaagagc ctggtgtcct 1980
ggcactactg catgattgac atagagaaga tcagggccat gacaatcgcc aagctgaaaa 2040
caatgcggca ggaagattac atgaagacga tagccgacct tgagttacat taccaagagt 2100
tcatcagaaa tagccaaggc tcagagatgt ttggagatga tgacaagcgg aaaatacagt 2160
ctcagttcac cgatgcccag aagcattacc agaccctggt cattcagctc cctggctatc 2220
cccagcacca gacagtgacc acaactgaaa tcactcatca tggaacctgc caagatgtca 2280
accataataa agtaattgaa accaacagag aaaatgacaa gcaagaaaca tggatgctga 2340

tggagctgca gaagattcgc aggcagatag agcactgcga gggcaggatg actctcaaaa 2400
acctccctct agcagaccag gggtcttctc accacatcac agtgaaaatt aacgagctta 2460
agagtgtgca gaatgattca caagcaattg ctgaggttct caaccagctt aaagatatgc 2520
ttgccaactt cagaggttct gaaaagtact gctatttaca gaatgaagta tttggactat 2580
ttcagaaact ggaaaatatc aatggtgtta cagatggcta cttaaatagc ttatgcacag 2640
taagggcact gctccaggct attctccaaa cagaagacat gttaaaggtt tatgaagcca 2700
ggctcactga ggaggaaact gtctgcctgg acctggataa agtggaagct taccgctgtg 2760
gactgaagaa aataaaaaat gacttgaact tgaagaagtc gttgttggcc actatgaaga 2820
cagaactaca gaaagcccag cagatccact ctcagacttc acagcagtat ccactttatg 2880
atctggactt gggcaagttc ggtgaaaaag tcacacagct gacagaccgc tggcaaagga 2940
tagataaaca gatcgacttt agattatggg acctggagaa acaaatcaag caattgagga 3000
attatcgtga taactatcag gctttctgca agtggctcta tgatcgtaaa cgccgccagg 3060
attccttaga atccatgaaa tttggagatt ccaacacagt catgcggttt ttgaatgagc 3120
agaagaactt gcacagtgaa atatctggca aacgagacaa atcagaggaa gtacaaaaaa 3180
ttgctgaact ttgcgccaat tcaattaagg attatgagct ccagctggcc tcatacacct 3240
caggactgga aactctgctg aacataccta tcaagaggac catgattcag tccccttctg 3300
gggtgattct gcaagaggct gcagatgttc atgctcggta cattgaacta cttacaagat 3360
ctggagacta ttacaggttc ttaagtgaga tgctgaagag tttggaagat ctgaagctga 3420
aaaataccaa gatcgaagtt ttggaagagg agctcagact ggcccgagat gccaactcgg 3480
aaaactgtaa taagaacaaa ttcctggatc agaacctgca gaaataccag gcagagtgtt 3540
cccagttcaa agcgaagctt gcgagcctgg aggagctgaa gagacaggct gagctggatg 3600
ggaagtcggc taagcaaaat ctagacaagt gctacggcca aataaaagaa ctcaatgaga 3660
agatcacccg actgacttat gagattgaag atgaaaagag aagaagaaaa tctgtggaag 3720
acagatttga ccaacagaag aatgactatg accaactgca gaaagcaagg caatgtgaaa 3780
aggagaacct tggttggcag aaattagagt ctgagaaagc catcaaggag aaggagtacg 3840
agattgaaag gttgagggtt ctactgcagg aagaaggcac ccggaagaga gaatatgaaa 3900
atgagctggc aaaggtaaga aaccactata atgaggagat gagtaattta aggaacaagt 3960
atgaaacaga gattaacatt acgaagacca ccatcaagga gatatccatg caaaaagagg 4020
atgattccaa aaatcttaga aaccagcttg atagactttc aagggaaaat cgagatctga 4080
aggatgaaat tgtcaggctc aatgacagca tcttgcaggc cactgagcag cgaaggcgag 4140
ctgaagaaaa cgcccttcag caaaaggcct gtggctctga gataatgcag aagaagcagc 4200
atctggagat agaactgaag caggtcatgc agcagcgctc tgaggacaat gcccggcaca 4260
agcagtccct ggaggaggct gccaagacca ttcaggacaa aaataaggag atcgagagac 4320
tcaaagctga gtttcaggag gaggccaagc gccgctggga atatgaaaat gaactgagta 4380
aggtaagaaa caattatgat gaggagatca ttagcttaaa aaatcagttt gagaccgaga 4440
tcaacatcac caagaccacc atccaccagc tcaccatgca gaaggaagag gataccagtg 4500
gctaccgggc tcagatagac aatctcaccc gagaaaacag gagcttatct gaagaaataa 4560
agaggctgaa gaacactcta acccagacca cagagaatct caggagggtg gaagaagaca 4620
tccaacagca aaaggccact ggctctgagg tgtctcagag gaaacagcag ctggaggttg 4680
agctgagaca agtcactcag atgcgaacag aggagagcgt aagatataag caatctcttg 4740
atgatgctgc caaaaccatc caggataaaa acaaggagat agaaaggtta aaacaactga 4800
tcgacaaaga aacaaatgac cggaaatgcc tggaagatga aaacgcgaga ttacaaaggg 4860
tccagtatga cctgcagaaa gcaaacagta gtgcgacgga gacaataaac aaactgaagg 4920
ttcaggagca agaactgaca cgcctgagga tcgactatga aagggtttcc caggagagga 4980
ctgtgaagga ccaggatatc acgcggttcc agaactctct gaaagagctg cagctgcaga 5040
agcagaaggt ggaagaggag ctgaatcggc tgaagaggac cgcgtcagaa gactcctgca 5100
agaggaagaa gctggaggaa gagctggaag gcatgaggag gtcgctgaag gagcaagcca 5160
tcaaaatcac caacctgacc cagcagctgg agcaggcatc cattgttaag aagaggagtg 5220
aggatgacct ccggcagcag agggacgtgc tggatggcca cctgagggaa aagcagagga 5280
cccaggaaga gctgaggagg ctctcttctg aggtcgaggc cctgaggcgg cagttactcc 5340
aggaacagga aagtgtcaaa caagctcact tgaggaatga gcatttccag aaggcgatag 5400
aagataaaag cagaagctta aatgaaagca aaatagaaat tgagaggctg cagtctctca 5460
cagagaacct gaccaaggag cacttgatgt tagaagaaga actgcggaac ctgaggctgg 5520
agtacgatga cctgaggaga ggacgaagcg aagcggacag tgataaaaat gcaaccatct 5580
tggaactaag gagccagctg cagatcagca acaaccggac cctggaactg caggggctga 5640

ttaatgattt acagagagag agggaaaatt tgagacagga aattgagaaa ttccaaaagc 5700
aggctttaga ggcatctaat aggattcagg aatcaaagaa tcagtgtact caggtggtac 5760
aggaaagaga gagccttctg gtgaaaatca aagtcctgga gcaagacaag gcaaggctgc 5820
agaggctgga ggatgagctg aatcgtgcaa aatcaactct agaggcagaa accaggtgaa 5880
aacagcgcct ggagtgtgag aaacagcaaa ttcagaatga cctgaatcag tggaagactc 5940
aatattcccg caaggaggag gctattagga agatagaatc ggaaagagaa aagagtgaga 6000
gagagaagaa cagtcttagg agtgagatcg aaagactcca agcagagatc aagagaattg 6060
aagagaggtg caggcgtaag ctggaggatt ctaccaggga gacacagtca cagttagaaa 6120
cagaacgctc ccgatatcag agggagattg ataaactcag acagcgccca tatgggtccc 6180
atcgagagac ccagactgag tgtgagtgga ccgttgacac ctccaagctg gtgtttgatg 6240
ggctgaggaa gaaggtgaca gcaatgcagc tctatgagtg tcagctgatc gacaaaacaa 6300
ccttggacaa actattgaag gggaagaagt cagtggaaga agttgcttct gaaatccagc 6360
cattccttcg gggtgcagga tctatcgctg gagcatctgc ttctcctaag gaaaaatact 6420
ctttggtaga ggccaagaga aagaaattaa tcagcccaga atccacagtc atgcttctgg 6480
aggcccaggc agctacaggt ggtataattg atccccatcg gaatgagaag ctgactgtcg 6540
acagtgccat agctcgggac ctcattgact tcgatgaccg tcagcagata tatgcagcag 6600
aaaaagctat cactggtttt gatgatccat tttcaggcaa gacagtatct gtttcagaag 6660
ccatcaagaa aaatttgatt gatagagaaa ccggaatgcg cctgctggaa gcccagattg 6720
cttcaggggg tgtagtagac cctgtgaaca gtgtcttttt gccaaaagat gtcgccttgg 6780
cccgggggct gattgataga gatttgtatc gatccctgaa tgatccccga gatagtcaga 6840
aaaactttgt ggatccagtc accaaaaaga aggtcagtta cgtgcagctg aaggaacggt 6900
gcagaatcga accacatact ggtctgctct tgctttcagt acagaagaga agcatgtcct 6960
tccaaggaat cagacaacct gtgaccgtca ctgagctagt agattctggt atattgagac 7020
cgtccactgt caatgaactg gaatctggtc agatttctta tgacgaggtt ggtgagagaa 7080
ttaaggactt cctccagggt tcaagctgca tagcaggcat atacaatgag accacaaaac 7140
agaagcttgg catttatgag gccatgaaaa ttggcttagt ccgacctggt actgctctgg 7200
agttgctgga agcccaagca gctactggct ttatagtgga tcctgttagc aacttgaggt 7260
taccagtgga ggaagcctac aagagaggtc tggtgggcat tgagttcaaa gagaagctcc 7320
tgtctgcaga acgagctgtc actgggtata atgatcctga aacaggaaac atcatctctt 7380
tgttccaagc catgaataag gaactcatcg aaaagggcca cggtattcgc ttattagaag 7440
cacagatcgc aaccgggggg atcattgacc caaaggagag ccatcgttta ccagttgaca 7500
tagcatataa gaggggctat ttcaatgagg aactcagtga gattctctca gatccaagtg 7560
atgataccaa aggatttttt gaccccaaca ctgaagaaaa tcttacctat ctgcaactaa 7620
aagaaagatg cattaaggat gaggaaacag ggctctgtct tctgcctctg aaagaaaaga 7680
agaaacaggt gcagacatca caaaagaata ccctcaggaa gcgtagagtg gtcatagttg 7740
acccagaaac caataaagaa atgtctgttc aggaggccta caagaagggc ctaattgatt 7800
atgaaacctt caaagaactg tgtgagcagg aatgtgaatg ggaagaaata accatcacgg 7860
gatcagatgg ctccaccagg gtggtcctgg tagatagaaa gacaggcagt cagtatgata 7920
ttcaagatgc tattgacaag ggccttgttg acaggaagtt ctttgatcag taccgatccg 7980
gcagcctcag cctcactcaa tttgctgaca tgatctcctt gaaaaatggt gtcggcacca 8040
gcagcagcat gggcagtggt gtcagcgatg atgtttttag cagctcccga catgaatcag 8100
taagtaagat ttccaccata tccagcgtca ggaatttaac cataaggagc agctcttttt 8160
cagacaccct ggaagaatcg agccccattg cagccatctt tgacacagaa aacctggaga 8220
aaatctccat tacagaaggt atagagcggg gcatcgttga cagcatcacg ggtcagaggc 8280
ttctggaggc tcaggcctgc acaggtggca tcatccaccc aaccacgggc cagaagctgt 8340
cacttcagga cgcagtctcc cagggtgtga ttgaccaaga catggccacc agcgtgaagc 8400
ctgctcagaa agccttcata ggcttcgagg gtgtgaaggg aaagaagaag atgtcagcag 8460
cagaggcagt gaaagaaaaa tggctcccgt atgaggctgg ccagcgcttc ctggagttcc 8520
agtacctcac gggaggtctt gttgacccgg aagtgcatgg gaggataagc accgaagaag 8580
ccatccggaa ggggttcata gatggccgcg ccgcacagag gctgcaagac accagcagct 8640
atgccaaaat cctgacctgc cccaaaacca aattaaaaat atcctataag gatgccataa 8700
atcgctccat ggtagaagat atcactgggc tgcgccttct ggaagccgcc tccgtgtcgt 8760
ccaagggctt acccagccct tacaacatgt cttcggctcc ggggtcccgc tccggctccc 8820
gctcgggatc tcgctccgga tctcgctccg ggtcccgcag tgggtcccgg agaggaagct 8880
ttgacgccac agggaattct tcctactctt attcctactc atttagcagt agttctattg 8940

ggcactag                                                          8948

<210> 120
<211> 587
<212> DNA
<213> Homo sapien

<220>
<221> misc_feature
<222> (1)...(587)
<223> n = A,T,C or G

<400> 120
cgtcctaagc acttagacta catcagggaa gaacacagac cacatccctg tcctcatgcg      60
gcttatgttt tctggaagaa agtggagacc nagtccttgg ctttagggct ccccggctgg     120
gggctgtgca ntccggtcag ggcgggaagg gaaatgcacc gctgcatgtg aacttacagc     180
ccaggcggat gccccttccc ttagcactac ctggcctcct gcatcccctc gcctcatgtt     240
cctcccacct tcaaanaatg aanaacccca tgggcccagc cccttgccct ggggaaccaa     300
ggcagccttc caaaactcag gggctgaagc anactattag ggcaggggct gactttgggt     360
gacactgccc attccctctc agggcagctc angtcacccn ggnctcttga acccagcctg     420
ttcctttgaa aaagggcaaa actgaaaagg gcttttccta naaaaagaaa aaccagggaa     480
ctttgccagg gcttcnntnt taccaaaacn ncttctcnng gatttttaat tccccattng     540
gcctccactt accnggggcn atgccccaaa attaanaatt tcccatc                   587

<210> 121
<211> 619
<212> DNA
<213> Homo sapien

<220>
<221> misc_feature
<222> (1)...(619)
<223> n = A,T,C or G

<400> 121
cactagtagg atagaaacac tgtgtcccga gagtaaggag agaagctact attgattaga      60
gcctaaccca ggttaactgc aagaagaggc gggatacttt cagctttcca tgtaactgta     120
tgcataaagc caatgtagtc cagtttctaa gatcatgttc caagctaact gaatcccact     180
tcaatacaca ctcatgaact cctgatggaa caataacagg cccaagcctg tggtatgatg     240
tgcacacttg ctagactcan aaaaaatact actctcataa atgggtggga gtattttggt     300
gacaacctac tttgcttggc tgagtgaagg aatgatattc atatattcat ttattccatg     360
gacatttagt tagtgctttt tatataccag gcatgatgct gagtgacact cttgtgtata     420
tttccaaatt tttgtacagt cgctgcacat atttgaaatc atatattaag acttccaaaa     480
aatgaagtcc ctggtttttc atggcaactt gatcagtaaa ggattcncct ctgtttggta     540
cttaaaacat ctactatatn gttnanatga aattcctttt ccccncctcc cgaaaaaana     600
aagtggtggg gaaaaaaaa                                                  619

<210> 122
<211> 1475
<212> DNA
<213> Homo sapien

<400> 122
tccacctgtc cccgcagcgc cggctcgcgc cctcctgccg cagccaccga gccgccgtct      60
agcgccccga cctcgccacc atgagagccc tgctggcgcg cctgcttctc tgcgtcctgg     120

tcgtgagcga ctccaaaggc agcaatgaac ttcatcaagt tccatcgaac tgtgactgtc 180
taaatggagg aacatgtgtg tccaacaagt acttctccaa cattcactgg tgcaactgcc 240
caaagaaatt cggagggcag cactgtgaaa tagataagtc aaaaacctgc tatgagggga 300
atggtcactt ttaccgagga aaggccagca ctgacaccat gggccggccc tgcctgccct 360
ggaactctgc cactgtcctt cagcaaacgt accatgccca cagatctgat gctcttcagc 420
tgggcctggg gaaacataat tactgcagga acccagacaa ccggaggcga ccctggtgct 480
atgtgcaggt gggcctaaag ccgcttgtcc aagagtgcat ggtgcatgac tgcgcagatg 540
gaaaaaagcc ctcctctcct ccagaagaat taaaatttca gtgtggccaa aagactctga 600
ggccccgctt taagattatt gggggagaat tcaccaccat cgagaaccag ccctggtttg 660
cggccatcta caggaggcac cgggggggct ctgtcaccta cgtgtgtgga ggcagcctca 720
tcagcccttg ctgggtgatc agcgccacac actgcttcat tgattaccca aagaaggagg 780
actacatcgt ctacctgggt cgctcaaggc ttaactccaa cacgcaaggg gagatgaagt 840
ttgaggtgga aaacctcatc ctacacaagg actacagcgc tgacacgctt gctcaccaca 900
acgacattgc cttgctgaag atccgttcca aggagggcag gtgtgcgcag ccatcccgga 960
ctatacagac catctgcctg ccctcgatgt ataacgatcc ccagtttggc acaagctgtg 1020
agatcactgg ctttggaaaa gagaattcta ccgactatct ctatccggag cagctgaaga 1080
tgactgttgt gaagctgatt tcccaccggg agtgtcagca gccccactac tacggctctg 1140
aagtcaccac caaaatgctg tgtgctgctg acccacagtg gaaaacagat tcctgccagg 1200
gagactcagg gggacccctc gtctgttccc tccaaggccg catgactttg actggaattg 1260
tgagctgggg ccgtggatgt gccctgaagg acaagccagg cgtctacacg agagtctcac 1320
acttcttacc ctggatccgc agtcacacca aggaagagaa tggcctggcc ctctgagggt 1380
ccccagggag gaaacgggca ccacccgctt tcttgctggt tgtcattttt gcagtagagt 1440
catctccatc agctgtaaga agagactggg aagat 1475

<210> 123
<211> 2294
<212> DNA
<213> Homo sapien

<400> 123
cagcgccggc tcgcgccctc ctgccgcagc caccgagccg ccgtctagcg ccccgacctc 60
gccaccatga gagccctgct ggcgcgcctg cttctctgcg tcctggtcgt gagcgactcc 120
aaaggcagca atgaacttca tcaagttcca tcgaactgtg actgtctaaa tggaggaaca 180
tgtgtgtcca acaagtactt ctccaacatt cactggtgca actgcccaaa gaaattcgga 240
gggcagcact gtgaaataga taagtcaaaa acctgctatg aggggaatgg tcacttttac 300
cgaggaaagg ccagcactga caccatgggc cggccctgcc tgccctggaa ctctgccact 360
gtccttcagc aaacgtacca tgcccacaga tctgatgctc ttcagctggg cctggggaaa 420
cataattact gcaggaaccc agacaaccgg aggcgaccct ggtgctatgt gcaggtgggc 480
ctaaagccgc ttgtccaaga gtgcatggtg catgactgcg cagatggaaa aaagccctcc 540
tctcctccag aagaattaaa atttcagtgt ggccaaaaga ctctgaggcc ccgctttaag 600
attattgggg gagaattcac caccatcgag aaccagccct ggtttgcggc catctacagg 660
aggcaccggg ggggctctgt cacctacgtg tgtggaggca gcctcatcag cccttgctgg 720
gtgatcagcg ccacacactg cttcattgat tacccaaaga aggaggacta catcgtctac 780
ctgggtcgct caaggcttaa ctccaacacg caaggggaga tgaagtttga ggtggaaaac 840
ctaatcctac acaaggacta cagcgctgac acgcttgctc accacaacga cattgccttg 900
ctgaagatcc gttccaagga gggcaggtgt gcgcagccat cccggactat acagaccatc 960
tgcctgccct cgatgtataa cgatccccag tttggcacaa gctgtgagat cactggcttt 1020
ggaaaagaga attctaccga ctatctctat ccggagcagc tgaaaatgac tgttgtgaag 1080
ctgatttccc accgggagtg tcagcagccc cactactacg gctctgaagt caccaccaaa 1140
atgctgtgtg ctgctgaccc acagtggaaa acagattcct gccagggaga ctcaggggga 1200
cccctcgtct gttccctcca aggccgcatg actttgactg gaattgtgag ctggggccgt 1260
ggatgtgccc tgaaggacaa gccaggcgtc tacacgagag tctcacactt cttaccctgg 1320
atccgcagtc acaccaagga agagaatggc ctggccctct gagggtcccc agggaggaaa 1380
cgggcaccac ccgctttctt gctggttgct attttgcagt agagtcatct ccatcagctg 1440
taagaagagc tgggaatata ggctctgcac agatggattt gcctgtgcca ccaccagggc 1500

gaacgacaat agctttaccc tcaggcatag gcctgggtgc tggctgccca gacccctctg 1560
gccaggatgg aggggtggtc ctgactcaac atgttactga ccagcaactt gtctttttct 1620
ggactgaagc ctgcaggagt taaaaagggc agggcatctc ctgtgcatgg gctcgaaggg 1680
agagccagct cccccgaccg gtgggcattt gtgaggccca tggttgagaa atgaataatt 1740
tcccaattag gaagtgtaag cagctgaggt ctcttgaggg agcttagcca atgtgggagc 1800
agcggtttgg ggagcagaga cactaacgac ttcagggcag ggctctgata ttccatgaat 1860
gtatcaggaa atatatatgt gtgtgtatgt ttgcacactt gtgtgtgggc tgtgagtgta 1920
agtgtgagta agagctggtg tctgattgtt aagtctaaat atttccttaa actgtgtgga 1980
ctgtgatgcc acacagagtg gtctttctgg agaggttata ggtcactcct ggggcctctt 2040
gggtccccca cgtgacagtg cctgggaatg tattattctg cagcatgacc tgtgaccagc 2100
actgtctcag tttcactttc acatagatgt ccctttcttg gccagttatc ccttcctttt 2160
agcctagttc atccaatcct cactgggtgg ggtgaggacc actcctgtac actgaatatt 2220
tatatttcac tatttttatt tatatttttg taattttaaa taaaagtgat caataaaatg 2280
tgatttttct gatg 2294

<210> 124
<211> 956
<212> DNA
<213> Homo sapien

<400> 124
gatgagttcc gcaccaagtt tgagacagac caggccctgc gcctgagtgt ggaggccgac 60
atcaatggcc tgcgcagggt gctggatgag ctgaccctgg ccagagccga cctggagatg 120
cagattgaga acctcaagga ggagctggcc tacctgaaga agaaccacga ggaggagatg 180
aacgccctgc gaggccaggt gggtggtgag atcaatgtgg agatggacgc tgccccaggc 240
gtggacctga gccgcatcct caacgagatg cgtgaccagt atgagaagat ggcagagaag 300
aaccgcaagg atgccgagga ttggttcttc agcaagacag aggaactgaa ccgcgaggtg 360
gccaccaaca gtgagctggt gcagagtggc aagagtgaga tctcggagct ccggcgcacc 420
atgcaggcct tggagataga gctgcagtcc cagctcagca tgaaagcatc cctggagggc 480
aacctggcgg agacagagaa ccgctactgc gtgcagctgt cccagatcca ggggctgatt 540
ggcagcgtgg aggagcagct ggcccagctt cgctgcgaga tggagcagca gaaccaggaa 600
tacaaaatcc tgctggatgt gaagacgcgg ctggagcagg agattgccac ctaccgccgc 660
ctgctggagg gagaggatgc ccacctgact cagtacaaga aagaaccggt gaccacccgt 720
caggtgcgta ccattgtgga agaggtccag gatggcaagg tcatctcctc ccgcgagcag 780
gtccaccaga ccacccgctg aggactcagc taccccggcc ggccacccag gaggcaggga 840
cgcagccgcc ccatctgccc cacagtctcc ggcctctcca gcctcagccc cctgcttcag 900
tcccttcccc atgcttcctt gcctgatgac aataaaagct tgttgactca gctatg 956

<210> 125
<211> 486
<212> DNA
<213> Homo sapien

<220>
<221> misc_feature
<222> (1)...(486)
<223> n = A,T,C or G

<400> 125
aaattatata tagtgnttca gctcccattg tggtgttcat agtcttctag gaacagataa 60
acttaagtat tcaattcact cttggcattt tttctttaat ataggctttt tagcctattt 120
ttggaaaact gcttttcttc tgagaacctt attctgaatg tcatcaactt taccaaacct 180
tctaagtcca gagctaactt agtactgttt aagttactat tgactgaatt ttcttcattt 240
tctgtttagc cagtgttacc aaggtaagct ggggaatgaa gtataccaac ttctttcaga 300
gcattttagg acattatggc agctttagaa ggctgtcttg tttctagcca agggagagcc 360

agcgcaggtt ttggatacta gagaaagtca tttgcttgta ctattgccat tttagaaagc 420
tctgatgtga attcaaattt tacctctgtt acttaaagcc aacaatttta aggcagtagt 480
tttact 486

<210> 126
<211> 3552
<212> DNA
<213> Homo sapien

<400> 126
cggcaggcag gtctcgtctc ggcaccctcc cggcgcccgc gttctcctgg ccctgcccgg 60
catcccgatg gccgccgctg ggccccggcg ctccgtgcgc ggagccgtct gcctgcatct 120
gctgctgacc ctcgtgatct tcagtcgtgc tggtgaagcc tgcaaaaagg tgatacttaa 180
tgtaccttct aaactagagg cagacaaaat aattggcaga gttaatttgg aagagtgctt 240
caggtctgca gacctcatcc ggtcaagtga tcctgatttc agagttctaa atgatgggtc 300
agtgtacaca gccagggctg ttgcgctgtc tgataagaaa agatcattta ccatatggct 360
ttctgacaaa aggaaacaga cacagaaaga ggttactgtg ctgctagaac atcagaagaa 420
ggtatcgaag acaagacaca ctagagaaac tgttctcagg cgtgccaaga ggagatgggc 480
acctattcct tgctctatgc aagagaattc cttgggccct ttcccattgt ttcttcaaca 540
agttgaatct gatgcagcac agaactatac tgtcttctac tcaataagtg gacgtggagt 600
tgataaagaa cctttaaatt tgttttatat agaaagagac actggaaatc tattttgcac 660
tcggcctgtg gatcgtgaag aatatgatgt ttttgatttg attgcttatg cgtcaactgc 720
agatggatat tcagcagatc tgcccctccc actacccatc agggtagagg atgaaaatga 780
caaccaccct gttttcacag aagcaattta taattttgaa gttttggaaa gtagtagacc 840
tggtactaca gtgggggtgg tttgtgccac agacagagat gaaccggaca caatgcatac 900
gcgcctgaaa tacagcattt tgcagcagac accaaggtca cctgggctct tttctgtgca 960
tcccagcaca ggcgtaatca ccacagtctc tcattatttg gacagagagg ttgtagacaa 1020
gtactcattg ataatgaaag tacaagacat ggatggccag ttttttggat tgataggcac 1080
atcaacttgt atcataacag taacagattc aaatgataat gcacccactt tcagacaaaa 1140
tgcttatgaa gcatttgtag aggaaaatgc attcaatgtg gaaatcttac gaatacctat 1200
agaagataag gatttaatta acactgccaa ttggagagtc aattttacca ttttaaaggg 1260
aaatgaaaat ggacatttca aaatcagcac agacaaagaa actaatgaag gtgttctttc 1320
tgttgtaaag ccactgaatt atgaagaaaa ccgtcaagtg aacctggaaa ttggagtaaa 1380
caatgaagcg ccatttgcta gagatattcc cagagtgaca gccttgaaca gagccttggt 1440
tacagttcat gtgagggatc tggatgaggg gcctgaatgc actcctgcag cccaatatgt 1500
gcggattaaa gaaaacttag cagtggggtc aaagatcaac ggctataagg catatgaccc 1560
cgaaaataga aatggcaatg gtttaaggta caaaaaattg catgatccta aaggttggat 1620
caccattgat gaaatttcag ggtcaatcat aacttccaaa atcctggata gggaggttga 1680
aactcccaaa aatgagttgt ataatattac agtcctggca atagacaaag atgatagatc 1740
atgtactgga acacttgctg tgaacattga agatgtaaat gataatccac cagaaatact 1800
tcaagaatat gtagtcattt gcaaaccaaa aatggggtat accgacattt tagctgttga 1860
tcctgatgaa cctgtccatg gagctccatt ttatttcagt ttgcccaata cttctccaga 1920
aatcagtaga ctgtggagcc tcaccaaagt taatgataca gctgcccgtc tttcatatca 1980
gaaaaatgct ggatttcaag aatataccat tcctattact gtaaaagaca gggccggcca 2040
agctgcaaca aaattattga gagttaatct gtgtgaatgt actcatccaa ctcagtgtcg 2100
tgcgacttca aggagtacag gagtaatact tggaaaatgg gcaatccttg caatattact 2160
gggtatagca ctgctctttt ctgtattgct aactttagta tgtggagttt ttggtgcaac 2220
taaagggaaa cgttttcctg aagatttagc acagcaaaac ttaattatat caaacacaga 2280
agcacctgga gacgatagag tgtgctctgc caatggattt atgacccaaa ctaccaacaa 2340
ctctagccaa ggtttttgtg gtactatggg atcaggaatg aaaaatggag ggcaggaaac 2400
cattgaaatg atgaaaggag gaaaccagac cttggaatcc tgccgggggg ctgggcatca 2460
tcataccctg gactcctgca ggggaggaca cacggaggtg gacaactgca gatacactta 2520
ctcggagtgg cacagtttta ctcaaccccg tctcggtgaa aaattgcatc gatgtaatca 2580
gaatgaagac cgcatgccat cccaagatta tgtcctcact tataactatg agggaagagg 2640
atctccagct ggttctgtgg gctgctgcag tgaaaagcag gaagaagatg gccttgactt 2700

tttaaataat ttggaaccca aatttattac attagcagaa gcatgcacaa agagataatg 2760
tcacagtgct acaattaggt ctttgtcaga cattctggag gtttccaaaa ataatattgt 2820
aaagttcaat ttcaacatgt atgtatatga tgattttttt ctcaattttg aattatgcta 2880
ctcaccaatt tatattttta aagcaagttg ttgcttatct tttccaaaaa gtgaaaaatg 2940
ttaaaacaga caactggtaa atctcaaact ccagcactgg aattaaggtc tctaaagcat 3000
ctgctctttt ttttttttac agatatttta gtaataaata tgctggataa atattagtcc 3060
aacaatagct aagttatgct aatatcacat tattatgtat tcactttaag tgatagttta 3120
aaaaataaac aagaaatatt gagtatcact atgtgaagaa agttttggaa aagaaacaat 3180
gaagactgaa ttaaattaaa aatgttgcag ctcataaaga attggactca cccctactgc 3240
actaccaaat tcatttgact ttggaggcaa aatgtgttga agtgccctat gaagtagcaa 3300
ttttctatag gaatatagtt ggaaataaat gtgtgtgtgt atattattat taatcaatgc 3360
aatatttaaa tgaaatgaga acaaagagga aaatggtaaa aacttgaaat gaggctgggg 3420
tatagtttgt cctacaatag aaaaaagaga gagcttccta ggcctgggct cttaaatgct 3480
gcattataac tgagtctatg aggaaatagt tcctgtccaa tttgtgtaat ttgtttaaaa 3540
ttgtaaataa at 3552

<210> 127
<211> 754
<212> DNA
<213> Homo sapien

<400> 127
tttttttttt ttgtcattgt tcattgattt taatgagaaa gctaagagag gaaataagta 60
gcctttcaaa ggtcacacag aagtaagtga cagatccagg attcatatcc aagcattctg 120
gctctagtgt ccatgcttct caaccattat gacccaatat tcaaccaaat caatactgaa 180
ggacacgtga aatgtatccg gtattttact attacaaaca aaaatccaat gaacattctt 240
gaagacatac acaaaaataa tggttacaat agaagttact ggaattgaaa ttttggttca 300
acctatatta aaatgtaagg cttttgatat agctaataga tttttgaaat gatcagtctt 360
aacgtttgta ggggagcaca ctcctgcatg gggaaaagat tcactgtgaa gcacagagca 420
cctttatggt tggatcatct tgtcattaaa gttcaggcgt tatctatcct gtaagtggca 480
gaatcaagac tgcaatatcg cctgcttttc tttttaactc atgttttccc ttgactacac 540
tggtcctcaa agtaaaaccc ctgtgtcagt gtactattca tggaatactc tgcaattata 600
accaccttct aatactttta atacccaatc aaaatttatt atacatatgt atcatagata 660
ctcatctgta aagctgtgct tcaaaatagt gatctcttcc caacattaca atatatatta 720
atgatgtcga acctgcccgg gcggccgctc gaag 754

<210> 128
<211> 374
<212> DNA
<213> Homo sapien

<400> 128
aggttttgat taaaaaggca aatgatttta ttgttcgata atcttttaaa aaaataagag 60
gaaggagtaa aattaaagat gaaagatgat ttttatttcc ttgtgacctc tatatccccc 120
ttcccctgcc cttggtaagt aactcttgat ggagaaagga ttaaagactc ttatttaacc 180
aaaaaacaga gccagctaat catttccaaa ggttagtatc tccctgctga cctcttcttt 240
ggtttaattg aataaaacta tatgttcata tatgtattaa aacaactcag aataacatct 300
tttcttcctt agttaaggca ttataagggc tatactatca tccataataa ccaaggcaat 360
aacttaaaaa gctg 374

<210> 129
<211> 546
<212> DNA
<213> Homo sapien

<400> 129
agtgtgatgg atatctgcag aattcgggct aagcgtggtc gcggcccgag gtctggaact 60
tcccagcacy tgaaaaggag cctcctgagc tgactcggct aaagccccac tttcgctcct 120
cctcatttct gcctactgat ttccttggag cattcatctg aatattaccg tttgctgtgt 180
aacctggtac atacatagca tgactccctg gaatagagtg ggctggggtg cttatgctgg 240
gagagtgatt gacatgcact ttcaagctat atctaccatt tgcagcaaag gagaaaaaat 300
acctcgagta aattccatca ttttttataa catcagcacc tgctccatca tcaaggagtc 360
tcagcgtaac aggatctcca gtctctggct caactgtggc agtgacagtg gcattaagaa 420
tgggataaaa tccctgtttc acattggcat aaatcatcac aggatgagga aaatggaggc 480
tgtctctttc cacaaaggct tccacagtgg ctgggggcac agacctgccc gggcggccgc 540
tcgaaa 546

<210> 130
<211> 5156
<212> DNA
<213> Homo sapien

<400> 130
accaaccgag gcgccgggca gcgacccctg cagcggagac agagactgag cggcccggca 60
ccgccatgcc tgcgctctgg ctgggctgct gcctctgctt gtcgctcctc ctgcccgcag 120
cccgggccac ctccaggagg gaagtctgtg attgcaatgg gaagtccagg cagtgtatct 180
ttgatcggga acttcacaga caaactggta atggattccg ctgcctcaac tgcaatgaca 240
acactgatgg cattcactgc gagaagtgca agaatggctt ttaccggcac agagaaaggg 300
accgctgttt gccctgcaat tgtaactcca aaggttctct tagtgctcga tgtgacaact 360
ccggacggtg cagctgtaaa ccaggtgtga caggagccag atgcgaccga tgtctgccag 420
gcttccacat gctcacggat gcggggtgca cccaagacca gagactgcta gactccaagt 480
gtgactgtga cccagctggc atcgcagggc cctgtgacgc gggccgctgt gtctgcaagc 540
cagctgtcac tggagaacgc tgtgataggt gtcgatcagg ttactataat ctggatgggg 600
ggaaccctga gggctgtacc cagtgtttct gctatgggca ttcagccagc tgccgcagct 660
ctgcagaata cagtgtccat aagatcacct ctacctttca tcaagatgtt gatggctgga 720
aggctgtcca acgaaatggg tctcctgcaa agctccaatg gtcacagcgc catcaagatg 780
tgtttagctc agcccaacga ctagaccctg tctattttgt ggctcctgcc aaatttcttg 840
ggaatcaaca ggtgagctat ggtcaaagcc tgtcctttga ctaccgtgtg gacagaggag 900
gcagacaccc atctgcccat gatgtgattc tggaaggtgc tggtctacgg atcacagctc 960
ccttgatgcc acttggcaag acactgcctt gtgggctcac caagacttac acattcaggt 1020
taaatgagca tccaagcaat aattggagcc cccagctgag ttactttgag tatcgaaggt 1080
tactgcggaa tctcacagcc ctccgcatcc gagctacata tggagaatac agtactgggt 1140
acattgacaa tgtgaccctg atttcagccc gccctgtctc tggagcccca gcaccctggg 1200
ttgaacagtg tatatgtcct gttgggtaca aggggcaatt ctgccaggat tgtgcttctg 1260
gctacaagag agattcagcg agactggggc cttttggcac ctgtattcct tgtaactgtc 1320
aagggggagg ggcctgtgat ccagacacag gagattgtta ttcaggggat gagaatcctg 1380
acattgagtg tgctgactgc ccaattggtt tctacaacga tccgcacgac ccccgcagct 1440
gcaagccatg tccctgtcat aacgggttca gctgctcagt gatgccggag acggaggagg 1500
tggtgtgcaa taactgccct cccggggtca ccggtgcccg ctgtgagctc tgtgctgatg 1560
gctactttgg ggaccccttt ggtgaacatg gcccagtgag gccttgtcag ccctgtcaat 1620
gcaacaacaa tgtggacccc agtgcctctg ggaattgtga ccggctgaca ggcaggtgtt 1680
tgaagtgtat ccacaacaca gccggcatct actgcgacca gtgcaaagca ggctacttcg 1740
gggacccatt ggctcccaac ccagcagaca agtgtcgagc ttgcaactgt aaccccatgg 1800
gctcagagcc tgtaggatgt cgaagtgatg gcacctgtgt ttgcaagcca ggatttggtg 1860
gccccaactg tgagcatgga gcattcagct gtccagcttg ctataatcaa gtgaagattc 1920
agatggatca gtttatgcag cagcttcaga gaatggaggc cctgatttca aaggctcagg 1980
gtggtgatgg agtagtacct gatacagagc tggaaggcag gatgcagcag gctgagcagg 2040
cccttcagga cattctgaga gatgcccaga tttcagaagg tgctagcaga tcccttggtc 2100
tccagttggc caaggtgagg agccaagaga acagctacca gagccgcctg gatgacctca 2160
agatgactgt ggaaagagtt cgggctctgg gaagtcagta ccagaaccga gttcgggata 2220

ctcacaggct catcactcag atgcagctga gcctggcaga aagtgaagct tccttgggaa 2280
acactaacat tcctgcctca gaccactacg tggggccaaa tggctttaaa agtctggctc 2340
aggaggccac aagattagca gaaagccacg ttgagtcagc cagtaacatg gagcaactga 2400
caagggaaac tgaggactat tccaaacaag ccctctcact ggtgcgcaag gccctgcatg 2460
aaggagtcgg aagcggaagc ggtagcccgg acggtgctgt ggtgcaaggg cttgtggaaa 2520
aattggagaa aaccaagtcc ctggcccagc agttgacaag ggaggccact caagcggaaa 2580
ttgaagcaga taggtcttat cagcacagtc tccgcctcct ggattcagtg tctcggcttc 2640
agggagtcag tgatcagtcc tttcaggtgg aagaagcaaa gaggatcaaa caaaaagcgg 2700
attcactctc aagcctggta accaggcata tggatgagtt caagcgtaca cagaagaatc 2760
tgggaaactg gaaagaagaa gcacagcagc tcttacagaa tggaaaaagt gggagagaga 2820
aatcagatca gctgctttcc cgtgccaatc ttgctaaaag cagagcacaa gaagcactga 2880
gtatgggcaa tgccactttt tatgaagttg agagcatcct taaaaacctc agagagtttg 2940
acctgcaggt ggacaacaga aaagcagaag ctgaagaagc catgaagaga ctctcctaca 3000
tcagccagaa ggtttcagat gccagtgaca agacccagca agcagaaaga gccctgggga 3060
gcgctgctgc tgatgcacag agggcaaaga atggggccgg ggaggccctg gaaatctcca 3120
gtgagattga acaggagatt gggagtctga acttggaagc caatgtgaca gcagatggag 3180
ccttggccat ggaaaaggga ctggcctctc tgaagagtga gatgagggaa gtggaaggag 3240
agctggaaag gaaggagctg gagtttgaca cgaatatgga tgcagtacag atggtgatta 3300
cagaagccca gaaggttgat accagagcca agaacgctgg ggttacaatc caagacacac 3360
tcaacacatt agacggcctc ctgcatctga tggaccagcc tctcagtgta gatgaagagg 3420
ggctggtctt actggagcag aagctttccc gagccaagac ccagatcaac agccaactgc 3480
ggcccatgat gtcagagctg gaagagaggg cacgtcagca gaggggccac ctccatttgc 3540
tggagacaag catagatggg attctggctg atgtgaagaa cttggagaac attagggaca 3600
acctgccccc aggctgctac aatacccagg ctcttgagca acagtgaagc tgccataaat 3660
atttctcaac tgaggttctt gggatacaga tctcagggct cgggagccat gtcatgtgag 3720
tgggtgggat ggggacattt gaacatgttt aatgggtatg ctcaggtcaa ctgacctgac 3780
cccattcctg atcccatggc caggtggttg tcttattgca ccatactcct tgcttcctga 3840
tgctgggcaa tgaggcagat agcactgggt gtgagaatga tcaaggatct ggaccccaaa 3900
gaatagactg gatggaaaga caaactgcac aggcagatgt ttgcctcata atagtcgtaa 3960
gtggagtcct ggaatttgga caagtgctgt tgggatatag tcaacttatt ctttgagtaa 4020
tgtgactaaa ggaaaaaact ttgactttgc ccaggcatga aattcttcct aatgtcagaa 4080
cagagtgcaa cccagtcaca ctgtggccag taaaatacta ttgcctcata ttgtcctctg 4140
caagcttctt gctgatcaga gttcctccta cttacaaccc agggtgtgaa catgttctcc 4200
attttcaagc tggaagaagt gagcagtgtt ggagtgagga cctgtaaggc aggcccattc 4260
agagctatgg tgcttgctgg tgcctgccac cttcaagttc tggacctggg catgacatcc 4320
tttcttttaa tgatgccatg gcaacttaga gattgcattt ttattaaagc atttcctacc 4380
agcaaagcaa atgttgggaa agtatttact ttttcggttt caaagtgata gaaaagtgtg 4440
gcttgggcat tgaaagaggt aaaattctct agatttatta gtcctaattc aatcctactt 4500
ttagaacacc aaaaatgatg cgcatcaatg tattttatct tattttctca atctcctctc 4560
tctttcctcc acccataata agagaatgtt cctactcaca cttcagctgg gtcacatcca 4620
tccctccatt catccttcca tccatctttc catccattac ctccatccat ccttccaaca 4680
tatatttatt gagtacctac tgtgtgccag gggctggtgg gacagtggtg acatagtctc 4740
tgccctcata gagttgattg tctagtgagg aagacaagca tttttaaaaa ataaatttaa 4800
acttacaaac tttgtttgtc acaagtggtg tttattgcaa taaccgcttg gtttgcaacc 4860
tctttgctca acagaacata tgttgcaaga ccctcccatg ggggcacttg agttttggca 4920
aggctgacag agctctgggt tgtgcacatt tctttgcatt ccagctgtca ctctgtgcct 4980
ttctacaact gattgcaaca gactgttgag ttatgataac accagtggga attgctggag 5040
gaaccagagg cacttccacc ttggctggga agactatggt gctgccttgc ttctgtattt 5100
ccttggattt tcctgaaagt gtttttaaat aaagaacaat tgttagaaaa aaaaaa 5156

<210> 131
<211> 671
<212> DNA
<213> Homo sapien

<400> 131
aggtctggag ggcccacagc cggatgtggg acaccgggaa aaagtggtca tagcacacat 60
ttttgcatcc cggttgcagt gtgttgcaga cgaagtcctc ttgctcgtca ccccacactt 120
cctgggcagc caycacgagg atcatgactc ggaaaataaa gatgactgtg atccacacct 180
tcccgatgct ggtggagtgt ttgttgacac ccccgatgaa agtgtgcagc gtcccccaat 240
ccattgcgct ggtttatccc tgagtcctgt ttccaacgac tgccagtgtt tcagacccaa 300
agaatgaggg caagatccct ctgcgagggt ttcagacctc cttctcctac cccactggag 360
tgcctagaag ccaatgggtg cacagtgatg atacgaatgt caatctttgc tcggtcagtg 420
aggatgtcgc ctggaatatt caaattgaat tacagatgca tgaagagggc gtacaagtta 480
gaatttttct ttcgccatac agaaattgtt tagccagatc ttctgtactt cttttccttc 540
cctgaccctt cctgctcccc aggaagggag gtcagccccg tttgcaaaac acaggatgcc 600
cgtgacaccg gagacaggtc ttcttcaccg acaggaagtg ccttctggtg cctgcacgtt 660
ttaactgcta t 671

<210> 132
<211> 590
<212> DNA
<213> Homo sapien

<400> 132
ctgaatggaa aagcttatgg ctctgtgatg atattagtga ccagcggaga tgataagctt 60
cttggcaatt gcttacccac tgtgctcagc agtggttcaa caattcactc cattgccctg 120
ggttcatctg cagccccaaa tctggaggaa ttatcacgtc ttacaggagg tttaaagttc 180
tttgttccag atatatcaaa ctccaatagc atgattgatg ctttcagtag aatttcctct 240
ggaactggag acattttcca gcaacatatt cagcttgaaa gtacaggtga aaatgtcaaa 300
cctcaccatc aattgaaaaa cacagtgact gtggataata ctgtgggcaa cgacactatg 360
tttctagtta cgtggcaggc cagtggtcct cctgagatta tattatttga tcctgatgga 420
cgaaaatact acacaaataa ttttatcacc aatctaactt ttcggacagc tagtctttgg 480
attccaggaa cagctaagcc tgggcactgg acttacaccc tgaacaatac ccatcattct 540
ctgcaagccc tgaaagtgac agtgacctct cgcgcctcca actcagacct 590

<210> 133
<211> 581
<212> DNA
<213> Homo sapien

<400> 133
aggtcctgtc cgggggcact gagaactccc tctggaattc ttggggggtg ttggggagag 60
actgtgggcc tggagataaa acttgtctcc tctaccacca ccctgtaccc tagcctgcac 120
ctgtcctcat ctctgcaaag ttcagcttcc ttccccaggt ctctgtgcac tctgtcttgg 180
atgctctggg gagctcatgg gtggaggagt ctccaccaga gggaggctca ggggactggt 240
tgggccaggg atgaatattt gagggataaa aattgtgtaa gagccaaaga attggtagta 300
gggggagaac agagaggagc tgggctatgg gaaatgattt gaataatgga gctgggaata 360
tggctggata tctggtacta aaaaagggtc tttaagaacc tacttcctaa tctcttcccc 420
aatccaaacc atagctgtct gtccagtgct ctcttcctgc ctccagctct gccccaggct 480
cctcctagac tctgtccctg ggctagggca ggggaggagg gagagcaggg ttgggggaga 540
ggctgaggag agtgtgacat gtggggagag gaccagacct c 581

<210> 134
<211> 4797
<212> DNA
<213> Homo sapien

<220>
<221> misc_feature

<222> (1)...(4797)
<223> n = A,T,C or G

<400> 134
cctgggacca aagtgctgcc cagagctgag ggtcctggag ccacatgaga aggcttctcc 60
ctgtgtacct gtgcagcaca gggtagggtg agtccactca gctgtctagg agaggaccca 120
ggagcagcag agacncgcca agcctttact cataccatat tctgatcctt ttccagcaaa 180
ttgtggctac taatttgccc cctgaagatc aagatggctc tggggatgac tctgacaact 240
tctccggctc aggtgcaggt gaggttgtca tgggggcccc ccccacccaa gacggcaaca 300
ggtcatgcct gggggcagtg gtcaggcagt ctcctgtgtt tactgagcat gtactgagtg 360
caccctgcct gccctgtctc cacccagctg gctccaaagg gcaatgctga ggagaggaat 420
ggggtcgtga gctgctgtta aggagagctc atgcttggag gtgaggtgaa ggctgtgagc 480
tccagaaggc cccagggcgc nctgctgcac gcaggctcat attcactagg aatagcttta 540
ctcactaaga aacctctgga acccccttca gaaggttatt tgactcctga gcctctattt 600
tctcatctgc aaaatgggaa taataccttg acctgataag cttgtggagc tgtaaggcag 660
cacagagcca gctggggtgt agctcttcca tccaagctcc cttccttact tcccctttcc 720
tgtggggact gggggagaga agtccctgag ctggaggtgg tcagggaagc ttcacagagg 780
aggtggctct tgagtggacc tcaggaagag gggtgagaga gctaaggaag gaggctgagg 840
tcatccctgg ggaagtgacc tagcggaggc ctgagagctg caaggtagga tatctgttgt 900
tggaagtgtc tgttgttgga agtgggggcc tttttttcag ggagggtggg gccagagaag 960
tgtgtgccct gggataagta ggataaccac agtagttatg cccctaaggg atgcccaccc 1020
cacccctgtg gtcacagaaa agctttccca ggtggcctag gcacctgtct cgtggctcca 1080
gagacaggct gcacctgaca cacacaatgg aaggacagct ctccttgtcc attttccaag 1140
gagcttagcc tcagctgcct tgtccaggta ctagcctccc tcatagcctg agcttggcca 1200
gcccaggtgc tctggagcct cccccgaccc acccaacaca ctctgcttct ggtcctcccc 1260
accccccacc tccccaacac actctgcttc tggtcctgca ggtgctttgc aagatatcac 1320
cttgtcacag cagaccccct ccacttggaa ggacacgcag ctcctgacgg ctattcccac 1380
gtctccagaa cccaccggcc tggaggctac agctgcctcc acctccaccc tgccggctgg 1440
agaggggccc aaggagggag aggctgtagt cctgccagaa gtggagcctg gcctcaccgc 1500
ccgggagcag gaggccaccc cccgacccag ggagaccaca cagctcccga ccactcatca 1560
ggcctcaacg accacagcca ccacggccca ggagcccgcc acctcccacc cccacaggga 1620
catgcagcct ggccaccatg agacctcaac ccctgcagga cccagccaag ctgaccttca 1680
cactccccac acagaggatg gaggtccttc tgccaccgag agggctgctg aggatggagc 1740
ctccagtcag ctcccagcag cagagggctc tggggagcag gtgagtggcc tctgcattcc 1800
ttgggaaatt gagtgggttg gtcctaatgc ctggcacttg gcaggcccta cacctgtgcc 1860
ctgcgcgatc tcgtattcct caccaggaag acagggcaca ggggccgcct tcccctaccc 1920
ccagggcctc gcagagcagg acagactaac tatgagatca gagcagaagc acccttaaag 1980
atcacccaag agagggctcc caaactcaca atccaaactt gcagccctcg tcgaagagtg 2040
aacgttatac cagtcatttt atttatagct tcgtggattt acgcttacac taaatagtct 2100
gctattcata caaaatgtgt gctttgtatc actttttgtg atatccatgc catggtccag 2160
ccagggtccg gagttgatgt ggcaagaagg cctggctttc gggccctgtg cgatcctggt 2220
ttgggtgcat ctgagtgggt ggtggcaaag atcagggagg caggagctgc ttctgggtct 2280
gtagtggagc tggttgctgc tgctggcggt gacctggcca acccaatctg cccctgccct 2340
cccacaggac ttcacctttg aaacctcggg ggagaatacg gctgtagtgg ccgtggagcc 2400
tgaccgccgg aaccagtccc cagtggatca gggggccacg ggggcctcac agggcctcct 2460
ggacaggaaa gaggtgctgg gaggtgagtt ttctttcagg ggggtagttt ggggtgaatt 2520
gctgctgtgg ggtcagggtg gggctgacca cagccaaggc cactgctttg ggagggtctg 2580
cacgagagcc caaggagccg ctgagctgag ctggccccgt ctacctgccc taggggtcat 2640
tgccggaggc ctcgtggggc tcatctttgc tgtgtgcctg gtgggtttca tgctgtaccg 2700
catgaagaag aaggacgaag gcagctactc cttggaggag ccgaaacaag ccaacggcgg 2760
ggcctaccag aagcccacca aacaggagga attctatgcc tgacgcggga gccatgcgcc 2820
ccctccgccc tgccactcac taggccccca cttgcctctt ccttgaagaa ctgcaggccc 2880
tggcctcccc tgccaccagg ccacctcccc agcattccag cccctctggt cgctcctgcc 2940
cacggagtcg tgggtgtgct gggagctcca ctctgcttct ctgacttctg cctggagact 3000
tagggcacca ggggtttctc gcataggacc tttccaccac agccagcacc tggcatcgca 3060

ccattctgac tcggtttctc caaactgaag cagcctctcc ccaggtccag ctctggaggg 3120
gagggggatc cgactgcttt ggacctaaat ggcctcatgt ggctggaaga tcctgcgggt 3180
ggggcttggg gctcacacac ctgtagcact tactggtagg accaagcatc ttgggggggt 3240
ggccgctgag tggcagggga caggagtcac tttgtttcgt ggggaggtct aatctagata 3300
tcgacttgtt tttgcacatg tttcctctag ttctttgttc atagcccagt agaccttgtt 3360
acttctgagg taagttaagt aagttgattc ggtatccccc catcttgctt ccctaatcta 3420
tggtcgggag acagcatcag ggttaagaag actttttttt ttttttttaa actaggagaa 3480
ccaaatctgg aagccaaaat gtaggcttag tttgtgtgtt gtctcttgag tttgtcgctc 3540
atgtgtgcaa cagggtatgg actatctgtc tggtggcccc gttctggtgg tctgttggca 3600
ggctggccag tccaggctgc cgtggggccg ccgcctcttt caagcagtcg tgcctgtgtc 3660
catgcgctca gggccatgct gaggcctggg ccgctgccac gttggagaag cccgtgtgag 3720
aagtgaatgc tgggactcag ccttcagaca gagaggactg tagggagggc ggcaggggcc 3780
tggagatcct cctgcaggct cacgcccgtc ctcctgtggc gccgtctcca ggggctgctt 3840
cctcctggaa attgacgagg ggtgtcttgg gcagagctgg ctctgagcgc ctccatccaa 3900
ggccaggttc tccgttagct cctgtggccc caccctgggc cctgggctgg aatcaggaat 3960
attttccaaa gagtgatagt cttttgcttt tggcaaaact ctacttaatc caatgggttt 4020
ttccctgtac agtagatttt ccaaatgtaa taaactttaa tataaagtag tctgtgaatg 4080
ccactgcctt cgcttcttgc ctctgtgctg tgtgtgacgt gaccggactt ttctgcaaac 4140
accaacatgt tgggaaactt ggctcgaatc tctgtgcctt cgtctttccc atggggaggg 4200
attctggttc cagggtccct ctgtgtattt gcttttttgt tttggctgaa attctcctgg 4260
aggtcggtag gttcagccaa ggttttataa ggctgatgtc aatttctgtg ttgccaagct 4320
ccaagcccat cttctaaatg gcaaaggaag gtggatggcc ccagcacagc ttgacctgag 4380
gctgtggtca cagcggaggt gtggagccga ggcctacccc ncagacacct tggacatcct 4440
cctcccaccc ggctgcagag gccaganncc agcccagggt cctgcactta cttgcttatt 4500
tgacaacgtt tcagcgactc cgttggccac tccgagagtg ggccagtctg tggatcagag 4560
atgcaccacc aagccaaggg aacctgtgtc cggtattcga tactgcgact ttctgcctgg 4620
agtgtatgac tgcacatgac tcgggggtgg ggaaaggggt cggctgacca tgctcatctg 4680
ctggtccgtg ggacggtncc caagccagag gtgggttcat ttgtgtaacg acaataaacg 4740
gtacttgtca tttcgggcaa cggctgctgt ggtggtggtt gagtctcttc ttggcct 4797

<210> 135
<211> 2856
<212> DNA
<213> Homo sapien

<400> 135
tagtcgcggg tccccgagtg agcacgccag ggagcaggag accaaacgac gggggtcgga 60
gtcagagtcg cagtgggagt ccccggaccg gagcacgagc ctgagcggga gagcgccgct 120
cgcacgcccg tcgccacccg cgtacccggc gcagccagag ccaccagcgc agcgctgcca 180
tggagcccag cagcaagaag ctgacgggtc gcctcatgct ggctgtggga ggagcagtgc 240
ttggctccct gcagtttggc tacaacactg gagtcatcaa tgccccccag aaggtgatcg 300
aggagttcta caaccagaca tgggtccacc gctatgggga gagcatcctg cccaccacgc 360
tcaccacgct ctggtccctc tcagtggcca tcttttctgt tgggggcatg attggctcct 420
tctctgtggg ccttttcgtt aaccgctttg gccggcggaa ttcaatgctg atgatgaacc 480
tgctggcctt cgtgtccgcc gtgctcatgg gcttctcgaa actgggcaag tcctttgaga 540
tgctgatcct gggccgcttc atcatcggtg tgtactgcgg cctgaccaca ggcttcgtgc 600
ccatgtatgt gggtgaagtg tcacccacag cctttcgtgg ggccctgggc accctgcacc 660
agctgggcat cgtcgtcggc atcctcatcg cccaggtgtt cggcctggac tccatcatgg 720
gcaacaagga cctgtggccc ctgctgctga gcatcatctt catcccggcc ctgctgcagt 780
gcatcgtgct gcccttctgc cccgagagtc cccgcttcct gctcatcaac cgcaacgagg 840
agaaccgggc caagagtgtg ctaaagaagc tgcgcgggac agctgacgtg acccatgacc 900
tgcaggagat gaaggaagag agtcggcaga tgatgcggga gaagaaggtc accatcctgg 960
agctgttccg ctcccccgcc taccgccagc ccatcctcat cgctgtggtg ctgcagctgt 1020
cccagcagct gtctggcatc aacgctgtct tctattactc cacgagcatc ttcgagaagg 1080
cgggggtgca gcagcctgtg tatgccacca ttggctccgg tatcgtcaac acggccttca 1140

ctgtcgtgtc gctgtttgtg gtggagcgag caggccggcg gaccctgcac ctcataggcc 1200
tcgctggcat ggcgggttgt gccatactca tgaccatcgc gctagcactg ctggagcagc 1260
taccctggat gtcctatctg agcatcgtgg ccatctttgg ctttgtggcc ttctttgaag 1320
tgggtcctgg ccccatccca tggttcatcg tggctgaact cttcagccag ggtccacgtc 1380
cagctgccat tgccgttgca ggcttctcca actggacctc aaatttcatt gtgggcatgt 1440
gcttccagta tgtggagcaa ctgtgtggtc cctacgtctt catcatcttc actgtgctcc 1500
tggttctgtt cttcatcttc acctacttca aagttcctga gactaaaggc cggaccttcg 1560
atgagatcgc ttccggcttc cggcaggggg gagccagcca aagtgataag acacccgagg 1620
agctgttcca tcccctgggg gctgattccc aagtgtgagt cgccccagat caccagcccg 1680
gcctgctccc agcagcccta aggatctctc aggagcacag gcagctggat gagacttcca 1740
aacctgacag atgtcagccg agccgggcct ggggctcctt tctccagcca gcaatgatgt 1800
ccagaagaat attcaggact taacggctcc aggattttaa caaaagcaag actgttgctc 1860
aaatctattc agacaagcaa caggttttat aattttttta ttactgattt tgttattttt 1920
atatcagcct gagtctcctg tgcccacatc ccaggcttca ccctgaatgg ttccatgcct 1980
gagggtggag actaagccct gtcgagacac ttgccttctt cacccagcta atctgtaggg 2040
ctggacctat gtcctaagga cacactaatc gaactatgaa ctacaaagct tctatcccag 2100
gaggtggcta tggccacccg ttctgctggc ctggatctcc ccactctagg ggtcaggctc 2160
cattaggatt tgccccttcc catctcttcc tacccaacca ctcaaattaa tctttcttta 2220
cctgagacca gttgggagca ctggagtgca gggaggagag gggaagggcc agtctgggct 2280
gccgggttct agtctccttt gcactgaggg ccacactatt accatgagaa gagggcctgt 2340
gggagcctgc aaactcactg ctcaagaaga catggagact cctgccctgt tgtgtataga 2400
tgcaagatat ttatatatat ttttggttgt caatattaaa tacagacact aagttatagt 2460
atatctggac aagccaactt gtaaatacac cacctcactc ctgttactta cctaaacaga 2520
tataaatggc tggtttttag aaacatggtt ttgaaatgct tgtggattga gggtaggagg 2580
tttggatggg agtgagacag aagtaagtgg ggttgcaacc actgcaacgg cttagacttc 2640
gactcaggat ccagtccctt acacgtacct ctcatcagtg tcctcttgct caaaaatctg 2700
tttgatccct gttacccaga gaatatatac attctttatc ttgacattca aggcatttct 2760
atcacatatt tgatagttgg tgttcaaaaa aacactagtt ttgtgccagc cgtgatgctc 2820
aggcttgaaa tcgcattatt ttgaatgtga agggaa 2856

<210> 136
<211> 356
<212> DNA
<213> Homo sapien

<400> 136
ggtggagcca aatgaagaaa atgaagatga aagagacaga cacctcagtt tttctggatc 60
aggcattgat gatgatgaag attttatctc cagcaccatt tcaaccacac cacgggcttt 120
tgaccacaca aaacagaacc aggactggac tcagtggaac ccaagccatt caaatccgga 180
agtgctactt cagacaacca caaggatgac tgatgtagac agaaatggca ccactgctta 240
tgaaggaaac tggaacccag aagcacaccc tcccctcatt caccatgagc atcatgagga 300
agaagagacc ccacattcta caagcacaat ccaggcaact cctagtagta caacgg 356

<210> 137
<211> 356
<212> DNA
<213> Homo sapien

<220>
<221> misc_feature
<222> (1)...(356)
<223> n = A,T,C or G

<400> 137
   gcaggtggag aagacatttt attgttcctg gggtctctgg aggcccattg gtggggctgg 60
   gtcactggct gcccccggaa cagggcgctg ctccatggct ctgcttgtgg tagtctgtgg 120
   ctatgtctcc cagcaaggac agaaactcag aaaaatcaat cttcttatcc tcattcttgt 180
   cctttttctc aaagacatcg gcgaggtaat ttgtgccctt tttacctcgg cccgcgacca 240
   cgctaaggcc aaanttccag acanayggcc gggccggtnc nataggggan cccaacttgg 300
   ggacccaaac tctggcgcgg aaacacangg gcataagctt gnttcctgtg gggaaa 356

<210> 138
<211> 353
<212> DNA
<213> Homo sapien

<400> 138
aggtccagtc ctccacttgg cctgatgaga gtggggagtg gcaagggacg tttctcctgc 60
aatagacact tagatttctc tcttgtggga agaaaccacc tgtccatcca ctgactcttc 120
tacattgatg tggaaattgc tgctgctacc accacctcct gaagaggctt ccctgatgcc 180
aatgccagcc atcttggcat cctggccctc gagcaggctg cggtaagtag cgatctcctg 240
ctccagccgt gtctttatgt caagcagcat cttgtactcc tggttctgag cctccatctc 300
gcatcggagc tcactcagac ctcgsccgsg mssmcgctam gccgaattcc agc 353

<210> 139
<211> 371
<212> DNA
<213> Homo sapien

<400> 139
agcgtggtcg cggccgaggt ccatccgaag caagattgca gatggcagtg tgaagagaga 60
agacatattc tacacttcaa agctttggtg caattcccat cgaccagagt tggtccgacc 120
agccttggaa aggtcactga aaaatcttca attggattat gttgacctct accttattca 180
ttttccagtg tctgtaaagc caggtgagga agtgatccca aaagatgaaa atggaaaaat 240
actatttgac acagtggatc tctgtgccac gtgggaggcc gtggagaagt gtaaagatgc 300
aggattggac ctgcccgggc ggccgctcga aagccgaatt ccagcacact ggcggccgtt 360
actagtggat c 371

<210> 140
<211> 370
<212> DNA
<213> Homo sapien

<400> 140
tagcgtggtc gcggccgagg tccatctccc tttgggaact agggggctgc tggtgggaaa 60
tgggagccag ggcagatgtt gcattccttt gtgtccctgt aaatgtggga ctacaagaag 120
aggagctgcc tgagtggtac tttctcttcc tggtaatcct ctggcccagc ctcatggcag 180
aatagaggta tttttaggct atttttgtaa tatggcttct ggtcaaaatc cctgtgtagc 240
tgaattccca agccctgcat tgtacagccc cccactcccc tcaccaccta ataaaggaat 300
agttaacact caaaaaaaaa aaaaaacctg cccgggcggc cgctcgaaag ccgaattcca 360
gcacactggc 370

<210> 141
<211> 371
<212> DNA
<213> Homo sapien

<400> 141
tagcgtggtc gcggccgagg tcctctgtgc tgcctgtcac agcccgatgg taccagcgca 60
gggtgtaggc agtgcaggag ccctcatcca gtggcaggga acaggggtca tcactatccc 120

aaggagcttc agggtcctgg tactcctcca cagaatactc ggagtattca gagtactcat 180
catcctcagg gggtacccgc tcttcctcct ctgcatgaga gacgcggagc acaggcacag 240
catggagctg ggagccggca gtgtctgcag cataactagg gaggggtcgt gatccagatg 300
cgatgaactg gccctggcag gcacagtgct gactcatctc ttggcgacct gcccgggcgg 360
ccgctcgaag c 371

<210> 142
<211> 343
<212> DNA
<213> Homo sapien

<400> 142
gcgttttgag gccaatggtg taaaaggaaa tatcttcaca taaaaactag atggaagcat 60
tgtcagaaac ctctttgtga tgtttgcttt caactcacag agttgaacat tccttttcat 120
agagcagttt tgaaacactc ttttgtagaa tttgcaagcg gatgattgga tcgctatgag 180
gtcttcattg gaaacgggat acctttacat aaaaactaga cagtagcatt ctcagaaatt 240
tctttgggat gtgggcattc aacccacaga ggagaacttc atttgataga gcagttttga 300
aacacccttt ttgtagaatc tacaggtgga catttagagt gct 343

<210> 143
<211> 354
<212> DNA
<213> Homo sapien

<400> 143
aggtctgatg gcagaaaaac tcagactgtc tgcaacttta cagatggtgc attggttcag 60
catcaggagt gggatgggaa ggaaagcaca ataacaagaa aattgaaaga tgggaaatta 120
gtggtggagt gtgtcatgaa caatgtcacc tgtactcgga tctatgaaaa agtagaataa 180
aaattccatc atcactttgg acaggagtta attaagagaa tgaccaagct cagttcaatg 240
agcaaatctc catactgttt ctttcttttt tttttcatta ctgtgttcaa ttatctttat 300
cataaacatt ttacatgcag ctatttcaaa gtgtgttgga ttaattagga tcat 354

<210> 144
<211> 353
<212> DNA
<213> Homo sapien

<400> 144
ggtcaaggac ctgggggacc cccaggtcca gcagccacat gattctgcag cagacaggga 60
cctagagcac atctggatct cagccccacc cctggcaacc tgcctgccta gagaactccc 120
aagatgacag actaagtagg attctgccat ttagaataat tctggtatcc tgggcgttgc 180
gttaagttgc ttaactttca ttctgtctta cgatagtctt cagaggtggg aacagatgaa 240
gaaaccatgc cccagagaag gttaagtgac ttcctcttta tggagccagt gttccaacct 300
aggtttgcct gataccagac ctgtggcccc acctcccatg caggtctctg tgg 353

<210> 145
<211> 371
<212> DNA
<213> Homo sapien

<400> 145
caggtctgtc ataaactggt ctggagtttc tgacgactcc ttgttcacca aatgcaccat 60
ttcctgagac ttgctggcct ctccgttgag tccacttggc tttctgtcct ccacagctcc 120
attgccactg ttgatcacta gctttttctt ctgcccacac cttcttcgac tgttgactgc 180
aatgcaaact gcaagaatca aagccaaggc caagagggat gccaagatga tcagccattc 240

tggaatttgg ggtgtcctta taggaccaga ggttgtgttt gctccacctt cttgactccc 300
atgtgagacc tcggccgcga ccacgctaag ccgaattcca gcacactggc ggcccgttac 360
tagtggatcc g 371

<210> 146
<211> 355
<212> DNA
<213> Homo sapien

<400> 146
ggtcctccgt cctcttccca gaggtgtcgg ggcttggccc cagcctccat cttcgtctct 60
caggatggcg agtagcagcg gctccaaggc tgaattcatt gtcggaggga aatataaact 120
ggtacggaag atcgggtctg gctccttcgg ggacatctat ttggcgatca acatcaccaa 180
cggcgaggaa gtggcagtga agctagaatc tcagaaggcc aggcatcccc agttgctgta 240
cgagagcaag ctctataaga ttcttcaagg tggggttggc atcccccaca tacggtggta 300
tggtcaggaa aaagactaca atgtactagt catggatctt ctgggaccta gcctc 355

<210> 147
<211> 355
<212> DNA
<213> Homo sapien

<400> 147
ggtctgttac aaaatgaaga cagacaacac aacatttact ctgtggagat atcctactca 60
tactatgcac gtgctgtgat tttgaacata actcgtccca aaaacttgtc acgatcatcc 120
tgacttttta ggttggctga tccatcaatc ttgcactcaa ctgttacttc tttcccagtg 180
ttgttaggag caaagctgac ctgaacagca accaatggct gtagataccc aacatgcagt 240
tttttcccat aatatgggaa atattttaag tctatcattc cattatgagg ataaactgct 300
acatttggta tatcttcatt ctttgaaaca caatctatcc ttggcactcc ttcag 355

<210> 148
<211> 369
<212> DNA
<213> Homo sapien

<400> 148
aggtctctct ccccctctcc ctctcctgcc agccaagtga agacatgctt acttcccctt 60
caccttcctt catgatgtgg gaagagtgct gcaacccagc cctagccaac accgcatgag 120
agggagtgtg ccgagggctt ctgagaaggt ttctctcaca tctagaaaga agcgcttaag 180
atgtggcagc ccctcttctt caagtggctc ttgtcctgtt gccctgggag ttctcaaatt 240
gctgcagcag cctccatcca gcctgaggat gacatcaata cacagaggaa gaagagtcag 300
gaaaagatga gagaagttac agactctcct gggcgacccc gagagcttac cattcctcag 360
acttcttca 369

<210> 149
<211> 620
<212> DNA
<213> Homo sapien

<220>
<221> misc_feature
<222> (1)...(620)
<223> n = A,T,C or G

<400> 149

actagtcaaa aatgctaaaa taatttggga gaaaatattt tttaagtagt gttatagttt 60
catgtttatc ttttattatg ttttgtgaag ttgtgtcttt tcactaatta cctatactat 120
gccaatattt ccttatatct atccataaca tttatactac atttgtaana naatatgcac 180
gtgaaactta acactttata aggtaaaaat gaggtttcca anatttaata atctgatcaa 240
gttcttgtta tttccaaata gaatggactt ggtctgttaa gggctaagga gaagaggaag 300
ataaggttaa aagttgttaa tgaccaaaca ttctaaaaga aatgcaaaaa aaaagtttat 360
tttcaagcct tcgaactatt taaggaaagc aaaatcattt cctaaatgca tatcatttgt 420
gagaatttct cattaatatc ctgaatcatt catttcacta aggctcatgt tnactccgat 480
atgtctctaa gaaagtacta tttcatggtc caaacctggt tgccatantt gggtaaaggc 540
tttcccttaa gtgtgaaant atttaaaatg aaattttcct ctttttaaaa attctttana 600
agggttaagg gtgttgggga 620

<210> 150
<211> 371
<212> DNA
<213> Homo sapien

<400> 150
   ggtccgatca aaacctgcta cctccccaag actttactag tgccgataaa ctttctcaaa 60
   gagcaaccag tatcacttcc ctgtttataa aacctctaac catctctttg ttctttgaac 120
   atgctgaaaa ccacctggtc tgcatgtatg cccgaatttg yaattctttt ctctcaaatg 180
   aaaatttaat tttagggatt catttctata ttttcacata tgtagtatta ttatttcctt 240
   atatgtgtaa ggtgaaattt atggtatttg agtgtgcaag aaaatatatt tttaaagctt 300
   tcatttttcc cccagtgaat gatttagaat tttttatgta aatatacaga atgttttttc 360
   ttacttttat a 371

<210> 151
<211> 4655
<212> DNA
<213> Homo sapien

<400> 151
gggacttgag ttctgttatc ttcttaagta gattcatatt gtaagggtct cggggtgggg 60
gggttggcaa aatcctggag ccagaagaaa ggacagcagc attgatcaat cttacagcta 120
acatgttgta cctggaaaac aatgcccaga ctcaatttag tgagccacag tacacgaacc 180
tggggctcct gaacagcatg gaccagcaga ttcagaacgg ctcctcgtcc accagtccct 240
ataacacaga ccacgcgcag aacagcgtca cggcgccctc gccctacgca cagcccagct 300
ccaccttcga tgctctctct ccatcacccg ccatcccctc caacaccgac tacccaggcc 360
cgcacagttt cgacgtgtcc ttccagcagt cgagcaccgc caagtcggcc acctggacgt 420
attccactga actgaagaaa ctctactgcc aaattgcaaa gacatgcccc atccagatca 480
aggtgatgac cccacctcct cagggagctg ttatccgcgc catgcctgtc tacaaaaaag 540
ctgagcacgt cacggaggtg gtgaagcggt gccccaacca tgagctgagc cgtgaattca 600
acgagggaca gattgcccct yctagtcatt tgattcgagt agaggggaac agccatgccc 660
agtatgtaga agatcccatc acaggaagac agagtgtgct ggtaccttat gagccacccc 720
aggttggcac tgaattcacg acagtcttgt acaatttcat gtgtaacagc agttgtgttg 780
gagggatgaa ccgccgtcca attttaatca ttgttactct ggaaaccaga gatgggcaag 840
tcctgggccg acgctgcttt gaggcccgga tctgtgcttg cccaggaaga gacaggaagg 900
cggatgaaga tagcatcaga aagcagcaag tttcggacag tacaaagaac ggtgatggta 960
cgaagcgccc gtttcgtcag aacacacatg gtatccagat gacatccatc aagaaacgaa 1020
gatccccaga tgatgaactg gtatacttac cagtgagggg ccgtgagact tatgaaatgc 1080
tggtgaagat caaagagtcc ctggaactca tgcagtacct tcttcagcac acaattgaaa 1140
cgtacaggca acagcaacag cagcagcacc agcacttact tcagaaacag acctcaatac 1200
agtctccatc ttcatatggt aacagctccc cacctctgaa caaaatgaac agcatgaaca 1260
agctgccttc tgtgagccag cttatcaacc ctcagcagcg caacgccctc actcctacaa 1320
ccattcctga tggcatggga gccaacattc ccatgatggg cacccacatg ccaatggctg 1380

gagacatgaa tggactcagc cccacccagg cactccctcc cccactctcc atgccatcca 1440
cctcccactg cacaccccca cctccgtatc ccacagattg cagcattgtc agtttcttag 1500
cgaggttggg ctgttcatca tgtctggact atttcacgac ccaggggctg accaccatct 1560
atcagattga gcattactcc atggatgatc tggcaagtct gaaaatccct gagcaatttc 1620
gacatgcgat ctggaagggc atcctggacc accggcagct ccacgaattc tcctcccctt 1680
ctcatctcct gcggacccca agcagtgcct ctacagtcag tgtgggctcc agtgagaccc 1740
ggggtgagcg tgttattgat gctgtgcgat tcaccctccg ccagaccatc tctttcccac 1800
cccgagatga gtggaatgac ttcaactttg acatggatgc tcgccgcaat aagcaacagc 1860
gcatcaaaga ggagggggag tgagcctcac catgtgagct cttcctatcc ctctcctaac 1920
tgccagcccc ctaaaagcac tcctgcttaa tcttcaaagc cttctcccta gctcctcccc 1980
ttcctcttgt ctgatttctt aggggaagga gaagtaagag gcttacttct taccctaacc 2040
atctgacctg gcatctaatt ctgattctgg ctttaagcct tcaaaactat agcttgcaga 2100
actgtagctt gccatggcta ggtagaagtg agcaaaaaag agttgggtgt ctccttaagc 2160
tgcagagatt tctcattgac ttttataaag catgttcacc cttatagtct aagactatat 2220
atataaatgt ataaatatac agtatagatt tttgggtggg ggcattgagt tattgtttaa 2280
aatgtaattt aaatgaaaga aaattgagtt gcacttattg accatttttt aatttacttg 2340
ttttggatgg cttgtctata ctccttccct taaggggtat catgtatggt gataggtatc 2400
tagagcttaa tgctacatgt gagtgacgat gatgtacaga ttctttcagt tctttggatt 2460
ctaaatacat gccacatcaa acctttgagt agatccattt ccattgctta ttatgtaggt 2520
aagactgtag atatgtattc ttttctcagt gttggtatat tttatattac tgacatttct 2580
tctagtgatg atggttcacg ttggggtgat ttaatccagt tataagaaga agttcatgtc 2640
caaacgtcct ctttagtttt tggttgggaa tgaggaaaat tcttaaaagg cccatagcag 2700
ccagttcaaa aacacccgac gtcatgtatt tgagcatatc agtaaccccc ttaaatttaa 2760
taccagatac cttatcttac aatattgatt gggaaaacat ttgctgccat tacagaggta 2820
ttaaaactaa atttcactac tagattgact aactcaaata cacatttgct actgttgtaa 2880
gaattctgat tgatttgatt gggatgaatg ccatctatct agttctaaca gtgaagtttt 2940
actgtctatt aatattcagg gtaaatagga atcattcaga aatgttgagt ctgtactaaa 3000
cagtaagata tctcaatgaa ccataaattc aactttgtaa aaatcttttg aagcatagat 3060
aatattgttt ggtaaatgtt tcttttgttt ggtaaatgtt tcytttaaag accctcctat 3120
tctataaaac tctgcatgta gaggcttgtt tacctttctc tctctaaggt ttacaatagg 3180
agtggtgatt tgaaaaatat aaaattatga gattggtttt cctgtggcat aaattgcatc 3240
actgtatcat ttttcttttt aaccggtaag agtttcagtt tgttggaaag taactgtgag 3300
aacccagttt cccgtccatc tcccttaggg actacccata gacatgaaag gtccccacag 3360
agcaagagat aagtctttca tggctgctgt tgcttaaacc acttaaacga agagttccct 3420
tgaaactttg ggaaaacatg ttaatgacaa tattccagat ctttcagaaa tataacacat 3480
ttttttgcat gcatgcaaat gagctctgaa atcttcccat gcattctggt caagggctgt 3540
cattgcacat aagcttccat ttttaattta aagtgcaaaa gggccagcgt ggctctaaaa 3600
ggtaatgtgt ggattgcctc tgaaaagtgt gtatatattt tgtgtgaaat tgcatacttt 3660
gtattttgat tatttttttt ttcttcttgg gatagtggga tttccagaac cacacttgaa 3720
accttttttt atcgtttttg tattttcatg aaaataccat ttagtaagaa taccacatca 3780
aataagaaat aatgctacaa ttttaagagg ggagggaagg gaaagttttt ttttttatta 3840
tttttttaaa attttgtatg ttaaagagaa tgagtccttg atttcaaagt tttgttgtac 3900
ttaaatggta ataagcactg taaacttctg caacaagcat gcagctttgc aaacccatta 3960
aggggaagaa tgaaagctgt tccttggtcc tagtaagaag acaaactgct tcccttactt 4020
tgctgagggt ttgaataaac ctaggacttc cgagctatgt cagtactatt caggtaacac 4080
tagggccttg gaaatccctg tactgtgtct catggatttg gcactagcca aagcgaggca 4140
ccccttactg gcttacctcc tcatggcagc ctactctcct tgagtgtatg agtagccagg 4200
gtaaggggta aaaggatagt aagcatagaa accactagaa agtggcttaa atggagttct 4260
tgtggcctca gctcaatgca gttagctgaa gaattgaaaa gtttttgttt ggagacgttt 4320
ataaacagaa atggaaagca gagttttcat taaatccttt tacctttttt ttttcttggt 4380
aatcccctaa aataacagta tgtgggatat tgaatgttaa agggatattt ttttctatta 4440
tttttataat tgtacaaaat taagcaaatg ttaaaagttt tatatgcttt attaatgttt 4500
tcaaaaggta ttatacatgt gatacatttt ttaagcttca gttgcttgtc ttctggtact 4560
ttctgttatg ggcttttggg gagccagaag ccaatctaca atctcttttt gtttgccagg 4620
acatgcaata aaatttaaaa aataaataaa aacta 4655

<210> 152
<211> 586
<212> PRT
<213> Homo sapien

<400> 152
Met Leu Tyr Leu Glu Asn Asn Ala Gln Thr Gln Phe Ser Glu Pro Gln
1               5                   10                  15
Tyr Thr Asn Leu Gly Leu Leu Asn Ser Met Asp Gln Gln Ile Gln Asn
            20                  25                  30
Gly Ser Ser Ser Thr Ser Pro Tyr Asn Thr Asp His Ala Gln Asn Ser
        35                  40                  45
Val Thr Ala Pro Ser Pro Tyr Ala Gln Pro Ser Ser Thr Phe Asp Ala
    50                  55                  60
Leu Ser Pro Ser Pro Ala Ile Pro Ser Asn Thr Asp Tyr Pro Gly Pro
65                  70                  75                  80
His Ser Phe Asp Val Ser Phe Gln Gln Ser Ser Thr Ala Lys Ser Ala
                85                  90                  95
Thr Trp Thr Tyr Ser Thr Glu Leu Lys Lys Leu Tyr Cys Gln Ile Ala
            100                 105                 110
Lys Thr Cys Pro Ile Gln Ile Lys Val Met Thr Pro Pro Pro Gln Gly
        115                 120                 125
Ala Val Ile Arg Ala Met Pro Val Tyr Lys Lys Ala Glu His Val Thr
    130                 135                 140
Glu Val Val Lys Arg Cys Pro Asn His Glu Leu Ser Arg Glu Phe Asn
145                 150                 155                 160
Glu Gly Gln Ile Ala Pro Ser Ser His Leu Ile Arg Val Glu Gly Asn
                165                 170                 175
Ser His Ala Gln Tyr Val Glu Asp Pro Ile Thr Gly Arg Gln Ser Val
            180                 185                 190
Leu Val Pro Tyr Glu Pro Pro Gln Val Gly Thr Glu Phe Thr Thr Val
        195                 200                 205
Leu Tyr Asn Phe Met Cys Asn Ser Ser Cys Val Gly Gly Met Asn Arg
    210                 215                 220
Arg Pro Ile Leu Ile Ile Val Thr Leu Glu Thr Arg Asp Gly Gln Val
225                 230                 235                 240
Leu Gly Arg Arg Cys Phe Glu Ala Arg Ile Cys Ala Cys Pro Gly Arg
                245                 250                 255
Asp Arg Lys Ala Asp Glu Asp Ser Ile Arg Lys Gln Gln Val Ser Asp
            260                 265                 270
Ser Thr Lys Asn Gly Asp Gly Thr Lys Arg Pro Phe Arg Gln Asn Thr
        275                 280                 285
His Gly Ile Gln Met Thr Ser Ile Lys Lys Arg Arg Ser Pro Asp Asp
    290                 295                 300
Glu Leu Val Tyr Leu Pro Val Arg Gly Arg Glu Thr Tyr Glu Met Leu
305                 310                 315                 320
Val Lys Ile Lys Glu Ser Leu Glu Leu Met Gln Tyr Leu Leu Gln His
                325                 330                 335
Thr Ile Glu Thr Tyr Arg Gln Gln Gln Gln Gln Gln His Gln His Leu
            340                 345                 350
Leu Gln Lys Gln Thr Ser Ile Gln Ser Pro Ser Ser Tyr Gly Asn Ser
        355                 360                 365
Ser Pro Pro Leu Asn Lys Met Asn Ser Met Asn Lys Leu Pro Ser Val
    370                 375                 380

Ser Gln Leu Ile Asn Pro Gln Gln Arg Asn Ala Leu Thr Pro Thr Thr
385                 390                 395                 400
Ile Pro Asp Gly Met Gly Ala Asn Ile Pro Met Met Gly Thr His Met
                405                 410                 415
Pro Met Ala Gly Asp Met Asn Gly Leu Ser Pro Thr Gln Ala Leu Pro
            420                 425                 430
Pro Pro Leu Ser Met Pro Ser Thr Ser His Cys Thr Pro Pro Pro Pro
        435                 440                 445
Tyr Pro Thr Asp Cys Ser Ile Val Ser Phe Leu Ala Arg Leu Gly Cys
    450                 455                 460
Ser Ser Cys Leu Asp Tyr Phe Thr Thr Gln Gly Leu Thr Thr Ile Tyr
465                 470                 475                 480
Gln Ile Glu His Tyr Ser Met Asp Asp Leu Ala Ser Leu Lys Ile Pro
                485                 490                 495
Glu Gln Phe Arg His Ala Ile Trp Lys Gly Ile Leu Asp His Arg Gln
            500                 505                 510
Leu His Glu Phe Ser Ser Pro Ser His Leu Leu Arg Thr Pro Ser Ser
        515                 520                 525
Ala Ser Thr Val Ser Val Gly Ser Ser Glu Thr Arg Gly Glu Arg Val
    530                 535                 540
Ile Asp Ala Val Arg Phe Thr Leu Arg Gln Thr Ile Ser Phe Pro Pro
545                 550                 555                 560
Arg Asp Glu Trp Asn Asp Phe Asn Phe Asp Met Asp Ala Arg Arg Asn
                565                 570                 575
Lys Gln Gln Arg Ile Lys Glu Glu Gly Glu
            580                 585

<210> 153
<211> 2007
<212> DNA
<213> Homo sapien

<400> 153
gaattcgtcg ctgctccagg gaaagttctg ttactccact gactctctct tttcctgata      60
acatggccag caagaaagta attacagtgt ttggagcaac aggagctcaa ggtggctctg     120
tggccagggc aattttggag agcaaaaaat ttgcagtgag agcagtgacc agggatgtga     180
cttgaccaaa tgccctggag ctccagcgcc ttggagctga ggtggtcaaa ggtgacctga     240
atgataaagc atcggtggac agtgccttaa aaggtgtcta tggggccttc ttggtgacca     300
acttctggga ccctctcaac caagataagg aagtgtgtcg ggggaagctg gtggcagact     360
ccgccaagca cctgggtctg aagcacgtgg tgtacagcgg cctggagaac gtcaagcgac     420
tgacggatgg caagctggag gtgccgcact ttgacagcaa gggcgaggtg gaggagtact     480
tctggtccat tggcatcccc atgaccagtg tccgcgtggc ggcctacttt gaaaactttc     540
tcgcggcgtg gcggcccgtg aaagcctctg atggagatta ctacaccttg gctgtaccga     600
tgggagatgt accaatggat ggtatctctg ttgctgatat tggagcagcc gtctctagca     660
tttttaattc tccagaggaa tttttaggca aggccgtggg gctcagtgca gaagcactaa     720
caatacagca atatgctgat gttttgtcca aggctttggg gaaagaagtc cgagatgcaa     780
agattacccc ggaagctttc gagaagctgg gattccctgc agcaaaggaa atagccaata     840
tgtgtcgttt ctatgaaatg aagccagacc gagatgtcaa tctcacccac caactaaatc     900
ccaaagtcaa aagcttcagc cagtttatct cagagaacca gggagccttc aagggcatgt     960
agaaaatcag ctgttcagat aggcctctgc accacacagc ctctttcctc tctgatcctt    1020
ttcctcttta cggcacaaca ttcatgttga cagaacatgc tggaatgcaa ttgtttgcaa    1080
caccgaagga tttcctgcgg tcgcctcttc agtaggaagc actgcattgg tgataggaca    1140
cggtaatttg attcacattt aacttgctag ttagtgataa gggtggtaca actgtttggt    1200
aaaatgagaa gcctcggaac ttggagcttc tctcctacca ctaatgggag ggcagattat    1260
actgggattt ctcctgggtg agtaatttca agccctaatg ctgaaattcc cctaggcagc    1320

tccagttttc tcaactgcat tgcaaaattc ccagtgaact tttaagtact tttaacttaa 1380
aaaaatgaac atctttgtag agaattttct ggggaacatg gtgttcaatg aacaagcaca 1440
agcattggaa atgctaaaat tcagttttgc ctcaagattg gaagtttatt ttctgactca 1500
ttcatgaagt catctattga gccaccattc aattattcat ctattaattc cttgatcctt 1560
catttatcca ttctgcaaac ttttcttgag caccagcacg ggtggccatt tgtggacttc 1620
tcttcattcc tatgtgtttt cttatcaaag tgatccactc tcgaaaggct cctttccagt 1680
ctgtggttgg gttcaagtca tgccagggcc aggggcccca tctcctcgtt tagctctagg 1740
caaaatccag gggatctgca gtggggagcg ggggcaggaa gctggaggga aggcctgtga 1800
agggtaggga tgtggaaaga caaggtgaca gaaggaccca ataggacctt tctatatctc 1860
tggcttagca ttttctacat catattgtaa tcgtcttatt tgctagtttt cttccttact 1920
gtgagtgact aacagtcatc tttatcccag tgcctggtac ataataagtg atcaataaat 1980
gttgattgac taaaaaaaaa aaaaaaa 2007

<210> 154
<211> 2148
<212> DNA
<213> Homo sapien

<400> 154
gaattcgtcg ctgctccagg gaaagttctg ttactccact gactctctct tttcctgata 60
acatggccag caagaaagta attacagtgt ttggagcaac aggagctcaa ggtggctctg 120
tggccagggc aattttggag agcaaaaaat ttgcagtgag agcagtgacc agggatgtga 180
cttgaccaaa tgccctggag ctccagcgcc ttggagctga ggtggtcaaa ggtgacctga 240
atgataaagc atcggtggac agtgccttaa aaggggaagc tggtggcaga ctccgccaag 300
cacctgggtc tgaagcacgt ggtgtacagc ggcctggaga acgtcaagcg actgacggat 360
ggcaagctgg aggtgccgca ctttgacagc aagggcgagg tggaggagta cttctggtcc 420
attggcatcc ccatgaccag tgtccgcgtg gcggcctact ttgaaaactt tctcgcggcg 480
tggcggcccg tgaaagcctc tgatggagat tactacacct tggctgtacc gatgggagat 540
gtaccaatgg atggtatctc tgttgctgat attggagcag ccgtctctag catttttaat 600
tctccagagg aatttttagg caaggccgtg gggctcagtg cagaagcact aacaatacag 660
caatatgctg atgttttgtc caaggctttg gggaaagaag tccgagatgc aaagactatc 720
tgtgctatag atgaccagaa aacagtggaa gaaggtttca tggaagacgt gggcttgagt 780
tggtccttga gggaacatga ccatgtatag acagaggagg catcaagaag gctggcctgg 840
ctaattctgg aataaacacg acaaaccaga ggcagtacgg gaaggaggca aattctggct 900
ctgcctctat ccttgattac cccggaagct ttcgagaagc tgggattccc tgcagcaaag 960
gaaatagcca atatgtgtcg tttctatgaa atgaagccag accgagatgt caatctcacc 1020
caccaactaa atcccaaagt caaaagcttc agccatttta tctcagagaa ccagggagcc 1080
ttcaagggca tgtagaaaat cagctgttca gataggcctc tgcaccacac agcctctttc 1140
ctctctgatc cttttcctct ttacggcaca acattcatgt tgacagaaca tgctggaatg 1200
caattgtttg caacaccgaa ggatttcctg cggtcgcctc ttcagtagga agcactgcat 1260
tggtgatagg acacggtaat ttgattcaca tttaacttgc tagttagtga taagggtggt 1320
acaactgttt ggtaaaatga gaagcctcgg aacttggagc ttctctccta ccactaatgg 1380
gagggcagat tatactggga tttctcctgg gtgagtaatt tcaagcccta atgctgaaat 1440
tcccctaggc agctccagtt ttctcaactg cattgcaaaa ttcccagtga acttttaagt 1500
acttttaact taaaaaaatg aacatctttg tagagaattt tctggggaac atggtgttca 1560
atgaacaagc acaagcattg gaaatgctaa aattcagttt tgcctcaaga ttggaagttt 1620
attttctgac tcattcatga agtcatctat tgagccacca ttcaattatt catctattaa 1680
ttccttgatc cttcatttat ccattctgca aacttttctt gagcaccagc acgggtggcc 1740
atttgtggac ttctcttcat tcctatgtgt tttcttatca aagtgatcca ctctcgaaag 1800
gctcctttcc agtctgtggt tgggttcaag tcatgccagg gccaggggcc ccatctcctc 1860
gtttagctct aggcaaaatc caggggatct gcagtgggga gcgggggcag gaagctggag 1920
ggaaggcctg tgaagggtag ggatgtggaa agacaaggtg acagaaggac ccaataggac 1980
ctttctatat ctctggctta gcattttcta catcatattg taatcgtctt atttgctagt 2040
tttcttcctt actgtgagtg actaacagtc atctttatcc cagtgcctgg tacataataa 2100
gtgatcaata aatgttgatt gactaaatga aaaaaaaaaa aaaaaaa 2148

<210> 155
<211> 153
<212> PRT
<213> Homo sapien

<400> 155
Met Thr Ser Val Arg Val Ala Ala Tyr Phe Glu Asn Phe Leu Ala Ala
1               5                   10                  15
Trp Arg Pro Val Lys Ala Ser Asp Gly Asp Tyr Tyr Thr Leu Ala Val
            20                  25                  30
Pro Met Gly Asp Val Pro Met Asp Gly Ile Ser Val Ala Asp Ile Gly
        35                  40                  45
Ala Ala Val Ser Ser Ile Phe Asn Ser Pro Glu Glu Phe Leu Gly Lys
    50                  55                  60
Ala Val Gly Leu Ser Ala Glu Ala Leu Thr Ile Gln Gln Tyr Ala Asp
65                  70                  75                  80
Val Leu Ser Lys Ala Leu Gly Lys Glu Val Arg Asp Ala Lys Ile Thr
                85                  90                  95
Pro Glu Ala Phe Glu Lys Leu Gly Phe Pro Ala Ala Lys Glu Ile Ala
            100                 105                 110
Asn Met Cys Arg Phe Tyr Glu Met Lys Pro Asp Arg Asp Val Asn Leu
        115                 120                 125
Thr His Gln Leu Asn Pro Lys Val Lys Ser Phe Ser Gln Phe Ile Ser
    130                 135                 140
Glu Asn Gln Gly Ala Phe Lys Gly Met
145                 150

<210> 156
<211> 128
<212> PRT
<213> Homo sapien

<400> 156
Met Thr Ser Val Arg Val Ala Ala Tyr Phe Glu Asn Phe Leu Ala Ala
1               5                   10                  15
Trp Arg Pro Val Lys Ala Ser Asp Gly Asp Tyr Tyr Thr Leu Ala Val
            20                  25                  30
Pro Met Gly Asp Val Pro Met Asp Gly Ile Ser Val Ala Asp Ile Gly
        35                  40                  45
Ala Ala Val Ser Ser Ile Phe Asn Ser Pro Glu Glu Phe Leu Gly Lys
    50                  55                  60
Ala Val Gly Leu Ser Ala Glu Ala Leu Thr Ile Gln Gln Tyr Ala Asp
65                  70                  75                  80
Val Leu Ser Lys Ala Leu Gly Lys Glu Val Arg Asp Ala Lys Thr Ile
                85                  90                  95
Cys Ala Ile Asp Asp Gln Lys Thr Val Glu Glu Gly Phe Met Glu Asp
            100                 105                 110
Val Gly Leu Ser Trp Ser Leu Arg Glu His Asp His Val Ala Gly Ala
        115                 120                 125

<210> 157
<211> 424
<212> DNA
<213> Homo sapien

<220>
<221> misc_feature
<222> (1)...(424)
<223> n = A,T,C or G

<400> 157
ctgcagcccg ggggatccac tagtccagtg tggtggaatt cattggtctt tacaagactt 60
ggatacatta cagcagacat ggaaatataa ttttaaaaaa tttctctcca acctccttca 120
aattcagtca ccactgttat attaccttct ccaggaaccc tccagtgggg aaggctgcga 180
tattagattt ccttgtatgc aaagtttttg ttgaaagctg tgctcagagg aggtgagagg 240
agaggaagga gaaaactgca tcataacttt acagaattga atctagagtc ttccccgaaa 300
agcccagaaa cttctctgcn gnatctggct tgtccatctg gtctaaggtg gctgcttctt 360
ccccagccat cgagtcagtt tgtgcccatg aataatacac gacctgctat ttcccatgac 420
tgct 424

<210> 158
<211> 2099
<212> DNA
<213> Homo sapien

<400> 158
ccgcggttaa aaggcgcagc aggtgggagc cggggccttc acccgaaacc cgacgagagc 60
ccgacagccg gcggcgcccg agcccgacct gcctgcccag ccggagcgaa gggcgccgcc 120
ccgcgcagag cccgcgccag ggccgccggc cgcagagcag ttaaaacgtg caggcaccag 180
aaggcacttc ctgtcggtga agaagacctg tctccggtgt cacgggcatc ctgtgttttg 240
caaacggggc tgacctccct tcctggggag caggaagggt cagggaagga aaagaagtac 300
agaagatctg gctaaacaat ttctgtatgg cgaaagaaaa attctaactt gtacgccctc 360
ttcatgcatc tttaattcaa tttgaatatt ccaggcgaca tcctcactga ccgagcaaag 420
attgacattc gtatcatcac tgtgcaccat tggcttctag gcactccagt ggggtaggag 480
aaggaggtct gaaaccctcg cagagggatc ttgccctcat tctttgggtc tgaaacactg 540
gcagtcgttg gaaacaggac tcagggataa accagcgcaa tggattgggg gacgctgcac 600
actttcatcg ggggtgtcaa caaacactcc accagcatcg ggaaggtgtg gatcacagtc 660
atctttattt tccgagtcat gatcctcgtg gtggctgccc aggaagtgtg gggtgacgag 720
caagaggact tcgtctgcaa cacactgcaa ccgggatgca aaaatgtgtg ctatgaccac 780
tttttcccgg tgtcccacat ccggctgtgg gccctccagc tgatcttcgt ctccacccca 840
gcgctgctgg tggccatgca tgtggcctac tacaggcacg aaaccactcg caagttcagg 900
cgaggagaga agaggaatga tttcaaagac atagaggaca ttaaaaagca gaaggttcgg 960
atagaggggt cgctgtggtg gacgtacacc agcagcatct ttttccgaat catctttgaa 1020
gcagccttta tgtatgtgtt ttacttcctt tacaatgggt accacctgcc ctgggtgttg 1080
aaatgtggga ttgacccctg ccccaacctt gttgactgct ttatttctag gccaacagag 1140
aagaccgtgt ttaccatttt tatgatttct gcgtctgtga tttgcatgct gcttaacgtg 1200
gcagagttgt gctacctgct gctgaaagtg tgttttagga gatcaaagag agcacagacg 1260
caaaaaaatc accccaatca tgccctaaag gagagtaagc agaatgaaat gaatgagctg 1320
atttcagata gtggtcaaaa tgcaatcaca ggttcccaag ctaaacattt caaggtaaaa 1380
tgtagctgcg tcataaggag acttctgtct tctccagaag gcaataccaa cctgaaagtt 1440
ccttctgtag cctgaagagt ttgtaaatga ctttcataat aaatagacac ttgagttaac 1500
tttttgtagg atacttgctc cattcataca caacgtaatc aaatatgtgg tccatctctg 1560
aaaacaagag actgcttgac aaaggagcat tgcagtcact ttgacaggtt ccttttaagt 1620
ggactctctg acaaagtggg tactttctga aaatttatat aactgttgtt gataaggaac 1680
atttatccag gaattgatac gtttattagg aaaagatatt tttataggct tggatgtttt 1740
tagttctgac tttgaattta tataaagtat ttttataatg actggtcttc cttacctgga 1800
aaaacatgcg atgttagttt tagaattaca ccacaagtat ctaaatttgg aacttacaaa 1860
gggtctatct tgtaaatatt gttttgcatt gtctgttggc aaatttgtga actgtcatga 1920
tacgcttaag gtggaaagtg ttcattgcac aatatatttt tactgctttc tgaatgtaga 1980

cggaacagtg tggaagcaga aggctttttt aactcatccg tttgccaatc attgcaaaca 2040
actgaaatgt ggatgtgatt gcctcaataa agctcgtccc cattgcttaa aaaaaaaaa 2099

<210> 159
<211> 291
<212> PRT
<213> Homo sapien

<400> 159
Met Asp Trp Gly Thr Leu His Thr Phe Ile Gly Gly Val Asn Lys His
1               5                   10                  15
Ser Thr Ser Ile Gly Lys Val Trp Ile Thr Val Ile Phe Ile Phe Arg
            20                  25                  30
Val Met Ile Leu Val Val Ala Ala Gln Glu Val Trp Gly Asp Glu Gln
        35                  40                  45
Glu Asp Phe Val Cys Asn Thr Leu Gln Pro Gly Cys Lys Asn Val Cys
    50                  55                  60
Tyr Asp His Phe Phe Pro Val Ser His Ile Arg Leu Trp Ala Leu Gln
65                  70                  75                  80
Leu Ile Phe Val Ser Thr Pro Ala Leu Leu Val Ala Met His Val Ala
                85                  90                  95
Tyr Tyr Arg His Glu Thr Thr Arg Lys Phe Arg Arg Gly Glu Lys Arg
            100                 105                 110
Asn Asp Phe Lys Asp Ile Glu Asp Ile Lys Lys Gln Lys Val Arg Ile
        115                 120                 125
Glu Gly Ser Leu Trp Trp Thr Tyr Thr Ser Ser Ile Phe Phe Arg Ile
    130                 135                 140
Ile Phe Glu Ala Ala Phe Met Tyr Val Phe Tyr Phe Leu Tyr Asn Gly
145                 150                 155                 160
Tyr His Leu Pro Trp Val Leu Lys Cys Gly Ile Asp Pro Cys Pro Asn
                165                 170                 175
Leu Val Asp Cys Phe Ile Ser Arg Pro Thr Glu Lys Thr Val Phe Thr
            180                 185                 190
Ile Phe Met Ile Ser Ala Ser Val Ile Cys Met Leu Leu Asn Val Ala
        195                 200                 205
Glu Leu Cys Tyr Leu Leu Leu Lys Val Cys Phe Arg Arg Ser Lys Arg
    210                 215                 220
Ala Gln Thr Gln Lys Asn His Pro Asn His Ala Leu Lys Glu Ser Lys
225                 230                 235                 240
Gln Asn Glu Met Asn Glu Leu Ile Ser Asp Ser Gly Gln Asn Ala Ile
                245                 250                 255
Thr Gly Ser Gln Ala Lys His Phe Lys Val Lys Cys Ser Cys Val Ile
            260                 265                 270
Arg Arg Leu Leu Ser Ser Pro Glu Gly Asn Thr Asn Leu Lys Val Pro
        275                 280                 285
Ser Val Ala
    290

<210> 160
<211> 3951
<212> DNA
<213> Homo sapien

<400> 160
tctgcatcca tattgaaaac ctgacacaat gtatgcagca ggctcagtgt gagtgaactg 60

gaggcttctc tacaacatga cccaaaggag cattgcaggt cctatttgca acctgaagtt 120
tgtgactctc ctggttgcct taagttcaga actcccattc ctgggagctg gagtacagct 180
tcaagacaat gggtataatg gattgctcat tgcaattaat cctcaggtac ctgagaatca 240
gaacctcatc tcaaacatta aggaaatgat aactgaagct tcattttacc tatttaatgc 300
taccaagaga agagtatttt tcagaaatat aaagatttta atacctgcca catggaaagc 360
taataataac agcaaaataa aacaagaatc atatgaaaag gcaaatgtca tagtgactga 420
ctggtatggg gcacatggag atgatccata caccctacaa tacagagggt gtggaaaaga 480
gggaaaatac attcatttca cacctaattt cctactgaat gataacttaa cagctggcta 540
cggatcacga ggccgagtgt ttgtccatga atgggcccac ctccgttggg gtgtgttcga 600
tgagtataac aatgacaaac ctttctacat aaatgggcaa aatcaaatta aagtgacaag 660
gtgttcatct gacatcacag gcatttttgt gtgtgaaaaa ggtccttgcc cccaagaaaa 720
ctgtattatt agtaagcttt ttaaagaagg atgcaccttt atctacaata gcacccaaaa 780
tgcaactgca tcaataatgt tcatgcaaag tttatcttct gtggttgaat tttgtaatgc 840
aagtacccac aaccaagaag caccaaacct acagaaccag atgtgcagcc tcagaagtgc 900
atgggatgta atcacagact ctgctgactt tcaccacagc tttcccatga acgggactga 960
gcttccacct cctcccacat tctcgcttgt agaggctggt gacaaagtgg tctgtttagt 1020
gctggatgtg tccagcaaga tggcagaggc tgacagactc cttcaactac aacaagccgc 1080
agaattttat ttgatgcaga ttgttgaaat tcataccttc gtgggcattg ccagtttcga 1140
cagcaaagga gagatcagag cccagctaca ccaaattaac agcaatgatg atcgaaagtt 1200
gctggtttca tatctgccca ccactgtatc agctaaaaca gacatcagca tttgttcagg 1260
gcttaagaaa ggatttgagg tggttgaaaa actgaatgga aaagcttatg gctctgtgat 1320
gatattagtg accagcggag atgataagct tcttggcaat tgcttaccca ctgtgctcag 1380
cagtggttca acaattcact ccattgccct gggttcatct gcagccccaa atctggagga 1440
attatcacgt cttacaggag gtttaaagtt ctttgttcca gatatatcaa actccaatag 1500
catgattgat gctttcagta gaatttcctc tggaactgga gacattttcc agcaacatat 1560
tcagcttgaa agtacaggtg aaaatgtcaa acctcaccat caattgaaaa acacagtgac 1620
tgtggataat actgtgggca acgacactat gtttctagtt acgtggcagg ccagtggtcc 1680
tcctgagatt atattatttg atcctgatgg acgaaaatac tacacaaata attttatcac 1740
caatctaact tttcggacag ctagtctttg gattccagga acagctaagc ctgggcactg 1800
gacttacacc ctgaacaata cccatcattc tctgcaagcc ctgaaagtga cagtgacctc 1860
tcgcgcctcc aactcagctg tgcccccagc cactgtggaa gcctttgtgg aaagagacag 1920
cctccatttt cctcatcctg tgatgattta tgccaatgtg aaacagggat tttatcccat 1980
tcttaatgcc actgtcactg ccacagttga gccagagact ggagatcctg ttacgctgag 2040
actccttgat gatggagcag gtgctgatgt tataaaaaat gatggaattt actcgaggta 2100
ttttttctcc tttgctgcaa atggtagata tagcttgaaa gtgcatgtca atcactctcc 2160
cagcataagc accccagccc actctattcc agggagtcat gctatgtatg taccaggtta 2220
cacagcaaac ggtaatattc agatgaatgc tccaaggaaa tcagtaggca gaaatgagga 2280
ggagcgaaag tggggcttta gccgagtcag ctcaggaggc tccttttcag tgctgggagt 2340
tccagctggc ccccaccctg atgtgtttcc accatgcaaa attattgacc tggaagctgt 2400
aaaagtagaa gaggaattga ccctatcttg gacagcacct ggagaagact ttgatcaggg 2460
ccaggctaca agctatgaaa taagaatgag taaaagtcta cagaatatcc aagatgactt 2520
taacaatgct attttagtaa atacatcaaa gcgaaatcct cagcaagctg gcatcaggga 2580
gatatttacg ttctcacccc aaatttccac gaatggacct gaacatcagc caaatggaga 2640
aacacatgaa agccacagaa tttatgttgc aatacgagca atggatagga actccttaca 2700
gtctgctgta tctaacattg cccaggcgcc tctgtttatt ccccccaatt ctgatcctgt 2760
acctgccaga gattatctta tattgaaagg agttttaaca gcaatgggtt tgataggaat 2820
catttgcctt attatagttg tgacacatca tactttaagc aggaaaaaga gagcagacaa 2880
gaaagagaat ggaacaaaat tattataaat aaatatccaa agtgtcttcc ttcttagata 2940
taagacccat ggccttcgac tacaaaaaca tactaacaaa gtcaaattaa catcaaaact 3000
gtattaaaat gcattgagtt tttgtacaat acagataaga tttttacatg gtagatcaac 3060
aaattctttt tgggggtaga ttagaaaacc cttacacttt ggctatgaac aaataataaa 3120
aattattctt taaagtaatg tctttaaagg caaagggaag ggtaaagtcg gaccagtgtc 3180
aaggaaagtt tgttttattg aggtggaaaa atagccccaa gcagagaaaa ggagggtagg 3240
tctgcattat aactgtctgt gtgaagcaat catttagtta ctttgattaa tttttctttt 3300
ctccttatct gtgcagaaca ggttgcttgt ttacaactga agatcatgct atatttcata 3360

tatgaagccc ctaatgcaaa gctctttacc tcttgctatt ttgttatata tattacagat 3420
gaaatctcac tgctaatgct cagagatctt ttttcactgt aagaggtaac ctttaacaat 3480
atgggtatta cctttgtctc ttcataccgg ttttatgaca aaggtctatt gaatttattt 3540
gtttgtaagt ttctactccc atcaaagcag ctttttaagt tattgccttg gttattatgg 3600
atgatagtta tagcccttat aatgccttaa ctaaggaaga aaagatgtta ttctgagttt 3660
gttttaatac atatatgaac atatagtttt attcaattaa accaaagaag aggtcagcag 3720
ggagatacta acctttggaa atgattagct ggctctgttt tttggttaaa taagagtctt 3780
taatcctttc tccatcaaga gttacttacc aagggcaggg gaagggggat atagaggtcc 3840
caaggaaata aaaatcatct ttcatcttta attttactcc ttcctcttat ttttttaaaa 3900
gattatcgaa caataaaatc atttgccttt ttaattaaaa acataaaaaa a 3951

<210> 161
<211> 943
<212> PRT
<213> Homo sapien

<400> 161

<210> 162
<211> 498
<212> DNA
<213> Homo sapien

<400> 162
tggagaacca cgtggacagc accatgaaca tgttgggcgg gggaggcagt gctggccgga 60
agcccctcaa gtcgggtatg aaggagctgg ccgtgttccg ggagaaggtc actgagcagc 120
accggcagat gggcaagggt ggcaagcatc accttggcct ggaggagccc aagaagctgc 180
gaccaccccc tgccaggact ccctgccaac aggaactgga ccaggtcctg gagcggatct 240
ccaccatgcg ccttccggat gagcggggcc ctctggagca cctctactcc ctgcacatcc 300
ccaactgtga caagcatggc ctgtacaacc tcaaacagtg gcaagatgtc tctgaacggg 360
cagcgtgggg agtgctggtg tgtgaacccc aacaccggga agctgatcca gggagccccc 420
accatccggg gggaccccga gtgtcatctc ttctacaatg agcagcagga ggctcgcggg 480
gtgcacaccc cagcggat 498

<210> 163
<211> 1128
<212> DNA
<213> Homo sapien

<400> 163
gccacctggc cctcctgatc gacgacacac gcacttgaaa cttgttctca gggtgtgtgg 60
aatcaacttt ccggaagcaa ccagcccacc agaggaggtc ccgagcgcga gcggagacga 120
tgcagcggag actggttcag cagtggagcg tcgcggtgtt cctgctgagc tacgcggtgc 180
cctcctgcgg gcgctcggtg gagggtctca gccgccgcct caaaagagct gtgtctgaac 240
atcagctcct ccatgacaag gggaagtcca tccaagattt acggcgacga ttcttccttc 300
accatctgat cgcagaaatc cacacagctg aaatcagagc tacctcggag gtgtccccta 360
actccaagcc ctctcccaac acaaagaacc accccgtccg atttgggtct gatgatgagg 420

gcagatacct aactcaggaa actaacaagg tggagacgta caaagagcag ccgctcaaga 480
cacctgggaa gaaaaagaaa ggcaagcccg ggaaacgcaa ggagcaggaa aagaaaaaac 540
ggcgaactcg ctctgcctgg ttagactctg gagtgactgg gagtggccta gaaggggacc 600
acctgtctga cacctccaca acgtcgctgg agctcgattc acggaggcat tgaaattttc 660
agcagagacc ttccaaggac atattgcagg attctgtaat agtgaacata tggaaagtat 720
tagaaatatt tattgtctgt aaatactgta aatgcattgg aataaaactg tctcccccat 780
tgctctatga aactgcacat tggtcattgt gaatattttt ttttttgcca aggctaatcc 840
aattattatt atcacattta ccataattta ttttgtccat tgatgtattt attttgtaaa 900
tgtatcttgg tgctgctgaa tttctatatt ttttgtaaca taatgcactt tagatataca 960
tatcaagtat gttgataaat gacacaatga agtgtctcta ttttgtggtt gattttaatg 1020
aatgcctaaa tataattatc caaattgatt ttcctttgtg catgtaaaaa taacagtatt 1080
ttaaatttgt aaagaatgtc taataaaata taatctaatt acatcatg 1128

<210> 164
<211> 1310
<212> DNA
<213> Homo sapien

<400> 164
gggcctggtt cgcaaagaag ctgacttcag agggggaaac tttcttcttt taggaggcgg 60
ttagccctgt tccacgaacc caggagaact gctggccaga ttaattagac attgctatgg 120
gagacgtgta aacacactac ttatcattga tgcatatata aaaccatttt attttcgcta 180
ttatttcaga ggaagcgcct ctgatttgtt tcttttttcc ctttttgctc tttctggctg 240
tgtggtttgg agaaagcaca gttggagtag ccggttgcta aataagtccc gagcgcgagc 300
ggagacgatg cagcggagac tggttcagca gtggagcgtc gcggtgttcc tgctgagcta 360
cgcggtgccc tcctgcgggc gctcggtgga gggtctcagc cgccgcctca aaagagctgt 420
gtctgaacat cagctcctcc atgacaaggg gaagtccatc caagatttac ggcgacgatt 480
cttccttcac catctgatcg cagaaatcca cacagctgaa atcagagcta cctcggaggt 540
gtcccctaac tccaagccct ctcccaacac aaagaaccac cccgtccgat ttgggtctga 600
tgatgagggc agatacctaa ctcaggaaac taacaaggtg gagacgtaca aagagcagcc 660
gctcaagaca cctgggaaga aaaagaaagg caagcccggg aaacgcaagg agcaggaaaa 720
gaaaaaacgg cgaactcgct ctgcctggtt agactctgga gtgactggga gtgggctaga 780
aggggaccac ctgtctgaca cctccacaac gtcgctggag ctcgattcac ggaggcattg 840
aaattttcag cagagacctt ccaaggacat attgcaggat tctgtaatag tgaacatatg 900
gaaagtatta gaaatattta ttgtctgtaa atactgtaaa tgcattggaa taaaactgtc 960
tcccccattg ctctatgaaa ctgcacattg gtcattgtga atattttttt ttttgccaag 1020
gctaatccaa ttattattat cacatttacc ataatttatt ttgtccattg atgtatttat 1080
tttgtaaatg tatcttggtg ctgctgaatt tctatatttt ttgtaacata atgcacttta 1140
gatatacata tcaagtatgt tgataaatga cacaatgaag tgtctctatt ttgtggttga 1200
ttttaatgaa tgcctaaata taattatcca aattgatttt cctttgtgcc cgtaaaaata 1260
acagtatttt aaatttgtaa agaatgtcta ataaaatata atctaattac 1310

<210> 165
<211> 177
<212> PRT
<213> Homo sapien

<400> 165

<210> 166
<211> 177
<212> PRT
<213> Homo sapien

<400> 166

<210> 167
<211> 3362
<212> DNA
<213> Homo sapien

<400> 167

cacaatgtat gcagcaggct cagtgtgagt gaactggagg cttctctaca acatgaccca 60
aaggagcatt gcaggtccta tttgcaacct gaagtttgtg actctcctgg ttgccttaag 120
ttcagaactc ccattcctgg gagctggagt acagcttcaa gacaatgggt ataatggatt 180
gctcattgca attaatcctc aggtacctga gaatcagaac ctcatctcaa acattaagga 240
aatgataact gaagcttcat tttacctatt taatgctacc aagagaagag tatttttcag 300
aaatataaag attttaatac ctgccacatg gaaagctaat aataacagca aaataaaaca 360
agaatcatat gaaaaggcaa atgtcatagt gactgactgg tatggggcac atggagatga 420
tccatacacc ctacaataca gagggtgtgg aaaagaggga aaatacattc atttcacacc 480
taatttccta ctgaatgata acttaacagc tggctacgga tcacgaggcc gagtgtttgt 540
ccatgaatgg gcccacctcc gttggggtgt gttcgatgag tataacaatg acaaaccttt 600
ctacataaat gggcaaaatc aaattaaagt gacaaggtgt tcatctgaca tcacaggcat 660
ttttgtgtgt gaaaaaggtc cttgccccca agaaaactgt attattagta agctttttaa 720
agaaggatgc acctttatct acaatagcac ccaaaatgca actgcatcaa taatgttcat 780
gcaaagttta tcttctgtgg ttgaattttg taatgcaagt acccacaacc aagaagcacc 840
aaacctacag aaccagatgt gcagcctcag aagtgcatgg gatgtaatca cagactctgc 900
tgactttcac cacagctttc ccatgaacgg gactgagctt ccacctcctc ccacattctc 960
gcttgtagag gctggtgaca aagtggtctg tttagtgctg gatgtgtcca gcaagatggc 1020
agaggctgac agactccttc aactacaaca agccgcagaa ttttatttga tgcagattgt 1080
tgaaattcat accttcgtgg gcattgccag tttcgacagc aaaggagaga tcagagccca 1140
gctacaccaa attaacagca atgatgatcg aaagttgctg gtttcatatc tgcccaccac 1200
tgtatcagct aaaacagaca tcagcatttg ttcagggctt aagaaaggat ttgaggtggt 1260
tgaaaaactg aatggaaaag cttatggctc tgtgatgata ttagtgacca gcggagatga 1320
taagcttctt ggcaattgct tacccactgt gctcagcagt ggttcaacaa ttcactccat 1380
tgccctgggt tcatctgcag ccccaaatct ggaggaatta tcacgtctta caggaggttt 1440
aaagttcttt gttccagata tatcaaactc caatagcatg attgatgctt tcagtagaat 1500
ttcctctgga actggagaca ttttccagca acatattcag cttgaaagta caggtgaaaa 1560
tgtcaaacct caccatcaat tgaaaaacac agtgactgtg gataatactg tgggcaacga 1620
cactatgttt ctagttacgt ggcaggccag tggtcctcct gagattatat tatttgatcc 1680
tgatggacga aaatactaca caaataattt tatcaccaat ctaacttttc ggacagctag 1740
tctttggatt ccaggaacag ctaagcctgg gcactggact tacaccctga tgtgtttcca 1800
ccatgcaaaa ttattgacct ggaagctgta aaagtagaag aggaattgac cctatcttgg 1860
acagcacctg gagaagactt tgatcagggc caggctacaa gctatgaaat aagaatgagt 1920
aaaagtctac agaatatcca agatgacttt aacaatgcta ttttagtaaa tacatcaaag 1980
cgaaatcctc agcaagctgg catcagggag atatttacgt tctcacccca aatttccacg 2040
aatggacctg aacatcagcc aaatggagaa acacatgaaa gccacagaat ttatgttgca 2100
atacgagcaa tggataggaa ctccttacag tctgctgtat ctaacattgc ccaggcgcct 2160
ctgtttattc cccccaattc tgatcctgta cctgccagag attatcttat attgaaagga 2220
gttttaacag caatgggttt gataggaatc atttgcctta ttatagttgt gacacatcat 2280
actttaagca ggaaaaagag agcagacaag aaagagaatg gaacaaaatt attataaata 2340
aatatccaaa gtgtcttcct tcttagatat aagacccatg gccttcgact acaaaaacat 2400
actaacaaag tcaaattaac atcaaaactg tattaaaatg cattgagttt ttgtacaata 2460
cagataagat ttttacatgg tagatcaaca aattcttttt gggggtagat tagaaaaccc 2520
ttacactttg gctatgaaca aataataaaa attattcttt aaagtaatgt ctttaaaggc 2580
aaagggaagg gtaaagtcgg accagtgtca aggaaagttt gttttattga ggtggaaaaa 2640
tagccccaag cagagaaaag gagggtaggt ctgcattata actgtctgtg tgaagcaatc 2700
atttagttac tttgattaat ttttcttttc tccttatctg tgcagaacag gttgcttgtt 2760
tacaactgaa gatcatgcta tatttcatat atgaagcccc taatgcaaag ctctttacct 2820
cttgctattt tgttatatat attacagatg aaatctcact gctaatgctc agagatcttt 2880
tttcactgta agaggtaacc tttaacaata tgggtattac ctttgtctct tcataccggt 2940
tttatgacaa aggtctattg aatttatttg tttgtaagtt tctactccca tcaaagcagc 3000
tttctaagtt attgccttgg ttattatgga tgatagttat agcccttata atgccttaac 3060
taaggaagaa aagatgttat tctgagtttg ttttaataca tatatgaaca tatagtttta 3120
ttcaattaaa ccaaagaaga ggtcagcagg gagatactaa cctttggaaa tgattagctg 3180
gctctgtttt ttggttaaat aagagtcttt aatcctttct ccatcaagag ttacttacca 3240
agggcagggg aagggggata tagaggtcac aaggaaataa aaatcatctt tcatctttaa 3300

ttttactcct tcctcttatt tttttaaaag attatcgaac aataaaatca tttgcctttt 3360
tt 3362

<210> 168
<211> 2784
<212> DNA
<213> Homo sapien

<400> 168
tctgcatcca tattgaaaac ctgacacaat gtatgcagca ggctcagtgt gagtgaactg 60
gaggcttctc tacaacatga cccaaaggag cattgcaggt cctatttgca acctgaagtt 120
tgtgactctc ctggttgcct taagttcaga actcccattc ctgggagctg gagtacagct 180
tcaagacaat gggtataatg gattgctcat tgcaattaat cctcaggtac ctgagaatca 240
gaacctcatc tcaaacatta aggaaatgat aactgaagct tcattttacc tatttaatgc 300
taccaagaga agagtatttt tcagaaatat aaagatttta atacctgcca catggaaagc 360
taataataac agcaaaataa aacaagaatc atatgaaaag gcaaatgtca tagtgactga 420
ctggtatggg gcacatggag atgatccata caccctacaa tacagagggt gtggaaaaga 480
gggaaaatac attcatttca cacctaattt cctactgaat gataacttaa cagctggcta 540
cggatcacga ggccgagtgt ttgtccatga atgggcccac ctccgttggg gtgtgttcga 600
tgagtataac aatgacaaac ctttctacat aaatgggcaa aatcaaatta aagtgacaag 660
gtgttcatct gacatcacag gcatttttgt gtgtgaaaaa ggtccttgcc cccaagaaaa 720
ctgtattatt agtaagcttt ttaaagaagg atgcaccttt atctacaata gcacccaaaa 780
tgcaactgca tcaataatgt tcatgcaaag tttatcttct gtggttgaat tttgtaatgc 840
aagtacccac aaccaagaag caccaaacct acagaaccag atgtgcagcc tcagaagtgc 900
atgggatgta atcacagact ctgctgactt tcaccacagc tttcccatga acgggactga 960
gcttccacct cctcccacat tctcgcttgt agaggctggt gacaaagtgg tctgtttagt 1020
gctggatgtg tccagcaaga tggcagaggc tgacagactc cttcaactac aacaagccgc 1080
agaattttat ttgatgcaga ttgttgaaat tcataccttc gtgggcattg ccagtttcga 1140
cagcaaagga gagatcagag cccagctaca ccaaattaac agcaatgatg atcgaaagtt 1200
gctggtttca tatctgccca ccactgtatc agctaaaaca gacatcagca tttgttcagg 1260
gcttaagaaa ggatttgagg tggttgaaaa actgaatgga aaagcttatg gctctgtgat 1320
gatattagtg accagcggag atgataagct tcttggcaat tgcttaccca ctgtgctcag 1380
cagtggttca acaattcact ccattgccct gggttcatct gcagccccaa atctggagga 1440
attatcacgt cttacaggag gtttaaagtt ctttgttcca gatatatcaa actccaatag 1500
catgattgat gctttcagta gaatttcctc tggaactgga gacattttcc agcaacatat 1560
tcagcttgaa agtacaggtg aaaatgtcaa acctcaccat caattgaaaa acacagtgac 1620
tgtggataat actgtgggca acgacactat gtttctagtt acgtggcagg ccagtggtcc 1680
tcctgagatt atattatttg atcctgatgg acgaaaatac tacacaaata attttatcac 1740
caatctaact tttcggacag ctagtctttg gattccagga acagctaagc ctgggcactg 1800
gacttacacc ctgaacaata cccatcattc tctgcaagcc ctgaaagtga cagtgacctc 1860
tcgcgcctcc aactcagctg tgcccccagc cactgtggaa gcctttgtgg aaagagacag 1920
cctccatttt cctcatcctg tgatgattta tgccaatgtg aaacagggat tttatcccat 1980
tcttaatgcc actgtcactg ccacagttga gccagagact ggagatcctg ttacgctgag 2040
actccttgat gatggagcag gtgctgatgt tataaaaaat gatggaattt actcgaggta 2100
ttttttctcc tttgctgcaa atggtagata tagcttgaaa gtgcatgtca atcactctcc 2160
cagcataagc accccagccc actctattcc agggagtcat gctatgtatg taccaggtta 2220
cacagcaaac ggtaatattc agatgaatgc tccaaggaaa tcagtaggca gaaatgagga 2280
ggagcgaaag tggggcttta gccgagtcag ctcaggaggc tccttttcag tgctgggagt 2340
tccagctggc ccccaccctg atgtgtttcc accatgcaaa attattgacc tggaagctgt 2400
aaatagaaga ggaattgacc ctatcttgga cagcacctgg agaagacttt gatcagggcc 2460
aggctacaag ctatgaaata agaatgagta aaagtctaca gaatatccaa gatgacttta 2520
acaatgctat tttagtaaat acatcaaagc gaaatcctca gcaagctggc atcagggaga 2580
tatttacgtt ctcaccccaa atttccacga atggacctga acatcagcca aatggagaaa 2640
cacatgaaag ccacagaatt tatgttgcaa tacgagcaat ggataggaac tccttacagt 2700
ctgctgtatc taacattgcc caggcgcctc tgtttattcc ccccaattct gatcctgtac 2760

ctgccagaga ttatcttata ttga                                                2784

<210> 169
<211> 592
<212> PRT
<213> Homo sapien

<400> 169
Met Thr Gln Arg Ser Ile Ala Gly Pro Ile Cys Asn Leu Lys Phe Val
1               5                   10                  15
Thr Leu Leu Val Ala Leu Ser Ser Glu Leu Pro Phe Leu Gly Ala Gly
            20                  25                  30
Val Gln Leu Gln Asp Asn Gly Tyr Asn Gly Leu Leu Ile Ala Ile Asn
        35                  40                  45
Pro Gln Val Pro Glu Asn Gln Asn Leu Ile Ser Asn Ile Lys Glu Met
    50                  55                  60
Ile Thr Glu Ala Ser Phe Tyr Leu Phe Asn Ala Thr Lys Arg Arg Val
65                  70                  75                  80
Phe Phe Arg Asn Ile Lys Ile Leu Ile Pro Ala Thr Trp Lys Ala Asn
                85                  90                  95
Asn Asn Ser Lys Ile Lys Gln Glu Ser Tyr Glu Lys Ala Asn Val Ile
            100                 105                 110
Val Thr Asp Trp Tyr Gly Ala His Gly Asp Asp Pro Tyr Thr Leu Gln
        115                 120                 125
Tyr Arg Gly Cys Gly Lys Glu Gly Lys Tyr Ile His Phe Thr Pro Asn
    130                 135                 140
Phe Leu Leu Asn Asp Asn Leu Thr Ala Gly Tyr Gly Ser Arg Gly Arg
145                 150                 155                 160
Val Phe Val His Glu Trp Ala His Leu Arg Trp Gly Val Phe Asp Glu
                165                 170                 175
Tyr Asn Asn Asp Lys Pro Phe Tyr Ile Asn Gly Gln Asn Gln Ile Lys
            180                 185                 190
Val Thr Arg Cys Ser Ser Asp Ile Thr Gly Ile Phe Val Cys Glu Lys
        195                 200                 205
Gly Pro Cys Pro Gln Glu Asn Cys Ile Ile Ser Lys Leu Phe Lys Glu
    210                 215                 220
Gly Cys Thr Phe Ile Tyr Asn Ser Thr Gln Asn Ala Thr Ala Ser Ile
225                 230                 235                 240
Met Phe Met Gln Ser Leu Ser Ser Val Val Glu Phe Cys Asn Ala Ser
                245                 250                 255
Thr His Asn Gln Glu Ala Pro Asn Leu Gln Asn Gln Met Cys Ser Leu
            260                 265                 270
Arg Ser Ala Trp Asp Val Ile Thr Asp Ser Ala Asp Phe His His Ser
        275                 280                 285
Phe Pro Met Asn Gly Thr Glu Leu Pro Pro Pro Pro Thr Phe Ser Leu
    290                 295                 300
Val Glu Ala Gly Asp Lys Val Val Cys Leu Val Leu Asp Val Ser Ser
305                 310                 315                 320
Lys Met Ala Glu Ala Asp Arg Leu Leu Gln Leu Gln Gln Ala Ala Glu
                325                 330                 335
Phe Tyr Leu Met Gln Ile Val Glu Ile His Thr Phe Val Gly Ile Ala
            340                 345                 350
Ser Phe Asp Ser Lys Gly Glu Ile Arg Ala Gln Leu His Gln Ile Asn
        355                 360                 365
Ser Asn Asp Asp Arg Lys Leu Leu Val Ser Tyr Leu Pro Thr Thr Val

370                 375                 380
Ser Ala Lys Thr Asp Ile Ser Ile Cys Ser Gly Leu Lys Lys Gly Phe
385                 390                 395                 400
Glu Val Val Glu Lys Leu Asn Gly Lys Ala Tyr Gly Ser Val Met Ile
                405                 410                 415
Leu Val Thr Ser Gly Asp Asp Lys Leu Leu Gly Asn Cys Leu Pro Thr
            420                 425                 430
Val Leu Ser Ser Gly Ser Thr Ile His Ser Ile Ala Leu Gly Ser Ser
        435                 440                 445
Ala Ala Pro Asn Leu Glu Glu Leu Ser Arg Leu Thr Gly Gly Leu Lys
    450                 455                 460
Phe Phe Val Pro Asp Ile Ser Asn Ser Asn Ser Met Ile Asp Ala Phe
465                 470                 475                 480
Ser Arg Ile Ser Ser Gly Thr Gly Asp Ile Phe Gln Gln His Ile Gln
                485                 490                 495
Leu Glu Ser Thr Gly Glu Asn Val Lys Pro His His Gln Leu Lys Asn
            500                 505                 510
Thr Val Thr Val Asp Asn Thr Val Gly Asn Asp Thr Met Phe Leu Val
        515                 520                 525
Thr Trp Gln Ala Ser Gly Pro Pro Glu Ile Ile Leu Phe Asp Pro Asp
    530                 535                 540
Gly Arg Lys Tyr Tyr Thr Asn Asn Phe Ile Thr Asn Leu Thr Phe Arg
545                 550                 555                 560
Thr Ala Ser Leu Trp Ile Pro Gly Thr Ala Lys Pro Gly His Trp Thr
                565                 570                 575
Tyr Thr Leu Met Cys Phe His His Ala Lys Leu Leu Thr Trp Lys Leu
            580                 585                 590

<210> 170
<211> 791
<212> PRT
<213> Homo sapien

<400> 170
Met Thr Gln Arg Ser Ile Ala Gly Pro Ile Cys Asn Leu Lys Phe Val
 1               5                  10                  15
Thr Leu Leu Val Ala Leu Ser Ser Glu Leu Pro Phe Leu Gly Ala Gly
            20                  25                  30
Val Gln Leu Gln Asp Asn Gly Tyr Asn Gly Leu Leu Ile Ala Ile Asn
        35                  40                  45
Pro Gln Val Pro Glu Asn Gln Asn Leu Ile Ser Asn Ile Lys Glu Met
    50                  55                  60
Ile Thr Glu Ala Ser Phe Tyr Leu Phe Asn Ala Thr Lys Arg Arg Val
65                  70                  75                  80
Phe Phe Arg Asn Ile Lys Ile Leu Ile Pro Ala Thr Trp Lys Ala Asn
                85                  90                  95
Asn Asn Ser Lys Ile Lys Gln Glu Ser Tyr Glu Lys Ala Asn Val Ile
            100                 105                 110
Val Thr Asp Trp Tyr Gly Ala His Gly Asp Asp Pro Tyr Thr Leu Gln
        115                 120                 125
Tyr Arg Gly Cys Gly Lys Glu Gly Lys Tyr Ile His Phe Thr Pro Asn
    130                 135                 140
Phe Leu Leu Asn Asp Asn Leu Thr Ala Gly Tyr Gly Ser Arg Gly Arg
145                 150                 155                 160
Val Phe Val His Glu Trp Ala His Leu Arg Trp Gly Val Phe Asp Glu

                165                 170                 175
Tyr Asn Asn Asp Lys Pro Phe Tyr Ile Asn Gly Gln Asn Gln Ile Lys
            180                 185                 190
Val Thr Arg Cys Ser Ser Asp Ile Thr Gly Ile Phe Val Cys Glu Lys
        195                 200                 205
Gly Pro Cys Pro Gln Glu Asn Cys Ile Ile Ser Lys Leu Phe Lys Glu
    210                 215                 220
Gly Cys Thr Phe Ile Tyr Asn Ser Thr Gln Asn Ala Thr Ala Ser Ile
225                 230                 235                 240
Met Phe Met Gln Ser Leu Ser Ser Val Val Glu Phe Cys Asn Ala Ser
                245                 250                 255
Thr His Asn Gln Glu Ala Pro Asn Leu Gln Asn Gln Met Cys Ser Leu
            260                 265                 270
Arg Ser Ala Trp Asp Val Ile Thr Asp Ser Ala Asp Phe His His Ser
        275                 280                 285
Phe Pro Met Asn Gly Thr Glu Leu Pro Pro Pro Pro Thr Phe Ser Leu
    290                 295                 300
Val Glu Ala Gly Asp Lys Val Val Cys Leu Val Leu Asp Val Ser Ser
305                 310                 315                 320
Lys Met Ala Glu Ala Asp Arg Leu Leu Gln Leu Gln Gln Ala Ala Glu
                325                 330                 335
Phe Tyr Leu Met Gln Ile Val Glu Ile His Thr Phe Val Gly Ile Ala
            340                 345                 350
Ser Phe Asp Ser Lys Gly Glu Ile Arg Ala Gln Leu His Gln Ile Asn
        355                 360                 365
Ser Asn Asp Asp Arg Lys Leu Leu Val Ser Tyr Leu Pro Thr Thr Val
    370                 375                 380
Ser Ala Lys Thr Asp Ile Ser Ile Cys Ser Gly Leu Lys Lys Gly Phe
385                 390                 395                 400
Glu Val Val Glu Lys Leu Asn Gly Lys Ala Tyr Gly Ser Val Met Ile
                405                 410                 415
Leu Val Thr Ser Gly Asp Asp Lys Leu Leu Gly Asn Cys Leu Pro Thr
            420                 425                 430
Val Leu Ser Ser Gly Ser Thr Ile His Ser Ile Ala Leu Gly Ser Ser
        435                 440                 445
Ala Ala Pro Asn Leu Glu Glu Leu Ser Arg Leu Thr Gly Gly Leu Lys
    450                 455                 460
Phe Phe Val Pro Asp Ile Ser Asn Ser Asn Ser Met Ile Asp Ala Phe
465                 470                 475                 480
Ser Arg Ile Ser Ser Gly Thr Gly Asp Ile Phe Gln Gln His Ile Gln
                485                 490                 495
Leu Glu Ser Thr Gly Glu Asn Val Lys Pro His His Gln Leu Lys Asn
            500                 505                 510
Thr Val Thr Val Asp Asn Thr Val Gly Asn Asp Thr Met Phe Leu Val
        515                 520                 525
Thr Trp Gln Ala Ser Gly Pro Pro Glu Ile Ile Leu Phe Asp Pro Asp
    530                 535                 540
Gly Arg Lys Tyr Tyr Thr Asn Asn Phe Ile Thr Asn Leu Thr Phe Arg
545                 550                 555                 560
Thr Ala Ser Leu Trp Ile Pro Gly Thr Ala Lys Pro Gly His Trp Thr
                565                 570                 575
Tyr Thr Leu Asn Asn Thr His His Ser Leu Gln Ala Leu Lys Val Thr
            580                 585                 590
Val Thr Ser Arg Ala Ser Asn Ser Ala Val Pro Pro Ala Thr Val Glu
        595                 600                 605

Ala Phe Val Glu Arg Asp Ser Leu His Phe Pro His Pro Val Met Ile
    610                 615                 620
Tyr Ala Asn Val Lys Gln Gly Phe Tyr Pro Ile Leu Asn Ala Thr Val
625                 630                 635                 640
Thr Ala Thr Val Glu Pro Glu Thr Gly Asp Pro Val Thr Leu Arg Leu
                645                 650                 655
Leu Asp Asp Gly Ala Gly Ala Asp Val Ile Lys Asn Asp Gly Ile Tyr
            660                 665                 670
Ser Arg Tyr Phe Phe Ser Phe Ala Ala Asn Gly Arg Tyr Ser Leu Lys
        675                 680                 685
Val His Val Asn His Ser Pro Ser Ile Ser Thr Pro Ala His Ser Ile
    690                 695                 700
Pro Gly Ser His Ala Met Tyr Val Pro Gly Tyr Thr Ala Asn Gly Asn
705                 710                 715                 720
Ile Gln Met Asn Ala Pro Arg Lys Ser Val Gly Arg Asn Glu Glu Glu
                725                 730                 735
Arg Lys Trp Gly Phe Ser Arg Val Ser Ser Gly Gly Ser Phe Ser Val
            740                 745                 750
Leu Gly Val Pro Ala Gly Pro His Pro Asp Val Phe Pro Pro Cys Lys
        755                 760                 765
Ile Ile Asp Leu Glu Ala Val Asn Arg Arg Gly Ile Asp Pro Ile Leu
    770                 775                 780
Asp Ser Thr Trp Arg Arg Leu
785                 790

<210> 171
<211> 1491
<212> DNA
<213> Homo sapien

<400> 171
cctcctgcca gccaagtgaa gacatgctta cttccccttc accttccttc atgatgtggg      60
aagagtgctg caacccagcc ctagccaacg ccgcatgaga gggagtgtgc cgagggcttc     120
tgagaaggtt tctctcacat ctagaaagaa gcgcttaaga tgtggcagcc cctcttcttc     180
aagtggctct tgtcctgttg ccctgggagt tctcaaattg ctgcagcagc ctccacccag     240
cctgaggatg acatcaatac acagaggaag aagagtcagg aaaagatgag agaagttaca     300
gactctcctg ggcgaccccg agagcttacc attcctcaga cttcttcaca tggtgctaac     360
agatttgttc ctaaaagtaa agctctagag gccgtcaaat tggcaataga agccgggttc     420
caccatattg attctgcaca tgtttacaat aatgaggagc aggttggact ggccatccga     480
agcaagattg cagatggcag tgtgaagaga gaagacatat tctacacttc aaagctttgg     540
agcaattccc atcgaccaga gttggtccga ccagccttgg aaaggtcact gaaaaatctt     600
caattggact atgttgacct ctatcttatt cattttccag tgtctgtaaa gccaggtgag     660
gaagtgatcc caaaagatga aaatggaaaa atactatttg acacagtgga tctctgtgcc     720
acatgggagg ccatggagaa gtgtaaagat gcaggattgg ccaagtccat cggggtgtcc     780
aacttcaacc acaggctgct ggagatgatc ctcaacaagc cagggctcaa gtacaagcct     840
gtctgcaacc aggtggaatg tcatccttac ttcaaccaga gaaaactgct ggatttctgc     900
aagtcaaaag acattgttct ggttgcctat agtgctctgg gatcccatcg agaagaacca     960
tgggtggacc cgaactcccc ggtgctcttg gaggacccag tcctttgtgc cttggcaaaa    1020
aagcacaagc gaaccccagc cctgattgcc ctgcgctacc agctgcagcg tggggttgtg    1080
gtcctggcca agagctacaa tgagcagcgc atcagacaga acgtgcaggt gtttgaattc    1140
cagttgactt cagaggagat gaaagccata gatggcctaa acagaaatgt gcgatatttg    1200
acccttgata tttttgctgg cccccctaat tatccatttt ctgatgaata ttaacatgga    1260
gggcattgca tgaggtctgc cagaaggccc tgcgtgtgga tggtgacaca gaggatggct    1320
ctatgctggt gactggacac atcgcctctg gttaaatctc tcctgcttgg cgacttcagt    1380
aagctacagc taagcccatc ggccggaaaa gaaagacaat aattttgttt ttcattttga    1440

aaaaattaaa tgctctctcc taaagattct tcacctaaaa aaaaaaaaaa a          1491

<210> 172
<211> 364
<212> PRT
<213> Homo sapien

<400> 172
Met Trp Gln Pro Leu Phe Phe Lys Trp Leu Leu Ser Cys Cys Pro Gly
1               5                   10                  15
Ser Ser Gln Ile Ala Ala Ala Ala Ser Thr Gln Pro Glu Asp Asp Ile
            20                  25                  30
Asn Thr Gln Arg Lys Lys Ser Gln Glu Lys Met Arg Glu Val Thr Asp
        35                  40                  45
Ser Pro Gly Arg Pro Arg Glu Leu Thr Ile Pro Gln Thr Ser Ser His
    50                  55                  60
Gly Ala Asn Arg Phe Val Pro Lys Ser Lys Ala Leu Glu Ala Val Lys
65                  70                  75                  80
Leu Ala Ile Glu Ala Gly Phe His His Ile Asp Ser Ala His Val Tyr
                85                  90                  95
Asn Asn Glu Glu Gln Val Gly Leu Ala Ile Arg Ser Lys Ile Ala Asp
            100                 105                 110
Gly Ser Val Lys Arg Glu Asp Ile Phe Tyr Thr Ser Lys Leu Trp Ser
        115                 120                 125
Asn Ser His Arg Pro Glu Leu Val Arg Pro Ala Leu Glu Arg Ser Leu
    130                 135                 140
Lys Asn Leu Gln Leu Asp Tyr Val Asp Leu Tyr Leu Ile His Phe Pro
145                 150                 155                 160
Val Ser Val Lys Pro Gly Glu Glu Val Ile Pro Lys Asp Glu Asn Gly
                165                 170                 175
Lys Ile Leu Phe Asp Thr Val Asp Leu Cys Ala Thr Trp Glu Ala Met
            180                 185                 190
Glu Lys Cys Lys Asp Ala Gly Leu Ala Lys Ser Ile Gly Val Ser Asn
        195                 200                 205
Phe Asn His Arg Leu Leu Glu Met Ile Leu Asn Lys Pro Gly Leu Lys
    210                 215                 220
Tyr Lys Pro Val Cys Asn Gln Val Glu Cys His Pro Tyr Phe Asn Gln
225                 230                 235                 240
Arg Lys Leu Leu Asp Phe Cys Lys Ser Lys Asp Ile Val Leu Val Ala
                245                 250                 255
Tyr Ser Ala Leu Gly Ser His Arg Glu Glu Pro Trp Val Asp Pro Asn
            260                 265                 270
Ser Pro Val Leu Leu Glu Asp Pro Val Leu Cys Ala Leu Ala Lys Lys
        275                 280                 285
His Lys Arg Thr Pro Ala Leu Ile Ala Leu Arg Tyr Gln Leu Gln Arg
    290                 295                 300
Gly Val Val Val Leu Ala Lys Ser Tyr Asn Glu Gln Arg Ile Arg Gln
305                 310                 315                 320
Asn Val Gln Val Phe Glu Phe Gln Leu Thr Ser Glu Glu Met Lys Ala
                325                 330                 335
Ile Asp Gly Leu Asn Arg Asn Val Arg Tyr Leu Thr Leu Asp Ile Phe
            340                 345                 350
Ala Gly Pro Pro Asn Tyr Pro Phe Ser Asp Glu Tyr
        355                 360

<210> 173
<211> 1988
<212> DNA
<213> Homo sapiens

<400> 173
cgggagccgc ctccccgcgg cctcttcgct tttgtggcgg cgcccgcgct cgcaggccac 60
tctctgctgt cgcccgtccc gcgcgctcct ccgacccgct ccgctccgct ccgctcggcc 120
ccgcgccgcc cgtcaacatg atccgctgcg gcctggcctg cgagcgctgc cgctggatcc 180
tgcccctgct cctactcagc gccatcgcct tcgacatcat cgcgctggcc ggccgcggct 240
ggttgcagtc tagcgaccac ggccagacgt cctcgctgtg gtggaaatgc tcccaagagg 300
gcggcggcag cgggtcctac gaggagggct gtcagagcct catggagtac gcgtggggta 360
gagcagcggc tgccatgctc ttctgtggct tcatcatcct ggtgatctgt ttcatcctct 420
ccttcttcgc cctctgtgga ccccagatgc ttgtcttcct gagagtgatt ggaggtctcc 480
ttgccttggc tgctgtgttc cagatcatct ccctggtaat ttaccccgtg aagtacaccc 540
agaccttcac ccttcatgcc aaccctgctg tcacttacat ctataactgg gcctacggct 600
ttgggtgggc agccacgatt atcctgatcg gctgtgcctt cttcttctgc tgcctcccca 660
actacgaaga tgaccttctg ggcaatgcca agcccaggta cttctacaca tctgcctaac 720
ttgggaatga atgtgggaga aaatcgctgc tgctgagatg gactccagaa gaagaaactg 780
tttctccagg cgactttgaa cccatttttt ggcagtgttc atattattaa actagtcaaa 840
aatgctaaaa taatttggga gaaaatattt tttaagtagt gttatagttt catgtttatc 900
ttttattatg ttttgtgaag ttgtgtcttt tcactaatta cctatactat gccaatattt 960
ccttatatct atccataaca tttatactac atttgtaaga gaatatgcac gtgaaactta 1020
acactttata aggtaaaaat gaggtttcca agatttaata atctgatcaa gttcttgtta 1080
tttccaaata gaatggactt ggtctgttaa gggctaagga gaagaggaag ataaggttaa 1140
aagttgttaa tgaccaaaca ttctaaaaga aatgcaaaaa aaaagtttat tttcaagcct 1200
tcgaactatt taaggaaagc aaaatcattt cctaaatgca tatcatttgt gagaatttct 1260
cattaatatc ctgaatcatt catttcagct aaggcttcat gttgactcga tatgtcatct 1320
aggaaagtac tatttcatgg tccaaacctg ttgccatagt tggtaaggct ttcctttaag 1380
tgtgaaatat ttagatgaaa ttttctcttt taaagttctt tatagggtta gggtgtggga 1440
aaatgctata ttaataaatc tgtagtgttt tgtgtttata tgttcagaac cagagtagac 1500
tggattgaaa gatggactgg gtctaattta tcatgactga tagatctggt taagttgtgt 1560
agtaaagcat taggagggtc attcytgtca caaaagtgcc actaaaacag cctcaggaga 1620
ataaatgact tgcttttcta aatctcaggt ttatctgggc tctatcatat agacaggctt 1680
ctgatagttt gcarctgtaa gcagaaacct acatatagtt aaaatcctgg tctttcttgg 1740
taaacagatt ttaaatgtct gatataaaac atgccacagg agaattcggg gatttgagtt 1800
tctctgaata gcatatatat gatgcatcgg ataggtcatt atgatttttt accatttcga 1860
cttacataat gaaaaccaat tcattttaaa tatcagatta ttattttgta agttgtggaa 1920
aaagctaatt gtagttttca ttatgaagtt ttcccaataa accaggtatt ctaaaaaaaa 1980
aaaaaaaa 1988

<210> 174
<211> 238
<212> PRT
<213> Homo sapiens

<400> 174
Gly Ala Ala Ser Pro Arg Pro Leu Arg Phe Cys Gly Gly Ala Arg Ala
                5                   10                  15

Arg Arg Pro Leu Ser Ala Val Ala Arg Pro Ala Arg Ser Ser Asp Pro
            20                  25                  30

Leu Arg Ser Ala Pro Leu Gly Pro Ala Pro Pro Val Asn Met Ile Arg

35                  40                  45

Cys Gly Leu Ala Cys Glu Arg Cys Arg Trp Ile Leu Pro Leu Leu Leu
    50                  55                  60

Leu Ser Ala Ile Ala Phe Asp Ile Ile Ala Leu Ala Gly Arg Gly Trp
65                  70                  75                  80

Leu Gln Ser Ser Asp His Gly Gln Thr Ser Ser Leu Trp Trp Lys Cys
                85                  90                  95

Ser Gln Glu Gly Gly Gly Ser Gly Ser Tyr Glu Glu Gly Cys Gln Ser
            100                 105                 110

Leu Met Glu Tyr Ala Trp Gly Arg Ala Ala Ala Ala Met Leu Phe Cys
        115                 120                 125

Gly Phe Ile Ile Leu Val Ile Cys Phe Ile Leu Ser Phe Phe Ala Leu
    130                 135                 140

Cys Gly Pro Gln Met Leu Val Phe Leu Arg Val Ile Gly Gly Leu Leu
145                 150                 155                 160

Ala Leu Ala Ala Val Phe Gln Ile Ile Ser Leu Val Ile Tyr Pro Val
                165                 170                 175

Lys Tyr Thr Gln Thr Phe Thr Leu His Ala Asn Pro Ala Val Thr Tyr
            180                 185                 190

Ile Tyr Asn Trp Ala Tyr Gly Phe Gly Trp Ala Ala Thr Ile Ile Leu
        195                 200                 205

Ile Gly Cys Ala Phe Phe Phe Cys Cys Leu Pro Asn Tyr Glu Asp Asp
    210                 215                 220

Leu Leu Gly Asn Ala Lys Pro Arg Tyr Phe Tyr Thr Ser Ala
225                 230                 235

<210> 175
<211> 4181
<212> DNA
<213> Homo sapiens

<220>
<221> unsure
<222> (3347)
<223> n=A,T,C or G
<221> unsure
<222> (3502)
<223> n=A,T,C or G
<221> unsure
<222> (3506)
<223> n=A,T,C or G

<221> unsure
<222> (3520)
<223> n=A,T,C or G
<221> unsure
<222> (3538)
<223> n=A,T,C or G
<221> unsure
<222> (3549)
<223> n=A,T,C or G
<221> unsure
<222> (3646)
<223> n=A,T,C or G
<221> unsure
<222> (3940)
<223> n=A,T,C or G
<221> unsure
<222> (3968)
<223> n=A,T,C or G
<221> unsure
<222> (3974)
<223> n=A,T,C or G
<221> unsure
<222> (4036)
<223> n=A,T,C or G
<221> unsure
<222> (4056)
<223> n=A,T,C or G
<221> unsure
<222> (4062)
<223> n=A,T,C or G
<221> unsure
<222> (4080)
<223> n=A,T,C or G
<221> unsure
<222> (4088)
<223> n=A,T,C or G
<221> unsure
<222> (4115)
<223> n=A,T,C or G

<400> 175
   ggtggatgcg tttgggttgt agctaggctt tttcttttct ttctctttta aaacacatct 60
   agacaaggaa aaaacaagcc tcggatctga tttttcactc ctcgttcttg tgcttggttc 120
   ttactgtgtt tgtgtatttt aaaggcgaga agacgagggg aacaaaacca gctggatcca 180
   tccatcaccg tgggtggttt taatttttcg ttttttctcg ttattttttt ttaaacaacc 240
   actcttcaca atgaacaaac tgtatatcgg aaacctcagc gagaacgccg ccccctcgga 300
   cctagaaagt atcttcaagg acgccaagat cccggtgtcg ggacccttcc tggtgaagac 360
   tggctacgcg ttcgtggact gcccggacga gagctgggcc ctcaaggcca tcgaggcgct 420
   ttcaggtaaa atagaactgc acgggaaacc catagaagtt gagcactcgg tcccaaaaag 480
   gcaaaggatt cggaaacttc agatacgaaa tatcccgcct catttacagt gggaggtgct 540
   ggatagttta ctagtccagt atggagtggt ggagagctgt gagcaagtga acactgactc 600
   ggaaactgca gttgtaaatg taacctattc cagtaaggac caagctagac aagcactaga 660
   caaactgaat ggatttcagt tagagaattt caccttgaaa gtagcctata tccctgatga 720
   aatggccgcc cagcaaaacc ccttgcagca gccccgaggt cgccgggggc ttgggcagag 780
   gggctcctca aggcaggggt ctccaggatc cgtatccaag cagaaaccat gtgatttgcc 840
   tctgcgcctg ctggttccca cccaatttgt tggagccatc ataggaaaag aaggtgccac 900
   cattcggaac atcaccaaac agacccagtc taaaatcgat gtccaccgta aagaaaatgc 960
   gggggctgct gagaagtcga ttactatcct ctctactcct gaaggcacct ctgcggcttg 1020
   taagtctatt ctggagatta tgcataagga agctcaagat ataaaattca cagaagagat 1080
   ccccttgaag attttagctc ataataactt tgttggacgt cttattggta aagaaggaag 1140
   aaatcttaaa aaaattgagc aagacacaga cactaaaatc acgatatctc cattgcagga 1200
   attgacgctg tataatccag aacgcactat tacagttaaa ggcaatgttg agacatgtgc 1260
   caaagctgag gaggagatca tgaagaaaat cagggagtct tatgaaaatg atattgcttc 1320
   tatgaatctt caagcacatt taattcctgg attaaatctg aacgccttgg gtctgttccc 1380
   acccacttca gggatgccac ctcccacctc agggccccct tcagccatga ctcctcccta 1440
   cccgcagttt gagcaatcag aaacggagac tgttcatcag tttatcccag ctctatcagt 1500
   cggtgccatc atcggcaagc agggccagca catcaagcag ctttctcgct ttgctggagc 1560
   ttcaattaag attgctccag cggaagcacc agatgctaaa gtgaggatgg tgattatcac 1620
   tggaccacca gaggctcagt tcaaggctca gggaagaatt tatggaaaaa ttaaagaaga 1680
   aaactttgtt agtcctaaag aagaggtgaa acttgaagct catatcagag tgccatcctt 1740
   tgctgctggc agagttattg gaaaaggagg caaaacggtg aatgaacttc agaatttgtc 1800
   aagtgcagaa gttgttgtcc ctcgtgacca gacacctgat gagaatgacc aagtggttgt 1860
   caaaataact ggtcacttct atgcttgcca ggttgcccag agaaaaattc aggaaattct 1920
   gactcaggta aagcagcacc aacaacagaa ggctctgcaa agtggaccac ctcagtcaag 1980
   acggaagtaa aggctcagga aacagcccac cacagaggca gatgccaaac caaagacaga 2040
   ttgcttaacc aacagatggg cgctgacccc ctatccagaa tcacatgcac aagtttttac 2100
   ctagccagtt gtttctgagg accaggcaac ttttgaactc ctgtctctgt gagaatgtat 2160
   actttatgct ctctgaaatg tatgacaccc agctttaaaa caaacaaaca aacaaacaaa 2220
   aaaagggtgg gggagggagg gaaagagaag agctctgcac ttccctttgt tgtagtctca 2280
   cagtataaca gatattctaa ttcttcttaa tattccccca taatgccaga aattggctta 2340
   atgatgcttt cactaaattc atcaaataga ttgctcctaa atccaattgt taaaattgga 2400
   tcagaataat tatcacagga acttaaatgt taagccatta gcatagaaaa actgttctca 2460
   gttttatttt tacctaacac taacatgagt aacctaaggg aagtgctgaa tggtgttggc 2520
   aggggtatta aacgtgcatt tttactcaac tacctcaggt attcagtaat acaatgaaaa 2580
   gcaaaattgt tccttttttt tgaaaatttt atatacttta taatgataga agtccaaccg 2640
   ttttttaaaa aataaattta aaatttaaca gcaatcagct aacaggcaaa ttaagatttt 2700
   tacttctggc tggtgacagt aaagctggaa aattaatttc agggtttttt gaggcttttg 2760
   acacagttat tagttaaatc aaatgttcaa aaatacggag cagtgcctag tatctggaga 2820
   gcagcactac catttattct ttcatttata gttgggaaag tttttgacgg tactaacaaa 2880
   gtggtcgcag gagattttgg aacggctggt ttaaatggct tcaggagact tcagtttttt 2940
   gtttagctac atgattgaat gcataataaa tgctttgtgc ttctgactat caatacctaa 3000
   agaaagtgca tcagtgaaga gatgcaagac tttcaactga ctggcaaaaa gcaagcttta 3060
   gcttgtctta taggatgctt agtttgccac tacacttcag accaatggga cagtcataga 3120
   tggtgtgaca gtgtttaaac gcaacaaaag gctacatttc catggggcca gcactgtcat 3180
   gagcctcact aagctatttt gaagattttt aagcactgat aaattaaaaa aaaaaaaaaa 3240
   aaattagact ccaccttaag tagtaaagta taacaggatt tctgtatact gtgcaatcag 3300
   ttctttgaaa aaaaagtcaa aagatagaga atacaagaaa agttttnggg atataatttg 3360
   aatgactgtg aaaacatatg acctttgata acgaactcat ttgctcactc cttgacagca 3420
   aagcccagta cgtacaattg tgttgggtgt gggtggtctc caaggccacg ctgctctctg 3480
   aattgatttt ttgagttttg gnttgnaaga tgatcacagn catgttacac tgatcttnaa 3540
   ggacatatnt tataaccctt taaaaaaaaa atcccctgcc tcattcttat ttcgagatga 3600
   atttcgatac agactagatg tctttctgaa gatcaattag acattntgaa aatgatttaa 3660
   agtgttttcc ttaatgttct ctgaaaacaa gtttcttttg tagttttaac caaaaaagtg 3720
   ccctttttgt cactggtttc tcctagcatt catgattttt ttttcacaca atgaattaaa 3780
   attgctaaaa tcatggactg gctttctggt tggatttcag gtaagatgtg tttaaggcca 3840
   gagcttttct cagtatttga tttttttccc caatatttga ttttttaaaa atatacacat 3900
   aggagctgca tttaaaacct gctggtttaa attctgtcan atttcacttc tagcctttta 3960
   gtatggcnaa tcanaattta cttttactta agcatttgta atttggagta tctggtacta 4020
   gctaagaaat aattcnataa ttgagttttg tactcnccaa anatgggtca ttcctcatgn 4080
   ataatgtncc cccaatgcag cttcattttc caganacctt gacgcaggat aaattttttc 4140
   atcatttagg tccccaaaaa aaaaaaaaaa aaaaaaaaaa a                    4181

<210> 176
<211> 580
<212> PRT
<213> Homo sapiens

<400> 176
Met Asn Lys Leu Tyr Ile Gly Asn Leu Ser Glu Asn Ala Ala Pro Ser
                5                   10                  15

Asp Leu Glu Ser Ile Phe Lys Asp Ala Lys Ile Pro Val Ser Gly Pro
            20                  25                  30

Phe Leu Val Lys Thr Gly Tyr Ala Phe Val Asp Cys Pro Asp Glu Ser
        35                  40                  45

Trp Ala Leu Lys Ala Ile Glu Ala Leu Ser Gly Lys Ile Glu Leu His
    50                  55                  60

Gly Lys Pro Ile Glu Val Glu His Ser Val Pro Lys Arg Gln Arg Ile
65                  70                  75                  80

Arg Lys Leu Gln Ile Arg Asn Ile Pro Pro His Leu Gln Trp Glu Val
                85                  90                  95

Leu Asp Ser Leu Leu Val Gln Tyr Gly Val Val Glu Ser Cys Glu Gln
            100                 105                 110

Val Asn Thr Asp Ser Glu Thr Ala Val Val Asn Val Thr Tyr Ser Ser
        115                 120                 125

Lys Asp Gln Ala Arg Gln Ala Leu Asp Lys Leu Asn Gly Phe Gln Leu
    130                 135                 140

Glu Asn Phe Thr Leu Lys Val Ala Tyr Ile Pro Asp Glu Met Ala Ala
145                 150                 155                 160

Gln Gln Asn Pro Leu Gln Gln Pro Arg Gly Arg Arg Gly Leu Gly Gln
                165                 170                 175

Arg Gly Ser Ser Arg Gln Gly Ser Pro Gly Ser Val Ser Lys Gln Lys
            180                 185                 190

Pro Cys Asp Leu Pro Leu Arg Leu Leu Val Pro Thr Gln Phe Val Gly
        195                 200                 205

Ala Ile Ile Gly Lys Glu Gly Ala Thr Ile Arg Asn Ile Thr Lys Gln
    210                 215                 220

Thr Gln Ser Lys Ile Asp Val His Arg Lys Glu Asn Ala Gly Ala Ala
225                 230                 235                 240

Glu Lys Ser Ile Thr Ile Leu Ser Thr Pro Glu Gly Thr Ser Ala Ala
                245                 250                 255

Cys Lys Ser Ile Leu Glu Ile Met His Lys Glu Ala Gln Asp Ile Lys
            260                 265                 270

Phe Thr Glu Glu Ile Pro Leu Lys Ile Leu Ala His Asn Asn Phe Val
        275                 280                 285

Gly Arg Leu Ile Gly Lys Glu Gly Arg Asn Leu Lys Lys Ile Glu Gln
    290                 295                 300

Asp Thr Asp Thr Lys Ile Thr Ile Ser Pro Leu Gln Glu Leu Thr Leu
305                 310                 315                 320

Tyr Asn Pro Glu Arg Thr Ile Thr Val Lys Gly Asn Val Glu Thr Cys
                325                 330                 335

Ala Lys Ala Glu Glu Glu Ile Met Lys Lys Ile Arg Glu Ser Tyr Glu
            340                 345                 350

Asn Asp Ile Ala Ser Met Asn Leu Gln Ala His Leu Ile Pro Gly Leu
        355                 360                 365

Asn Leu Asn Ala Leu Gly Leu Phe Pro Pro Thr Ser Gly Met Pro Pro
    370                 375                 380

Pro Thr Ser Gly Pro Pro Ser Ala Met Thr Pro Pro Tyr Pro Gln Phe
385                 390                 395                 400

Glu Gln Ser Glu Thr Glu Thr Val His Gln Phe Ile Pro Ala Leu Ser
                405                 410                 415

Val Gly Ala Ile Ile Gly Lys Gln Gly Gln His Ile Lys Gln Leu Ser
            420                 425                 430

Arg Phe Ala Gly Ala Ser Ile Lys Ile Ala Pro Ala Glu Ala Pro Asp
        435                 440                 445

Ala Lys Val Arg Met Val Ile Ile Thr Gly Pro Pro Glu Ala Gln Phe
    450                 455                 460

Lys Ala Gln Gly Arg Ile Tyr Gly Lys Ile Lys Glu Glu Asn Phe Val
465                 470                 475                 480

Ser Pro Lys Glu Glu Val Lys Leu Glu Ala His Ile Arg Val Pro Ser
                485                 490                 495

Phe Ala Ala Gly Arg Val Ile Gly Lys Gly Gly Lys Thr Val Asn Glu
            500                 505                 510

Leu Gln Asn Leu Ser Ser Ala Glu Val Val Val Pro Arg Asp Gln Thr
        515                 520                 525

Pro Asp Glu Asn Asp Gln Val Val Val Lys Ile Thr Gly His Phe Tyr
    530                 535                 540

Ala Cys Gln Val Ala Gln Arg Lys Ile Gln Glu Ile Leu Thr Gln Val
545                 550                 555                 560

Lys Gln His Gln Gln Gln Lys Ala Leu Gln Ser Gly Pro Pro Gln Ser
                565                 570                 575

Arg Arg Lys

<210> 177
<211> 401
<212> DNA
<213> Homo sapiens

<400> 177
atgccccgta aatgtcttca gtgttcttca gggtagttgg gatctcaaaa gatttggttc 60
agatccaaac aaatacacat tctgtgtttt agctcagtgt tttctaaaaa aagaaactgc 120
cacacagcaa aaaattgttt actttgttgg acaaaccaaa tcagttctca aaaaatgacc 180
ggtgcttata aaaagttata aatatcgagt agctctaaaa caaaccacct gaccaagagg 240
gaagtgagct tgtgcttagt atttacattg gatgccagtt ttgtaatcac tgacttatgt 300
gcaaactggt gcagaaattc tataaactct ttgctgtttt tgatacctgc ttttgttttc 360
attttgtttt gttttgtaaa aatgataaaa cttcagaaaa t 401

<210> 178
<211> 561
<212> DNA
<213> Homo sapiens

<400> 178
acgcctttca agggtgtacg caaagcactc attgataccc ttttggatgg ctatgaaaca 60
gcccgctatg ggacaggggt ctttggccag aatgagtacc tacgctatca ggaggccctg 120
agtgagctgg ccactgcggt taaagcacga attgggagct ctcagcgaca tcaccagtca 180
gcagccaaag acctaactca gtcccctgag gtctccccaa caaccatcca ggtgacatac 240
ctcccctcca gtcagaagag taaacgtgcc aagcacttcc ttgaattgaa gagctttaag 300
gataactata acacattgga gagtactctg tgacggagct gaaggactct tgccgtagat 360
taagccagtc agttgcaatg tgcaagacag gctgcttgcc gggccgccct cggaacatct 420
ggcccagcag gcccagactg tatccatcca agttcccgtt gtatccagag ttcttagagc 480
ttgtgtctaa agggtaattc cccaaccctt ccttatgagc atttttagaa cattggctaa 540
gactattttc ccccagtagc g 561

<210> 179
<211> 521
<212> DNA
<213> Homo sapiens

<400> 179
cccaacgcgt ttgcaaatat tcccctggta gcctacttcc ttacccccga atattggtaa 60
gatcgagcaa tggcttcagg acatgggttc tcttctcctg tgatcattca agtgctcact 120
gcatgaagac tggcttgtct cagtgtttca acctcaccag ggctgtctct tggtccacac 180
ctcgctccct gttagtgccg tatgacagcc cccatcaaat gaccttggcc aagtcacggt 240
ttctctgtgg tcaaggttgg ttggctgatt ggtggaaagt agggtggacc aaaggaggcc 300
acgtgagcag tcagcaccag ttctgcacca gcagcgcctc cgtcctagtg ggtgttcctg 360
tttctcctgg ccctgggtgg gctagggcct gattcgggaa gatgcctttg cagggagggg 420
aggataagtg ggatctacca attgattctg gcaaaacaat ttctaagatt tttttgcttt 480

atgtgggaaa cagatctaaa tctcatttta tgctgtattt t 521

<210> 180
<211> 417
<212> DNA
<213> Homo sapiens

<400> 180
ggtggaattc gccgaagatg gcggaggtgc aggtcctggt gcttgatggt cgaggccatc 60
tcctgggccg cctggcggcc atcgtggcta aacaggtact gctgggccgg aaggtggtgg 120
tcgtacgctg tgaaggcatc aacatttctg gcaatttcta cagaaacaag ttgaagtacc 180
tggctttcct ccgcaagcgg atgaacacca acccttcccg aggcccctac cacttccggg 240
cccccagccg catcttctgg cggaccgtgc gaggtatgct gccccacaaa accaagcgag 300
gccaggccgc tctggaccgt ctcaaggtgt ttgacggcat cccaccgccc tacgacaaga 360
aaaagcggat ggtggttcct gctgccctca aggtcgtgcg tctgaagcct acaagaa 417

<210> 181
<211> 283
<212> DNA
<213> Homo sapiens

<220>
<221> unsure
<222> (35)
<223> n=A,T,C or G

<400> 181
gatttcttct aaataggatg taaaacttct ttcanattac tcttcctcag tcctgcctgc 60
caagaactca agtgtaactg tgataaaata acctttccca ggtatattgg caggtatgtg 120
tgtaatctca gaatacacag gtgacataga tatgatatga caactggtaa tggtggattc 180
atttacattg tttacacttc tatgaccagg ccttaaggga aggtcagttt tttaaaaaac 240
caagtagtgt cttcctacct atctccagat acatgtcaaa aaa 283

<210> 182
<211> 401
<212> DNA
<213> Homo sapiens

<400> 182
atattcttgc tgcttatgca gctgacattg ttgccctccc taaagcaacc aagtagcctt 60
tatttcccac agtgaaagaa aacgctggcc tatcagttac attacaaaag gcagatttca 120
agaggattga gtaagtagtt ggatggcttt cataaaaaca agaattcaag aagaggattc 180
atgctttaag aaacatttgt tatacattcc tcacaaatta tacctgggat aaaaactatg 240
tagcaggcag tgtgttttcc ttccatgtct ctctgcacta cctgcagtgt gtcctctgag 300
gctgcaagtc tgtcctatct gaattcccag cagaagcact aagaagctcc accctatcac 360
ctagcagata aaactatggg gaaaacttaa atctgtgcat a 401

<210> 183
<211> 366
<212> DNA
<213> Homo sapiens

<220>
<221> unsure
<222> (325)

<223> n=A,T,C or G

<400> 183
accgtgtcca agtttttaga acccttgtta gccagaccga ggtgtcctgg tcaccgtttc 60
accatcatgc tttgatgttc ccctgtcttt ctctcttctg ctctcaagag caaaggttaa 120
tttaaggaca aagatgaagt cactgtaaac taatctgtca ttgtttttac cttccttttc 180
tttttcagtg cagaaattaa aagtaagtat aaagcaccgt gattgggagt gtttttgcgt 240
gtgtcggaat cactggtaaa tgttggctga gaacaatccc tccccttgca cttgtgaaaa 300
cactttgagc gctttaagag attancctga gaaataatta aatatctttt ctcttcaaaa 360
aaaaaa 366

<210> 184
<211> 370
<212> DNA
<213> Homo sapiens

<400> 184
tcttacttca aaagaaaaat aaacataaaa aataagttgc tggttcctaa caggaaaaat 60
tttaataatt gtactgagag aaactgctta cgtacacatt gcagatcaaa tatttggagt 120
taaaatgtta gtctacatag atgggtgatt gtaactttat tgccattaaa agatttcaaa 180
ttgcattcat gcttctgtgt acacataatg aaaaatgggc aaataatgaa gatctctcct 240
tcagtctgct ctgtttaatt ctgctgtctg ctcttctcta atgctgcgtc cctaattgta 300
cacagtttag tgatatctag gagtataaag ttgtcgccca tcaataaaaa tcacaaagtt 360
ggtttaaaaa 370

<210> 185
<211> 107
<212> DNA
<213> Homo sapiens

<400> 185
ctcatattat tttccttttg agaaattgga aactctttct gttgctatta tattaataaa 60
gttggtgttt attttctggt agtcaccttc cccatttaaa aaaaaaa 107

<210> 186
<211> 309
<212> DNA
<213> Homo sapiens

<400> 186
gaaaggatgg ctctggttgc cacagagctg ggacttcatg ttcttctaga gagggccaca 60
agagggccac aggggtggcc gggagttgtc agctgatgcc tgctgagagg caggaattgt 120
gccagtgagt gacagtcatg agggagtgtc tcttcttggg gaggaaagaa ggtagagcct 180
ttctgtctga atgaaaggcc aaggctacag tacagggccc cgccccagcc agggtgttaa 240
tgcccacgta gtggaggcct ctggcagatc ctgcattcca aggtcactgg actgtacgtt 300
tttatggtt 309

<210> 187
<211> 477
<212> DNA
<213> Homo sapiens

<400> 187
ttcagtccta gcaagaagcg agaattctga gatcctccag aaagtcgagc agcacccacc 60
tccaacctcg ggccagtgtc ttcaggcttt actggggacc tgcgagctgg cctaatgtgg 120

tggcctgcaa gccaggccat ccctgggcgc cacagacgag ctccgagcca ggtcaggctt 180
cggaggccac aagctcagcc tcaggcccag gcactgattg tggcagaggg gccactaccc 240
aaggtctagc taggcccaag acctagttac ccagacagtg agaagcccct ggaaggcaga 300
aaagttggga gcatggcaga cagggaaggg aaacattttc agggaaaaga catgtatcac 360
atgtcttcag aagcaagtca ggtttcatgt aaccgagtgt cctcttgcgt gtccaaaagt 420
agcccagggc tgtagcacag gcttcacagt gattttgtgt tcagccgtga gtcacac 477

<210> 188
<211> 220
<212> DNA
<213> Homo sapiens

<400> 188
taaatatggt agatattaat attcctctta gatgaccagt gattccaatt gtcccaagtt 60
ttaaataagt accctgtgag tatgagataa attagtgaca atcagaacaa gtttcagtat 120
cagatgttca agaggaagtt gctattgcat tgattttaat atttgtacat aaacactgat 180
ttttttgagc attattttgt atttgttgta ctttaatacc 220

<210> 189
<211> 417
<212> DNA
<213> Homo sapiens

<220>
<221> unsure
<222> (76)
<223> n=A,T,C or G
<221> unsure
<222> (77)
<223> n=A,T,C or G

<400> 189
accatcttga cagaggatac atgctcccaa aacgtttgtt accacactta aaaatcactg 60
ccatcattaa gcatcnnttt caaaattata gccattcatg atttactttt tccagatgac 120
tatcattatt ctagtccttt gaatttgtaa ggggaaaaaa aacaaaaaca aaaacttacg 180
atgcactttt ctccagcaca tcagatttca aattgaaaat taaagacatg ctatggtaat 240
gcacttgcta gtactacaca ctttgtacaa caaaaaacag aggcaagaaa caacggaaag 300
agaaaagcct tcctttgttg gcccttaaac tgagtcaaga tctgaaatgt agagatgatc 360
tctgacgata cctgtatgtt cttattgtgt aaataaaatt gctggtatga aatgaca 417

<210> 190
<211> 497
<212> DNA
<213> Homo sapiens

<400> 190
gcactgcggc gctctcccgt cccgcggtgg ttgctgctgc tgccgctgct gctgggcctg 60
aacgcaggag ctgtcattga ctggcccaca gaggagggca aggaagtatg ggattatgtg 120
acggtccgca aggatgccta catgttctgg tggctctatt atgccaccaa ctcctgcaag 180
aacttctcag aactgcccct ggtcatgtgg cttcagggcg gtccaggcgg ttctagcact 240
ggatttggaa actttgagga aattgggccc cttgacagtg atctcaaacc acggaaaacc 300
acctggctcc aggctgccag tctcctattt gtggataatc ccgtgggcac tgggttcagt 360
tatgtgaatg gtagtggtgc ctatgccaag gacctggcta tggtggcttc agacatgatg 420
gttctcctga agaccttctt cagttgccac aaagaattcc agacagttcc attctacatt 480
ttctcagagt cctatgg 497

<210> 191
<211> 175
<212> DNA
<213> Homo sapiens

<400> 191
atgttgaata ttttgcttat taactttgtt tattgtcttc tccctcgatt agaatattag 60
ctacttgagt acaaggattt gagcctgtta cattcactgc tgaattttag gctcctggaa 120
gatacccagc attcaataga gaccacacaa taaatatatg tcaaataaaa aaaaa 175

<210> 192
<211> 526
<212> DNA
<213> Homo sapiens

<400> 192
agtaaacatt attatttttt ttatatttgc aaaggaaaca tatctaatcc ttcctataga 60
aagaacagta ttgctgtaat tccttttctt ttcttcctca tttcctctgc cccttaaaag 120
attgaagaaa gagaaacttg tcaactcata tccacgttat ctagcaaagt acataagaat 180
ctatcactaa gtaatgtatc cttcagaatg tgttggttta ccagtgacac cccatattca 240
tcacaaaatt aaagcaagaa gtccatagta atttatttgc taatagtgga tttttaatgc 300
tcagagtttc tgaggtcaaa ttttatcttt tcacttacaa gctctatgat cttaaataat 360
ttacttaatg tattttggtg tattttcctc aaattaatat tggtgttcaa gactatatct 420
aattcctctg atcactttga gaaacaaact tttattaaat gtaaggcact tttctatgaa 480
ttttaaatat aaaaataaat attgttctga ttattactga aaaaaa 526

<210> 193
<211> 553
<212> DNA
<213> Homo sapiens

<220>
<221> unsure
<222> (290)
<223> n=A,T,C or G
<221> unsure
<222> (300)
<223> n=A,T,C or G
<221> unsure
<222> (411)
<223> n=A,T,C or G
<221> unsure
<222> (441)
<223> n=A,T,C or G

<400> 193
tccattgtgg tggaattcgc tctctggtaa aggcgtgcag gtgttggccg cggcctctga 60
gctgggatga gccgtgctcc cggtggaagc aagggagccc agccggagcc atggccagta 120
cagtggtagc agttggactg accattgctg ctgcaggatt tgcaggccgt tacgttttgc 180
aagccatgaa gcatatggag cctcaagtaa aacaagtttt tcaaagccta ccaaaatctg 240
ccttcagtgg tggctattat agaggtgggt ttgaacccaa aatgacaaan cgggaagcan 300
cattaatact aggtgtaagc cctactgcca ataaagggaa aataagagat gctcatcgac 360
gaattatgct tttaaatcat cctgacaaag gaggatctcc ttatatagca nccaaaatca 420
atgaagctaa agatttacta naaggtcaag ctaaaaaatg aagtaaatgt atgatgaatt 480

ttaagttcgt attagtttat gtatatgagt actaagtttt tataataaaa tgcctcagag 540
ctacaatttt aaa 553

<210> 194
<211> 320
<212> DNA
<213> Homo sapiens

<400> 194
cccttcccaa tccatcagta aagaccccat ctgccttgtc catgccgttt cccaacaggg 60
atgtcacttg atatgagaat ctcaaatctc aatgccttat aagcattcct tcctgtgtcc 120
attaagactc tgataattgt ctcccctcca taggaatttc tcccaggaaa gaaatatatc 180
cccatctccg tttcatatca gaactaccgt ccccgatatt cccttcagag agattaaaga 240
ccagaaaaaa gtgagcctct tcatctgcac ctgtaatagt ttcagttcct attttcttcc 300
attgacccat atttatacct 320

<210> 195
<211> 320
<212> DNA
<213> Homo sapiens

<220>
<221> unsure
<222> (203)
<223> n=A,T,C or G
<221> unsure
<222> (218)
<223> n=A,T,C or G

<400> 195
aagcatgacc tggggaaatg gtcagacctt gtattgtgtt tttggccttg aaagtagcaa 60
gtgaccagaa tctgccatgg caacaggctt taaaaaagac ccttaaaaag acactgtctc 120
aactgtggtg ttagcaccag ccagctctct gtacatttgc tagcttgtag ttttctaaga 180
ctgagtaaac ttcttatttt tanaaagggg aggctggntt gtaactttcc ttgtacttaa 240
ttgggtaaaa gtcttttcca caaaccacca tctattttgt gaactttgtt agtcatcttt 300
tatttggtaa attatgaact 320

<210> 196
<211> 357
<212> DNA
<213> Homo sapiens

<220>
<221> unsure
<222> (36)
<223> n=A,T,C or G

<400> 196
atataaaata atacgaaact ttaaaaagca ttggantgtc agtatgttga atcagtagtt 60
tcactttaac tgtaaacaat ttcttaggac accatttggg ctagtttctg tgtaagtgta 120
aatactacaa aaacttattt atactgttct tatgtcattt gttatattca tagatttata 180
tgatgatatg acatctggct aaaaagaaat tattgcaaaa ctaaccacta tgtacttttt 240
tataaatact gtatggacaa aaaatggcat tttttatatt aaattgttta gctctggcaa 300
aaaaaaaaaa ttttaagagc tggtactaat aaaggattat tatgactgtt aaaaaaa 357

<210> 197
<211> 565
<212> DNA
<213> Homo sapiens

<220>
<221> unsure
<222> (27)
<223> n=A,T,C or G

<400> 197
tcagctgagt accatcagga tatttanccc tttaagtgct gttttgggag tagaaaacta 60
aagcaacaat acttcctctt gacagctttg attggaatgg ggttattaga tcattcacct 120
tggtcctaca ctttttagga tgcttggtga acataacacc acttataatg aacatccctg 180
gttcctatat tttgggctat gtgggtagga attgttactt gttactgcag cagcagccct 240
agaaagtaag cccagggctt cagatctaag ttagtccaaa agctaaatga tttaaagtca 300
agttgtaatg ctaggcataa gcactctata atacattaaa ttataggccg agcaattagg 360
gaatgtttct gaaacattaa acttgtattt atgtcactaa aattctaaca caaacttaaa 420
aaatgtgtct catacatatg ctgtactagg cttcatcatg catttctaaa tttgtgtatg 480
atttgaatat atgaaagaat ttatacaaga gtgttattta aaattattaa aaataaatgt 540
atataatttg tacctattgt aaaaa 565

<210> 198
<211> 484
<212> DNA
<213> Homo sapiens

<400> 198
tatgtaagta ttggtgtctg ctttaaaaaa ggagacccag acttcacctg tcctttttaa 60
acatttgaga acagtgttac tctgagcagt tgggccacct tcaccttatc cgacagctga 120
ctgttggatg tgtccattgt cgccagtttg gctgttgccc ggacaggaca ggacctccat 180
tgggcgcagc agcaggtggc aggggtgtgg cttgaggtgg gtggcagcgt ctggtcctcc 240
tctctggtgc tttctgagag ggtctctaaa gcagagtgtg gttggcctgg gggaaggcag 300
agcacgtatt tctcccctct agtacctctg catttgtgag tgttccctct ggctttctga 360
agggcagcag actcttgagt atactgcaga ggacatgctt tatcagtagg tcctgagggc 420
tccaggggct caactgacca agtaacacag aagttggggt atgtggccta tttgggtcgg 480
aaac 484

<210> 199
<211> 429
<212> DNA
<213> Homo sapiens

<220>
<221> unsure
<222> (77)
<223> n=A,T,C or G
<221> unsure
<222> (88)
<223> n=A,T,C or G
<221> unsure
<222> (134)
<223> n=A,T,C or G
<221> unsure
<222> (151)

<223> n=A,T,C or G
<221> unsure
<222> (189)
<223> n=A,T,C or G
<221> unsure
<222> (227)
<223> n=A,T,C or G
<221> unsure
<222> (274)
<223> n=A,T,C or G
<221> unsure
<222> (319)
<223> n=A,T,C or G

<400> 199
gcttatgttt tttgttttaa cttttgtttt ttaacattta gaatattaca ttttgtatta 60
tacagtacct ttctcanaca ttttgtanaa ttcatttcgg cagctcacta ggattttgct 120
gaacattaaa aagngtgata gcgatattag ngccaatcaa atggaaaaaa ggtagtctta 180
ataaacaana cacaacgttt ttatacaaca tactttaaaa tattaanaaa actccttaat 240
attgtttcct attaagtatt attctttggg caanattttc tgatgctttt gattttctct 300
caatttagca tttgctttng gtttttttct ctatttagca ttctgttaag gcacaaaaac 360
tatgtactgt atgggaaatg ttgtaaatat taccttttcc acattttaaa cagacaactt 420
tgaatccaa 429

<210> 200
<211> 279
<212> DNA
<213> Homo sapiens

<400> 200
gctttttgga ggaattacag ggaagctcct ggaattgtac atggatatct ttatccctag 60
ggggaaatca aggagctggg cacccctaat tctttatgga agtgtttaaa actattttaa 120
ttttattaca agtattacta gagtagtggt tctactctaa gatttcaaaa gtgcatttaa 180
aatcatacat gttcccgcct gcaaatatat tgttattttg gtggagaaaa aaatagtata 240
ttctacataa aaaattaaag atattaacta agaaaaaaa 279

<210> 201
<211> 569
<212> DNA
<213> Homo sapiens

<400> 201
taggtcagta tttttagaaa ctcttaatag ctcatactct tgataccaaa agcagccctg 60
attgttaaag cacacacctg cacaagaagc agtgatggtt gcatttacat ttcctgggtg 120
cacaaaaaaa aattctcaaa aagcaaggac ttacgctttt tgcaaagcct ttgagaagtt 180
actggatcat aggaagctta taacaagaat ggaagattct taaataactc actttctttg 240
gtatccagta acagtagatg ttcaaaatat gtagctgatt aataccagca ttgtgaacgc 300
tgtacaacct tgtggttatt actaagcaag ttactactag cttctgaaaa gtagcttcat 360
aattaatgtt atttatacac tgccttccat gacttttact ttgccctaag ctaatctcca 420
aaatctgaaa tgctactcca atatcagaaa aaaaggggga ggtggaatta tatttcctgt 480
gattttaaga gtacagagaa tcatgcacat ctctgattag ttcatatatg tctagtgtgt 540
aataaaagtc aaagatgaac tctcaaaaa 569

<210> 202
<211> 501

<212> DNA
<213> Homo sapiens

<400> 202
attaataggc ttaataattg ttggcaagga tccttttgct ttctttggca tgcaagctcc 60
tagcatctgg cagtggggcc aagaaaataa ggtttatgca tgtatgatgg ttttcttctt 120
gagcaacatg attgagaacc agtgtatgtc aacaggtgca tttgagataa ctttaaatga 180
tgtacctgtg tggtctaagc tggaatctgg tcaccttcca tccatgcaac aacttgttca 240
aattcttgac aatgaaatga agctcaatgt gcatatggat tcaatcccac accatcgatc 300
atagcaccac ctatcagcac tgaaaactct tttgcattaa gggatcattg caagagcagc 360
gtgactgaca ttatgaaggc ctgtactgaa gacagcaagc tgttagtaca gaccagatgc 420
tttcttggca ggctcgttgt acctcttgga aaacctcaat gcaagatagt gtttcagtgc 480
tggcatattt tggaattctg c 501

<210> 203
<211> 261
<212> DNA
<213> Homo sapiens

<220>
<221> unsure
<222> (36)
<223> n=A,T,C or G
<221> unsure
<222> (96)
<223> n=A,T,C or G

<400> 203
gacaagctcc tggtcttgag atgtcttctc gttaangaga tgggcctttt ggaggtaaag 60
gataaaatga atgagttctg tcatgattca ctattntata acttgcatga cctttactgt 120
gttagctctt tgaatgttct tgaaatttta gactttcttt gtaaacaaat gatatgtcct 180
tatcattgta taaaagctgt tatgtgcaac agtgtggaga ttccttgtct gatttaataa 240
aatacttaaa cactgaaaaa a 261

<210> 204
<211> 421
<212> DNA
<213> Homo sapiens

<400> 204
agcatctttt ctacaacgtt aaaattgcag aagtagctta tcattaaaaa acaacaacaa 60
caacaataac aataaatcct aagtgtaaat cagttattct accccctacc aaggatatca 120
gcctgttttt tccctttttt ctcctgggaa taattgtggg cttcttccca aatttctaca 180
gcctctttcc tcttctcatg cttgagcttc cctgtttgca cgcatgcgtg tgcaggactg 240
gcttgtgtgc ttggactcgg ctccaggtgg aagcatgctt tcccttgtta ctgttggaga 300
aactcaaacc ttcaagccct aggtgtagcc attttgtcaa gtcatcaact gtatttttgt 360
actggcatta acaaaaaaag aagataaaat attgtaccat taaactttaa taaaacttta 420
a 421

<210> 205
<211> 460
<212> DNA
<213> Homo sapiens

<400> 205

tactctcaca atgaaggacc tggaatgaaa aatctgtgtc taaacaagtc ctctttagat 60
tttagtgcaa atccagagcc agcgtcggtt gcctcgagta attctttcat gggtaccttt 120
ggaaaagctc tcaggagacc tcacctagat gcctattcaa gctttggaca gccatcagat 180
tgtcagccaa gagcctttta tttgaaagct cattcttccc cagacttgga ctctgggtca 240
gaggaagatg ggaaagaaag gacagatttt caggaagaaa atcacatttg tacctttaaa 300
cagactttag aaaactacag gactccaaat tttcagtctt atgacttgga cacatagact 360
gaatgagacc aaaggaaaag cttaacatac tacctcaagg tgaactttta tttaaaagag 420
agagaatctt atgtttttta aatggagtta tgaattttaa 460

<210> 206
<211> 481
<212> DNA
<213> Homo sapiens

<400> 206
tgtggtggaa ttcgggacgc ccccagaccc tgactttttc ctgcgtgggc cgtctcctcc 60
tgcggaagca gtgacctctg acccctggtg accttcgctt tgagtgcctt ttgaacgctg 120
gtcccgcggg acttggtttt ctcaagctct gtctgtccaa agacgctccg gtcgaggtcc 180
cgcctgccct gggtggatac ttgaacccca gacgcccctc tgtgctgctg tgtccggagg 240
cggccttccc atctgcctgc ccacccggag ctctttccgc cggcgcaggg tcccaagccc 300
acctcccgcc ctcagtcctg cggtgtgcgt ctgggcacgt cctgcacaca caatgcaagt 360
cctggcctcc gcgcccgccc gcccacgcga gccgtacccg ccgccaactc tgttatttat 420
ggtgtgaccc cctggaggtg ccctcggccc accggggcta tttattgttt aatttatttg 480
t 481

<210> 207
<211> 605
<212> DNA
<213> Homo sapiens

<400> 207
accctttttg gattcagggc tcctcacaat taaaatgagt gtaatgaaac aaggtgaaaa 60
tatagaagca tccctttgta tactgttttg ctacttacag tgtacttggc attgctttat 120
ctcactggat tctcacggta ggatttctga gatcttaatc taagctccaa agttgtctac 180
ttttttgatc ctagggtgct ccttttgttt tacagagcag ggtcacttga tttgctagct 240
ggtggcagaa ttggcaccat tacccaggtc tgactgacca ccagtcagag gcactttatt 300
tgtatcatga aatgatttga aatcattgta aagcagcgaa gtctgataat gaatgccagc 360
tttccttgtg ctttgataac aaagactcca aatattctgg agaacctgga taaaagtttg 420
aagggctaga ttgggatttg aagacaaaat tgtaggaaat cttacatttt tgcaataaca 480
aacattaatg aaagcaaaac attataaaag taattttaat tcaccacata cttatcaatt 540
tcttgatgct tccaaatgac atctaccaga tatggttttg tggacatctt tttctgttta 600
cataa 605

<210> 208
<211> 655
<212> DNA
<213> Homo sapiens

<400> 208
ggcgttgttc tggattcccg tcgtaactta aagggaaact ttcacaatgt ccggagccct 60
tgatgtcctg caaatgaagg aggaggatgt ccttaagttc cttgcagcag gaacccactt 120
aggtggcacc aatcttgact tccagatgga acagtacatc tataaaagga aaagtgatgg 180
catctatatc ataaatctca agaggacctg ggagaagctt ctgctggcag ctcgtgcaat 240
tgttgccatt gaaaaccctg ctgatgtcag tgttatatcc tccaggaata ctggccagag 300
ggctgtgctg aagtttgctg ctgccactgg agccactcca attgctggcc gcttcactcc 360

tggaaccttc actaaccaga tccaggcagc cttccgggag ccacggcttc ttgtggttac 420
tgaccccagg gctgaccacc agcctctcac ggaggcatct tatgttaacc tacctaccat 480
tgcgctgtgt aacacagatt ctcctctgcg ctatgtggac attgccatcc catgcaacaa 540
caagggagct cactcagtgg gtttgatgtg gtggatgctg gctcgggaag ttctgcgcat 600
gcgtggcacc atttcccgtg aacacccatg ggaggtcatg cctgatctgt acttc 655

<210> 209
<211> 621
<212> DNA
<213> Homo sapiens

<400> 209
catttagaac atggttatca tccaagacta ctctaccctg caacattgaa ctcccaagag 60
caaatccaca ttcctcttga gttctgcagc ttctgtgtaa atagggcagc tgtcgtctat 120
gccgtagaat cacatgatct gaggaccatt catggaagct gctaaatagc ctagtctggg 180
gagtcttcca taaagttttg catggagcaa acaaacagga ttaaactagg tttggttcct 240
tcagccctct aaaagcatag ggcttagcct gcaggcttcc ttgggctttc tctgtgtgtg 300
tagttttgta aacactatag catctgttaa gatccagtgt ccatggaaac cttcccacat 360
gccgtgactc tggactatat cagtttttgg aaagcagggt tcctctgcct gctaacaagc 420
ccacgtggac cagtctgaat gtctttcctt tacacctatg tttttaaata gtcaaacttc 480
aagaaacaat ctaaacaagt ttctgttgca tatgtgtttg tgaacttgta tttgtattta 540
gtaggcttct atattgcatt taacttgttt ttgtaactcc tgattcttcc ttttcggata 600
ctattgatga ataaagaaat t 621

<210> 210
<211> 533
<212> DNA
<213> Homo sapiens

<220>
<221> unsure
<222> (20)
<223> n=A,T,C or G
<221> unsure
<222> (21)
<223> n=A,T,C or G
<221> unsure
<222> (61)
<223> n=A,T,C or G

<400> 210
cgccttgggg agccggcggn ngagtccggg acgtggagac ccggggtccc ggcagccggg 60
nggcccgcgg gcccagggtg gggatgcacc gccgcggggt gggagctggc gccatcgcca 120
agaagaaact tgcagaggcc aagtataagg agcgagggac ggtcttggct gaggaccagc 180
tagcccagat gtcaaagcag ttggacatgt tcaagaccaa cctggaggaa tttgccagca 240
aacacaagca ggagatccgg aagaatcctg agttccgtgt gcagttccag gacatgtgtg 300
caaccattgg cgtggatccg ctggcctctg gaaaaggatt ttggtctgag atgctgggcg 360
tgggggactt ctattacgaa ctaggtgtcc aaattatcga agtgtgcctg gcgctgaagc 420
atcggaatgg aggtctgata actttggagg aactacatca acaggtgttg aagggaaggg 480
gcaagttcgc ccaggatgtc agtcaagatg acctgatcag agccatcaag aaa 533

<210> 211
<211> 451
<212> DNA
<213> Homo sapiens

<400> 211
ttagcttgag ccgagaacga ggcgagaaag ctggagaccg aggagaccgc ctagagcgga 60
gtgaacgggg aggggaccgt ggggaccggc ttgatcgtgc gcggacacct gctaccaagc 120
ggagcttcag caaggaagtg gaggagcgga gtagagaacg gccctcccag cctgaggggc 180
tgcgcaaggc agctagcctc acggaggatc gggaccgtgg gcgggatgcc gtgaagcgag 240
aagctgccct acccccagtg agccccctga aggcggctct ctctgaggag gagttagaga 300
agaaatccaa ggctatcatt gaggaatatc tccatctcaa tgacatgaaa gaggcagtcc 360
agtgcgtgca ggagctggcc tcaccctcct tgctcttcat ctttgtacgg catggtgtcg 420
agtctacgct ggagcgcagt gccattgctc g 451

<210> 212
<211> 471
<212> DNA
<213> Homo sapiens

<220>
<221> unsure
<222> (54)
<223> n=A,T,C or G

<400> 212
gtgattattc ttgatcaggg agaagatcat ttagatttgt tttgcattcc ttanaatgga 60
gggcaacatt ccacagctgc cctggctgtg atgagtgtcc ttgcaggggc cggagtagga 120
gcactggggt gggggcggaa ttggggttac tcgatgtaag ggattccttg ttgttgtgtt 180
gagatccagt gcagttgtga tttctgtgga tcccagcttg gttccaggaa ttttgtgtga 240
ttggcttaaa tccagttttc aatcttcgac agctgggctg gaacgtgaac tcagtagctg 300
aacctgtctg acccggtcac gttcttggat cctcagaact ctttgctctt gtcggggtgg 360
gggtgggaac tcacgtgggg agcggtggct gagaaaatgt aaggattctg gaatacatat 420
tccatgggac tttccttccc tctcctgctt cctcttttcc tgctccctaa c 471

<210> 213
<211> 511
<212> DNA
<213> Homo sapiens

<220>
<221> unsure
<222> (27)
<223> n=A,T,C or G
<221> unsure
<222> (63)
<223> n=A,T,C or G
<221> unsure
<222> (337)
<223> n=A,T,C or G
<221> unsure
<222> (442)
<223> n=A,T,C or G

<400> 213
ctaattagaa acttgctgta ctttttnttt tcttttaggg gtcaaggacc ctctttatag 60
ctnccatttg cctacaataa attattgcag cagtttgcaa tactaaaata ttttttatag 120
actttatatt tttccttttg ataaagggat gctgcatagt agagttggtg taattaaact 180
atctcagccg tttccctgct ttcccttctg ctccatatgc ctcattgtcc ttccagggag 240

ctcttttaat cttaaagttc tacatttcat gctcttagtc aaattctgtt acctttttaa 300
taactcttcc cactgcatat ttccatcttg aattggnggt tctaaattct gaaactgtag 360
ttgagataca gctatttaat atttctggga gatgtgcatc cctcttcttt gtggttgccc 420
aaggttgttt tgcgtaactg anactccttg atatgcttca gagaatttag gcaaacactg 480
gccatggccg tgggagtact gggagtaaaa t 511

<210> 214
<211> 521
<212> DNA
<213> Homo sapiens

<400> 214
agcattgcca aataatccct aattttccac taaaaatata atgaaatgat gttaagcttt 60
ttgaaaagtt taggttaaac ctactgttgt tagattaatg tatttgttgc ttccctttat 120
ctggaatgtg gcattagctt ttttatttta accctcttta attcttattc aattccatga 180
cttaaggttg gagagctaaa cactgggatt tttggataac agactgacag ttttgcataa 240
ttataatcgg cattgtacat agaaaggata tggctacctt ttgttaaatc tgcactttct 300
aaatatcaaa aaagggaaat gaagtataaa tcaatttttg tataatctgt ttgaaacatg 360
agttttattt gcttaatatt agggctttgc cccttttctg taagtctctt gggatcctgt 420
gtagaagctg ttctcattaa acaccaaaca gttaagtcca ttctctggta ctagctacaa 480
attcggtttc atattctact taacaattta aataaactga a 521

<210> 215
<211> 381
<212> DNA
<213> Homo sapiens

<220>
<221> unsure
<222> (17)
<223> n=A,T,C or G
<221> unsure
<222> (20)
<223> n=A,T,C or G
<221> unsure
<222> (60)
<223> n=A,T,C or G
<221> unsure
<222> (61)
<223> n=A,T,C or G
<221> unsure
<222> (365)
<223> n=A,T,C or G

<400> 215
gagcggagag cggaccngtn agagccctga gcagccccac cgccgccgcc ggcctagttn 60
ncatcacacc ccgggaggag ccgcagctgc cgcagccggc cccagtcacc atcaccgcaa 120
ccatgagcag cgaggccgag acccagcagc cgcccgccgc cccccccgcc gcccccgccc 180
tcagcgccgc cgacaccaag cccggcacta cgggcagcgg cgcagggagc ggtggcccgg 240
gcggcctcac atcggcggcg cctgccggcg gggacaagaa ggtcatcgca acgaaggttt 300
tgggaacagt aaaatggttc aatgtaagga acggatatgg tttcatcaac aggaatgaca 360
ccaangaaga tgtatttgta c 381

<210> 216
<211> 425

<212> DNA
<213> Homo sapiens

<400> 216
ttactaacta ggtcattcaa ggaagtcaag ttaacttaaa catgtcacct aaatgcactt 60
gatggtgttg aaatgtccac cttcttaaat ttttaagatg aacttagttc taaagaagat 120
aacaggccaa tcctgaaggt actccctgtt tgctgcagaa tgtcagatat tttggatgtt 180
gcataagagt cctatttgcc ccagttaatt caacttttgt ctgcctgttt tgtggactgg 240
ctggctctgt tagaactctg tccaaaaagt gcatggaata taacttgtaa agcttcccac 300
aattgacaat atatatgcat gtgtttaaac caaatccaga aagcttaaac aatagagctg 360
cataatagta tttattaaag aatcacaact gtaaacatga gaataactta aggattctag 420
tttag 425

<210> 217
<211> 181
<212> DNA
<213> Homo sapiens

<400> 217
gagaaaccaa atgataggtt gtagagcctg atgactccaa acaaagccat cacccgcatt 60
cttcctcctt cttctggtgc tacagctcca agggcccttc accttcatgt ctgaaatgga 120
actttggctt tttcagtgga agaatatgtt gaaggtttca ttttgttcta gaaaaaaaaa 180
a 181

<210> 218
<211> 405
<212> DNA
<213> Homo sapiens

<400> 218
caggccttcc agttcactga caaacatggg gaagtgtgcc cagctggctg gaaacctggc 60
agtgatacca tcaagcctga tgtccaaaag agcaaagaat atttctccaa gcagaagtga 120
gcgctgggct gttttagtgc caggctgcgg tgggcagcca tgagaacaaa acctcttctg 180
tatttttttt ttccattagt aaaacacaag acttcagatt cagccgaatt gtggtgtctt 240
acaaggcagg cctttcctac agggggtgga gagaccagcc tttcttcctt tggtaggaat 300
ggcctgagtt ggcgttgtgg gcaggctact ggtttgtatg atgtattagt agagcaaccc 360
attaatcttt tgtagtttgt attaaacttg aactgagaaa aaaaa 405

<210> 219
<211> 216
<212> DNA
<213> Homo sapiens

<220>
<221> unsure
<222> (207)
<223> n=A,T,C or G
<221> unsure
<222> (210)
<223> n=A,T,C or G

<400> 219
actccaagag ttagggcagc agagtggagc gatttagaaa gaacatttta aaacaatcag 60
ttaatttacc atgtaaaatt gctgtaaatg ataatgtgta cagattttct gttcaaatat 120
tcaattgtaa acttcttgtt aagactgtta cgtttctatt gcttttgtat gggatattgc 180

aaaaataaaa aggaaagaac cctcttnaan aaaaaa 216

<210> 220
<211> 380
<212> DNA
<213> Homo sapiens

<400> 220
cttacaaatt gcccccatgt gtaggggaca cagaaccctt tgagaaaact tagatttttg 60
tctgtacaaa gtctttgcct ttttccttct tcattttttt ccagtacatt aaatttgtca 120
atttcatctt tgagggaaac tgattagatg ggttgtgttt gtgttctgat ggagaaaaca 180
gcaccccaag gactcagaag atgattttaa cagttcagaa cagatgtgtg caatattggt 240
gcatgtaata atgttgagtg gcagtcaaaa gtcatgattt ttatcttagt tcttcattac 300
tgcattgaaa aggaaaacct gtctgagaaa atgcctgaca gtttaattta aaactatggt 360
gtaagtcttt gacaaaaaaa 380

<210> 221
<211> 398
<212> DNA
<213> Homo sapiens

<400> 221
ggttagtaag ctgtcgactt tgtaaaaaag ttaaaaatga aaaaaaaagg aaaaatgaat 60
tgtatattta atgaatgaac atgtacaatt tgccactggg aggaggttcc tttttgttgg 120
gtgagtctgc aagtgaattt cactgatgtt gatattcatt gtgtgtagtt ttatttcggt 180
cccagccccg tttcctttta ttttggagct aatgccagct gcgtgtctag ttttgagtgc 240
agtaaaatag aatcagcaaa tcactcttat ttttcatcct tttccggtat tttttgggtt 300
gtttctgtgg gagcagtgta caccaactct tcctgtatat tgcctttttg ctggaaaatg 360
ttgtatgttg aataaaattt tctataaaaa ttaaaaaa 398

<210> 222
<211> 301
<212> DNA
<213> Homo sapiens

<220>
<221> unsure
<222> (49)
<223> n=A,T,C or G
<221> unsure
<222> (64)
<223> n=A,T,C or G

<400> 222
ttcgataatt gatctcatgg gctttccctg gaggaaaggt tttttttgnt gtttattttt 60
taanaacttg aaacttgtaa actgagatgt ctgtagcttt tttgcccatc tgtagtgtat 120
gtgaagattt caaaacctga gagcactttt tctttgttta gaattatgag aaaggcacta 180
gatgacttta ggatttgcat ttttcccttt attgcctcat ttcttgtgac gccttgttgg 240
ggagggaaat ctgtttattt tttcctacaa ataaaaagct aagattctat atcgcaaaaa 300
a 301

<210> 223
<211> 200
<212> DNA
<213> Homo sapiens

<400> 223
gtaagtgctt aggaagaaac tttgcaaaca tttaatgagg atacactgtt catttttaaa 60
attccttcac actgtaattt aatgtgtttt atattctttt gtagtaaaac aacataactc 120
agatttctac aggagacagt ggttttattt ggattgtctt ctgtaatagg tttcaataaa 180
gctggatgaa cttaaaaaaa 200

<210> 224
<211> 385
<212> DNA
<213> Homo sapiens

<400> 224
gaaaggtttg atccggactc aaagaaagca aaggagtgtg agccgccatc tgctggagca 60
gctgtaactg caagacctgg acaagagatt cgtcagcgaa ctgcagctca aagaaacctt 120
tctccaacac cagcaagccc taaccagggc cctcctccac aagttccagt atctcctgga 180
ccaccaaagg acagttctgc ccctggtgga cccccagaaa ggactgttac tccagcccta 240
tcatcaaatg tgttaccaag acatcttgga tcccctgcta cttcagtgcc tggaatgggt 300
aaacagagca cttaatgtta tttacagttt atattgtttt ctctggttac caataaaacg 360
ggccattttc aggtggtaaa aaaaa 385

<210> 225
<211> 560
<212> PRT
<213> Homo sapien

<400> 225
Met Glu Cys Leu Tyr Tyr Phe Leu Gly Phe Leu Leu Leu Ala Ala Arg
1               5                   10                  15
Leu Pro Leu Asp Ala Ala Lys Arg Phe His Asp Val Leu Gly Asn Glu
            20                  25                  30
Arg Pro Ser Ala Tyr Met Arg Glu His Asn Gln Leu Asn Gly Trp Ser
        35                  40                  45
Ser Asp Glu Asn Asp Trp Asn Glu Lys Leu Tyr Pro Val Trp Lys Arg
    50                  55                  60
Gly Asp Met Arg Trp Lys Asn Ser Trp Lys Gly Gly Arg Val Gln Ala
65                  70                  75                  80
Val Leu Thr Ser Asp Ser Pro Ala Leu Val Gly Ser Asn Ile Thr Phe
                85                  90                  95
Ala Val Asn Leu Ile Phe Pro Arg Cys Gln Lys Glu Asp Ala Asn Gly
            100                 105                 110
Asn Ile Val Tyr Glu Lys Asn Cys Arg Asn Glu Ala Gly Leu Ser Ala
        115                 120                 125
Asp Pro Tyr Val Tyr Asn Trp Thr Ala Trp Ser Glu Asp Ser Asp Gly
    130                 135                 140
Glu Asn Gly Thr Gly Gln Ser His His Asn Val Phe Pro Asp Gly Lys
145                 150                 155                 160
Pro Phe Pro His His Pro Gly Trp Arg Arg Trp Asn Phe Ile Tyr Val
                165                 170                 175
Phe His Thr Leu Gly Gln Tyr Phe Gln Lys Leu Gly Arg Cys Ser Val
            180                 185                 190
Arg Val Ser Val Asn Thr Ala Asn Val Thr Leu Gly Pro Gln Leu Met
        195                 200                 205
Glu Val Thr Val Tyr Arg Arg His Gly Arg Ala Tyr Val Pro Ile Ala

210                 215                 220
Gln Val Lys Asp Val Tyr Val Val Thr Asp Gln Ile Pro Val Phe Val
225                 230                 235                 240
Thr Met Phe Gln Lys Asn Asp Arg Asn Ser Ser Asp Glu Thr Phe Leu
                245                 250                 255
Lys Asp Leu Pro Ile Met Phe Asp Val Leu Ile His Asp Pro Ser His
            260                 265                 270
Phe Leu Asn Tyr Ser Thr Ile Asn Tyr Lys Trp Ser Phe Gly Asp Asn
        275                 280                 285
Thr Gly Leu Phe Val Ser Thr Asn His Thr Val Asn His Thr Tyr Val
    290                 295                 300
Leu Asn Gly Thr Phe Ser Leu Asn Leu Thr Val Lys Ala Ala Ala Pro
305                 310                 315                 320
Gly Pro Cys Pro Pro Pro Pro Pro Pro Pro Arg Pro Ser Lys Pro Thr
                325                 330                 335
Pro Ser Leu Gly Pro Ala Gly Asp Asn Pro Leu Glu Leu Ser Arg Ile
            340                 345                 350
Pro Asp Glu Asn Cys Gln Ile Asn Arg Tyr Gly His Phe Gln Ala Thr
        355                 360                 365
Ile Thr Ile Val Glu Gly Ile Leu Glu Val Asn Ile Ile Gln Met Thr
    370                 375                 380
Asp Val Leu Met Pro Val Pro Trp Pro Glu Ser Ser Leu Ile Asp Phe
385                 390                 395                 400
Val Val Thr Cys Gln Gly Ser Ile Pro Thr Glu Val Cys Thr Ile Ile
                405                 410                 415
Ser Asp Pro Thr Cys Glu Ile Thr Gln Asn Thr Val Cys Ser Pro Val
            420                 425                 430
Asp Val Asp Glu Met Cys Leu Leu Thr Val Arg Arg Thr Phe Asn Gly
        435                 440                 445
Ser Gly Thr Tyr Cys Val Asn Leu Thr Leu Gly Asp Asp Thr Ser Leu
    450                 455                 460
Ala Leu Thr Ser Thr Leu Ile Ser Val Pro Asp Arg Asp Pro Ala Ser
465                 470                 475                 480
Pro Leu Arg Met Ala Asn Ser Ala Leu Ile Ser Val Gly Cys Leu Ala
                485                 490                 495
Ile Phe Val Thr Val Ile Ser Leu Leu Val Tyr Lys Lys His Lys Glu
            500                 505                 510
Tyr Asn Pro Ile Glu Asn Ser Pro Gly Asn Val Val Arg Ser Lys Gly
        515                 520                 525
Leu Ser Val Phe Leu Asn Arg Ala Lys Ala Val Phe Phe Pro Gly Asn
    530                 535                 540
Gln Glu Lys Asp Pro Leu Leu Lys Asn Gln Glu Phe Lys Gly Val Ser
545                 550                 555                 560

<210> 226
<211> 9
<212> PRT
<213> Homo sapien

<400> 226
Ile Leu Ile Pro Ala Thr Trp Lys Ala
1               5

<210> 227
<211> 9

<212> PRT
<213> Homo sapien

<400> 227
Phe Leu Leu Asn Asp Asn Leu Thr Ala
1               5

<210> 228
<211> 9
<212> PRT
<213> Homo sapien

<400> 228
Leu Leu Gly Asn Cys Leu Pro Thr Val
1               5

<210> 229
<211> 10
<212> PRT
<213> Homo sapien

<400> 229
Lys Leu Leu Gly Asn Cys Leu Pro Thr Val
1               5                   10

<210> 230
<211> 10
<212> PRT
<213> Homo sapien

<400> 230
Arg Leu Thr Gly Gly Leu Lys Phe Phe Val
1               5                   10

<210> 231
<211> 9
<212> PRT
<213> Homo sapien

<400> 231
Ser Leu Gln Ala Leu Lys Val Thr Val
1               5

<210> 232
<211> 20
<212> PRT
<213> Homo sapiens

<400> 232
Ala Gly Ala Asp Val Ile Lys Asn Asp Gly Ile Tyr Ser Arg Tyr Phe
                5                   10                  15

Phe Ser Phe Ala
            20

<210> 233
<211> 21
<212> PRT
<213> Homo sapiens

<400> 233
Phe Phe Ser Phe Ala Ala Asn Gly Arg Tyr Ser Leu Lys Val His Val
                5                   10                  15

Asn His Ser Pro Ser
            20


<210> 234
<211> 20
<212> PRT
<213> Homo sapiens

<400> 234
Phe Leu Val Thr Trp Gln Ala Ser Gly Pro Pro Glu Ile Ile Leu Phe
                5                   10                  15

Asp Pro Asp Gly
            20


<210> 235
<211> 20
<212> PRT
<213> Homo sapiens

<400> 235
Leu Gln Ser Ala Val Ser Asn Ile Ala Gln Ala Pro Leu Phe Ile Pro
                5                   10                  15

Pro Asn Ser Asp
            20


<210> 236
<211> 20
<212> PRT
<213> Homo sapiens

<400> 236
Ile Gln Asp Asp Phe Asn Asn Ala Ile Leu Val Asn Thr Ser Lys Arg
                5                   10                  15

Asn Pro Gln Gln
            20


<210> 237

<211> 21
<212> PRT
<213> Homo sapiens

<400> 237
Arg Asn Ser Leu Gln Ser Ala Val Ser Asn Ile Ala Gln Ala Pro Leu
                5                   10                  15

Phe Ile Pro Pro Asn
            20


<210> 238
<211> 20
<212> PRT
<213> Homo sapiens

<400> 238
Thr His Glu Ser His Arg Ile Tyr Val Ala Ile Arg Ala Met Asp Arg
                5                   10                  15

Asn Ser Leu Gln
            20


<210> 239
<211> 20
<212> PRT
<213> Homo sapiens

<400> 239
Arg Asn Pro Gln Gln Ala Gly Ile Arg Glu Ile Phe Thr Phe Ser Pro
                5                   10                  15

Gln Ile Ser Thr
            20


<210> 240
<211> 21
<212> PRT
<213> Homo sapiens

<400> 240
Gly Gln Ala Thr Ser Tyr Glu Ile Arg Met Ser Lys Ser Leu Gln Asn
                5                   10                  15

Ile Gln Asp Asp Phe
            20


<210> 241
<211> 20
<212> PRT
<213> Homo sapiens

<400> 241
Glu Arg Lys Trp Gly Phe Ser Arg Val Ser Ser Gly Gly Ser Phe Ser
1               5                   10                  15

Val Leu Gly Val
            20

<210> 242
<211> 20
<212> PRT
<213> Homo sapiens

<400> 242
Gly Ser His Ala Met Tyr Val Pro Gly Tyr Thr Ala Asn Gly Asn Ile
1               5                   10                  15

Gln Met Asn Ala
            20

<210> 243
<211> 20
<212> PRT
<213> Homo sapiens

<400> 243
Val Asn His Ser Pro Ser Ile Ser Thr Pro Ala His Ser Ile Pro Gly
1               5                   10                  15

Ser His Ala Met
            20

<210> 244
<211> 20
<212> PRT
<213> Homo sapiens

<400> 244
Ala Val Pro Pro Ala Thr Val Glu Ala Phe Val Glu Arg Asp Ser Leu
1               5                   10                  15

His Phe Pro His
            20

<210> 245
<211> 20
<212> PRT
<213> Homo sapiens

<400> 245
Lys Pro Gly His Trp Thr Tyr Thr Leu Asn Asn Thr His His Ser Leu

5                   10                   15

Gln Ala Leu Lys
            20



<210> 246
<211> 20
<212> PRT
<213> Homo sapiens

<400> 246
Asn Leu Thr Phe Arg Thr Ala Ser Leu Trp Ile Pro Gly Thr Ala Lys
                5                   10                   15

Pro Gly His Trp
            20



<210> 247
<211> 20
<212> PRT
<213> Homo sapiens

<400> 247
Leu His Phe Pro His Pro Val Met Ile Tyr Ala Asn Val Lys Gln Gly
                5                   10                   15

Phe Tyr Pro Ile
            20



<210> 248
<211> 20
<212> PRT
<213> Homo sapiens

<400> 248
Pro Glu Thr Gly Asp Pro Val Thr Leu Arg Leu Leu Asp Asp Gly Ala
                5                   10                   15

Gly Ala Asp Val
            20



<210> 249
<211> 20
<212> PRT
<213> Homo sapiens

<400> 249
Gly Phe Tyr Pro Ile Leu Asn Ala Thr Val Thr Ala Thr Val Glu Pro
                5                   10                   15

Glu Thr Gly Asp

            20

<210> 250
<211> 20
<212> PRT
<213> Homo sapiens

<400> 250
Phe Asp Pro Asp Gly Arg Lys Tyr Tyr Thr Asn Asn Phe Ile Thr Asn
                5                   10                  15

Leu Thr Phe Arg
            20

<210> 251
<211> 20
<212> PRT
<213> Homo sapiens

<400> 251
Leu Gln Ala Leu Lys Val Thr Val Thr Ser Arg Ala Ser Asn Ser Ala
                5                   10                  15

Val Pro Pro Ala
            20

<210> 252
<211> 153
<212> PRT
<213> Homo sapien

<400> 252
Met Ala Ser Val Arg Val Ala Ala Tyr Phe Glu Asn Phe Leu Ala Ala
 1              5                   10                  15
Trp Arg Pro Val Lys Ala Ser Asp Gly Asp Tyr Tyr Thr Leu Ala Val
            20                  25                  30
Pro Met Gly Asp Val Pro Met Asp Gly Ile Ser Val Ala Asp Ile Gly
        35                  40                  45
Ala Ala Val Ser Ser Ile Phe Asn Ser Pro Glu Glu Phe Leu Gly Lys
    50                  55                  60
Ala Val Gly Leu Ser Ala Glu Ala Leu Thr Ile Gln Gln Tyr Ala Asp
65                  70                  75                  80
Val Leu Ser Lys Ala Leu Gly Lys Glu Val Arg Asp Ala Lys Ile Thr
                85                  90                  95
Pro Glu Ala Phe Glu Lys Leu Gly Phe Pro Ala Ala Lys Glu Ile Ala
            100                 105                 110
Asn Met Cys Arg Phe Tyr Glu Met Lys Pro Asp Arg Asp Val Asn Leu
        115                 120                 125
Thr His Gln Leu Asn Pro Lys Val Lys Ser Phe Ser Gln Phe Ile Ser
    130                 135                 140
Glu Asn Gln Gly Ala Phe Lys Gly Met
145                 150

<210> 253
<211> 462
<212> DNA
<213> Homo sapien

<400> 253
atggccagtg tccgcgtggc ggcctacttt gaaaactttc tcgcggcgtg gcggcccgtg 60
aaagcctctg atggagatta ctacaccttg gctgtaccga tgggagatgt accaatggat 120
ggtatctctg ttgctgatat tggagcagcc gtctctagca tttttaattc tccagaggaa 180
tttttaggca aggccgtggg gctcagtgca gaagcactaa caatacagca atatgctgat 240
gttttgtcca aggctttggg gaaagaagtc cgagatgcaa agattacccc ggaagctttc 300
gagaagctgg gattccctgc agcaaaggaa atagccaata tgtgtcgttt ctatgaaatg 360
aagccagacc gagatgtcaa tctcacccac caactaaatc ccaaagtcaa aagcttcagc 420
cagtttatct cagagaacca gggagccttc aagggcatgt ag 462

<210> 254
<211> 8031
<212> DNA
<213> Homo sapien

<400> 254
tggcgaatgg gacgcgccct gtagcggcgc attaagcgcg gcgggtgtgg tggttacgcg 60
cagcgtgacc gctacacttg ccagcgccct agcgcccgct cctttcgctt tcttcccttc 120
ctttctcgcc acgttcgccg gctttccccg tcaagctcta aatcgggggc tccctttagg 180
gttccgattt agtgctttac ggcacctcga ccccaaaaaa cttgattagg gtgatggttc 240
acgtagtggg ccatcgccct gatagacggt ttttcgccct ttgacgttgg agtccacgtt 300
ctttaatagt ggactcttgt tccaaactgg aacaacactc aaccctatct cggtctattc 360
ttttgattta taagggattt tgccgatttc ggcctattgg ttaaaaaatg agctgattta 420
acaaaaattt aacgcgaatt ttaacaaaat attaacgttt acaatttcag gtggcacttt 480
tcggggaaat gtgcgcggaa cccctatttg tttatttttc taaatacatt caaatatgta 540
tccgctcatg aattaattct tagaaaaact catcgagcat caaatgaaac tgcaatttat 600
tcatatcagg attatcaata ccatattttt gaaaaagccg tttctgtaat gaaggagaaa 660
actcaccgag gcagttccat aggatggcaa gatcctggta tcggtctgcg attccgactc 720
gtccaacatc aatacaacct attaatttcc cctcgtcaaa aataaggtta tcaagtgaga 780
aatcaccatg agtgacgact gaatccggtg agaatggcaa aagtttatgc atttctttcc 840
agacttgttc aacaggccag ccattacgct cgtcatcaaa atcactcgca tcaaccaaac 900
cgttattcat tcgtgattgc gcctgagcga gacgaaatac gcgatcgctg ttaaaaggac 960
aattacaaac aggaatcgaa tgcaaccggc gcaggaacac tgccagcgca tcaacaatat 1020
tttcacctga atcaggatat tcttctaata cctggaatgc tgttttcccg gggatcgcag 1080
tggtgagtaa ccatgcatca tcaggagtac ggataaaatg cttgatggtc ggaagaggca 1140
taaattccgt cagccagttt agtctgacca tctcatctgt aacatcattg gcaacgctac 1200
ctttgccatg tttcagaaac aactctggcg catcgggctt cccatacaat cgatagattg 1260
tcgcacctga ttgcccgaca ttatcgcgag cccatttata cccatataaa tcagcatcca 1320
tgttggaatt taatcgcggc ctagagcaag acgtttcccg ttgaatatgg ctcataacac 1380
cccttgtatt actgtttatg taagcagaca gttttattgt tcatgaccaa aatcccttaa 1440
cgtgagtttt cgttccactg agcgtcagac cccgtagaaa agatcaaagg atcttcttga 1500
gatccttttt ttctgcgcgt aatctgctgc ttgcaaacaa aaaaaccacc gctaccagcg 1560
gtggtttgtt tgccggatca agagctacca actctttttc cgaaggtaac tggcttcagc 1620
agagcgcaga taccaaatac tgtccttcta gtgtagccgt agttaggcca ccacttcaag 1680
aactctgtag caccgcctac atacctcgct ctgctaatcc tgttaccagt ggctgctgcc 1740
agtggcgata agtcgtgtct taccgggttg gactcaagac gatagttacc ggataaggcg 1800
cagcggtcgg gctgaacggg gggttcgtgc acacagccca gcttggagcg aacgacctac 1860
accgaactga gatacctaca gcgtgagcta tgagaaagcg ccacgcttcc cgaagggaga 1920
aaggcggaca ggtatccggt aagcggcagg gtcggaacag gagagcgcac gagggagctt 1980

ccagggggaa acgcctggta tctttatagt cctgtcgggt ttcgccacct ctgacttgag 2040
cgtcgatttt tgtgatgctc gtcagggggg cggagcctat ggaaaaacgc cagcaacgcg 2100
gcctttttac ggttcctggc cttttgctgg ccttttgctc acatgttctt tcctgcgtta 2160
tcccctgatt ctgtggataa ccgtattacc gcctttgagt gagctgatac cgctcgccgc 2220
agccgaacga ccgagcgcag cgagtcagtg agcgaggaag cggaagagcg cctgatgcgg 2280
tattttctcc ttacgcatct gtgcggtatt tcacaccgca tatatggtgc actctcagta 2340
caatctgctc tgatgccgca tagttaagcc agtatacact ccgctatcgc tacgtgactg 2400
ggtcatggct gcgccccgac acccgccaac acccgctgac gcgccctgac gggcttgtct 2460
gctcccggca tccgcttaca gacaagctgt gaccgtctcc gggagctgca tgtgtcagag 2520
gttttcaccg tcatcaccga aacgcgcgag gcagctgcgg taaagctcat cagcgtggtc 2580
gtgaagcgat tcacagatgt ctgcctgttc atccgcgtcc agctcgttga gtttctccag 2640
aagcgttaat gtctggcttc tgataaagcg ggccatgtta agggcggttt tttcctgttt 2700
ggtcactgat gcctccgtgt aagggggatt tctgttcatg ggggtaatga taccgatgaa 2760
acgagagagg atgctcacga tacgggttac tgatgatgaa catgcccggt tactggaacg 2820
ttgtgagggt aaacaactgg cggtatggat gcggcgggac cagagaaaaa tcactcaggg 2880
tcaatgccag cgcttcgtta atacagatgt aggtgttcca cagggtagcc agcagcatcc 2940
tgcgatgcag atccggaaca taatggtgca gggcgctgac ttccgcgttt ccagacttta 3000
cgaaacacgg aaaccgaaga ccattcatgt tgttgctcag gtcgcagacg ttttgcagca 3060
gcagtcgctt cacgttcgct cgcgtatcgg tgattcattc tgctaaccag taaggcaacc 3120
ccgccagcct agccgggtcc tcaacgacag gagcacgatc atgcgcaccc gtggggccgc 3180
catgccggcg ataatggcct gcttctcgcc gaaacgtttg gtggcgggac cagtgacgaa 3240
ggcttgagcg agggcgtgca agattccgaa taccgcaagc gacaggccga tcatcgtcgc 3300
gctccagcga aagcggtcct cgccgaaaat gacccagagc gctgccggca cctgtcctac 3360
gagttgcatg ataaagaaga cagtcataag tgcggcgacg atagtcatgc cccgcgccca 3420
ccggaaggag ctgactgggt tgaaggctct caagggcatc ggtcgagatc ccggtgccta 3480
atgagtgagc taacttacat taattgcgtt gcgctcactg cccgctttcc agtcgggaaa 3540
cctgtcgtgc cagctgcatt aatgaatcgg ccaacgcgcg gggagaggcg gtttgcgtat 3600
tgggcgccag ggtggttttt cttttcacca gtgagacggg caacagctga ttgcccttca 3660
ccgcctggcc ctgagagagt tgcagcaagc ggtccacgct ggtttgcccc agcaggcgaa 3720
aatcctgttt gatggtggtt aacggcggga tataacatga gctgtcttcg gtatcgtcgt 3780
atcccactac cgagatatcc gcaccaacgc gcagcccgga ctcggtaatg gcgcgcattg 3840
cgcccagcgc catctgatcg ttggcaacca gcatcgcagt gggaacgatg ccctcattca 3900
gcatttgcat ggtttgttga aaaccggaca tggcactcca gtcgccttcc cgttccgcta 3960
tcggctgaat ttgattgcga gtgagatatt tatgccagcc agccagacgc agacgcgccg 4020
agacagaact taatgggccc gctaacagcg cgatttgctg gtgacccaat gcgaccagat 4080
gctccacgcc cagtcgcgta ccgtcttcat gggagaaaat aatactgttg atgggtgtct 4140
ggtcagagac atcaagaaat aacgccggaa cattagtgca ggcagcttcc acagcaatgg 4200
catcctggtc atccagcgga tagttaatga tcagcccact gacgcgttgc gcgagaagat 4260
tgtgcaccgc cgctttacag gcttcgacgc cgcttcgttc taccatcgac accaccacgc 4320
tggcacccag ttgatcggcg cgagatttaa tcgccgcgac aatttgcgac ggcgcgtgca 4380
gggccagact ggaggtggca acgccaatca gcaacgactg tttgcccgcc agttgttgtg 4440
ccacgcggtt gggaatgtaa ttcagctccg ccatcgccgc ttccactttt tcccgcgttt 4500
tcgcagaaac gtggctggcc tggttcacca cgcgggaaac ggtctgataa gagacaccgg 4560
catactctgc gacatcgtat aacgttactg gtttcacatt caccaccctg aattgactct 4620
cttccgggcg ctatcatgcc ataccgcgaa aggttttgcg ccattcgatg gtgtccggga 4680
tctcgacgct ctcccttatg cgactcctgc attaggaagc agcccagtag taggttgagg 4740
ccgttgagca ccgccgccgc aaggaatggt gcatgcaagg agatggcgcc caacagtccc 4800
ccggccacgg ggcctgccac catacccacg ccgaaacaag cgctcatgag cccgaagtgg 4860
cgagcccgat cttccccatc ggtgatgtcg gcgatatagg cgccagcaac cgcacctgtg 4920
gcgccggtga tgccggccac gatgcgtccg gcgtagagga tcgagatctc gatcccgcga 4980
aattaatacg actcactata ggggaattgt gagcggataa caattcccct ctagaaataa 5040
ttttgtttaa ctttaagaag gagatataca tatgcagcat caccaccatc accacggagt 5100
acagcttcaa gacaatgggt ataatggatt gctcattgca attaatcctc aggtacctga 5160
gaatcagaac ctcatctcaa acattaagga aatgataact gaagcttcat tttacctatt 5220
taatgctacc aagagaagag tatttttcag aaatataaag attttaatac ctgccacatg 5280

gaaagctaat aataacagca aaataaaaca agaatcatat gaaaaggcaa atgtcatagt 5340
gactgactgg tatggggcac atggagatga tccatacacc ctacaataca gagggtgtgg 5400
aaaagaggga aaatacattc atttcacacc taatttccta ctgaatgata acttaacagc 5460
tggctacgga tcacgaggcc gagtgtttgt ccatgaatgg gcccacctcc gttggggtgt 5520
gttcgatgag tataacaatg acaaaccttt ctacataaat gggcaaaatc aaattaaagt 5580
gacaaggtgt tcatctgaca tcacaggcat ttttgtgtgt gaaaaaggtc cttgccccca 5640
agaaaactgt attattagta agctttttaa agaaggatgc acctttatct acaatagcac 5700
ccaaaatgca actgcatcaa taatgttcat gcaaagttta tcttctgtgg ttgaattttg 5760
taatgcaagt acccacaacc aagaagcacc aaacctacag aaccagatgt gcagcctcag 5820
aagtgcatgg gatgtaatca cagactctgc tgactttcac cacagctttc ccatgaacgg 5880
gactgagctt ccacctcctc ccacattctc gcttgtagag gctggtgaca aagtggtctg 5940
tttagtgctg gatgtgtcca gcaagatggc agaggctgac agactccttc aactacaaca 6000
agccgcagaa ttttatttga tgcagattgt tgaaattcat accttcgtgg gcattgccag 6060
tttcgacagc aaaggagaga tcagagccca gctacaccaa attaacagca atgatgatcg 6120
aaagttgctg gtttcatatc tgcccaccac tgtatcagct aaaacagaca tcagcatttg 6180
ttcagggctt aagaaaggat ttgaggtggt tgaaaaactg aatggaaaag cttatggctc 6240
tgtgatgata ttagtgacca gcggagatga taagcttctt ggcaattgct tacccactgt 6300
gctcagcagt ggttcaacaa ttcactccat tgccctgggt tcatctgcag ccccaaatct 6360
ggaggaatta tcacgtctta caggaggttt aaagttcttt gttccagata tatcaaactc 6420
caatagcatg attgatgctt tcagtagaat ttcctctgga actggagaca ttttccagca 6480
acatattcag cttgaaagta caggtgaaaa tgtcaaacct caccatcaat tgaaaaacac 6540
agtgactgtg gataatactg tgggcaacga cactatgttt ctagttacgt ggcaggccag 6600
tggtcctcct gagattatat tatttgatcc tgatggacga aaatactaca caaataattt 6660
tatcaccaat ctaacttttc ggacagctag tctttggatt ccaggaacag ctaagcctgg 6720
gcactggact tacaccctga acaataccca tcattctctg caagccctga aagtgacagt 6780
gacctctcgc gcctccaact cagctgtgcc cccagccact gtggaagcct ttgtggaaag 6840
agacagcctc catttcctc atcctgtgat gatttatgcc aatgtgaaac aggattttta 6900
tcccattctt aatgccactg tcactgccac agttgagcca gagactggag atcctgttac 6960
gctgagactc cttgatgatg gagcaggtgc tgatgttata aaaaatgatg gaatttactc 7020
gaggtatttt ttctcctttg ctgcaaatgg tagatatagc ttgaaagtgc atgtcaatca 7080
ctctcccagc ataagcaccc cagcccactc tattccaggg agtcatgcta tgtatgtacc 7140
aggttacaca gcaaacggta atattcagat gaatgctcca aggaaatcag taggcagaaa 7200
tgaggaggag cgaaagtggg gctttagccg agtcagctca ggaggctcct tttcagtgct 7260
gggagttcca gctggccccc accctgatgt gtttccacca tgcaaaatta ttgacctgga 7320
agctgtaaaa gtagaagagg aattgaccct atcttggaca gcacctggag aagactttga 7380
tcagggccag gctacaagct atgaaataag aatgagtaaa agtctacaga atatccaaga 7440
tgactttaac aatgctattt tagtaaatac atcaaagcga aatcctcagc aagctggcat 7500
cagggagata tttacgttct caccccaaat ttccacgaat ggacctgaac atcagccaaa 7560
tggagaaaca catgaaagcc acagaattta tgttgcaata cgagcaatgg ataggaactc 7620
cttacagtct gctgtatcta acattgccca ggcgcctctg tttattcccc ccaattctga 7680
tcctgtacct gccagagatt atcttatatt gaaaggagtt ttaacagcaa tgggtttgat 7740
aggaatcatt tgccttatta tagttgtgac acatcatact ttaagcagga aaaagagagc 7800
agacaagaaa gagaatggaa caaaattatt ataatgaatt ctgcagatat ccatcacact 7860
ggcggccgct cgagcaccac caccaccacc actgagatcc ggctgctaac aaagcccgaa 7920
aggaagctga gttggctgct gccaccgctg agcaataact agcataaccc cttggggcct 7980
ctaaacgggt cttgaggggt tttttgctga aaggaggaac tatatccgga t 8031

<210> 255
<211> 401
<212> DNA
<213> Homo sapien

<220>
<221> misc_feature

<222> (1)...(401)
<223> n = A,T,C or G

<400> 255
gtggccagng actagaaggc gaggcgccgc gggaccatgg cggcggcggc ggacgagcgg 60
agtccanagg acggagaaga cgaggaagag gaggagcagt tggttctggt ggaattatca 120
ggaattattg attcagactt cctctcaaaa tgtgaaaata aatgcaaggt tttgggcatt 180
gacactgaga ggcccattct gcaagtggac agctgtgtct ttgctgggga gtatgaagac 240
actctangga cctgtgttat atttgaagaa aatgntnaac atgctgatac agaaggcaat 300
aataaaacag tgctaaaata taaatgccat acaatgaaga agctcagcat gacaagaact 360
ctcctgacag agaagaagga aggagaagaa aacatangtg g 401

<210> 256
<211> 401
<212> DNA
<213> Homo sapien

<220>
<221> misc_feature
<222> (1)...(401)
<223> n = A,T,C or G

<400> 256
tggtggncct gggatgggga accgcggtgg cttccgngga ggtttcggca ntggcatccg 60
gggccggggt cgcggccgng gacggggccg gggccnangc cgnnganctc gcggangcaa 120
ggccgaggat aaggagtgga tgcccgtcac caacttgggc cgcttgncca aggacatgaa 180
nancaagccc ctgnaggaga tctatntctt cttccctgcc ccattaagga atcaagagat 240
catttgattt cttcctgggg gcctctctca aggatnaggt ttttgaagat tatgccagtg 300
canaaannan accccgttgc ccngtccatc tncacccaac ncttccaagg gcnatttttg 360
tttaggcctc attncngggg ggaaccttaa cccaatttgg g 401

<210> 257
<211> 401
<212> DNA
<213> Homo sapien

<220>
<221> misc_feature
<222> (1)...(401)
<223> n = A,T,C or G

<400> 257
atgtatgtaa aacacttcat aaaatgtaaa gggctataac aaatatgtta taaagtgatt 60
ctctcagccc tgaggtatac agaatcattt gcctcagact gctgttggat tttaaaattt 120
ttaaaatatc tgctaagtaa tttgctatgt cttctcccac actatcaata tgcctgcttc 180
taacaggctc cccactttct tttaatgtgc tgttatgagc tttggacatg agataaccgt 240
gcctgttcag agtgtctaca gtaagagctg gacaaactct ggagggacac agtctttgag 300
acagctcttt tggttgcttt ccacttttct gaaaggttca cagtaacctt ctagataata 360
gaaactccca gttaaagcct angctancaa ttttttttag t 401

<210> 258
<211> 401
<212> DNA
<213> Homo sapien

<400> 258
ggagcgctag gtcggtgtac gaccgagatt agggtgcgtg ccagctccgg gaggccgcgg 60
tgaggggccg ggcccaagct gccgacccga gccgatcgtc agggtcgcca gcgcctcagc 120
tctgtggagg agcagcagta gtcggagggt gcaggatatt agaaatggct actccccagt 180
caattttcat ctttgcaatc tgcattttaa tgataacaga attaattctg gcctcaaaaa 240
gctactatga tatcttaggt gtgccaaaat cggcatcaga gcgccaaatc aagaaggcct 300
ttcacaagtt ggccatgaag taccaccctg acaaaaataa gacccagatg ctgaagcaaa 360
attcagagag attgcagaag catatgaaac actctcagat g 401

<210> 259
<211> 401
<212> DNA
<213> Homo sapien

<400> 259
attgggtttg gagggaggat gatgacagag gaatgccctt tggccatcac ggttttgatt 60
ctccagaata ttgtgggttt gatcatcaat gcagtcatgt taggctgcat tttcatgaaa 120
acagctcagg ctcacagaag ggcagaaact ttgattttca gccgccatgc tgtgattgcc 180
gtccgaaatg gcaagctgtg cttcatgttc cgagtgggtg acctgaggaa aagcatgatc 240
attagtgcct ctgtgcgcat ccaggtggtc aagaaaacaa ctacacctga aggggaggtg 300
gttcctattc accaactgga cattcctgtt gataacccaa tcgagagcaa taacattttt 360
ctggtggccc ctttgatcat ctgccacgtg attgacaagc g 401

<210> 260
<211> 363
<212> DNA
<213> Homo sapien

<220>
<221> misc_feature
<222> (1)...(363)
<223> n = A,T,C or G

<400> 260
aggaganang gagggggana tgaataggga tggagaggga natagtggat gagcagggca 60
canggagagg aancagaaag gagaggcaag acagggagac acacancaca nangangana 120
caggtggggg ctggggtggg gcatggagag cctttnangt cncccaggcc accctgctct 180
cgctggnctg ttgaaaccca ctccatggct tcctgccact gcagttgggc ccagggctgg 240
cttattnctg gaatgcaagt ggctgtggct tggagcctcc cctctggnnn anggaaannn 300
attgctccct tatctgcttg gaatatctga gtttttccan cccggaaata aaacacacac 360
aca 363

<210> 261
<211> 401
<212> DNA
<213> Homo sapien

<220>
<221> misc_feature
<222> (1)...(401)
<223> n = A,T,C or G

<400> 261
cggctctccg ccgctctccc ggggtttcgg ggcacttggg tcccacagtc tggtcctgct 60
tcaccttccc ctgacctgag tagtcgccat ggcacaggtt ctcagaggca ctgngactga 120

cttccctgga tttgatgagc gggctgatgc anaaactctt cggaaggcta tgaaaggctt 180
gggcacagat gaggagagca tcctgactct gttgacatcc cgaagtaatg ctcagcgcca 240
ggaaatctct gcagctttta agactctgtt tggcagggat cttctggatg acctgaaatc 300
agaactaact ggaaaatttg aaaaattaat tgtggctctg atgaaaccct ctcggcttta 360
tgatgcttat gaactgaaac atgccttgaa gggagctgga a 401

<210> 262
<211> 401
<212> DNA
<213> Homo sapien

<220>
<221> misc_feature
<222> (1)...(401)
<223> n = A,T,C or G

<400> 262
agtctanaac atttctaata ttttgngctt tcatatatca aaggagatta tgtgaaacta 60
tttttaaata ctgtaaagtg acatatagtt ataagatata tttctgtaca gtagagaaag 120
agtttataac atgaagaata ttgtaccatt atacattttc attctcgatc tcataagaaa 180
ttcaaaagaa taatgataga ggtgaaaata tgtttacttt ctctaaatca agcctagttg 240
tcaactcaaa aattatgntg catagtttta ttttgaattt aggttttggg actacttttt 300
tccancttca atgagaaaat aaaatctaca actcaggagt tactacagaa gttctaanta 360
tttttttgct aannagcnaa aaatataaac atatgaaaat g 401

<210> 263
<211> 401
<212> DNA
<213> Homo sapien

<220>
<221> misc_feature
<222> (1)...(401)
<223> n = A,T,C or G

<400> 263
ctgtccgacc aagagaggcc ggccgagccc gaggcttggg cttttgcttt ctggcggagg 60
gatctgcggc ggtttaggag gcggcgctga tcctgggagg aagaggcagc tacggcggcg 120
gcggcggtgg cggctagggc ggcggcgaat aaaggggccg ccgccgggtg atgcggtgac 180
cactgcggca ggcccaggag ctgagtgggc cccggccctc agcccgtccc gncggacccg 240
ctttcctcaa ctctccatct tctcctgccg accgagatcg ccgaggcggn ctcaggctcc 300
ctancccctt ccccgtccct tccccncccc cgtccccgcc ccgggggccg ccgccacccg 360
cctcccacca tggctctgaa ganaatccac aaggaattga a 401

<210> 264
<211> 401
<212> DNA
<213> Homo sapien

<400> 264
aacaccagcc actccaggac ccctgaaggc ctctaccagg tcaccagtgt tctgcgccta 60
aagccacccc ctggcagaaa cttcagctgt gtgttctgga atactcacgt gagggaactt 120
actttggcca gcattgacct tcaaagtcag atggaaccca ggacccatcc aacttggctg 180
cttcacattt tcatcccctc ctgcatcatt gctttcattt tcatagccac agtgatagcc 240
ctaagaaaac aactctgtca aaagctgtat tcttcaaaag acacaacaaa aagacctgtc 300

accacaacaa agagggaagt gaacagtgct gtgaatctga acctgtggtc ttgggagcca 360
gggtgacctg atatgacatc taaagaagct tctggactct g 401

<210> 265
<211> 271
<212> DNA
<213> Homo sapien

<220>
<221> misc_feature
<222> (1)...(271)
<223> n = A,T,C or G

<400> 265
gccacttcct gtggacatgg gcagagcgct gctgccagtt cctggtagcc ttgaccacna 60
cgctgggggg tctttgtgat ggtcatgggt ctcatttgca cttgggggtg tgggattcaa 120
gttagaagtt tctagatctg gccgggcgca gtggctcaca cctgtaatcc cagcacttta 180
ggaggctgag gcaggcggat catgaggtca ggagatcgag accgtcctgg ctaacacagt 240
gaaaccccgt ctctactaaa aatacaaaaa a 271

<210> 266
<211> 401
<212> DNA
<213> Homo sapien

<220>
<221> misc_feature
<222> (1)...(401)
<223> n = A,T,C or G

<400> 266
attcataaat ttagctgaaa gatactgatt caatttgtat acagngaata taaatgagac 60
gacagcaaaa ttttcatgaa atgtaaaata tttttatagt ttgttcatac tatatgaggt 120
tctattttaa atgactttct ggattttaaa aaatttcttt aaatacaatc atttttgtaa 180
tatttatttt atgcttatga tctagataat tgcagaatat cattttatct gactctgtct 240
tcataagaga gctgtggccg aattttgaac atctgttata gggagtgatc aaattagaag 300
gcaatgtgga aaaacaattc tgggaaagat ttctttatat gaagtccctg ccactagcca 360
gccatcctaa ttgatgaaag ttatctgttc acaggcctgc a 401

<210> 267
<211> 401
<212> DNA
<213> Homo sapien

<220>
<221> misc_feature
<222> (1)...(401)
<223> n = A,T,C or G

<400> 267
gaagaggcat cacctgatcc cggagacctt tggagttaag aggcggcgga agcgagggcc 60
tgtggagtcg gatcctcttc ggggtgagcc agggtcggcg cgcgcggctg tctcanaact 120
catgcagctg ttcccgcgag gcctgtttga ggacgcgctg ccgcccatcg tgctgaggag 180
ccaggtgtac agccttgtgc ctgacaggac cgtggccgac cggcagctga aggagcttca 240
agagcanggg gagacaaaat cgtccagctg ggcttcnact tggatgccca tggaanttat 300

tctttcnctt ganggactta cnngggaccc aagaanccct tncaaggggc ccttngtgga 360
tgggncccga aaccccnnta tttgcccttg gggggnccca a 401

<210> 268
<211> 223
<212> DNA
<213> Homo sapien

<400> 268
tcgccatgtt ggccaggctg gtcttgaact cctgacttta agtgatccac ccgcctcaac 60
ctcccaaagt gctgggatta caggtgtgag ccaccgcgcc tggcctgata catactttta 120
gaatcaagta gtcacgcact ttttctgttc atttttctaa aaagtaaata tacaaatgtt 180
ttgtttttg tttttttgt ttgtttgttt ctgtttttt ttt 223

<210> 269
<211> 401
<212> DNA
<213> Homo sapien

<400> 269
actatgtaaa ccacattgta ctttttttta ctttggcaac aaatatttat acatacaaga 60
tgctagttca tttgaatatt tctcccaact tatccaagga tctccagctc taacaaaatg 120
gtttattttt atttaaatgt caatagttgt tttttaaaat ccaaatcaga ggtgcaggcc 180
accagttaaa tgccgtctat caggttttgt gccttaagag actacagagt caaagctcat 240
ttttaaagga gtaggacaaa gttgtcacag gtttttgttg ttgtttttat tgcccccaaa 300
attacatgtt aatttccatt tatatcaggg attctattta cttgaagact gtgaagttgc 360
cattttgtct cattgttttc tttgacataa ctaggatcca t 401

<210> 270
<211> 401
<212> DNA
<213> Homo sapien

<220>
<221> misc_feature
<222> (1)...(401)
<223> n = A,T,C or G

<400> 270
tggctgttga ttcacctcag cactgcttgg tatctgcacc ctacctctct ttagaggctg 60
ccttgtcaac tgaaaaatgc acctgacttc gagcaagact ctttccttag gttctggatc 120
tgtttgagcc ccatggcact gagctggaat ctgagggtct tgttccaagg atgtgatgat 180
gtgggagaat gttctttgaa agagcagaaa tccagtctgc atggaaacag cctgtagagn 240
agaagtttcc agtgataagt gttcactgtt ctaaggaggt acaccacagc tacctgaatt 300
ttcccaaaat gagtgcttct gtgcgttaca actggccttt gtacttgact gtgatgactt 360
tgttttttct tttcaattct anatgaacat gggaaaaaat g 401

<210> 271
<211> 329
<212> DNA
<213> Homo sapien

<400> 271
ccacagcctc caagtcaggt ggggtggagt cccagagctg cacagggttt ggcccaagtt 60
tctaagggag gcacttcctc ccctcgccca tcagtgccag cccctgctgg ctggtgcctg 120

agcccctcag acagccccct gccccgcagg cctgccttct cagggacttc tgcggggcct 180
gaggcaagcc atggagtgag acccaggagc cggacacttc tcaggaaatg gcttttccca 240
acccccagcc cccacccggt ggttcttcct gttctgtgac tgtgtatagt gccaccacag 300
cttatggcat ctcattgagg acaaaaaaa 329

<210> 272
<211> 401
<212> DNA
<213> Homo sapien

<220>
<221> misc_feature
<222> (1)...(401)
<223> n = A,T,C or G

<400> 272
nggctgntaa cntcggaggt nacttcctgg actatcctgg agaccccctc cgcttccacg 60
nncatnatat cnctcatngc tgggcccntn angacacnat cccactccaa cacctgngng 120
atgctggncn cctnggaacc ancntcagaa ngaccctgnt cntntgtnnt ccgcaanctg 180
aagnnaangc gggntacacc tncntgcant ggnccacnct gcngggaact ntacacacct 240
acgggatgtg gctgcgccan gagccaagag cntttctgga tgattcccca gcctcttgnn 300
agggantcta caacattgct nnntaccttt ntccnncngc nnntnntgga ntacaggngn 360
tnntaacact acatcttttt tactgcnccn tncttggtgg g 401

<210> 273
<211> 401
<212> DNA
<213> Homo sapien

<220>
<221> misc_feature
<222> (1)...(401)
<223> n = A,T,C or G

<400> 273
cagcaccatg aagatcaaga tcatcgcacc cccagagcgc aagtactcgg tgtggatcgg 60
tggctccatc ctggcctcac tgtccacctt ccagcagatg tggattagca agcaggagta 120
cgacgagtcg ggcccctcca tcgtccaccg caaatgcttc taaacggact cagcagatgc 180
gtagcatttg ctgcatgggt taattgagaa tagaaatttg cccctggcaa atgcacacac 240
ctcatgctag cctcacgaaa ctggaataag ccttcgaaaa gaaattgtcc ttgaagcttg 300
tatctgatat cagcactgga ttgtagaact tgttgctgat tttgaccttg tattgaagtt 360
aactgttccc cttggtatta acgtgtcagg gctgagtgnt c 401

<210> 274
<211> 401
<212> DNA
<213> Homo sapien

<400> 274
ccacccacac ccaccgcgcc ctcgttcgcc tcttctccgg gagccagtcc gcgccaccgc 60
cgccgcccag gccatcgcca ccctccgcag ccatgtccac caggtccgtg tcctcgtcct 120
cctaccgcag gatgttcggc ggcccgggca ccgcgagccg gccgagctcc agccggagct 180
acgtgactac gtccacccgc acctacagcc tgggcagcgc gctgcgcccc agcaccagcc 240
gcagcctcta cgcctcgtcc ccgggcggcg tgtatgccac gcgctcctct gccgtgcgcc 300
tgcggagcag cgtgcccggg gtgcggctcc tgcaggactc ggtggacttc tcgctggccg 360

acgccatcaa caccgagttc aagaacaccc gcaccaacga g 401

<210> 275
<211> 401
<212> DNA
<213> Homo sapien

<400> 275
ccacttccac cactttgtgg agcagtgcct tcagcgcaac ccggatgcca ggtatccctg 60
ctggcctggg cctgggcttc gggagagcag agggtgctca ggagggtaag gccagggtgt 120
gaagggactt acctcccaaa ggttctgcag gggaatctgg agctacacac aggagggatc 180
agctcctggg tgtgtcagag gccagcctgg ggagctctgg ccactgcttc ccatgagctg 240
agggagaggg agaggggacc cgaggctgag gcataagtgg caggatttcg ggaagctggg 300
gacacggcag tgatgctgcg gtctctcctc ccctttccct ccaggcccag tgccagcacc 360
ctcctgaacc actctttctt caagcagatc aagcgacgtg c 401

<210> 276
<211> 401
<212> DNA
<213> Homo sapien

<220>
<221> misc_feature
<222> (1)...(401)
<223> n = A,T,C or G

<400> 276
tctgatattg ntacccttga gccacctaag ttagaagaaa ttggaaatca agaagttgtc 60
attgttgaag aagcacagag ttcagaagac tttaacatgg gctcttcctc tagcagccag 120
tatactttct gtcagccaga aactgtattt tcatctcagc ctagtgatga tgaatcaagt 180
agtgatgaaa ccagtaatca gcccagtcct gcctttagac gacgccgtgc taggaagaag 240
accgtttctg cttcagaatc tgaagaccgg ctagttggtg aacaagaaac tgaaccttct 300
aaggagttga gtaaacgtca gttcagtagt ggtctcaata agtgtgttat acttgctttg 360
gtgattgcaa tcagcatggg atttggccat ttctatggca c 401

<210> 277
<211> 401
<212> DNA
<213> Homo sapien

<220>
<221> misc_feature
<222> (1)...(401)
<223> n = A,T,C or G

<400> 277
aactttggca acatatctca gcaaaaacta cagctatgtt attcatgcca aaataaaagc 60
tgtgcagagg agtggctgca atgaggtcac aacggtggtg gatgtaaaag agatcttcaa 120
gtcctcatca cccatccctc gaactcaagt cccgctcatt acaaattctt cttgccagtg 180
tccacacatc ctgccccatc aagatgttct catcatgtgt tacgagnggc gctcaaggat 240
gatgcttctt gaaaattgct tagttgaaaa atggagagat cagcttagta aaagatccat 300
acagtgggaa gagaggctgc aggaacagcg ganaacagtt caggacaaga agaaaacagc 360
cgggcgcacc agtcgtagta atccccccaa accaaaggga a 401

<210> 278

<211> 401
<212> DNA
<213> Homo sapien

<220>
<221> misc_feature
<222> (1)...(401)
<223> n = A,T,C or G

<400> 278
aatgagtgtg agaccacaaa tgaatgccgg gaggatgaaa tgtgttggaa ttatcatggc 60
ggcttccgtt gttatccacg aaatccttgt caagatccct acattctaac accagagaac 120
cgatgtgttt gcccagtctc aaatgccatg tgccgagaac tgccccagtc aatagtctac 180
aaatacatga gcatccgatc tgataggtct gtgccatcag acatcttcca gatacaggcc 240
acaactattt atgccaacac catcaatact tttcggatta aatctggaaa tgaaaatgga 300
gagtctacct acgacaacaa anccctgtaa gtgcaatgct tgtgctcgtg aagncattat 360
caggaccaag agaacatatc gtggacctgg agatgctgac a 401

<210> 279
<211> 401
<212> DNA
<213> Homo sapien

<220>
<221> misc_feature
<222> (1)...(401)
<223> n = A,T,C or G

<400> 279
aaattattgc ctctgataca tacctaagtn aacanaacat taatacctaa gtaaacataa 60
cattacttgg agggttgcag nttctaantg aaactgtatt tgaaactttt aagtatactt 120
taggaaacaa gcatgaacgg cagtctagaa taccagaaac atctacttgg gtagcttggn 180
gccattatcc tgtggaatct gatatgtctg gnagcatgtc attgatggga catgaagaca 240
tctttggaaa tgatgagatt atttcctgtg ttaaaaaaaa aaaaaatctt aaattcctac 300
aatgtgaaac tgaaactaat aattttgatc ctgatgtatg ggacagcgta tctgtaccag 360
gctctaaata acaaaagnta gggngacaag nacatgttcc t 401

<210> 280
<211> 326
<212> DNA
<213> Homo sapien

<400> 280
gaagtggaat tgtataattc aattcgataa ttgatctcat gggctttccc tggaggaaag 60
gttttttttg ttgttttttt tttaagaact tgaaacttgt aaactgagat gtctgtagct 120
tttttgccca tctgtagtgt atgtgaagat ttcaaaacct gagagcactt tttctttgtt 180
tagaattatg agaaaggcac tagatgactt taggatttgc atttttccct ttattgcctc 240
atttcttgtg acgccttgtt ggggagggaa atctgtttat tttttcctac aaataaaaag 300
ctaagattct atatcgcaaa aaaaaa 326

<210> 281
<211> 374
<212> DNA
<213> Homo sapien

<400> 281
caacgcgttt gcaaatattc ccctggtagc ctacttcctt acccccgaat attggtaaga 60
tcgagcaatg gcttcaggac atgggttctc ttctcctgtg atcattcaag tgctcactgc 120
atgaagactg gcttgtctca gtgtttcaac ctcaccaggg ctgtctcttg gtccacacct 180
cgctccctgt tagtgccgta tgacagcccc catcaaatga ccttggccaa gtcacggttt 240
ctctgtggtc aaggttggtt ggctgattgg tggaaagtag ggtggaccaa aggaggccac 300
gtgagcagtc agcaccagtt ctgcaccagc agcgcctccg tcctagtggg tgttcctgtt 360
tctcctggcc ctgg 374

<210> 282
<211> 404
<212> DNA
<213> Homo sapien

<220>
<221> misc_feature
<222> (1)...(404)
<223> n = A,T,C or G

<400> 282
agtgtggtgg aattcccgca tcctanncgc cgactcacac aaggcagagt ngccatggag 60
aaaattccag tgtcagcatt cttgctcctt gtggccctct cctacactct ggccagagat 120
accacagtca aacctgnagc caaaaaggac acaaaggact ctcgacccaa actgccccan 180
accctctcca gaggttgggg tgaccaactc atctggactc anacatatga agaagctcta 240
tataaatcca agacaagcaa caaacccttg atgattattc atcacttgga tgagtgccca 300
cacagtcaag ctttaaagaa agtgtttgct gaaaataaag aaatccagaa attggcagag 360
cagtttgtcc tcctcaatct ggtttatgaa acaactgaca aaca 404

<210> 283
<211> 184
<212> DNA
<213> Homo sapien

<220>
<221> misc_feature
<222> (1)...(184)
<223> n = A,T,C or G

<400> 283
agtgtggtgg aattcacttg cttaanttgt gggcaaaaga gaaaaagaag gattgatcag 60
agcattgtgc aatacagttt cattaactcc ttccctcgct cccccaaaaa tttgaatttt 120
tttttcaaca ctcttacacc tgttatggaa aatgtcaacc tttgtaagaa aaccaaaata 180
aaaa 184

<210> 284
<211> 421
<212> DNA
<213> Homo sapien

<220>
<221> misc_feature
<222> (1)...(421)
<223> n = A,T,C or G

<400> 284

ctattaatcc tgccacaata tttttaatta cgtacaaaga tctgacatgt cacccaggga 60
cccatttcac ccactgctct gtttggccgc cagtcttttg tctctctctt cagcaatggt 120
gaggcggata ccctttcctc ggggaanana aatccatggt ttgttgccct tgccaataac 180
aaaaatgttg gaaagtcgag tggcaaagct gttgccattg gcatctttca cgtgaaccac 240
gtcaaaagat ccagggtgcc tctctctgtt ggtgatcaca ccaattcttc ctaggttagc 300
acctccagtc accatacaca ggttaccagt gtcgaacttg atgaaatcag taatcttgcc 360
agtctctaaa tcaatctgaa tggtatcatt caccttgatg aggggatcgg ggtagcggat 420
g 421

<210> 285
<211> 361
<212> DNA
<213> Homo sapien

<220>
<221> misc_feature
<222> (1)...(361)
<223> n = A,T,C or G

<400> 285
ctgggtggta actctttatt tcattgtccg gaanaaagat gggagtggga acagggtgga 60
cactgtgcag gcttcagctt ccactccggg caggattcag gctatctggg accgcaggga 120
ctgccaggtg cacagccctg gctcccgagg caggcaggca aggtgacggg actggaagcc 180
cttttcanag ccttggagga gctggtccgt ccacaagcaa tgagtgccac tctgcagttt 240
gcaggggatg gataaacagg gaaacactgt gcattcctca cagccaacag tgtaggtctt 300
ggtgaagccc cggcgctgag ctaagctcag gctgttccag ggagccacga aactgcaggt 360
a 361

<210> 286
<211> 336
<212> DNA
<213> Homo sapien

<220>
<221> misc_feature
<222> (1)...(336)
<223> n = A,T,C or G

<400> 286
tttgagtggc agcgccttta tttgtggggg ccttcaaggn agggtcgtgg ggggcagcgg 60
ggaggaanag ccganaaact gtgtgaccgg ggcctcaggt ggtgggcatt gggggctcct 120
cttgcanatg cccattggca tcaccggtgc agccattggt ggcagcgggt accggtcctt 180
tcttgttcaa catagggtag gtggcagcca cgggtccaac tcgcttgagg ctgggccctg 240
ggcgctccat tttgtgttcc angagcatgt ggttctgtgg cgggagcccc acgcaggccc 300
tgaggatgtt ctcgatgcag ctgcgctggc ggaaaa 336

<210> 287
<211> 301
<212> DNA
<213> Homo sapien

<220>
<221> misc_feature
<222> (1)...(301)
<223> n = A,T,C or G

<400> 287
tgggtaccaa atttntttat ttgaaggaat ggnacaaatc aaanaactta agnggatgtt 60
ttggtacaac ttatanaaaa ggnaaaggaa accccaacat gcatgcnctg ccttggngac 120
cagggaagtc accccacggc tatggggaaa ttancccgag gcttancttt cattatcact 180
gtctcccagg gngngcttgt caaaaanata ttccnccaag ccaaattcgg gcgctcccat 240
nttgcncaag ttggtcacgt ggtcacccaa ttctttgatg gctttcacct gctcattcag 300
g 301

<210> 288
<211> 358
<212> DNA
<213> Homo sapien

<220>
<221> misc_feature
<222> (1)...(358)
<223> n = A,T,C or G

<400> 288
aagtttttaa actttttatt tgcatattaa aaaaattgng cattccaata attaaaatca 60
tttgaacaaa aaaaaaaatg gcactctgat taaactgcat tacagcctgc aggacacctt 120
gggccagctt ggttttactc tanatttcac tgtcgtccca ccccacttct tccaccccac 180
ttcttccttc accaacatgc aagttctttc cttccctgcc agccanatag atagacagat 240
gggaaaggca ggcgcggcct tcgttgtcag tagttctttg atgtgaaagg ggcagcacag 300
tcatttaaac ttgatccaac ctctttgcat cttacaaagt taaacagcta aaagaagt 358

<210> 289
<211> 462
<212> DNA
<213> Homo sapien

<220>
<221> misc_feature
<222> (1)...(462)
<223> n = A,T,C or G

<400> 289
   ggcatcagaa atgctgttta tttctctgct gctcccaagc tggctggcct ttgcagagga 60
   gcagacaaca gatgcatagt tggggganaa gggaggacag gttccaggat agagggtgca 120
   ggctgaggga ggaagggtaa naggaaggaa ggccatcctg gatccccaca tttcagtctc 180
   anatgaggac aaagggactc ccaagccccc aaatcatcan aaaacaccaa ggagcaggag 240
   gagcttgagc aggccccagg gagcctcana gccataccag ccactgtcta cttcccatcc 300
   tcctctccca ttccctgtct gcttcanacc acctcccagc taagccccag ctccattccc 360
   ccaatcctgg cccttgccag cttgacagtc acagtgcctg gaattccacc actgaggctt 420
   ctcccagttg gattaggacg tcgccctgtt agcatgctgc cc 462

<210> 290
<211> 481
<212> DNA
<213> Homo sapien

<220>
<221> misc_feature
<222> (1)...(481)

<223> n = A,T,C or G

<400> 290
tactttccta aactttatta aagaaaaaag caataagcaa tggnggtaaa tctctanaac 60
atacccaatt ttctgggctt cctcccccga gaatgtgaca ttttgatttc caaacatgcc 120
anaagtgtat ggttcccaac tgtactaaag taggtganaa gctgaagtcc tcaagtgttc 180
atcttccaac ttttcccagt ctgtggtctg tctttggatc agcaataatt gcctgaacag 240
ctactatggc ttcgttgatt tttgtctgta gctctctgag ctcctctatg tgcagcaatc 300
gcanaatttg agcagcttca ttaanaactg catctcctgt gtcaaaacca anaatatgtt 360
tgtctaaagc aacaggtaag ccctcttttg tttgatttgc cttancaact gcatcctgtg 420
tcaggcgctc ctgaaccaaa atccgaattg ccttaagcat taccaggtaa tcatcatgac 480
g 481

<210> 291
<211> 381
<212> DNA
<213> Homo sapien

<220>
<221> misc_feature
<222> (1)...(381)
<223> n = A,T,C or G

<400> 291
tcatagtaat gtaaaaccat ttgtttaatt ctaaatcaaa tcactttcac aacagtgaaa 60
attagtgact ggttaaggng tgccactgta catatcatca ttttctgact ggggtcagga 120
cctggtccta gtccacaagg gtggcaggag gagggtggag gctaanaaca cagaaaacac 180
acaaaanaaa ggaaagctgc cttggcanaa ggatgaggng gtgagcttgc cgaaggatgg 240
tgggaagggg gctccctgtt ggggccgagc caggagtccc aagtcagctc tcctgcctta 300
cttagctcct ggcanagggt gagtggggac ctacgaggtt caaaatcaaa tggcatttgg 360
ccagcctggc tttactaaca g 381

<210> 292
<211> 371
<212> DNA
<213> Homo sapien

<220>
<221> misc_feature
<222> (1)...(371)
<223> n = A,T,C or G

<400> 292
gaaaaaataa tccgtttaat tgaaaaacct gnaggatact attccactcc cccanatgag 60
gaggctgagg anaccaaacc cctacatcac ctcgtagcca cttctgatac tcttcacgag 120
gcagcaggca aagacaattc ccaaaacctc nacaaaagca attccaaggg ctgctgcagc 180
taccaccanc acatttttcc tcagccagcc cccaatcttc tccacacagc cctccttatg 240
gatcgccttc tcgttgaaat taatcccaca gcccacagta acattaatgc ancaggagtc 300
ggggactcgg ttcttcgaca tggaagggat tttctcccaa tctgtgtagt tagcagcccc 360
acagcactta a 371

<210> 293
<211> 361
<212> DNA
<213> Homo sapien

<220>
<221> misc_feature
<222> (1)...(361)
<223> n = A,T,C or G

<400> 293
gatttaaaag aaaacacttt attgttcagc aattaaaagt tagccaaata tgtatttttc 60
tccataattt attgngatgt tatcaacatc aagtaaaatg ctcattttca tcatttgctt 120
ctgttcatgt tttcttgaac acgtcttcaa ttttccttcc aaaatgctgc atgccacact 180
tgaggtaacg aagcanaagt atttttaaac atgacagcta anaacattca tctacagcaa 240
cctatatgct caatacatgc cgcgtgatcc tagtagtttt ttcacaacct tctacaagtt 300
tttggaaaac atctgttatg atgactttca tacaccttca cctcaaaggc tttcttgcac 360
c 361

<210> 294
<211> 391
<212> DNA
<213> Homo sapien

<220>
<221> misc_feature
<222> (1)...(391)
<223> n = A,T,C or G

<400> 294
tattttaaag tttaattatg attcanaaaa aatcgagcga ataactttct ctgaaaaaat 60
atattgactc tgtatanacc acagttattg gggganaagg gctggtaggt taaattatcc 120
tattttttat tctgaaaatg atattaatan aaagtcccgt ttccagtctg attataaaga 180
tacatatgcc caaaatggct ganaataaat acaacaggaa atgcaaaagc tgtaaagcta 240
agggcatgca ananaaaatc tcanaatacc caaagnggca acaaggaacg tttggctgga 300
atttgaagtt atttcagtca tctttgtctt tggctccatg tttcaggatg cgtgtgaact 360
cgatgtaatt gaaattcccc tttttatcaa t 391

<210> 295
<211> 343
<212> DNA
<213> Homo sapien

<220>
<221> misc_feature
<222> (1)...(343)
<223> n = A,T,C or G

<400> 295
ttcttttgtt ttattgataa cagaaactgt gcataattac agatttgatg aggaatctgc 60
aaataataaa gaatgtgtct actgccagca aaatacaatt attccatgcc ctctcaacat 120
acaaatatag agttcttcac accanatggc tctggtgtaa caaagccatt ttanatgttt 180
aattgtgctt ctacaaaacc ttcanagcat gaggtagttt cttttaccta cnatattttc 240
cacatttcca ttattacact tttagtgagc taaaatcctt ttaacatagc ctgcggatga 300
tctttcacaa aagccaagcc tcatttacaa agggtttatt tct 343

<210> 296
<211> 241
<212> DNA

<213> Homo sapien

<220>
<221> misc_feature
<222> (1)...(241)
<223> n = A,T,C or G

<400> 296
ttcttggata ttggttgttt ttgtgaaaaa gtttttgttt ttcttctcag tcaactgaat 60
tatttctcta ctttgccctc ctgatgccca catgananaa cttaanataa tttctaacag 120
cttccacttt ggaaaaaaaa aaaacctgtt ttcctcatgg aaccccagga gttgaaagtg 180
gatanatcgc tctcaaaatc taaggctctg ttcagcttta cattatgtta cctgacgttt 240
t 241

<210> 297
<211> 391
<212> DNA
<213> Homo sapien

<220>
<221> misc_feature
<222> (1)...(391)
<223> n = A,T,C or G

<400> 297
gttgtggctg anaatgctgg agatgctcag ttctctccct cacaaggtag gccacaaatt 60
cttggtggtg ccctcacatc tggggtcttc aggcaccagc catgcctgcc gaggagtgct 120
gtcaggacan accatgtccg tgctaggccc aggcacagcc caaccactcc tcatccaagt 180
ctctcccagg tttctggtcc cgatgggcaa ggatgacccc tccagtggct ggtaccccac 240
catcccacta cccctcacat gctctcactc tccatcaggt ccccaatcct ggcttccctc 300
ttcacgaact ctcaaagaaa aggaaggata aaacctaaat aaaccagaca gaagcagctc 360
tggaaaagta caaaaagaca gccagaggtg t 391

<210> 298
<211> 321
<212> DNA
<213> Homo sapien

<220>
<221> misc_feature
<222> (1)...(321)
<223> n = A,T,C or G

<400> 298
caagccaaac tgtntccagc tttattaaan atactttcca taaacaatca tggtatttca 60
ggcaggacat gggcanacaa tcgttaacag tatacaacaa ctttcaaact cccttnttca 120
atggactacc aaaaatcaaa aagccactat aaaacccaat gaagtcttca tctgatgctc 180
tgaacaggga aagtttaaag ngagggttga catttcacat ttagcatgtt gtttaacaac 240
ttttcacaag ccgaccctga ctttcaggaa gtgaaatgaa aatggcanaa tttatctgaa 300
natccacaat ctaaaaatgg a 321

<210> 299
<211> 401
<212> DNA
<213> Homo sapien

<220>
<221> misc_feature
<222> (1)...(401)
<223> n = A,T,C or G

<400> 299
tatcataaag agtgttgaag tttatttatt atagcaccat tgagacattt tgaaattgga 60
attggtaaaa aaataaaaca aaaagcattt gaattgtatt tggnggaaca gcaaaaaaag 120
agaagtatca tttttctttg tcaaattata ctgtttccaa acattttgga aataaataac 180
tggaattttg tcggtcactt gcactggttg acaagattag aacaagagga acacatatgg 240
agttaaattt tttttgttgg gatttcanat agagtttggt ttataaaaag caaacagggc 300
caacgtccac accaaattct tgatcaggac caccaatgtc atagggngca atatctacaa 360
taggtagtct cacagccttg cgtgttcgat attcaaagac t 401

<210> 300
<211> 188
<212> DNA
<213> Homo sapien

<220>
<221> misc_feature
<222> (1)...(188)
<223> n = A,T,C or G

<400> 300
tgaatgcttt gtcatattaa gaaagttaaa gtgcaataat gtttgaanac aataagtggt 60
ggtgtatctt gtttctaata agataaactt ttttgtcttt gctttatctt attagggagt 120
tgtatgtcag tgtataaaac atactgtgtg gtataacagg cttaataaat tctttaaaag 180
gaaaaaaa 188

<210> 301
<211> 291
<212> DNA
<213> Homo sapien

<400> 301
   aagattttgt ttttatttat tatggctaga aagacactgt tatagccaaa atcggcaatg 60
   acactaaaga aatcctctgt gcttttcaat atgcaaatat atttcttcca agagttgccc 120
   tggtgtgact tcaagagttc atgttaactt cttttctgga aacttccttt tcttagttgt 180
   tgtattcttg aagagcctgg gccatgaaga gcttgcctaa gttttgggca gtgaactcct 240
   tgatgttctg gcagtaagtg tttatctggc ctgcaatgag cagcgagtcc a 291

<210> 302
<211> 341
<212> DNA
<213> Homo sapien

<220>
<221> misc_feature
<222> (1)...(341)
<223> n = A,T,C or G

<400> 302
tgatttttca taattttatt aaatnatcac tgggaaaact aatggttcgc gtatcacaca 60

attacactac aatctgatag gagtggtaaa accagccaat ggaatccagg taaagtacaa 120
aaacgccacc ttttattgtc ctgtcttatt tctcgggaag gagggttcta ctttacacat 180
ttcatgagcc agcagtggac ttgagttaca atgtgtaggt tccttgtggt tatagctgca 240
gaagaagcca tcaaattctt gaggacttga catctctcgg aaagaagcaa actagtggat 300
cccccgggct gcaggaattc gatatcaagc ttatcgatac c 341

<210> 303
<211> 361
<212> DNA
<213> Homo sapien

<220>
<221> misc_feature
<222> (1)...(361)
<223> n = A,T,C or G

<400> 303
tgcagacagt aaatnaattt tatttgngtt cacagaacat actaggcgat ctcgacagtc 60
gctccgtgac agcccaccaa cccccaaccc tntacctcgc agccacccta aaggcgactt 120
caanaanatg gaaggatctc acggatctca ttcctaatgg tccgccgaag tctcacacag 180
tanacagacg gagttganat gctggaggat gcagtcacct cctaaactta cgacccacca 240
ccanacttca tcccagccgg gacgtcctcc cccacccgag tcctccccat ttcttctcct 300
actttgccgc agttccaggn gtcctgcttc caccagtccc acaaagctca ataaatacca 360
a 361

<210> 304
<211> 301
<212> DNA
<213> Homo sapien

<220>
<221> misc_feature
<222> (1)...(301)
<223> n = A,T,C or G

<400> 304
ctctttacaa cagcctttat ttncggccct tgatcctgct cggatgctgg tggaggccct 60
tagctccgcc cgccaggctc tgtgccgcct ccccgcaggc gcanattcat gaacacggtg 120
ctcaggggct tgaggccgta ctcccccagc gggagctggt cctccagggg cttcccctcg 180
aaggtcagcc anaacaggtc gtcctgcaca ccctccagcc cgctcacttg ctgcttcagg 240
tgggccacgg tctgcgtcag ccgcacctcg taggtgctgc tgcggccctt gttattcctc 300
a 301

<210> 305
<211> 331
<212> DNA
<213> Homo sapien

<220>
<221> misc_feature
<222> (1)...(331)
<223> n = A,T,C or G

<400> 305
   ganaggctag taacatcagt tttattgggt tggggnggca accatagcct ggctgggggn 60
   ggggctggcc ctcacaggtt gttgagttcc agcagggtct ggtccaaggt ctggtgaatc 120
   tcgacgttct cctccttggc actggccaag gtctcttcta ggtcatcgat ggttttctcc 180
   aactttgcca canacctctc ggcaaactct gctcgggtct cancctcctt cagcttctcc 240
   tccaacagtt tgatctcctc ttcatattta tcttctttgg gggaatactc ctcctctgag 300
   gccatcaggg acttgagggc ctggtccatg g 331

<210> 306
<211> 457
<212> DNA
<213> Homo sapien

<400> 306
aatatgtaaa ggtaataact tttattatat taaagacaat gcaaacgaaa aacagaattg 60
agcagtgcaa aatttaaagg actgttttgt tctcaaagtt gcaagtttca aagccaaaag 120
aattatatgt atcaaatata taagtaaaaa aaagttagac tttcaagcct gtaatcccag 180
cactttggga ggctgaggca ggtggatcac taacattaaa aagacaacat tagattttgt 240
cgatttatag caattttata aatatataac tttgtcactt ggatcctgaa gcaaaataat 300
aaagtgaatt tgggattttt gtacttggta aaaagtttaa caccctaaat tcacaactag 360
tggatccccc gggctgcagg aattcgatat caagcttatc gataccgtcg acctcgaggg 420
ggggcccggt acccaattcg ccctatagtg agtcgta 457

<210> 307
<211> 491
<212> DNA
<213> Homo sapien

<400> 307
gtgcttggac ggaacccggc gctcgttccc caccccggcc ggccgcccat agccagccct 60
ccgtcacctc ttcaccgcac cctcggactg ccccaaggcc cccgccgccg ctccagcgcc 120
gcgcagccac cgccgccgcc gccgcctctc cttagtcgcc gccatgacga ccgcgtccac 180
ctcgcaggtg cgccagaact accaccagga ctcagaggcc gccatcaacc gccagatcaa 240
cctggagctc tacgcctcct acgtttacct gtccatgtct tactactttg accgcgatga 300
tgtggctttg aagaactttg ccaaatactt tcttcaccaa tctcatgagg agagggaaca 360
tgctgagaaa ctgatgaagc tgcagaacca acgaggtggc cgaatcttcc ttcaggatat 420
caagaaacca gactgtgatg actgggagag cgggctgaat gcaatggagt gtgcattaca 480
tttggaaaaa a 491

<210> 308
<211> 421
<212> DNA
<213> Homo sapien

<400> 308
ctcagcgctt cttctttctt ggtttgatcc tgactgctgt catggcgtgc cctctggaga 60
aggccctgga tgtgatggtg tccaccttcc acaagtactc gggcaaagag ggtgacaagt 120
tcaagctcaa caagtcagaa ctaaaggagc tgctgacccg ggagctgccc agcttcttgg 180
ggaaaaggac agatgaagct gctttccaga agctgatgag caacttggac agcaacaggg 240
acaacgaggt ggacttccaa gagtactgtg tcttcctgtc ctgcatcgcc atgatgtgta 300
acgaattctt tgaaggcttc ccagataagc agcccaggaa gaaatgaaaa ctcctctgat 360
gtggttgggg ggtctgccag ctggggccct ccctgtcgcc agtgggcact tttttttttc 420
c 421

<210> 309
<211> 321
<212> DNA

<213> Homo sapien

<400> 309
accaaatggc ggatgacgcc ggtgcagcgg gggggcccgg gggccctggt ggccctggga 60
tggggaaccg cggtggcttc cgcggaggtt tcggcagtgg catccggggc cggggtcgcg 120
gccgtggacg gggccggggc cgaggccgcg gagctcgcgg aggcaaggcc gaggataagg 180
agtggatgcc cgtcaccaag ttgggccgct tggtcaagga catgaagatc aagtccctgg 240
aggagatcta tctcttctcc ctgcccatta aggaatcaga gatcattgat ttcttcctgg 300
gggcctctct caaggatgag g 321

<210> 310
<211> 381
<212> DNA
<213> Homo sapien

<400> 310
ttaaccagcc atattggctc aataaatagc ttcggtaagg agttaatttc cttctagaaa 60
tcagtgccta tttttcctgg aaactcaatt ttaaatagtc caattccatc tgaagccaag 120
ctgttgtcat tttcattcgg tgacattctc tcccatgaca cccagaaggg gcagaagaac 180
cacatttttc atttatagat gtttgcatcc tttgtattaa aattattttg aaggggttgc 240
ctcattggat ggcttttttt tttttcctcc agggagaagg ggagaaatgt acttggaaat 300
taatgtatgt ttacatctct ttgcaaattc ctgtacatag agatatattt tttaagtgtg 360
aatgtaacaa catactgtga a 381

<210> 311
<211> 538
<212> DNA
<213> Homo sapien

<400> 311
tttgaattta caccaagaac ttctcaataa aagaaaatca tgaatgctcc acaatttcaa 60
cataccacaa gagaagttaa tttcttaaca ttgtgttcta tgattatttg taagaccttc 120
accaagttct gatatctttt aaagacatag ttcaaaattg cttttgaaaa tctgtattct 180
tgaaaatatc cttgttgtgt attaggtttt taaataccag ctaaaggatt acctcactga 240
gtcatcagta ccctcctatt cagctcccca agatgatgtg tttttgctta ccctaagaga 300
ggttttcttc ttatttttag ataattcaag tgcttagata aattatgttt tctttaagtg 360
tttatggtaa actcttttaa agaaaattta atatgttata gctgaatctt tttggtaact 420
ttaaatcttt atcatagact ctgtacatat gttcaaatta gctgcttgcc tgatgtgtgt 480
atcatcggtg ggatgacaga acaaacatat ttatgatcat gaataatgtg ctttgtaa 538

<210> 312
<211> 176
<212> DNA
<213> Homo sapien

<400> 312
ggaggagcag ctgagagata gggtcagtga atgcggttca gcctgctacc tctcctgtct 60
tcatagaacc attgccttag aattattgta tgacacgttt tttgttggtt aagctgtaag 120
gttttgttct ttgtgaacat gggtattttg aggggagggt ggagggagta gggaag 176

<210> 313
<211> 396
<212> DNA
<213> Homo sapien

<400> 313
ccagcacccc caggccctgg gggacctggg ttctcagact gccaaagaag ccttgccatc 60
tggcgctccc atggctcttg caacatctcc ccttcgtttt tgagggggtc atgccggggg 120
agccaccagc ccctcactgg gttcggagga gagtcaggaa gggccaagca cgacaaagca 180
gaaacatcgg atttggggaa cgcgtgtcaa tcccttgtgc cgcagggctg ggcgggagag 240
actgttctgt tccttgtgta actgtgttgc tgaaagacta cctcgttctt gtcttgatgt 300
gtcaccgggg caactgcctg ggggcgggga tgggggcagg gtggaagcgg ctccccattt 360
tataccaaag gtgctacatc tatgtgatgg gtgggg 396

<210> 314
<211> 311
<212> DNA
<213> Homo sapien

<400> 314
cctcaacatc ctcagagagg actggaagcc agtccttacg ataaactcca taatttatgg 60
cctgcagtat ctcttcttgg agcccaaccc cgaggaccca ctgaacaagg aggccgcaga 120
ggtcctgcag aacaaccggc ggctgtttga gcagaacgtg cagcgctcca tgcggggtgg 180
ctacatcggc tccacctact ttgagcgctg cctgaaatag ggttggcgca tacccacccc 240
cgccacggcc acaagccctg gcatcccctg caaatattta ttgggggcca tgggtagggg 300
tttgggggc g 311

<210> 315
<211> 336
<212> DNA
<213> Homo sapien

<400> 315
tttagaacat ggttatcatc caagactact ctaccctgca acattgaact cccaagagca 60
aatccacatt cctcttgagt tctgcagctt ctgtgtaaat agggcagctg tcgtctatgc 120
cgtagaatca catgatctga ggaccattca tggaagctgc taaatagcct agtctgggga 180
gtcttccata aagttttgca tggagcaaac aaacaggatt aaactaggtt tggttccttc 240
agccctctaa aagcataggg cttagcctgc aggcttcctt gggctttctc tgtgtgtgta 300
gttttgtaaa cactatagca tctgttaaga tccagt 336

<210> 316
<211> 436
<212> DNA
<213> Homo sapien

<400> 316
aacatggtct gcgtgcctta agagagacgc ttcctgcaga acaggacctg actacaaaga 60
atgtttccat tggaattgtt ggtaaagact tggagtttac aatctatgat gatgatgatg 120
tgtctccatt cctggaaggt cttgaagaaa gaccacagag aaaggcacag cctgctcaac 180
ctgctgatga acctgcagaa aaggctgatg aaccaatgga acattaagtg ataagccagt 240
ctatatatgt attatcaaat atgtaagaat acaggcacca catactgatg acaataatct 300
atactttgaa ccaaaagttg cagagtggtg gaatgctatg ttttaggaat cagtccagat 360
gtgagttttt tccaagcaac ctcactgaaa cctatataat ggaatacatt tttctttgaa 420
agggtctgta taatca 436

<210> 317
<211> 196
<212> DNA
<213> Homo sapien

<400> 317
   tattccttgt gaagatgata tactattttt gttaagcgtg tctgtattta tgtgtgagga      60
   gctgctggct tgcagtgcgc gtgcacgtgg agagctggtg cccggagatt ggacggcctg     120
   atgctccctc ccctgccctg gtccagggaa gctggccgag ggtcctggct cctgaggggc     180
   atctgcccct ccccca                                                    196

<210> 318
<211> 381
<212> DNA
<213> Homo sapien

<220>
<221> misc_feature
<222> (1)...(381)
<223> n = A,T,C or G

<400> 318
gacgcttnng ccgtaacgat gatcggagac atcctgctgt tcgggacgtt gctgatgaat      60
gccggggcgg tgctgaactt taagctgaaa aagaaggaca cncagggctt tggggaggag     120
tncagggagc ccaacacagg tgacaacatc cgggaattct tgctgancct cagatacttt     180
cnaatcttca tcnccctgtg gaacatcttc atgatgttct gcatgattgt gctgntcggc     240
tcttgaatcc cancgatgaa accannaact cactttcccg ggatgccgan tctccattcc     300
tccattcctg atgacttcaa naatgttttt gaccaaaaaa ccgacaacct tcccagaaag     360
tccaagctcg tggtgggngg a                                               381

<210> 319
<211> 506
<212> DNA
<213> Homo sapien

<400> 319
ctaagcttta cgaatggggt gacaacttat gataaaaact agagctagtg aattagccta      60
tttgtaaata cctttgttat aattgatagg atacatcttg gacatggaat tgttaagcca     120
cctctgagca gtgtatgtca ggacttgttc attaggttgg cagcagaggg gcagaaggaa     180
ttatacaggt agagatgtat gcagatgtgt ccatatatgt ccatatttac attttgatag     240
ccattgatgt atgcatctct tggctgtact ataagaacac attaattcaa tggaaataca     300
ctttgctaat attttaatgg tatagatctg ctaatgaatt ctcttaaaaa catactgtat     360
tctgttgctg tgtgtttcat tttaaattga gcattaaggg aatgcagcat ttaaatcaga     420
actctgccaa tgcttttatc tagaggcgtg ttgccatttt tgtcttatat gaaatttctg     480
tcccaagaaa ggcaggatta catctt                                          506

<210> 320
<211> 351
<212> DNA
<213> Homo sapien

<400> 320
ctgacctgca ggacgaaacc atgaagagcc tgatccttct tgccatcctg gccgccttag      60
cggtagtaac tttgtgttat gaatcacatg aaagcatgga atcttatgaa cttaatccct     120
tcattaacag gagaaatgca aataccttca tatcccctca gcagagatgg agagctaaag     180
tccaagagag gatccgagaa cgctctaagc ctgtccacga gctcaatagg gaagcctgtg     240
atgactacag actttgcgaa cgctacgcca tggtttatgg atacaatgct gcctataatc     300
gctacttcag gaagcgccga gggaccaaat gagactgagg gaagaaaaaa a              351

<210> 321

<211> 421
<212> DNA
<213> Homo sapien

<400> 321
ctcggaggcg ttcagctgct tcaagatgaa gctgaacatc tccttcccag ccactggctg 60
ccagaaactc attgaagtgg acgatgaacg caaacttcgt actttctatg agaagcgtat 120
ggccacagaa gttgctgctg acgctctggg tgaagaatgg aagggttatg tggtccgaat 180
cagtggtggg aacgacaaac aaggtttccc catgaagcag ggtgtcttga cccatggccg 240
tgtccgcctg ctactgagta aggggcattc ctgttacaga ccaaggagaa ctggagaaag 300
aaagagaaaa tcagttcgtg gttgcattgt ggatgcaaat ctgagcgttc tcaacttggt 360
tattgtaaaa aaaggagaga aggatattcc tggactgact gatactacag tgcctcgccg 420
c 421

<210> 322
<211> 521
<212> DNA
<213> Homo sapien

<400> 322
agcagctctc ctgccacagc tcctcacccc ctgaaaatgt tcgcctgctc caagtttgtc 60
tccactccct ccttggtcaa gagcacctca cagctgctga gccgtccgct atctgcagtg 120
gtgctgaaac gaccggagat actgacagat gagagcctca gcagcttggc agtctcatgt 180
ccccttacct cacttgtctc tagccgcagc ttccaaacca gcgccatttc aagggacatc 240
gacacagcag ccaagttcat tggagctggg gctgccacag ttggggtggc tggttctggg 300
gctgggattg gaactgtgtt tgggagcctc atcattggtt atgccaggaa cccttctctg 360
aagcaacagc tcttctccta cgccattctg ggctttgccc tctcggaggc catggggctc 420
ttttgtctga tggtagcctt tctcatcctc tttgccatgt gaaggagccg tctccacctc 480
ccatagttct cccgcgtctg gttggccccg tgtgttcctt t 521

<210> 323
<211> 435
<212> DNA
<213> Homo sapien

<400> 323
ccgaggtcgc acgcgtgaga cttctccgcc gcagacgccg ccgcgatgcg ctacgtcgcc 60
tcctacctgc tggctgccct agggggcaac tcctccccca gcgccaagga catcaagaag 120
atcttggaca gcgtgggtat cgaggcggac gacgaccggc tcaacaaggt tatcagtgag 180
ctgaatggaa aaaacattga agacgtcatt gcccagggta ttggcaagct tgccagtgta 240
cctgctggtg gggctgtagc cgtctctgct gccccaggct ctgcagcccc tgctgctggt 300
tctgcccctg ctgcagcaga ggagaagaaa gatgagaaga aggaggagtc tgaagagtca 360
gatgatgaca tgggatttgg cctttttgat taaattcctg ctcccctgca aataaagcct 420
ttttacacat ctcaa 435

<210> 324
<211> 521
<212> DNA
<213> Homo sapien

<400> 324
aggagatcga ctttcggtgc ccgcaagacc agggctggaa cgccgagatc acgctgcaga 60
tggtgcagta caagaatcgt caggccatcc tggcggtcaa atccacgcgg cagaagcagc 120
agcacctggt ccagcagcag cccccctcgc agccgcagcc gcagccgcag ctccagcccc 180
aaccccagcc tcagcctcag ccgcaacccc agccccaatc acaaccccag cctcagcccc 240

aacccaagcc tcagccccag cagctccacc cgtatccgca tccacatcca catccacact 300
ctcatcctca ctcgcaccca caccctcacc cgcacccgca tccgcaccaa ataccgcacc 360
cacacccaca gccgcactcg cagccgcacg ggcaccggct tctccgcagc acctccaact 420
ctgcctgaaa ggggcagctc ccgggcaaga caaggttttg aggacttgag gaagtgggac 480
gagcacattt ctattgtctt cacttggatc aaaagcaaaa c 521

<210> 325
<211> 451
<212> DNA
<213> Homo sapien

<400> 325
attttcattt ccattaacct ggaagctttc atgaatattc tcttctttta aaacatttta 60
acattattta aacagaaaaa gatgggctct ttctggttag ttgttacatg atagcagaga 120
tatttttact tagattactt tgggaatgag agattgttgt cttgaactct ggcactgtac 180
agtgaatgtg tctgtagttg tgttagtttg cattaagcat gtataacatt caagtatgtc 240
atccaaataa gaggcatata cattgaattg tttttaatcc tctgacaagt tgactcttcg 300
acccccaccc ccacccaaga cattttaata gtaaatagag agagagagaa gagttaatga 360
acatgaggta gtgttccact ggcaggatga cttttcaata gctcaaatca atttcagtgc 420
ctttatcact tgaattatta acttaatttg a 451

<210> 326
<211> 421
<212> DNA
<213> Homo sapien

<220>
<221> misc_feature
<222> (1)...(421)
<223> n = A,T,C or G

<400> 326
cgcggtcgta agggctgagg atttttggtc cgcacgctcc tgctcctgac tcaccgctgt 60
tcgctctcgc cgaggaacaa gtcggtcagg aagcccgcgc gcaacagcca tggcttttaa 120
ggataccgga aaaacacccg tggagccgga ggtggcaatt caccgaattc gaatcaccct 180
aacaagccgc aacgtaaaat ccttggaaaa ggtgtgtgct gacttgataa gaggcgcaaa 240
agaaaagaat ctcaaagtga aaggaccagt tcgaatgcct accaagactt tgagantcac 300
tacaagaaaa actccttgtg gtgaaggttc taagacgtgg gatcgtttcc agatgagaat 360
tcacaagcga ctcattgact tgcacagtcc ttctgagatt gttaagcaga ttacttccat 420
c 421

<210> 327
<211> 456
<212> DNA
<213> Homo sapien

<400> 327
atcttgacga ggctgcggtg tctgctgcta ttctccgagc ttcgcaatgc cgcctaagga 60
cgacaagaag aagaaggacg ctggaaagtc ggccaagaaa gacaaagacc cagtgaacaa 120
atccgggggc aaggccaaaa agaagaagtg gtccaaaggc aaagttcggg acaagctcaa 180
taacttagtc ttgtttgaca aagctaccta tgataaactc tgtaaggaag ttcccaacta 240
taaacttata accccagctg tggtctctga gagactgaag attcgaggct ccctggccag 300
ggcagccctt caggagctcc ttagtaaagg acttatcaaa ctggtttcaa agcacagagc 360
tcaagtaatt tacaccagaa ataccaaggg tggagatgct ccagctgctg gtgaagatgc 420
atgaataggt ccaaccagct gtacatttgg aaaaat 456

<210> 328
<211> 471
<212> DNA
<213> Homo sapien

<400> 328
gtggaagtga catcgtcttt aaaccctgcg tggcaatccc tgacgcaccg ccgtgatgcc 60
cagggaagac agggcgacct ggaagtccaa ctacttcctt aagatcatcc aactattgga 120
tgattatccg aaatgtttca ttgtgggagc agacaatgtg ggctccaagc agatgcagca 180
gatccgcatg tcccttcgcg ggaaggctgt ggtgctgatg ggcaagaaca ccatgatgcg 240
caaggccatc cgagggcacc tggaaaacaa cccagctctg gagaaactgc tgcctcatat 300
ccgggggaat gtgggctttg tgttcaccaa ggaggacctc actgagatca gggacatgtt 360
gctggccaat aaggtgccag ctgctgcccg tgctggtgcc attgccccat gtgaagtcac 420
tgtgccagcc cagaacactg gtctcgggcc cgagaagacc tcctttttcc a 471

<210> 329
<211> 278
<212> DNA
<213> Homo sapien

<220>
<221> misc_feature
<222> (1)...(278)
<223> n = A,T,C or G

<400> 329
gtttaaactt aagcttggta ccgagctcgg atccactagt ccagtgtggt ggaattctag 60
aaattgagat gcccccccag gccagcaaat gttccttttt gttcaaagtc tatttttatt 120
ccttgatatt tttctttttt tttttttttt ttgnggatgg ggacttgtga atttttctaa 180
aggtgctatt taacatggga gganagcgtg tgcggctcca gcccagcccg ctgctcactt 240
tccaccctct ctccacctgc ctctggcttc tcaggcct 278

<210> 330
<211> 338
<212> DNA
<213> Homo sapien

<400> 330
ctcaggcttc aacatcgaat acgccgcagg ccccttcgcc ctattcttca tagccgaata 60
cacaaacatt attataataa acaccctcac cactacaatc ttcctaggaa caacatatga 120
cgcactctcc cctgaactct acacaacata ttttgtcacc aagaccctac ttctaacctc 180
cctgttctta tgaattcgaa cagcataccc ccgattccgc tacgaccaac tcatacacct 240
cctatgaaaa aacttcctac cactcaccct agcattactt atatgatatg tctccatacc 300
cattacaatc tccagcattc cccctcaaac ctaaaaaa 338

<210> 331
<211> 2820
<212> DNA
<213> Homo sapiens

<400> 331
tggcaaaatc ctggagccag aagaaaggac agcagcattg atcaatctta cagctaacat 60
gttgtacctg gaaaacaatg cccagactca atttagtgag ccacagtaca cgaacctggg 120

gctcctgaac agcatggacc agcagattcg gaacggctcc tcgtccacca gtccctataa 180
cacagaccac gcgcagaaca gcgtcacggc gccctcgccc tacgcacagc ccagccccac 240
cttcgatgct ctctctccat cacccgccat cccctccaac accgactacc caggcccgca 300
cagttccgac gtgtccttcc agcagtcgag caccgccaag tcggccacct ggacgtattc 360
cactgaactg aagaaactct actgccaaat tgcaaagaca tgccccatcc agatcaaggt 420
gatgacccca cctcctcagg gagctgttat ccgcgccatg cctgtctaca aaaaagctga 480
gcacgtcacg gaggtggtga agcggtgccc caaccatgag ctgagccgtg agttcaacga 540
gggacagatt gcccctccta gtcatttgat tcgagtagag gggaacagcc atgcccagta 600
tgtagaagat cccatcacag gaagacagag tgtgctggta ccttatgagc caccccaggt 660
tggcactgaa ttcacgacag tcttgtacaa tttcatgtgt aacagcagtt gtgttggagg 720
gatgaaccgc cgtccaattt taatcattgt tactctggaa accagagatg ggcaagtcct 780
gggccgacgc tgctttgagg cccggatctg tgcttgccca ggaagagaca ggaaggcgga 840
tgaagatagc atcagaaagc agcaagtttc ggacagtaca aagaacggtg atggtacgaa 900
gcgcccgttt cgtcagaaca cacatggtat ccagatgaca tccatcaaga aacgaagatc 960
cccagatgat gaactgttat acttaccagt gaggggccgt gagacttatg aaatgctgtt 1020
gaagatcaaa gagtccctgg aactcatgca gtaccttcct cagcacacaa ttgaaacgta 1080
caggcaacag caacagcagc agcaccagca cttacttcag aaacagacct caatacagtc 1140
tccatcttca tatggtaaca gctccccacc tctgaacaaa atgaacagca tgaacaagct 1200
gccttctgtg agccagctta tcaaccctca gcagcgcaac gccctcactc ctacaaccat 1260
tcctgatggc atgggagcca acattcccat gatgggcacc cacatgccaa tggctggaga 1320
catgaatgga ctcagcccca cccaggcact ccctccccca ctctccatgc catccacctc 1380
ccactgcaca cccccacctc cgtatcccac agattgcagc attgtcagtt tcttagcgag 1440
gttgggctgt tcatcatgtc tggactattt cacgacccag gggctgacca ccatctatca 1500
gattgagcat tactccatgg atgatctggc aagtctgaaa atccctgagc aatttcgaca 1560
tgcgatctgg aagggcatcc tggaccaccg gcagctccac gaattctcct ccccttctca 1620
tctcctgcgg accccaagca gtgcctctac agtcagtgtg ggctccagtg agacccgggg 1680
tgagcgtgtt attgatgctg tgcgattcac cctccgccag accatctctt tcccaccccg 1740
agatgagtgg aatgacttca actttgacat ggatgctcgc cgcaataagc aacagcgcat 1800
caaagaggag ggggagtgag cctcaccatg tgagctcttc ctatccctct cctaactgcc 1860
agccccctaa aagcactcct gcttaatctt caaagccttc tccctagctc ctccccttcc 1920
tcttgtctga tttcttaggg gaaggagaag taagaggcta cctcttacct aacatctgac 1980
ctggcatcta attctgattc tggctttaag ccttcaaaac tatagcttgc agaactgtag 2040
ctgccatggc taggtagaag tgagcaaaaa agagttgggt gtctccttaa gctgcagaga 2100
tttctcattg acttttataa agcatgttca cccttatagt ctaagactat atatataaat 2160
gtataaatat acagtataga tttttgggtg gggggcattg agtattgttt aaaatgtaat 2220
ttaaatgaaa gaaaattgag ttgcacttat tgaccatttt ttaatttact tgttttggat 2280
ggcttgtcta tactccttcc cttaaggggt atcatgtatg gtgataggta tctagagctt 2340
aatgctacat gtgagtgcga tgatgtacag attctttcag ttctttggat tctaaataca 2400
tgccacatca aacctttgag tagatccatt tccattgctt attatgtagg taagactgta 2460
gatatgtatt cttttctcag tgttggtata ttttatatta ctgacatttc ttctagtgat 2520
gatggttcac gttggggtga tttaatccag ttataagaag aagttcatgt ccaaacggtc 2580
ctctttagtt tttggttggg aatgaggaaa attcttaaaa ggcccatagc agccagttca 2640
aaaacacccg acgtcatgta tttgagcata tcagtaaccc ccttaaattt aatacccaga 2700
taccttatct tacaatgttg attgggaaaa catttgctgc ccattacaga ggtattaaaa 2760
ctaaatttca ctactagatt gactaactca aatacacatt tgctactgtt gtaagaattc 2820

<210> 332
<211> 2270
<212> DNA
<213> Homo sapiens

<400> 332
tcgttgatat caaagacagt tgaaggaaat gaattttgaa acttcacggt gtgccaccct 60
acagtactgc cctgaccctt acatccagcg tttcgtagaa acccagctca tttctcttgg 120

aaagaaagtt attaccgatc caccatgtcc cagagcacac agacaaatga attcctcagt 180
ccagaggttt tccagcatat ctgggatttt ctggaacagc ctatatgttc agttcagccc 240
attgacttga actttgtgga tgaaccatca gaagatggtg cgacaaacaa gattgagatt 300
agcatggact gtatccgcat gcaggactcg gacctgagtg accccatgtg gccacagtac 360
acgaacctgg ggctcctgaa cagcatggac cagcagattc agaacggctc ctcgtccacc 420
agtccctata acacagacca cgcgcagaac agcgtcacgg cgccctcgcc ctacgcacag 480
cccagctcca ccttcgatgc tctctctcca tcacccgcca tcccctccaa caccgactac 540
ccaggcccgc acagtttcga cgtgtccttc cagcagtcga gcaccgccaa gtcggccacc 600
tggacgtatt ccactgaact gaagaaactc tactgccaaa ttgcaaagac atgccccatc 660
cagatcaagg tgatgacccc acctcctcag ggagctgtta tccgcgccat gcctgtctac 720
aaaaaagctg agcacgtcac ggaggtggtg aagcggtgcc ccaaccatga gctgagccgt 780
gaattcaacg agggacagat tgcccctcct agtcatttga ttcgagtaga ggggaacagc 840
catgcccagt atgtagaaga tcccatcaca ggaagacaga gtgtgctggt accttatgag 900
ccaccccagg ttggcactga attcacgaca gtcttgtaca atttcatgtg taacagcagt 960
tgtgttggag ggatgaaccg ccgtccaatt ttaatcattg ttactctgga aaccagagat 1020
gggcaagtcc tgggccgacg ctgctttgag gcccggatct gtgcttgccc aggaagagac 1080
aggaaggcgg atgaagatag catcagaaag cagcaagttt cggacagtac aaagaacggt 1140
gatggtacga agcgcccgtt tcgtcagaac acacatggta tccagatgac atccatcaag 1200
aaacgaagat ccccagatga tgaactgtta tacttaccag tgagggggcg tgagacttat 1260
gaaatgctgt tgaagatcaa agagtccctg gaactcatgc agtaccttcc tcagcacaca 1320
attgaaacgt acaggcaaca gcaacagcag cagcaccagc acttacttca gaaacagacc 1380
tcaatacagt ctccatcttc atatggtaac agctccccac ctctgaacaa aatgaacagc 1440
atgaacaagc tgccttctgt gagccagctt atcaaccctc agcagcgcaa cgccctcact 1500
cctacaacca ttcctgatgg catgggagcc aacattccca tgatgggcac ccacatgcca 1560
atggctggag acatgaatgg actcagcccc acccaggcac tccctccccc actctccatg 1620
ccatccacct cccactgcac acccccacct ccgtatccaa cagattgcag cattgtcggt 1680
ttcttagcga ggttgggctg ttcatcatgt ctggactatt tcacgaccca ggggctgacc 1740
accatctatc agattgagca ttactccatg gatgatctgg caagtctgaa aatccctgag 1800
caatttcgac atgcgatctg gaagggcatc ctggaccacc ggcagctcca cgaattctcc 1860
tccccttctc atctcctgcg gaccccaagc agtgcctcta cagtcagtgt gggctccagt 1920
gagacccggg gtgagcgtgt tattgatgct gtgcgattca ccctccgcca gaccatctct 1980
ttcccacccc gagatgagtg gaatgacttc aactttgaca tggatgctcg ccgcaataag 2040
caacagcgca tcaaagagga gggggagtga gcctcaccat gtgagctctt cctatccctc 2100
tcctaactgc cagcccccta aaagcactcc tgcttaatct tcaaagcctt ctccctagct 2160
cctccccttc ctcttgtctg atttcttagg ggaaggagaa gtaagaggct acctcttacc 2220
taacatctga cctggcatct aattctgatt ctggctttaa gccttcaaaa 2270

<210> 333
<211> 2816
<212> DNA
<213> Homo sapiens

<400> 333
tcgttgatat caaagacagt tgaaggaaat gaattttgaa acttcacggt gtgccaccct 60
acagtactgc cctgaccctt acatccagcg tttcgtagaa acccagctca tttctcttgg 120
aaagaaagtt attaccgatc caccatgtcc cagagcacac agacaaatga attcctcagt 180
ccagaggttt tccagcatat ctgggatttt ctggaacagc ctatatgttc agttcagccc 240
attgacttga actttgtgga tgaaccatca gaagatggtg cgacaaacaa gattgagatt 300
agcatggact gtatccgcat gcaggactcg gacctgagtg accccatgtg gccacagtac 360
acgaacctgg ggctcctgaa cagcatggac cagcagattc agaacggctc ctcgtccacc 420
agtccctata acacagacca cgcgcagaac agcgtcacgg cgccctcgcc ctacgcacag 480
cccagctcca ccttcgatgc tctctctcca tcacccgcca tcccctccaa caccgactac 540
ccaggcccgc acagtttcga cgtgtccttc cagcagtcga gcaccgccaa gtcggccacc 600
tggacgtatt ccactgaact gaagaaactc tactgccaaa ttgcaaagac atgccccatc 660

cagatcaagg tgatgacccc acctcctcag ggagctgtta tccgcgccat gcctgtctac 720
aaaaaagctg agcacgtcac ggaggtggtg aagcggtgcc ccaaccatga gctgagccgt 780
gaattcaacg agggacagat tgcccctcct agtcatttga ttcgagtaga ggggaacagc 840
catgcccagt atgtagaaga tcccatcaca ggaagacaga gtgtgctggt accttatgag 900
ccaccccagg ttggcactga attcacgaca gtcttgtaca atttcatgtg taacagcagt 960
tgtgttggag ggatgaaccg ccgtccaatt ttaatcattg ttactctgga aaccagagat 1020
gggcaagtcc tgggccgacg ctgctttgag gcccggatct gtgcttgccc aggaagagac 1080
aggaaggcgg atgaagatag catcagaaag cagcaagttt cggacagtac aaagaacggt 1140
gatggtacga agcgcccgtt tcgtcagaac acacatggta tccagatgac atccatcaag 1200
aaacgaagat ccccagatga tgaactgtta tacttaccag tgaggggccg tgagacttat 1260
gaaatgctgt tgaagatcaa agagtccctg gaactcatgc agtaccttcc tcagcacaca 1320
attgaaacgt acaggcaaca gcaacagcag cagcaccagc acttacttca gaaacatctc 1380
ctttcagcct gcttcaggaa tgagcttgtg gagccccgga gagaaactcc aaaacaatct 1440
gacgtcttct ttagacattc caagccccca aaccgatcag tgtacccata gagccctatc 1500
tctatatttt aagtgtgtgt gttgtatttc catgtgtata tgtgagtgtg tgtgtgtgta 1560
tgtgtgtgcg tgtgtatcta gccctcataa acaggacttg aagacacttt ggctcagaga 1620
cccaactgct caaaggcaca aagccactag tgagagaatc ttttgaaggg actcaaacct 1680
ttacaagaaa ggatgttttc tgcagatttt gtatccttag accggccatt ggtgggtgag 1740
gaaccactgt gtttgtctgt gagctttctg ttgtttcctg ggagggaggg gtcaggtggg 1800
gaaaggggca ttaagatgtt tattggaacc cttttctgtc ttcttctgtt gtttttctaa 1860
aattcacagg gaagcttttg agcaggtctc aaacttaaga tgtcttttta agaaaaggag 1920
aaaaaagttg ttattgtctg tgcataagta agttgtaggt gactgagaga ctcagtcaga 1980
cccttttaat gctggtcatg taataatatt gcaagtagta agaaacgaag gtgtcaagtg 2040
tactgctggg cagcgaggtg atcattacca aaagtaatca actttgtggg tggagagttc 2100
tttgtgagaa cttgcattat ttgtgtcctc ccctcatgtg taggtagaac atttcttaat 2160
gctgtgtacc tgcctctgcc actgtatgtt ggcatctgtt atgctaaagt ttttcttgta 2220
catgaaaccc tggaagacct actacaaaaa aactgttgtt tggcccccat agcaggtgaa 2280
ctcattttgt gcttttaata gaaagacaaa tccaccccag taatattgcc cttacgtagt 2340
tgtttaccat tattcaaagc tcaaaataga atttgaagcc ctctcacaaa atctgtgatt 2400
aatttgctta attagagctt ctatccctca agcctaccta ccataaaacc agccatatta 2460
ctgatactgt tcagtgcatt tagccaggag acttacgttt tgagtaagtg agatccaagc 2520
agacgtgtta aaatcagcac tcctggactg gaaattaaag attgaaaggg tagactactt 2580
ttcttttttt tactcaaaag tttagagaat ctctgtttct ttccatttta aaaacatatt 2640
ttaagataat agcataaaga ctttaaaaat gttcctcccc tccatcttcc cacacccagt 2700
caccagcact gtattttctg tcaccaagac aatgatttct tgttattgag gctgttgctt 2760
ttgtggatgt gtgattttaa ttttcaataa acttttgcat cttggtttaa aagaaa 2816

<210> 334
<211> 2082
<212> DNA
<213> Homo sapiens

<400> 334
agatgctaca gcgactgcac acccaggctg tatgatacag cctattgctc ccgggctgca 60
aacctgtcca gcatgtgatg tggtgggata ctgaattgaa taccgaatac tgtaggcaat 120
tgtaacacag tggtaagtct ttgtgtatct aaacatagct aaacaccaaa aggtatagta 180
agaatatggt attataatct tatggaacta tcattgtata tgtggtttgt caaccagaat 240
gtagttatac agcacaggac tgtgcttatg atgtgccaag cacagctctc agtactaact 300
cctttaatct tcatatcaac cctaggaggt aacttcttaa gtagattcat attgtaaggg 360
tctcggggtg ggggggttgg caaaatcctg gagccagaag aaaggacagc agcattgatc 420
aatcttacag ctaacatgtt gtacctggaa aacaatgccc agactcaatt tagtgagcca 480
cagtacacga acctggggct cctgaacagc atggaccagc agattcagaa cggctcctcg 540
tccaccagtc cctataacac agaccacgcg cagaacagcg tcacggcgcc ctcgccctac 600
gcacagccca gctccacctt cgatgctctc tctccatcac ccgccatccc ctccaacacc 660

gactacccag gcccgcacag tttcgacgtg tccttccagc agtcgagcac cgccaagtcg 720
gccacctgga cgtattccac tgaactgaag aaactctact gccaaattgc aaagacatgc 780
cccatccaga tcaaggtgat gaccccacct cctcagggag ctgttatccg cgccatgcct 840
gtctacaaaa aagctgagca cgtcacggag gtggtgaagc ggtgccccaa ccatgagctg 900
agccgtgaat tcaacgaggg acagattgcc cctcctagtc atttgattcg agtagagggg 960
aacagccatg cccagtatgt agaagatccc atcacaggaa gacagagtgt gctggtacct 1020
tatgagccac cccaggttgg cactgaattc acgacagtct tgtacaattt catgtgtaac 1080
agcagttgtg ttggagggat gaaccgccgt ccaattttaa tcattgttac tctggaaacc 1140
agagatgggc aagtcctggg ccgacgctgc tttgaggccc ggatctgtgc ttgcccagga 1200
agagacagga aggcggatga agatagcatc agaaagcagc aagtttcgga cagtacaaag 1260
aacggtgatg gtacgaagcg cccgtctcgt cagaacacac atggtatcca gatgacatcc 1320
atcaagaaac gaagatcccc agatgatgaa ctgttatact taccagtgag gggccgtgag 1380
acttatgaaa tgctgttgaa gatcaaagag tccctggaac tcatgcagta ccttcctcag 1440
cacacaattg aaacgtacag gcaacagcaa cagcagcagc accagcactt acttcagaaa 1500
cagtgagtgt atcaacgtgt cattttagga ggcatgagtg acggtgactt tatttggatc 1560
agcaataggg tgattgatga gcaatgtgga acataatggg agatagcaga ttgtcataga 1620
ttcagatgac ctggtatggc aaccctcttt cagttgcaac cttttttacg tgtcttatta 1680
taaccttccc ttcagaattc cacttatgtt ctgaaattaa atacaaacca tttctggtga 1740
attacaaaga aactcacact aacagttctc ttctctatat gcctggtcca tacacactaa 1800
cagtaagtac acactctatt tggtagtgat gtgtatattt gaaaacatga aatcttttct 1860
catcccaatg gattgtctta taaatctcct gggatgcaca ctatccactt ttgggaataa 1920
cactgtagac cagggatagc aaataggctt tactataata taaagtgact tgtttgaatg 1980
ctgtaatgag aagaattctg agacctagtg catgataatt ggggaaatat ctgggtgcag 2040
aaggataagg tagcatcatg ttgccgtatt ttagcatctc tg 2082

<210> 335
<211> 4849
<212> DNA
<213> Homo sapiens

<400> 335
cgttgatatc aaagacagtt gaaggaaatg aattttgaaa cttcacggtg tgccacccta 60
cagtactgcc ctgaccctta catccagcgt ttcgtagaaa ccccagctca tttctcttgg 120
aaagaaagtt attaccgatc caccatgtcc cagagcacac agacaaatga attcctcagt 180
ccagaggttt tccagcatat ctgggatttt ctggaacagc ctatatgttc agttcagccc 240
attgacttga actttgtgga tgaaccatca gaagatggtg cgacaaacaa gattgagatt 300
agcatggact gtatccgcat gcaggactcg gacctgagtg accccatgtg gccacagtac 360
acgaacctgg ggctcctgaa cagcatggac cagcagattc agaacggctc ctcgtccacc 420
agtccctata acacagacca cgcgcagaac agcgtcacgg cgccctcgcc ctacgcacag 480
cccagctcca ccttcgatgc tctctctcca tcacccgcca tcccctccaa caccgactac 540
ccaggcccgc acagtttcga cgtgtccttc cagcagtcga gcaccgccaa gtcggccacc 600
tggacgtatt ccactgaact gaagaaactc tactgccaaa ttgcaaagac atgccccatc 660
cagatcaagg tgatgacccc acctcctcag ggagctgtta tccgcgccat gcctgtctac 720
aaaaaagctg agcacgtcac ggaggtggtg aagcggtgcc ccaaccatga gctgagccgt 780
gaattcaacg agggacagat tgcccctcct agtcatttga ttcgagtaga ggggaacagc 840
catgcccagt atgtagaaga tcccatcaca ggaagacaga gtgtgctggt accttatgag 900
ccaccccagg ttggcactga attcacgaca gtcttgtaca atttcatgtg taacagcagt 960
tgtgttggag ggatgaaccg ccgtccaatt ttaatcattg ttactctgga aaccagagat 1020
gggcaagtcc tgggccgacg ctgctttgag gcccggatct gtgcttgccc aggaagagac 1080
aggaaggcgg atgaagatag catcagaaag cagcaagttt cggacagtac aaagaacggt 1140
gatggtacga agcgcccgtt tcgtcagaac acacatggta tccagatgac atccatcaag 1200
aaacgaagat ccccagatga tgaactgtta tacttaccag tgaggggccg tgagacttat 1260
gaaatgctgt tgaagatcaa agagtccctg gaactcatgc agtaccttcc tcagcacaca 1320
attgaaacgt acaggcaaca gcaacagcag cagcaccagc acttacttca gaaacagacc 1380

tcaatacagt ctccatcttc atatggtaac agctccccac ctctgaacaa aatgaacagc 1440
atgaacaagc tgccttctgt gagccagctt atcaaccctc agcagcgcaa cgccctcact 1500
cctacaacca ttcctgatgg catgggagcc aacattccca tgatgggcac ccacatgcca 1560
atggctggag acatgaatgg actcagcccc acccaggcac tccctccccc actctccatg 1620
ccatccacct cccagtgcac acccccacct ccgtatccca cagattgcag cattgtcagt 1680
ttcttagcga ggttgggctg ttcatcatgt ctggactatt tcacgaccca ggggctgacc 1740
accatctatc agattgagca ttactccatg gatgatctgg caagtctgaa aatccctgag 1800
caatttcgac atgcgatctg gaagggcatc ctggaccacc ggcagctcca cgaattctcc 1860
tccccttctc atctcctgcg gaccccaagc agtgcctcta cagtcagtgt gggctccagt 1920
gagacccggg gtgagcgtgt tattgatgct gtgcgattca ccctccgcca gaccatctct 1980
ttcccacccc gagatgagtg gaatgacttc aactttgaca tggatgctcg ccgcaataag 2040
caacagcgca tcaaagagga gggggagtga gcctcaccat gtgagctctt cctatccctc 2100
tcctaactgc cagcycccta aaagcactcc tgcttaatct tcaaagcctt ctccctagct 2160
cctccccttc ctcttgtctg atttcttagg ggaaggagaa gtaagaggct acctcttacc 2220
taacatctga cctggcatct aattctgatt ctggctttaa gccttcaaaa ctatagcttg 2280
cagaactgta gctgccatgg ctaggtagaa gtgagcaaaa aagagttggg tgtctcctta 2340
agctgcagag atttctcatt gacttttata aagcatgttc acccttatag tctaagacta 2400
tatatataaa tgtataaata tacagtatag atttttgggt ggggggcatt gagtattgtt 2460
taaaatgtaa tttaaatgaa agaaaattga gttgcactta ttgaccattt tttaatttac 2520
ttgttttgga tggcttgtct atactccttc ccttaagggg tatcatgtat ggtgataggt 2580
atctagagct taatgctaca tgtgagtgac gatgatgtac agattctttc agttctttgg 2640
attctaaata catgccacat caaacctttg agtagatcca tttccattgc ttattatgta 2700
ggtaagactg tagatatgta ttcttttctc agtgttggta tattttatat tactgacatt 2760
tcttctagtg atgatggttc acgttggggt gatttaatcc agttataaga agaagttcat 2820
gtccaaacgt cctctttagt ttttggttgg gaatgaggaa aattcttaaa aggcccatag 2880
cagccagttc aaaaacaccc gacgtcatgt atttgagcat atcagtaacc cccttaaatt 2940
taataccaga taccttatct tacaatattg attgggaaaa catttgctgc cattacagag 3000
gtattaaaac taaatttcac tactagattg actaactcaa atacacattt gctactgttg 3060
taagaattct gattgatttg attgggatga atgccatcta tctagttcta acagtgaagt 3120
tttactgtct attaatattc agggtaaata ggaatcattc agaaatgttg agtctgtact 3180
aaacagtaag atatctcaat gaaccataaa ttcaactttg taaaaatctt ttgaagcata 3240
gataatattg tttggtaaat gtttcttttg tttggtaaat gtttctttta aagaccctcc 3300
tattctataa aactctgcat gtagaggctt gtttaccttt ctctctctaa ggtttacaat 3360
aggagtggtg atttgaaaaa tataaaatta tgagattggt tttcctgtgg cataaattgc 3420
atcactgtat cattttcttt tttaaccggt aagagtttca gtttgttgga aagtaactgt 3480
gagaacccag tttcccgtcc atctccctta gggactaccc atagacatga aaggtcccca 3540
cagagcaaga gataagtctt tcatggctgc tgttgcttaa accacttaaa cgaagagttc 3600
ccttgaaact ttgggaaaac atgttaatga caatattcca gatctttcag aaatataaca 3660
catttttttg catgcatgca aatgagctct gaaatcttcc catgcattct ggtcaagggc 3720
tgtcattgca cataagcttc cattttaatt ttaaagtgca aaagggccag cgtggctcta 3780
aaaggtaatg tgtggattgc ctctgaaaag tgtgtatata ttttgtgtga aattgcatac 3840
tttgtatttt gattattttt tttttcttct tgggatagtg ggatttccag aaccacactt 3900
gaaacctttt tttatcgttt ttgtattttc atgaaaatac catttagtaa gaataccaca 3960
tcaaataaga aataatgcta caattttaag aggggaggga agggaaagtt tttttttatt 4020
atttttttaa aattttgtat gttaaagaga atgagtcctt gatttcaaag ttttgttgta 4080
cttaaatggt aataagcact gtaaacttct gcaacaagca tgcagctttg caaacccatt 4140
aaggggaaga atgaaagctg ttccttggtc ctagtaagaa gacaaactgc ttcccttact 4200
ttgctgaggg tttgaataaa cctaggactt ccgagctatg tcagtactat tcaggtaaca 4260
ctagggcctt ggaaattcct gtactgtgtc tcatggattt ggcactagcc aaagcgaggc 4320
acccttactg gcttacctcc tcatggcagc ctactctcct tgagtgtatg agtagccagg 4380
gtaaggggta aaaggatagt aagcatagaa accactagaa agtgggctta atggagttct 4440
tgtggcctca gctcaatgca gttagctgaa gaattgaaaa gtttttgttt ggagacgttt 4500
ataaacagaa atggaaagca gagttttcat taaatccttt tacctttttt ttttcttggt 4560
aatcccctaa aataacagta tgtgggatat tgaatgttaa agggatattt tttttctatt 4620
atttttataa ttgtacaaaa ttaagcaaat gttaaaagtt ttatatgctt tattaatgtt 4680

ttcaaaaggt attatacatg tgatacattt tttaagcttc agttgcttgt cttctggtac 4740
tttctgttat gggcttttgg ggagccagaa gccaatctac aatctctttt tgtttgccag 4800
gacatgcaat aaaatttaaa aaataaataa aaactaatta agaaataaa 4849

<210> 336
<211> 1386
<212> DNA
<213> Homo sapiens

<400> 336
atgttgtacc tggaaaacaa tgcccagact caatttagtg agccacagta cacgaacctg 60
gggctcctga acagcatgga ccagcagatt cagaacggct cctcgtccac cagtccctat 120
aacacagacc acgcgcagaa cagcgtcacg gcgccctcgc cctacgcaca gcccagctcc 180
accttcgatg ctctctctcc atcacccgcc atcccctcca acaccgacta cccaggcccg 240
cacagtttcg acgtgtcctt ccagcagtcg agcaccgcca agtcggccac ctggacgtat 300
tccactgaac tgaagaaact ctactgccaa attgcaaaga catgccccat ccagatcaag 360
gtgatgaccc cacctcctca gggagctgtt atccgcgcca tgcctgtcta caaaaaagct 420
gagcacgtca cggaggtggt gaagcggtgc cccaaccatg agctgagccg tgaattcaac 480
gagggacaga ttgcccctcc tagtcatttg attcgagtag aggggaacag ccatgcccag 540
tatgtagaag atcccatcac aggaagacag agtgtgctgg taccttatga gccaccccag 600
gttggcactg aattcacgac agtcttgtac aatttcatgt gtaacagcag ttgtgttgga 660
gggatgaacc gccgtccaat tttaatcatt gttactctgg aaaccagaga tgggcaagtc 720
ctgggccgac gctgctttga ggcccggatc tgtgcttgcc caggaagaga caggaaggcg 780
gatgaagata gcatcagaaa gcagcaagtt tcggacagta caaagaacgg tgatggtacg 840
aagcgcccgt ttcgtcagaa cacacatggt atccagatga catccatcaa gaaacgaaga 900
tccccagatg atgaactgtt atacttacca gtgaggggcc gtgagactta tgaaatgctg 960
ttgaagatca aagagtccct ggaactcatg cagtaccttc ctcagcacac aattgaaacg 1020
tacaggcaac agcaacagca gcagcaccag cacttacttc agaaacagac ctcaatacag 1080
tctccatctt catatggtaa cagctcccca cctctgaaca aaatgaacag catgaacaag 1140
ctgccttctg tgagccagct tatcaaccct cagcagcgca acgccctcac tcctacaacc 1200
attcctgatg gcatgggagc caacattccc atgatgggca cccacatgcc aatggctgga 1260
gacatgaatg gactcagccc cacccaggca ctccctcccc cactctccat gccatccacc 1320
tcccactgca cacccccacc tccgtatccc acagattgca gcattgtcag gatctggcaa 1380
gtctga 1386

<210> 337
<211> 1551
<212> DNA
<213> Homo sapiens

<400> 337
atgtcccaga gcacacagac aaatgaattc ctcagtccag aggttttcca gcatatctgg 60
gatttcctgg aacagcctat atgttcagtt cagcccattg acttgaactt tgtggatgaa 120
ccatcagaag atggtgcgac aaacaagatt gagattagca tggactgtat ccgcatgcag 180
gactcggacc tgagtgaccc catgtggcca cagtacacga acctggggct cctgaacagc 240
atggaccagc agattcagaa cggctcctcg tccaccagtc cctataacac agaccacgcg 300
cagaacagcg tcacggcgcc ctcgccctac gcacagccca gctccacctt cgatgctctc 360
tctccatcac ccgccatccc ctccaacacc gactacccag gcccgcacag tttcgacgtg 420
tccttccagc agtcgagcac cgccaagtcg gccacctgga cgtattccac tgaactgaag 480
aaactctact gccaaattgc aaagacatgc cccatccaga tcaaggtgat gaccccacct 540
cctcagggag ctgttatccg cgccatgcct gtctacaaaa aagctgagca cgtcacggag 600
gtggtgaagc ggtgccccaa ccatgagctg agccgtgaat tcaacgaggg acagattgcc 660
cctcctagtc atttgattcg agtagagggg aacagccatg cccagtatgt agaagatccc 720

atcacaggaa gacagagtgt gctggtacct tatgagccac cccaggttgg cactgaattc 780
acgacagtct tgtacaattt catgtgtaac agcagttgtg ttggagggat gaaccgccgt 840
ccaattttaa tcattgttac tctggaaacc agagatgggc aagtcctggg ccgacgctgc 900
tttgaggccc ggatctgtgc ttgcccagga agagacagga aggcggatga agatagcatc 960
agaaagcagc aagtttcgga cagtacaaag aacggtgatg gtacgaagcg cccgtttcgt 1020
cagaacacac atggtatcca gatgacatcc atcaagaaac gaagatcccc agatgatgaa 1080
ctgttatact taccagtgag gggccgtgag acttatgaaa tgctgttgaa gatcaaagag 1140
tccctggaac tcatgcagta ccttcctcag cacacaattg aaacgtacag gcaacagcaa 1200
cagcagcagc accagcactt acttcagaaa cagacctcaa tacagtctcc atcttcatat 1260
ggtaacagct ccccacctct gaacaaaatg aacagcatga acaagctgcc ttctgtgagc 1320
cagcttatca accctcagca gcgcaacgcc ctcactccta caaccattcc tgatggcatg 1380
ggagccaaca ttcccatgat gggcacccac atgccaatgg ctggagacat gaatggactc 1440
agccccaccc aggcactccc tcccccactc tccatgccat ccacctccca ctgcacaccc 1500
ccacctccgt atcccacaga ttgcagcatt gtcaggatct ggcaagtctg a 1551

<210> 338
<211> 586
<212> PRT
<213> Homo sapiens

<400> 338

<210> 339
<211> 641
<212> PRT
<213> Homo sapiens

<400> 339


<210> 340
<211> 448
<212> PRT
<213> Homo sapiens

<400> 340
Met Ser Gln Ser Thr Gln Thr Asn Glu Phe Leu Ser Pro Glu Val Phe
                5                   10                  15

Gln His Ile Trp Asp Phe Leu Glu Gln Pro Ile Cys Ser Val Gln Pro
            20                  25                  30

Ile Asp Leu Asn Phe Val Asp Glu Pro Ser Glu Asp Gly Ala Thr Asn
        35                  40                  45

Lys Ile Glu Ile Ser Met Asp Cys Ile Arg Met Gln Asp Ser Asp Leu
    50                  55                  60

Ser Asp Pro Met Trp Pro Gln Tyr Thr Asn Leu Gly Leu Leu Asn Ser
65                  70                  75                  80

Met Asp Gln Gln Ile Gln Asn Gly Ser Ser Ser Thr Ser Pro Tyr Asn
                85                  90                  95

Thr Asp His Ala Gln Asn Ser Val Thr Ala Pro Ser Pro Tyr Ala Gln
            100                 105                 110

Pro Ser Ser Thr Phe Asp Ala Leu Ser Pro Ser Pro Ala Ile Pro Ser
        115                 120                 125

Asn Thr Asp Tyr Pro Gly Pro His Ser Phe Asp Val Ser Phe Gln Gln
    130                 135                 140

Ser Ser Thr Ala Lys Ser Ala Thr Trp Thr Tyr Ser Thr Glu Leu Lys
145                 150                 155                 160

Lys Leu Tyr Cys Gln Ile Ala Lys Thr Cys Pro Ile Gln Ile Lys Val
                165                 170                 175

Met Thr Pro Pro Pro Gln Gly Ala Val Ile Arg Ala Met Pro Val Tyr
            180                 185                 190

Lys Lys Ala Glu His Val Thr Glu Val Val Lys Arg Cys Pro Asn His
        195                 200                 205

Glu Leu Ser Arg Glu Phe Asn Glu Gly Gln Ile Ala Pro Pro Ser His
    210                 215                 220

Leu Ile Arg Val Glu Gly Asn Ser His Ala Gln Tyr Val Glu Asp Pro
225                 230                 235                 240

Ile Thr Gly Arg Gln Ser Val Leu Val Pro Tyr Glu Pro Pro Gln Val
                245                 250                 255

Gly Thr Glu Phe Thr Thr Val Leu Tyr Asn Phe Met Cys Asn Ser Ser
            260                 265                 270

Cys Val Gly Gly Met Asn Arg Arg Pro Ile Leu Ile Ile Val Thr Leu
        275                 280                 285

Glu Thr Arg Asp Gly Gln Val Leu Gly Arg Arg Cys Phe Glu Ala Arg
    290                 295                 300

Ile Cys Ala Cys Pro Gly Arg Asp Arg Lys Ala Asp Glu Asp Ser Ile
305                 310                 315                 320

Arg Lys Gln Gln Val Ser Asp Ser Thr Lys Asn Gly Asp Gly Thr Lys
                325                 330                 335

Arg Pro Phe Arg Gln Asn Thr His Gly Ile Gln Met Thr Ser Ile Lys
            340                 345                 350

Lys Arg Arg Ser Pro Asp Asp Glu Leu Leu Tyr Leu Pro Val Arg Gly
        355                 360                 365

Arg Glu Thr Tyr Glu Met Leu Leu Lys Ile Lys Glu Ser Leu Glu Leu
    370                 375                 380

Met Gln Tyr Leu Pro Gln His Thr Ile Glu Thr Tyr Arg Gln Gln Gln
385                 390                 395                 400

Gln Gln Gln His Gln His Leu Leu Gln Lys His Leu Leu Ser Ala Cys
                405                 410                 415

Phe Arg Asn Glu Leu Val Glu Pro Arg Arg Glu Thr Pro Lys Gln Ser
            420                 425                 430

Asp Val Phe Phe Arg His Ser Lys Pro Pro Asn Arg Ser Val Tyr Pro
        435                 440                 445

<210> 341
<211> 356
<212> PRT
<213> Homo sapiens

<400> 341

<210> 342
<211> 680
<212> PRT
<213> Homo sapiens

<400> 342
Met Asn Phe Glu Thr Ser Arg Cys Ala Thr Leu Gln Tyr Cys Pro Asp
                5                   10                  15

Pro Tyr Ile Gln Arg Phe Val Glu Thr Pro Ala His Phe Ser Trp Lys
            20                  25                  30

Glu Ser Tyr Tyr Arg Ser Thr Met Ser Gln Ser Thr Gln Thr Asn Glu
        35                  40                  45

Phe Leu Ser Pro Glu Val Phe Gln His Ile Trp Asp Phe Leu Glu Gln
    50                  55                  60

Pro Ile Cys Ser Val Gln Pro Ile Asp Leu Asn Phe Val Asp Glu Pro
65                  70                  75                  80

Ser Glu Asp Gly Ala Thr Asn Lys Ile Glu Ile Ser Met Asp Cys Ile
                85                  90                  95

Arg Met Gln Asp Ser Asp Leu Ser Asp Pro Met Trp Pro Gln Tyr Thr
            100                 105                 110

Asn Leu Gly Leu Leu Asn Ser Met Asp Gln Gln Ile Gln Asn Gly Ser
        115                 120                 125

Ser Ser Thr Ser Pro Tyr Asn Thr Asp His Ala Gln Asn Ser Val Thr
    130                 135                 140

Ala Pro Ser Pro Tyr Ala Gln Pro Ser Ser Thr Phe Asp Ala Leu Ser
145                 150                 155                 160

Pro Ser Pro Ala Ile Pro Ser Asn Thr Asp Tyr Pro Gly Pro His Ser
                165                 170                 175

Phe Asp Val Ser Phe Gln Gln Ser Ser Thr Ala Lys Ser Ala Thr Trp
            180                 185                 190

Thr Tyr Ser Thr Glu Leu Lys Lys Leu Tyr Cys Gln Ile Ala Lys Thr
        195                 200                 205

Cys Pro Ile Gln Ile Lys Val Met Thr Pro Pro Pro Gln Gly Ala Val
    210                 215                 220

Ile Arg Ala Met Pro Val Tyr Lys Lys Ala Glu His Val Thr Glu Val
225                 230                 235                 240

Val Lys Arg Cys Pro Asn His Glu Leu Ser Arg Glu Phe Asn Glu Gly
                245                 250                 255

Gln Ile Ala Pro Pro Ser His Leu Ile Arg Val Glu Gly Asn Ser His
            260                 265                 270

Ala Gln Tyr Val Glu Asp Pro Ile Thr Gly Arg Gln Ser Val Leu Val
        275                 280                 285

Pro Tyr Glu Pro Pro Gln Val Gly Thr Glu Phe Thr Thr Val Leu Tyr
    290                 295                 300

Asn Phe Met Cys Asn Ser Ser Cys Val Gly Gly Met Asn Arg Arg Pro
305                 310                 315                 320

Ile Leu Ile Ile Val Thr Leu Glu Thr Arg Asp Gly Gln Val Leu Gly
                325                 330                 335

Arg Arg Cys Phe Glu Ala Arg Ile Cys Ala Cys Pro Gly Arg Asp Arg
            340                 345                 350

Lys Ala Asp Glu Asp Ser Ile Arg Lys Gln Gln Val Ser Asp Ser Thr
        355                 360                 365

Lys Asn Gly Asp Gly Thr Lys Arg Pro Phe Arg Gln Asn Thr His Gly
    370                 375                 380

Ile Gln Met Thr Ser Ile Lys Lys Arg Arg Ser Pro Asp Asp Glu Leu
385                 390                 395                 400

Leu Tyr Leu Pro Val Arg Gly Arg Glu Thr Tyr Glu Met Leu Leu Lys
                405                 410                 415

Ile Lys Glu Ser Leu Glu Leu Met Gln Tyr Leu Pro Gln His Thr Ile
            420                 425                 430

Glu Thr Tyr Arg Gln Gln Gln Gln Gln Gln His Gln His Leu Leu Gln
        435                 440                 445

Lys Gln Thr Ser Ile Gln Ser Pro Ser Ser Tyr Gly Asn Ser Ser Pro
    450                 455                 460

Pro Leu Asn Lys Met Asn Ser Met Asn Lys Leu Pro Ser Val Ser Gln
465                 470                 475                 480

Leu Ile Asn Pro Gln Gln Arg Asn Ala Leu Thr Pro Thr Thr Ile Pro
                485                 490                 495

Asp Gly Met Gly Ala Asn Ile Pro Met Met Gly Thr His Met Pro Met
            500                 505                 510

Ala Gly Asp Met Asn Gly Leu Ser Pro Thr Gln Ala Leu Pro Pro Pro
        515                 520                 525

Leu Ser Met Pro Ser Thr Ser Gln Cys Thr Pro Pro Pro Pro Tyr Pro
    530                 535                 540

Thr Asp Cys Ser Ile Val Ser Phe Leu Ala Arg Leu Gly Cys Ser Ser
545                 550                 555                 560

Cys Leu Asp Tyr Phe Thr Thr Gln Gly Leu Thr Thr Ile Tyr Gln Ile
                565                 570                 575

Glu His Tyr Ser Met Asp Asp Leu Ala Ser Leu Lys Ile Pro Glu Gln
            580                 585                 590

Phe Arg His Ala Ile Trp Lys Gly Ile Leu Asp His Arg Gln Leu His
        595                 600                 605

Glu Phe Ser Ser Pro Ser His Leu Leu Arg Thr Pro Ser Ser Ala Ser
    610                 615                 620

Thr Val Ser Val Gly Ser Ser Glu Thr Arg Gly Glu Arg Val Ile Asp

625                 630                 635                 640

Ala Val Arg Phe Thr Leu Arg Gln Thr Ile Ser Phe Pro Pro Arg Asp
                645                 650                 655

Glu Trp Asn Asp Phe Asn Phe Asp Met Asp Ala Arg Arg Asn Lys Gln
            660                 665                 670

Gln Arg Ile Lys Glu Glu Gly Glu
        675                 680

<210> 343
<211> 461
<212> PRT
<213> Homo sapiens

<400> 343
Met Leu Tyr Leu Glu Asn Asn Ala Gln Thr Gln Phe Ser Glu Pro Gln
                5                   10                  15

Tyr Thr Asn Leu Gly Leu Leu Asn Ser Met Asp Gln Gln Ile Gln Asn
            20                  25                  30

Gly Ser Ser Ser Thr Ser Pro Tyr Asn Thr Asp His Ala Gln Asn Ser
        35                  40                  45

Val Thr Ala Pro Ser Pro Tyr Ala Gln Pro Ser Ser Thr Phe Asp Ala
    50                  55                  60

Leu Ser Pro Ser Pro Ala Ile Pro Ser Asn Thr Asp Tyr Pro Gly Pro
65                  70                  75                  80

His Ser Phe Asp Val Ser Phe Gln Gln Ser Ser Thr Ala Lys Ser Ala
                85                  90                  95

Thr Trp Thr Tyr Ser Thr Glu Leu Lys Lys Leu Tyr Cys Gln Ile Ala
            100                 105                 110

Lys Thr Cys Pro Ile Gln Ile Lys Val Met Thr Pro Pro Pro Gln Gly
        115                 120                 125

Ala Val Ile Arg Ala Met Pro Val Tyr Lys Lys Ala Glu His Val Thr
    130                 135                 140

Glu Val Val Lys Arg Cys Pro Asn His Glu Leu Ser Arg Glu Phe Asn
145                 150                 155                 160

Glu Gly Gln Ile Ala Pro Pro Ser His Leu Ile Arg Val Glu Gly Asn
                165                 170                 175

Ser His Ala Gln Tyr Val Glu Asp Pro Ile Thr Gly Arg Gln Ser Val
            180                 185                 190

Leu Val Pro Tyr Glu Pro Pro Gln Val Gly Thr Glu Phe Thr Thr Val
        195                 200                 205

Leu Tyr Asn Phe Met Cys Asn Ser Ser Cys Val Gly Gly Met Asn Arg
    210                 215                 220

Arg Pro Ile Leu Ile Ile Val Thr Leu Glu Thr Arg Asp Gly Gln Val
225                 230                 235                 240

Leu Gly Arg Arg Cys Phe Glu Ala Arg Ile Cys Ala Cys Pro Gly Arg
                245                 250                 255

Asp Arg Lys Ala Asp Glu Asp Ser Ile Arg Lys Gln Gln Val Ser Asp
            260                 265                 270

Ser Thr Lys Asn Gly Asp Gly Thr Lys Arg Pro Phe Arg Gln Asn Thr
        275                 280                 285

His Gly Ile Gln Met Thr Ser Ile Lys Lys Arg Arg Ser Pro Asp Asp
    290                 295                 300

Glu Leu Leu Tyr Leu Pro Val Arg Gly Arg Glu Thr Tyr Glu Met Leu
305                 310                 315                 320

Leu Lys Ile Lys Glu Ser Leu Glu Leu Met Gln Tyr Leu Pro Gln His
                325                 330                 335

Thr Ile Glu Thr Tyr Arg Gln Gln Gln Gln Gln Gln His Gln His Leu
            340                 345                 350

Leu Gln Lys Gln Thr Ser Ile Gln Ser Pro Ser Ser Tyr Gly Asn Ser
        355                 360                 365

Ser Pro Pro Leu Asn Lys Met Asn Ser Met Asn Lys Leu Pro Ser Val
    370                 375                 380

Ser Gln Leu Ile Asn Pro Gln Gln Arg Asn Ala Leu Thr Pro Thr Thr
385                 390                 395                 400

Ile Pro Asp Gly Met Gly Ala Asn Ile Pro Met Met Gly Thr His Met
                405                 410                 415

Pro Met Ala Gly Asp Met Asn Gly Leu Ser Pro Thr Gln Ala Leu Pro
            420                 425                 430

Pro Pro Leu Ser Met Pro Ser Thr Ser His Cys Thr Pro Pro Pro Pro
        435                 440                 445

Tyr Pro Thr Asp Cys Ser Ile Val Arg Ile Trp Gln Val
    450                 455                 460

<210> 344
<211> 516
<212> PRT
<213> Homo sapiens

<400> 344

Met Ser Gln Ser Thr Gln Thr Asn Glu Phe Leu Ser Pro Glu Val Phe
                5                   10                  15

Gln His Ile Trp Asp Phe Leu Glu Gln Pro Ile Cys Ser Val Gln Pro
            20                  25                  30

Ile Asp Leu Asn Phe Val Asp Glu Pro Ser Glu Asp Gly Ala Thr Asn
        35                  40                  45

Lys Ile Glu Ile Ser Met Asp Cys Ile Arg Met Gln Asp Ser Asp Leu
    50                  55                  60

Ser Asp Pro Met Trp Pro Gln Tyr Thr Asn Leu Gly Leu Leu Asn Ser
65                  70                  75                  80

Met Asp Gln Gln Ile Gln Asn Gly Ser Ser Ser Thr Ser Pro Tyr Asn
                85                  90                  95

Thr Asp His Ala Gln Asn Ser Val Thr Ala Pro Ser Pro Tyr Ala Gln
            100                 105                 110

Pro Ser Ser Thr Phe Asp Ala Leu Ser Pro Ser Pro Ala Ile Pro Ser
        115                 120                 125

Asn Thr Asp Tyr Pro Gly Pro His Ser Phe Asp Val Ser Phe Gln Gln
    130                 135                 140

Ser Ser Thr Ala Lys Ser Ala Thr Trp Thr Tyr Ser Thr Glu Leu Lys
145                 150                 155                 160

Lys Leu Tyr Cys Gln Ile Ala Lys Thr Cys Pro Ile Gln Ile Lys Val
                165                 170                 175

Met Thr Pro Pro Pro Gln Gly Ala Val Ile Arg Ala Met Pro Val Tyr
            180                 185                 190

Lys Lys Ala Glu His Val Thr Glu Val Val Lys Arg Cys Pro Asn His
        195                 200                 205

Glu Leu Ser Arg Glu Phe Asn Glu Gly Gln Ile Ala Pro Pro Ser His
    210                 215                 220

Leu Ile Arg Val Glu Gly Asn Ser His Ala Gln Tyr Val Glu Asp Pro
225                 230                 235                 240

Ile Thr Gly Arg Gln Ser Val Leu Val Pro Tyr Glu Pro Pro Gln Val
                245                 250                 255

Gly Thr Glu Phe Thr Thr Val Leu Tyr Asn Phe Met Cys Asn Ser Ser
            260                 265                 270

Cys Val Gly Gly Met Asn Arg Arg Pro Ile Leu Ile Ile Val Thr Leu
        275                 280                 285

Glu Thr Arg Asp Gly Gln Val Leu Gly Arg Arg Cys Phe Glu Ala Arg

290                 295                 300

Ile Cys Ala Cys Pro Gly Arg Asp Arg Lys Ala Asp Glu Asp Ser Ile
305                 310                 315                 320

Arg Lys Gln Gln Val Ser Asp Ser Thr Lys Asn Gly Asp Gly Thr Lys
                325                 330                 335

Arg Pro Phe Arg Gln Asn Thr His Gly Ile Gln Met Thr Ser Ile Lys
            340                 345                 350

Lys Arg Arg Ser Pro Asp Asp Glu Leu Leu Tyr Leu Pro Val Arg Gly
        355                 360                 365

Arg Glu Thr Tyr Glu Met Leu Leu Lys Ile Lys Glu Ser Leu Glu Leu
    370                 375                 380

Met Gln Tyr Leu Pro Gln His Thr Ile Glu Thr Tyr Arg Gln Gln Gln
385                 390                 395                 400

Gln Gln Gln His Gln His Leu Leu Gln Lys Gln Thr Ser Ile Gln Ser
                405                 410                 415

Pro Ser Ser Tyr Gly Asn Ser Ser Pro Pro Leu Asn Lys Met Asn Ser
            420                 425                 430

Met Asn Lys Leu Pro Ser Val Ser Gln Leu Ile Asn Pro Gln Gln Arg
        435                 440                 445

Asn Ala Leu Thr Pro Thr Thr Ile Pro Asp Gly Met Gly Ala Asn Ile
    450                 455                 460

Pro Met Met Gly Thr His Met Pro Met Ala Gly Asp Met Asn Gly Leu
465                 470                 475                 480

Ser Pro Thr Gln Ala Leu Pro Pro Pro Leu Ser Met Pro Ser Thr Ser
                485                 490                 495

His Cys Thr Pro Pro Pro Pro Tyr Pro Thr Asp Cys Ser Ile Val Arg
            500                 505                 510

Ile Trp Gln Val
        515

<210> 345
<211> 1800
<212> DNA
<213> Homo sapiens

<400> 345
gcgcctcatt gccactgcag tgactaaagc tgggaagacg ctggtcagtt cacctgcccc 60
actggttgtt ttttaaacaa attctgatac aggcgacatc ctcactgacc gagcaaagat 120
tgacattcgt atcatcactg tgcaccattg gcttctaggc actccagtgg ggtaggagaa 180

ggaggtctga aaccctcgca gagggatctt gccctcattc tttgggtctg aaacactggc 240
agtcgttgga aacaggactc agggataaac cagcgcaatg gattggggga cgctgcacac 300
tttcatcggg ggtgtcaaca aacactccac cagcatcggg aaggtgtgga tcacagtcat 360
ctttattttc cgagtcatga tcctagtggt ggctgcccag gaagtgtggg gtgacgagca 420
agaggacttc gtctgcaaca cactgcaacc gggatgcaaa aatgtgtgct atgaccactt 480
tttcccggtg tcccacatcc ggctgtgggc cctccagctg atcttcgtct ccaccccagc 540
gctgctggtg gccatgcatg tggcctacta caggcacgaa accactcgca agttcaggcg 600
aggagagaag aggaatgatt tcaaagacat agaggacatt aaaaagcaca aggttcggat 660
agaggggtcg ctgtggtgga cgtacaccag cagcatcttt ttccgaatca tctttgaagc 720
agcctttatg tatgtgtttt acttccttta caatgggtac cacctgccct gggtgttgaa 780
atgtgggatt gacccctgcc ccaaccttgt tgactgcttt atttctaggc caacagagaa 840
gaccgtgttt accattttta tgatttctgc gtctgtgatt tgcatgctgc ttaacgtggc 900
agagttgtgc tacctgctgc tgaaagtgtg ttttaggaga tcaaagagag cacagacgca 960
aaaaaatcac cccaatcatg ccctaaagga gagtaagcag aatgaaatga atgagctgat 1020
ttcagatagt ggtcaaaatg caatcacagg tttcccaagc taaacatttc aaggtaaaat 1080
gtagctgcgt cataaggaga cttctgtctt ctccagaagg caataccaac ctgaaagttc 1140
cttctgtagc ctgaagagtt tgtaaatgac tttcataata aatagacact tgagttaact 1200
ttttgtagga tacttgctcc attcatacac aacgtaatca aatatgtggt ccatctctga 1260
aaacaagaga ctgcttgaca aaggagcatt gcagtcactt tgacaggttc cttttaagtg 1320
gactctctga caaagtgggt actttctgaa aatttatata actgttgttg ataaggaaca 1380
tttatccagg aattgatacg tttattagga aaagatattt ttataggctt ggatgttttt 1440
agttccgact ttgaatttat ataaagtatt tttataatga ctggtcttcc ttacctggaa 1500
aaacatgcga tgttagtttt agaattacac cacaagtatc taaatttcca acttacaaag 1560
ggtcctatct tgtaaatatt gttttgcatt gtctgttggc aaatttgtga actgtcatga 1620
tacgcttaag gtgggaaagt gttcattgca caatatattt ttactgcttt ctgaatgtag 1680
acggaacagt gtggaagcag aaggcttttt taactcatcc gtttggccga tcgttgcaga 1740
ccactgggag atgtggatgt ggttgcctcc ttttgctcgt ccccgtggct taacccttct 1800

<210> 346
<211> 261
<212> PRT
<213> Homo sapiens

<400> 346
Met Asp Trp Gly Thr Leu His Thr Phe Ile Gly Gly Val Asn Lys His
1               5                   10                  15

Ser Thr Ser Ile Gly Lys Val Trp Ile Thr Val Ile Phe Ile Phe Arg
            20                  25                  30

Val Met Ile Leu Val Val Ala Ala Gln Glu Val Trp Gly Asp Glu Gln
        35                  40                  45

Glu Asp Phe Val Cys Asn Thr Leu Gln Pro Gly Cys Lys Asn Val Cys
    50                  55                  60

Tyr Asp His Phe Phe Pro Val Ser His Ile Arg Leu Trp Ala Leu Gln
65                  70                  75                  80

Leu Ile Phe Val Ser Thr Pro Ala Leu Leu Val Ala Met His Val Ala
                85                  90                  95

Tyr Tyr Arg His Glu Thr Thr Arg Lys Phe Arg Arg Gly Glu Lys Arg
            100                 105                 110

Asn Asp Phe Lys Asp Ile Glu Asp Ile Lys Lys His Lys Val Arg Ile
        115                 120                 125

Glu Gly Ser Leu Trp Trp Thr Tyr Thr Ser Ser Ile Phe Phe Arg Ile
    130                 135                 140

Ile Phe Glu Ala Ala Phe Met Tyr Val Phe Tyr Phe Leu Tyr Asn Gly
145                 150                 155                 160

Tyr His Leu Pro Trp Val Leu Lys Cys Gly Ile Asp Pro Cys Pro Asn
                165                 170                 175

Leu Val Asp Cys Phe Ile Ser Arg Pro Thr Glu Lys Thr Val Phe Thr
            180                 185                 190

Ile Phe Met Ile Ser Ala Ser Val Ile Cys Met Leu Leu Asn Val Ala
        195                 200                 205

Glu Leu Cys Tyr Leu Leu Leu Lys Val Cys Phe Arg Arg Ser Lys Arg
    210                 215                 220

Ala Gln Thr Gln Lys Asn His Pro Asn His Ala Leu Lys Glu Ser Lys
225                 230                 235                 240

Gln Asn Glu Met Asn Glu Leu Ile Ser Asp Ser Gly Gln Asn Ala Ile
                245                 250                 255

Thr Gly Phe Pro Ser
            260

<210> 347
<211> 1740
<212> DNA
<213> Homo sapiens

<400> 347
atgaacaaac tgtatatcgg aaacctcagc gagaacgccg ccccctcgga cctagaaagt 60
atcttcaagg acgccaagat cccggtgtcg ggacccttcc tggtgaagac tggctacgcg 120
ttcgtggact gcccggacga gagctgggcc ctcaaggcca tcgaggcgct ttcaggtaaa 180
atagaactgc acgggaaacc catagaagtt gagcactcgg tcccaaaaag gcaaaggatt 240
cggaaacttc agatacgaaa tatcccgcct catttacagt gggaggtgct ggatagttta 300
ctagtccagt atggagtggt ggagagctgt gagcaagtga acactgactc ggaaactgca 360
gttgtaaatg taacctattc cagtaaggac caagctagac aagcactaga caaactgaat 420
ggatttcagt tagagaattt caccttgaaa gtagcctata tccctgatga aacggccgcc 480
cagcaaaacc ccttgcagca gccccgaggt cgccgggggc ttgggcagag gggctcctca 540
aggcaggggt ctccaggatc cgtatccaag cagaaaccat gtgatttgcc tctgcgcctg 600
ctggttccca cccaatttgt tggagccatc ataggaaaag aaggtgccac cattcggaac 660
atcaccaaac agacccagtc taaaatcgat gtccaccgta aagaaaatgc gggggctgct 720
gagaagtcga ttactatcct ctctactcct gaaggcacct ctgcggcttg taagtctatt 780
ctggagatta tgcataagga agctcaagat ataaaattca cagaagagat ccccttgaag 840
attttagctc ataataactt tgttggacgt cttattggta aagaaggaag aaatcttaaa 900
aaaattgagc aagacacaga cactaaaatc acgatatctc cattgcagga attgacgctg 960

tataatccag aacgcactat tacagttaaa ggcaatgttg agacatgtgc caaagctgag 1020
gaggagatca tgaagaaaat cagggagtct tatgaaaatg atattgcttc tatgaatctt 1080
caagcacatt taattcctgg attaaatctg aacgccttgg gtctgttccc acccacttca 1140
gggatgccac ctcccacctc agggccccct tcagccatga ctcctcccta cccgcagttt 1200
gagcaatcag aaacggagac tgttcatctg tttatcccag ctctatcagt cggtgccatc 1260
atcggcaagc agggccagca catcaagcag ctttctcgct ttgctggagc ttcaattaag 1320
attgctccag cggaagcacc agatgctaaa gtgaggatgg tgattatcac tggaccacca 1380
gaggctcagt tcaaggctca gggaagaatt tatggaaaaa ttaaagaaga aaactttgtt 1440
agtcctaaag aagaggtgaa acttgaagct catatcagag tgccatcctt tgctgctggc 1500
agagttattg gaaaaggagg caaaacggtg aatgaacttc agaatttgtc aagtgcagaa 1560
gttgttgtcc ctcgtgacca gacacctgat gagaatgacc aagtggttgt caaaataact 1620
ggtcacttct atgcttgcca ggttgcccag agaaaaattc aggaaattct gactcaggta 1680
aagcagcacc aacaacagaa ggctctgcaa agtggaccac ctcagtcaag acggaagtaa 1740

<210> 348
<211> 579
<212> PRT
<213> Homo sapiens

<400> 348
Met Asn Lys Leu Tyr Ile Gly Asn Leu Ser Glu Asn Ala Ala Pro Ser
1               5                   10                  15

Asp Leu Glu Ser Ile Phe Lys Asp Ala Lys Ile Pro Val Ser Gly Pro
            20                  25                  30

Phe Leu Val Lys Thr Gly Tyr Ala Phe Val Asp Cys Pro Asp Glu Ser
        35                  40                  45

Trp Ala Leu Lys Ala Ile Glu Ala Leu Ser Gly Lys Ile Glu Leu His
    50                  55                  60

Gly Lys Pro Ile Glu Val Glu His Ser Val Pro Lys Arg Gln Arg Ile
65                  70                  75                  80

Arg Lys Leu Gln Ile Arg Asn Ile Pro Pro His Leu Gln Trp Glu Val
                85                  90                  95

Leu Asp Ser Leu Leu Val Gln Tyr Gly Val Val Glu Ser Cys Glu Gln
            100                 105                 110

Val Asn Thr Asp Ser Glu Thr Ala Val Val Asn Val Thr Tyr Ser Ser
        115                 120                 125

Lys Asp Gln Ala Arg Gln Ala Leu Asp Lys Leu Asn Gly Phe Gln Leu
    130                 135                 140

Glu Asn Phe Thr Leu Lys Val Ala Tyr Ile Pro Asp Glu Thr Ala Ala
145                 150                 155                 160

Gln Gln Asn Pro Leu Gln Gln Pro Arg Gly Arg Arg Gly Leu Gly Gln
                165                 170                 175

Arg Gly Ser Ser Arg Gln Gly Ser Pro Gly Ser Val Ser Lys Gln Lys

            180                 185                 190

Pro Cys Asp Leu Pro Leu Arg Leu Leu Val Pro Thr Gln Phe Val Gly
        195                 200                 205

Ala Ile Ile Gly Lys Glu Gly Ala Thr Ile Arg Asn Ile Thr Lys Gln
    210                 215                 220

Thr Gln Ser Lys Ile Asp Val His Arg Lys Glu Asn Ala Gly Ala Ala
225                 230                 235                 240

Glu Lys Ser Ile Thr Ile Leu Ser Thr Pro Glu Gly Thr Ser Ala Ala
                245                 250                 255

Cys Lys Ser Ile Leu Glu Ile Met His Lys Glu Ala Gln Asp Ile Lys
            260                 265                 270

Phe Thr Glu Glu Ile Pro Leu Lys Ile Leu Ala His Asn Asn Phe Val
        275                 280                 285

Gly Arg Leu Ile Gly Lys Glu Gly Arg Asn Leu Lys Lys Ile Glu Gln
    290                 295                 300

Asp Thr Asp Thr Lys Ile Thr Ile Ser Pro Leu Gln Glu Leu Thr Leu
305                 310                 315                 320

Tyr Asn Pro Glu Arg Thr Ile Thr Val Lys Gly Asn Val Glu Thr Cys
                325                 330                 335

Ala Lys Ala Glu Glu Glu Ile Met Lys Lys Ile Arg Glu Ser Tyr Glu
            340                 345                 350

Asn Asp Ile Ala Ser Met Asn Leu Gln Ala His Leu Ile Pro Gly Leu
        355                 360                 365

Asn Leu Asn Ala Leu Gly Leu Phe Pro Pro Thr Ser Gly Met Pro Pro
    370                 375                 380

Pro Thr Ser Gly Pro Pro Ser Ala Met Thr Pro Pro Tyr Pro Gln Phe
385                 390                 395                 400

Glu Gln Ser Glu Thr Glu Thr Val His Leu Phe Ile Pro Ala Leu Ser
                405                 410                 415

Val Gly Ala Ile Ile Gly Lys Gln Gly Gln His Ile Lys Gln Leu Ser
            420                 425                 430

Arg Phe Ala Gly Ala Ser Ile Lys Ile Ala Pro Ala Glu Ala Pro Asp
        435                 440                 445

Ala Lys Val Arg Met Val Ile Ile Thr Gly Pro Pro Glu Ala Gln Phe
    450                 455                 460

Lys Ala Gln Gly Arg Ile Tyr Gly Lys Ile Lys Glu Glu Asn Phe Val
465                 470                 475                 480

Ser Pro Lys Glu Glu Val Lys Leu Glu Ala His Ile Arg Val Pro Ser
                485                 490                 495

Phe Ala Ala Gly Arg Val Ile Gly Lys Gly Gly Lys Thr Val Asn Glu
            500                 505                 510

Leu Gln Asn Leu Ser Ser Ala Glu Val Val Val Pro Arg Asp Gln Thr
        515                 520                 525

Pro Asp Glu Asn Asp Gln Val Val Val Lys Ile Thr Gly His Phe Tyr
    530                 535                 540

Ala Cys Gln Val Ala Gln Arg Lys Ile Gln Glu Ile Leu Thr Gln Val
545                 550                 555                 560

Lys Gln His Gln Gln Gln Lys Ala Leu Gln Ser Gly Pro Pro Gln Ser
                565                 570                 575

Arg Arg Lys


<210> 349
<211> 207
<212> DNA
<213> Homo sapiens

<400> 349
atgtggcagc ccctcttctt caagtggctc ttgtcctgtt gccctgggag ttctcaaatt 60
gctgcagcag cctccaccca gcctgaggat gacatcaata cacagaggaa gaagagtcag 120
gaaaagatga gagaagttac agactctcct gggcgacccc gagagcttac cattcctcag 180
acttcttcac atggtgctaa cagattt                                     207


<210> 350
<211> 69
<212> PRT
<213> Homo sapiens

<400> 350
Met Trp Gln Pro Leu Phe Phe Lys Trp Leu Leu Ser Cys Cys Pro Gly
                5                   10                  15

Ser Ser Gln Ile Ala Ala Ala Ala Ser Thr Gln Pro Glu Asp Asp Ile
            20                  25                  30

Asn Thr Gln Arg Lys Lys Ser Gln Glu Lys Met Arg Glu Val Thr Asp
        35                  40                  45

Ser Pro Gly Arg Pro Arg Glu Leu Thr Ile Pro Gln Thr Ser Ser His
    50                  55                  60

Gly Ala Asn Arg Phe
65

## Description

### TECHNICAL FIELD

The present invention relates generally to therapy and diagnosis of cancer, such as lung cancer. The invention is more specifically related to polypeptides comprising at least a portion of a lung tumor protein, and to polynucleotides encoding such polypeptides. Such polypeptides and polynucleotides may be used in vaccines and pharmaceutical compositions for prevention and treatment of lung cancer, and for the diagnosis and monitoring of such cancers.

### BACKGROUND OF THE INVENTION

Lung cancer is the primary cause of cancer death among both men and women in the U.S., with an estimated 172,000 new cases being reported in 1994. The five-year survival rate among all lung cancer patients, regardless of the stage of disease at diagnosis, is only 13%. This contrasts with a five-year survival rate of 46% among cases detected while the disease is still localized. However, only 16% of lung cancers are discovered before the disease has spread.

Early detection is difficult since clinical symptoms are often not seen until the disease has reached an advanced stage. Currently, diagnosis is aided by the use of chest x-rays, analysis of the type of cells contained in sputum and fiberoptic examination of the bronchial passages. Treatment regimens are determined by the type and stage of the cancer, and include surgery, radiation therapy and/or chemotherapy. In spite of considerable research into therapies for the disease, lung cancer remains difficult to treat.

Accordingly, there remains a need in the art for improved vaccines, treatment methods and diagnostic techniques for lung cancer.

### SUMMARY OF THE INVENTION

Briefly stated, the present invention provides compositions and methods for the diagnosis and therapy of cancer, such as lung cancer. In one aspect, the present invention provides polypeptides comprising at least a portion of a lung tumor protein, or a variant thereof. Certain portions and other variants are immunogenic, such that the ability of the variant to react with antigen-specific antisera is not substantially diminished. Within certain embodiments, the polypeptide comprises a sequence that is encoded by a polynucleotide sequence selected from the group consisting of (a) sequences recited in any one of SEQ ID NO: 1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347 and 349; (b) variants of a sequence recited in any one of SEQ ID NO: 1-3,6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 179, 182, 184-186, 188-191, 193, 194, 198-207, 209, 210, 213, 214, 217, 220-224, 253-337, 345, 347 and 349; and (c) complements of a sequence of (a) or (b). In specific embodiments, the polypeptides of the present invention comprise at least a portion of a tumor protein that includes an amino acid sequence selected from the group consisting of sequences recited in any one of SEQ ID NO: 152, 155, 156, 165, 166, 169, 170, 172, 174, 176, 226-252, 338-344 and 346, and variants thereof.

The present invention further provides polynucleotides that encode a polypeptide as described above, or a portion thereof (such as a portion encoding at least 15 amino acid residues of a lung tumor protein), expression vectors comprising such polynucleotides and host cells transformed or transfected with such expression vectors.

Within other aspects, the present invention provides pharmaceutical compositions comprising a polypeptide or polynucleotide as described above and a physiologically acceptable carrier.

Within a related aspect of the present invention, vaccines for prophylactic or therapeutic use are provided. Such vaccines comprise a polypeptide or polynucleotide as described above and an immunostimulant.

The present invention further provides pharmaceutical compositions that comprise: (a) an antibody or antigen-binding fragment thereof that specifically binds to a lung tumor protein; and (b) a physiologically acceptable carrier.

Within further aspects, the present invention provides pharmaceutical compositions comprising: (a) an antigen presenting cell that expresses a polypeptide as described above and (b) a pharmaceutically acceptable carrier or excipient. Antigen presenting cells include dendritic cells, macrophages, monocytes, fibroblasts and B cells.

Within related aspects, vaccines are provided that comprise: (a) an antigen presenting cell that expresses a polypeptide as described above, and (b) an immunostimulant.

The present invention further provides, in other aspects, fusion proteins that comprise at least one polypeptide as described above, as well as polynucleotides encoding such fusion proteins.

Within related aspects, pharmaceutical compositions comprising a fusion protein, or a polynucleotide encoding a fusion protein, in combination with a physiologically acceptable carrier are provided.

Vaccines are further provided, within other aspects, that comprise a fusion protein, or a polynucleotide encoding a fusion protein, in combination with an immunostimulant.

Within further aspects, the present invention provides methods for inhibiting the development of a cancer in a patient, comprising administering to a patient a pharmaceutical composition or vaccine as recited above.

The present invention further provides, within other aspects, methods for removing tumor cells from a biological sample, comprising contacting a biological sample with T cells that specifically react with a lung tumor protein, wherein the step of contacting is performed under conditions and for a time sufficient to permit the removal of cells expressing the protein from the sample.

Within related aspects, methods are provided for inhibiting the development of a cancer in a patient, comprising administering to a patient a biological sample treated as described above.

Methods are further provided, within other aspects, for stimulating and/or expanding T cells specific for a lung tumor protein, comprising contacting T cells with one or more of: (i) a polypeptide as described above; (ii) a polynucleotide encoding such a polypeptide; and/or (iii) an antigen presenting cell that expresses such a polypeptide; under conditions and for a time sufficient to permit the stimulation and/or expansion of T cells. Determined T cell populations comprising T cells prepared as described above are also provided.

Within further aspects, the present invention provides methods for inhibiting the development of a cancer in a patient, comprising administering to a patient an effective amount of a T cell population as described above.

The present invention further provides methods for inhibiting the development of a cancer in a patient, comprising the steps of: (a) incubating CD4⁺ and/or CD8⁺ T cells determined from a patient with one or more of: (i) a polypeptide comprising at least an immunogenic portion of a lung tumor protein; (ii) a polynucleotide encoding such a polypeptide; and (iii) an antigen-presenting cell that expressed such a polypeptide; and (b) administering to the patient an effective amount of the proliferated T cells, and thereby inhibiting the development of a cancer in the patient. Proliferated cells may, but need not, be cloned prior to administration to the patient.

Within further aspects, the present invention provides methods for determining the presence or absence of a cancer in a patient, comprising: (a) contacting a biological sample obtained from a patient with a binding agent that binds to a polypeptide as recited above; (b) detecting in the sample an amount of polypeptide that binds to the binding agent; and (c) comparing the amount of polypeptide with a predetermined cut-off value, and therefrom determining the presence or absence of a cancer in the patient. Within preferred embodiments, the binding agent is an antibody, more preferably a monoclonal antibody. The cancer may be lung cancer.

The present invention also provides, within other aspects, methods for monitoring the progression of a cancer in a patient. Such methods comprise the steps of: (a) contacting a biological sample obtained from a patient at a first point in time with a binding agent that binds to a polypeptide as recited above; (b) detecting in the sample an amount of polypeptide that binds to the binding agent; (c) repeating steps (a) and (b) using a biological sample obtained from the patient at a subsequent point in time; and (d) comparing the amount of polypeptide detected in step (c) with the amount detected in step (b) and therefrom monitoring the progression of the cancer in the patient.

The present invention further provides, within other aspects, methods for determining the presence or absence of a cancer in a patient, comprising the steps of: (a) contacting a biological sample obtained from a patient with an oligonucleotide that hybridizes to a polynucleotide that encodes a lung tumor protein; (b) detecting in the sample a level of a polynucleotide, preferably mRNA, that hybridizes to the oligonucleotide; and (c) comparing the level of polynucleotide that hybridizes to the oligonucleotide with a predetermined cut-off value, and therefrom determining the presence or absence of a cancer in the patient. Within certain embodiments, the amount of mRNA is detected via polymerase chain reaction using, for example, at least one oligonucleotide primer that hybridizes to a polynucleotide encoding a polypeptide as recited above, or a complement of such a polynucleotide. Within other embodiments, the amount of mRNA is detected using a hybridization technique, employing an oligonucleotide probe that hybridizes to a polynucleotide that encodes a polypeptide as recited above, or a complement of such a polynucleotide.

In related aspects, methods are provided for monitoring the progression of a cancer in a patient, comprising the steps of: (a) contacting a biological sample obtained from a patient with an oligonucleotide that hybridizes to a polynucleotide that encodes a lung tumor protein; (b) detecting in the sample an amount of a polynucleotide that hybridizes to the oligonucleotide; (c) repeating steps (a) and (b) using a biological sample obtained from the patient at a subsequent point in time; and (d) comparing the amount of polynucleotide detected in step (c) with the amount detected in step (b) and therefrom monitoring the progression of the cancer in the patient.

Within further aspects, the present invention provides antibodies, such as monoclonal antibodies, that bind to a polypeptide as described above, as well as diagnostic kits comprising such antibodies. Diagnostic kits comprising one or more oligonucleotide probes or primers as described above are also provided.
These and other aspects of the present invention will become apparent upon reference to the following detailed description and attached drawings. All references disclosed herein are hereby incorporated by reference in their entirety as if each was incorporated individually.

### SEQUENCE IDENTIFIERS

SEQ ID NO: 1 is the determined cDNA sequence for LST-S1-2
SEQ ID NO: 2 is the determined cDNA sequence for LST-S1-28
SEQ ID NO: 3 is the determined cDNA sequence for LST-S1-90
SEQ ID NO: 4 is the determined cDNA sequence for LST-S1-144
SEQ ID NO: 5 is the determined cDNA sequence for LST-S1-133
SEQ ID NO: 6 is the determined cDNA sequence for LST-S 1-169
SEQ ID NO: 7 is the determined cDNA sequence for LST-S2-6
SEQ ID NO: 8 is the determined cDNA sequence for LST-S2-11
SEQ ID NO: 9 is the determined cDNA sequence for LST-S2-17
SEQ ID NO: 10 is the determined cDNA sequence for LST-S2-25
SEQ ID NO: 11 is the determined cDNA sequence for LST-S2-39
SEQ ID NO: 12 is a first determined cDNA sequence for LST-S2-43
SEQ ID NO: 13 is a second determined cDNA sequence for LST-S2-43
SEQ ID NO: 14 is the determined cDNA sequence for LST-S2-65
SEQ ID NO: 15 is the determined cDNA sequence for LST-S2-68
SEQ ID NO: 16 is the determined cDNA sequence for LST-S2-72
SEQ ID NO: 17 is the determined cDNA sequence for LST-S2-74
SEQ ID NO: 18 is the determined cDNA sequence for LST-S2-103
SEQ ID NO: 19 is the determined cDNA sequence for LST-S2-N1-1F
SEQ ID NO: 20 is the determined cDNA sequence for LST-S2-N1-2A
SEQ ID NO: 21 is the determined cDNA sequence for LST-S2-N1-4H
SEQ ID NO: 22 is the determined cDNA sequence for LST-S2-N1-5A
SEQ ID NO: 23 is the determined cDNA sequence for LST-S2-N1-6B
SEQ ID NO: 24 is the determined cDNA sequence for LST-S2-N1-7B
SEQ ID NO; 25 is the determined cDNA sequence for LST-S2-N1-7H
SEQ ID NO: 26 is the determined cDNA sequence for LST-S2-N1-8A
SEQ ID NO: 27 is the determined cDNA sequence for LST-S2-N1-8D
SEQ ID NO: 28 is the determined cDNA sequence for LST-S2-N1-9A
SEQ ID NO: 29 is the determined cDNA sequence for LST-S2-N1-9E
SEQ ID NO: 30 is the determined cDNA sequence for LST-S2-N1-10A
SEQ ID NO: 31 is the determined cDNA sequence for LST-S2-N1-10G
SEQ ID NO: 32 is the determined cDNA sequence for LST-S2 N1-11A
SEQ ID NO: 33 is the determined cDNA sequence for LST-S2-N1-12C
SEQ ID NO: 34 is the determined cDNA sequence for LST-S2-N1-12E
SEQ ID NO: 35 is the determined cDNA sequence for LST-S2-B1-3D
SEQ ID NO: 36 is the determined cDNA sequence for LST-S2-B1-6C
SEQ ID NO: 37 is the determined cDNA sequence for LST-S2-B1-5D
SEQ ID NO: 38 is the determined cDNA sequence for LST-S2-B1-5F
SEQ ID NO: 39 is the determined cDNA sequence for LST-S2-B1-6G
SEQ ID NO: 40 is the determined cDNA sequence for LST-S2-B1-8A
SEQ ID NO: 41 is the determined cDNA sequence for LST-S2-B1-8D
SEQ ID NO: 42 is the determined cDNA sequence for LST-S2-B1-10A
SEQ ID NO: 43 is the determined cDNA sequence for LST-S2-B1-9B
SEQ ID NO: 44 is the determined cDNA sequence for LST-S2-B1-9F
SEQ ID NO: 45 is the determined cDNA sequence for LST-S2-B1-12D
SEQ ID NO: 46 is the determined cDNA sequence for LST-S2-I2-2B
SEQ ID NO: 47 is the determined cDNA sequence for LST-S2-I2-5F
SEQ ID NO: 48 is the determined cDNA sequence for LST-S2-I2-6B
SEQ ID NO: 49 is the determined cDNA sequence for LST-S2-I2-7F
SEQ ID NO: 50 is the determined cDNA sequence for LST-S2-I2-8G
SEQ ID NO: 51 is the determined cDNA sequence for LST-S2-I2-9E
SEQ ID NO: 52 is the determined cDNA sequence for LST-52-I2-12B
SEQ ID NO: 53 is the determined cDNA sequence for LST-S2-H2-2C
SEQ ID NO: 54 is the determined cDNA sequence for LST-S2-H2-1 G
SEQ ID NO: 55 is the determined cDNA sequence for LST-S2-H2-4G
SEQ ID NO: 56 is the determined cDNA sequence for LST-S2-H2-3H
SEQ ID NO: 57 is the determined cDNA sequence for LST-S2-H2-5G
SEQ ID NO: 58 is the determined cDNA sequence for LST-S2-H2-9B
SEQ ID NO: 59 is the determined cDNA sequence for LST-S2-H2-10H
SEQ ID NO: 60 is the determined cDNA sequence for LST-S2-H2-12D
SEQ ID NO: 61 is the determined cDNA sequence for LST-S3-2
SEQ ID NO: 62 is the determined cDNA sequence for LST-S3-4
SEQ ID NO: 63 is the determined cDNA sequence for LST-S3-7
SEQ ID NO: 64 is the determined cDNA sequence for LST-S3-8
SEQ ID NO: 65 is the determined cDNA sequence for LST-S3-12
SEQ ID NO: 66 is the determined cDNA sequence for LST-S3-13
SEQ ID NO: 67 is the determined cDNA sequence for LST-S3-14
SEQ ID NO: 68 is the determined cDNA sequence for LST-S3-16
SEQ ID NO: 69 is the determined cDNA sequence for LST-S3-21
SEQ ID NO: 70 is the determined cDNA sequence for LST-S3-22
SEQ ID NO: 71 is the determined cDNA sequence for LST-S1-7
SEQ ID NO: 72 is the determined cDNA sequence for LST-S1-A-1E
SEQ ID NO: 73 is the determined cDNA sequence for LST-S1-A-1G
SEQ ID NO: 74 is the determined cDNA sequence for LST-S 1-A-3E
SEQ ID NO: 75 is the determined cDNA sequence for LST-S1-A-4E
SEQ ID NO: 76 is the determined cDNA sequence for LST-S1-A-6D
SEQ ID NO: 77 is the determined cDNA sequence for LST-S1-A-8D
SEQ ID NO: 78 is the determined cDNA sequence for LST-S1-A-10A
SEQ ID NO: 79 is the determined cDNA sequence for LST-S1-A-10C
SEQ ID NO: 80 is the determined cDNA sequence for LST-S 1-A-9D
SEQ ID NO: 81 is the determined cDNA sequence for LST-S1-A-10D
SEQ ID NO: 82 is the determined cDNA sequence for LST-S1-A-9H
SEQ ID NO: 83 is the determined cDNA sequence for LST-S1-A-110
SEQ ID NO: 84 is the determined cDNA sequence for LST-S1-A-12D
SEQ ID NO: 85 is the determined cDNA sequence for LST-S1-A-11E
SEQ ID NO: 86 is the determined cDNA sequence for LST-S1-A-12E
SEQ ID NO: 87 is the determined cDNA sequence for L513S (T3).
SEQ ID NO: 88 is the determined cDNA sequence for L513S contig 1.
SEQ ID NO: 89 is a first determined cDNA sequence for L514S.
SEQ ID NO: 90 is a second determined cDNA sequence for L514S.
SEQ ID NO: 91 is a first determined cDNA sequence for L516S.
SEQ ID NO: 92 is a second determined cDNA sequence for L516S.
SEQ ID NO: 93 is the determined cDNA sequence for L517S.
SEQ ID NO: 94 is the extended cDNA sequence for LST-S1-169 (also known as L519S).
SEQ ID NO: 95 is a first determined cDNA sequence for L520S.
SEQ ID NO: 96 is a second determined cDNA sequence for L520S.
SEQ ID NO: 97 is a first determined cDNA sequence for L521S,
SEQ ID NO: 98 is a second determined cDNA sequence for L521 S.
SEQ ID NO: 99 is the determined cDNA sequence for L522S.
SEQ ID NO: 100 is the determined cDNA sequence for L523S.
SEQ ID NO: 101 is the determined cDNA sequence for L524S.
SEQ ID NO: 102 is the determined cDNA sequence for L525S.
SEQ ID NO: 103 is the determined cDNA sequence for L526S.
SEQ ID NO: 104 is the determined cDNA sequence for L527S.
SEQ ID NO: 105 is the determined cDNA sequence for L528S.
SEQ ID NO: 106 is the determined cDNA sequence for L529S.
SEQ ID NO: 107 is a first determined cDNA sequence for L530S.
SEQ ID NO: 108 is a second determined cDNA sequence for L530S.
SEQ ID NO: 109 is the determined full-length cDNA sequence for L531S short form
SEQ ID NO: 110 is the predicted amino acid sequence encoded by SEQ ID NO: 109.
SEQ ID NO: 111 is the determined full-length cDNA sequence for L531 S long form
SEQ ID NO: 112 is the predicted amino acid sequence encoded by SEQ ID NO: 111.
SEQ ID NO: 113 is the determined full-length cDNA sequence for L520S.
SEQ ID NO: 114 is the predicted amino acid sequence encoded by SEQ ID NO: 113.
SEQ ID NO: 115 is the determined cDNA sequence for contig 1.
SEQ ID NO: 116 is the determined cDNA sequence for contig 3.
SEQ ID NO: 117 is the determined cDNA sequence for contig 4.
SEQ ID NO: 118 is the determined cDNA sequence for contig 5.
SEQ ID NO: 119 is the determined cDNA sequence for contig 7.
SEQ ID NO: 120 is the determined cDNA sequence for contig 8.
SEQ ID NO: 121 is the determined cDNA sequence for contig 9.
SEQ ID NO: 122 is the determined cDNA sequence for contig 10.
SEQ ID NO: 123 is the determined cDNA sequence for contig 12.
SEQ ID NO: 124 is the determined cDNA sequence for contig 11.
SEQ ID NO: 125 is the determined cDNA sequence for contig 13.
SEQ ID NO: 126 is the determined cDNA sequence for contig 15.
SEQ ID NO: 127 is the determined cDNA sequence for contig 16.
SEQ ID NO: 128 is the determined cDNA sequence for contig 17.
SEQ ID NO: 129 is the determined cDNA sequence for contig 19.
SEQ ID NO: 130 is the determined cDNA sequence for contig 20.
SEQ ID NO: 131 is the determined cDNA sequence for contig 22.
SEQ ID NO: 132 is the determined cDNA sequence for contig 24.
SEQ ID NO: 133 is the determined cDNA sequence for contig 29.
SEQ ID NO: 134 is the determined cDNA sequence for contig 31.
SEQ ID NO: 135 is the determined cDNA sequence for contig 33.
SEQ ID NO: 136 is the determined cDNA sequence for contig 38.
SEQ ID NO: 137 is the determined cDNA sequence for contig 39.
SEQ ID NO: 138 is the determined cDNA sequence for contig 41.
SEQ ID NO: 139 is the determined cDNA sequence for contig 43.
SEQ ID NO: 140 is the determined cDNA sequence for contig 44.
SEQ ID NO: 141 is the determined cDNA sequence for contig 45.
SEQ ID NO: 142 is the determined cDNA sequence for contig 47.
SEQ ID NO: 143 is the determined cDNA sequence for contig 48.
SEQ ID NO: 144 is the determined cDNA sequence for contig 49.
SEQ ID NO: 145 is the determined cDNA sequence for contig 50.
SEQ ID NO: 146 is the determined cDNA sequence for contig 53.
SEQ ID NO: 147 is the determined cDNA sequence for contig 54.
SEQ ID NO: 148 is the determined cDNA sequence for contig 56.
SEQ ID NO: 149 is the determined cDNA sequence for contig 57.
SEQ ID NO: 150 is the determined cDNA sequence for contig 58.
SEQ ID NO: 151 is the full-length cDNA sequence for L530S.
SEQ ID NO: 152 is the amino acid sequence encoded by SEQ ID NO: 151
SEQ ID NO: 153 is the full-length cDNA sequence of a first variant of L514S
SEQ ID NO: 154 is the full-length cDNA sequence of a second variant of L514S
SEQ ID NO: 155 is the amino acid sequence encoded by SEQ ID NO: 153.
SEQ ID NO: 156 is the amino acid sequence encoded by SEQ ID NO: 154.
SEQ ID NO: 157 is the determined cDNA sequence for contig 59.
SEQ ID NO: 158 is the full-length cDNA sequence for L763P (also referred to as contig 22).
SEQ ID NO: 159 is the amino acid sequence encoded by SEQ ID NO: 158.
SEQ ID NO: 160 is the full-length cDNA sequence for L762P (also referred to as contig 17).
SEQ ID NO: 161 is the amino acid sequence encoded by SEQ ID NO: 160.
SEQ ID NO: 162 is the determined cDNA sequence for L515S.
SEQ ID NO: 163 is the full-length cDNA sequence of a first variant of L524S.
SEQ ID NO: 164 is the full-length cDNA sequence of a second variant of L524S.
SEQ ID NO: 165 is the amino acid sequence encoded by SEQ ID NO: 163.
SEQ ID NO: 166 is the amino acid sequence encoded by SEQ ID NO: 164.
SEQ ID NO: 167 is the full-length cDNA sequence of a first variant of L762P.
SEQ ID NO: 168 is the full-length cDNA sequence of a second variant of L762P.
SEQ ID NO: 169 is the amino acid sequence encoded by SEQ ID NO: 167.
SEQ ID NO: 170 is the amino acid sequence encoded by SEQ ID NO: 168.
SEQ ID NO: 171 is the full-length cDNA sequence for L773P (also referred to as contig 56).
SEQ ID NO: 172 is the amino acid sequence encoded by SEQ ID NO: 171.
SEQ ID NO: 173 is an extended cDNA sequence for L519S.
SEQ ID NO: 174 is the predicted amino acid sequence encoded by SEQ ID NO: 174.
SEQ ID NO: 175 is the full-length cDNA sequence for L523S.
SEQ ID NO: 176 is the predicted amino acid sequence encoded by SEQ ID NO: 175.
SEQ ID NO: 177 is the determined cDNA sequence for LST-sub5-7A.
SEQ ID NO: 178 is the determined cDNA sequence for LST-sub5-8G.
SEQ ID NO: 179 is the determined cDNA sequence for LST-sub5-8H.
SEQ ID NO: 180 is the determined cDNA sequence for LST-sub5-10B.
SEQ ID NO: 181 is the determined cDNA sequence for LST-sub5-10H.
SEQ ID NO: 182 is the determined cDNA sequence for LST-sub5-12B.
SEQ ID NO: 183 is the determined cDNA sequence for LST-sub5-11C.
SEQ ID NO: 184 is the determined cDNA sequence for LST-sub5-1c.
SEQ ID NO: 185 is the determined cDNA sequence for LST-sub6-2f.
SEQ ID NO: 186 is the determined cDNA sequence for LST-sub6-2G.
SEQ ID NO: 187 is the determined cDNA sequence for LST-sub6-4d.
SEQ ID NO: 188 is the determined cDNA sequence for LST-sub6-4e.
SEQ ID NO: 189 is the determined cDNA sequence for LST-sub6-4f.
SEQ ID NO: 190 is the determined cDNA sequence for LST-sub6-3h.
SEQ ID NO: 191 is the determined cDNA sequence for LST-sub6-5d.
SEQ ID NO: 192 is the determined cDNA sequence for LST-sub6-5h.
SEQ ID NO: 193 is the determined cDNA sequence for LST-sub6-6h.
SEQ ID NO: 194 is the determined cDNA sequence for LST-sub6-7a.
SEQ ID NO: 195 is the determined cDNA sequence for LST-sub6-8a.
SEQ ID NO: 196 is the determined cDNA sequence for LST-sub6-7d.
SEQ ID NO: 197 is the determined cDNA sequence for LST-sub6-7e.
SEQ ID NO: 198 is the determined cDNA sequence for LST-sub6-8e.
SEQ ID NO: 199 is the determined cDNA sequence for LST-sub6-7g.
SEQ ID NO: 200 is the determined cDNA sequence for LST-sub6-9f.
SEQ ID NO: 201 is the determined cDNA sequence for LST-sub6-9h.
SEQ ID NO: 202 is the determined cDNA sequence for LST-sub6-11b.
SEQ ID NO: 203 is the determined cDNA sequence for LST-sub6-11c.
SEQ ID NO: 204 is the determined cDNA sequence for LST-sub6-12c.
SEQ ID NO: 205 is the determined cDNA sequence for LST-sub6-12e.
SEQ ID NO: 206 is the determined cDNA sequence for LST-sub6-12f.
SEQ ID NO: 207 is the determined cDNA sequence for LST-sub6-11 g.
SEQ ID NO: 208 is the determined cDNA sequence for LST-sub6-12g.
SEQ ID NO: 209 is the determined cDNA sequence for LST-sub6-12h.
SEQ ID NO: 210 is the determined cDNA sequence for LST-sub6-II-1a.
SEQ ID NO: 211 is the determined cDNA sequence for LST-sub6-II-2b.
SEQ ID NO: 212 is the determined cDNA sequence for LST-sub6-II-2g.
SEQ ID NO: 213 is the determined cDNA sequence for LST-sub6-II-1h.
SEQ ID NO: 214 is the determined cDNA sequence for LST-sub6-II-4a.
SEQ ID NO: 215 is the determined cDNA sequence for LST-sub6-II-4b.
SEQ ID NO: 216 is the determined cDNA sequence for LST-sub6-II-3e.
SEQ ID NO: 217 is the determined cDNA sequence for LST-sub6-II-4f.
SEQ ID NO: 218 is the determined cDNA sequence for LST-sub6-II-4g.
SEQ ID NO: 219 is the determined cDNA sequence for LST-sub6-II-4h.
SEQ ID NO: 220 is the determined cDNA sequence for LST-sub6-II-5c.
SEQ ID NO: 221 is the determined cDNA sequence for LST-sub6-II-5e.
SEQ ID NO: 222 is the determined cDNA sequence for LST-sub6-II-6f.
SEQ ID NO: 223 is the determined cDNA sequence for LST-sub6-II-5g.
SEQ ID NO: 224 is the determined cDNA sequence for LST-sub6-II-6g.
SEQ ID NO: 225 is the amino acid sequence for L528S.
SEQ ID NO: 226-251 are synthetic peptides derived from L762P.
SEQ ID NO: 252 is the expressed amino acid sequence of L514S.
SEQ ID NO: 253 is the DNA sequence corresponding to SEQ ID NO: 252.
SEQ ID NO: 254 is the DNA sequence of a L762P expression construct.
SEQ ID NO: 255 is the determined cDNA sequence for clone 23785.
SEQ ID NO: 256 is the determined cDNA sequence for clone 23786.
SEQ ID NO: 257 is the determined cDNA sequence for clone 23788.
SEQ ID NO: 258 is the determined cDNA sequence for clone 23790.
SEQ ID NO: 259 is the determined cDNA sequence for clone 23793.
SEQ ID NO: 260 is the determined cDNA sequence for clone 23794.
SEQ ID NO: 261 is the determined cDNA sequence for clone 23795.
SEQ ID NO: 262 is the determined cDNA sequence for clone 23796.
SEQ ID NO: 263 is the determined cDNA sequence for clone 23797,
SEQ ID NO: 264 is the determined cDNA sequence for clone 23798.
SEQ ID NO: 265 is the determined cDNA sequence for clone 23799.
SEQ ID NO: 266 is the determined cDNA sequence for clone 23800.
SEQ ID NO: 267 is the determined cDNA sequence for clone 23802.
SEQ ID NO: 268 is the determined cDNA sequence for clone 23803.
SEQ ID NO: 269 is the determined cDNA sequence for clone 23804.
SEQ ID NO: 270 is the determined cDNA sequence for clone 23805.
SEQ ID NO: 271 is the determined cDNA sequence for clone 23806.
SEQ ID NO: 272 is the determined cDNA sequence for clone 23807.
SEQ ID NO: 273 is the determined cDNA sequence for clone 23808.
SEQ ID NO: 274 is the determined cDNA sequence for clone 23809.
SEQ ID NO: 275 is the determined cDNA sequence for clone 23810.
SEQ ID NO: 276 is the determined cDNA sequence for clone 23811.
SEQ ID NO: 277 is the determined cDNA sequence for clone 23812.
SEQ ID NO: 278 is the determined cDNA sequence for clone 23813.
SEQ ID NO: 279 is the determined cDNA sequence for clone 23 815.
SEQ ID NO: 280 is the determined cDNA sequence for clone 25298.
SEQ ID NO: 281 is the determined cDNA sequence for clone 25299.
SEQ ID NO: 282 is the determined cDNA sequence for clone 25300.
SEQ ID NO: 283 is the determined cDNA sequence for clone 25301
SEQ ID NO: 284 is the determined cDNA sequence for clone 25304
SEQ ID NO: 285 is the determined cDNA sequence for clone 25309.
SEQ ID NO: 286 is the determined cDNA sequence for clone 25312.
SEQ ID NO: 287 is the determined cDNA sequence for clone 25317.
SEQ ID NO: 288 is the determined cDNA sequence for clone 25321.
SEQ ID NO: 289 is the determined cDNA sequence for clone 25323.
SEQ ID NO: 290 is the determined cDNA sequence for clone 25327.
SEQ ID NO: 291 is the determined cDNA sequence for clone 25328.
SEQ ID NO: 292 is the determined cDNA sequence for clone 25332.
SEQ ID NO: 293 is the determined cDNA sequence for clone 25333.
SEQ ID NO: 294 is the determined cDNA sequence for clone 25336.
SEQ ID NO: 295 is the determined cDNA sequence for clone 25340.
SEQ ID NO: 296 is the determined cDNA sequence for clone 25342.
SEQ ID NO: 297 is the determined cDNA sequence for clone 25356.
SEQ ID NO: 298 is the determined cDNA sequence for clone 25357.
SEQ ID NO: 299 is the determined cDNA sequence for clone 25361.
SEQ ID NO: 300 is the determined cDNA sequence for clone 25363.
SEQ ID NO: 301 is the determined cDNA sequence for clone 25397.
SEQ ID NO: 302 is the determined cDNA sequence for clone 25402.
SEQ ID NO: 303 is the determined cDNA sequence for clone 25403.
SEQ ID NO: 304 is the determined cDNA sequence for clone 25405.
SEQ ID NO: 305 is the determined cDNA sequence for clone 25407.
SEQ ID NO: 306 is the determined cDNA sequence for clone 25409.
SEQ ID NO: 307 is the determined cDNA sequence for clone 25396.
SEQ ID NO: 308 is the determined cDNA sequence for clone 25414.
SEQ ID NO: 309 is the determined cDNA sequence for clone 25410.
SEQ ID NO: 310 is the determined cDNA sequence for clone 25406.
SEQ ID NO: 311 is the determined cDNA sequence for clone 25306.
SEQ ID NO: 312 is the determined cDNA sequence for clone 25362.
SEQ ID NO: 313 is the determined cDNA sequence for clone 25360.
SEQ ID NO: 314 is the determined cDNA sequence for clone 25398.
SEQ ID NO: 315 is the determined cDNA sequence for clone 25355.
SEQ ID NO: 316 is the determined cDNA sequence for clone 25351.
SEQ ID NO: 317 is the determined cDNA sequence for clone 25331.
SEQ ID NO: 318 is the determined cDNA sequence for clone 25338.
SEQ ID NO: 319 is the determined cDNA sequence for clone 25335.
SEQ ID NO: 320 is the determined cDNA sequence for clone 25329.
SEQ ID NO: 321 is the determined cDNA sequence for clone 25324.
SEQ ID NO: 322 is the determined cDNA sequence for clone 25322.
SEQ ID NO: 323 is the determined cDNA sequence for clone 25319.
SEQ ID NO: 324 is the determined cDNA sequence for clone 25316.
SEQ ID NO: 325 is the determined cDNA sequence for clone 25311.
SEQ ID NO: 326 is the determined cDNA sequence for clone 25310.
SEQ ID NO: 327 is the determined cDNA sequence for clone 25302.
SEQ ID NO: 328 is the determined cDNA sequence for clone 25315.
SEQ ID NO: 329 is the determined cDNA sequence for clone 25308.
SEQ ID NO: 330 is the determined cDNA sequence for clone 25303.
SEQ ID NO: 331-337 are the cDNA sequences of isoforms of the p53 tumor suppressor homologue, p63 (also referred to as L530S).
SEQ ID NO: 338-344 are the amino acid sequences encoded by SEQ ID NO: 331-337, respectively.
SEQ ID NO: 345 is a second cDNA sequence for the antigen L763P.
SEQ ID NO: 346 is the amino acid sequence encoded by the sequence of SEQ ID NO: 345.
SEQ ID NO: 347 is a determined full-length cDNA sequence for L523S.
SEQ ID NO: 348 is the predicted amino acid sequence encoded by SEQ ID NO: 347.
SEQ ID NO: 349 is the cDNA sequence encoding the N-terminal portion of L773P.
SEQ ID NO: 350 is the amino acid sequence of the N-terminal portion of L773P.

### DETAILED DESCRIPTION OF THE INVENTION

As noted above, the present invention is generally directed to compositions and methods for the therapy and diagnosis of cancer, such as lung cancer. The compositions described herein may include lung tumor polypeptides, polynucleotides encoding such polypeptides, binding agents such as antibodies, antigen presenting cells (APCs) and/or immune system cells (e.g., T cells). Polypeptides of the present invention generally comprise at least a portion (such as an immunogenic portion) of a lung tumor protein or a variant thereof. A "lung tumor protein" is a protein that is expressed in lung tumor cells at a level that is at least two fold, and preferably at least five fold, greater than the level of expression in a normal tissue, as determined using a representative assay provided herein. Certain lung tumor proteins are tumor proteins that react detectably (within an immunoassay, such as an ELISA or Western blot) with antisera of a patient afflicted with lung cancer. Polynucleotides of the subject invention generally comprise a DNA or RNA sequence that encodes all or a portion of such a polypeptide, or that is complementary to such a sequence. Antibodies are generally immune system proteins, or antigen-binding fragments thereof, that are capable of binding to a polypeptide as described above. Antigen presenting cells include dendritic cells, macrophages, monocytes, fibroblasts and B-cells that express a polypeptide as described above. T cells that may be employed within such compositions are generally T cells that are specific for a polypeptide as described above.

The present invention is based on the discovery human lung tumor proteins. Sequences of polynucleotides encoding specific tumor proteins are provided in SEQ ID NO: 1-109, 111, 113, 115-151, 153, 154,157, 158, 160, 162-164, 167, 168, 171, 173, 175, 177-224, 255-337, 345, 347 and 349.

### LUNG TUMOR PROTEIN POLYNUCLEOTIDES

Any polynucleotide that encodes a lung tumor protein or a portion or other variant thereof as described herein is encompassed by the present invention. Preferred polynucleotides comprise at least 15 consecutive nucleotides, preferably at least 30 consecutive nucleotides and more preferably at least 45 consecutive nucleotides, that encode a portion of a lung tumor protein. More preferably, a polynucleotide encodes an immunogenic portion of a lung tumor protein. Polynucleotides complementary to any such sequences are also encompassed by the present invention. Polynucleotides may be single-stranded (coding or antisense) or double-stranded, and may be DNA (genomic, cDNA or synthetic) or RNA molecules. RNA molecules include HnRNA molecules, which contain introns and correspond to a DNA molecule in a one-to-one manner, and mRNA molecules, which do not contain introns. Additional coding or non-coding sequences may, but need not, be present within a polynucleotide of the present invention, and a polynucleotide may, but need not, be linked to other molecules and/or support materials.

Polynucleotides may comprise a native sequence (*i.e.,* an endogenous sequence that encodes a lung tumor protein or a portion thereof) or may comprise a variant of such a sequence. Polynucleotide variants may contain one or more substitutions, additions, deletions and/or insertions such that the immunogenicity of the encoded polypeptide is not diminished, relative to a native tumor protein. The effect on the immunogenicity of the encoded polypeptide may generally be assessed as described herein. Variants preferably exhibit at least about 70% identity, more preferably at least about 80% identity and most preferably at least about 90% identity to a polynucleotide sequence that encodes a native lung tumor protein or a portion thereof. The term "variants" also encompasses homologous genes of xenogenic origin.

Two polynucleotide or polypeptide sequences are said to be "identical" if the sequence of nucleotides or amino acids in the two sequences is the same when aligned for maximum correspondence as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships.

In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 Methods in Enzymology vol. 183, Academic Press, Inc., San Diego, CA; Higgins, D.G. and Sharp, P.M. (1989) CABIOS 5:151-153; Myers, E.W, and Muller W. (1988) CABIOS 4:11-17; Robinson, E.D. (1971) Comb. Theor 11:105; Santou, N. Nes, M. (1987) Mol. Biol. Evol. 4:406-425; Sneath, P.H.A. and Sokal, R.R. (1973) Numerical Taxonomy - the Principles and Practice of Numerical Taxonomy, Freeman Press, San Francisco, CA; Wilbur, W.J. and Lipman, D.J. (1983) Proc. Natl. Acad., Sci. USA 80:726-730.

Preferably, the "percentage of sequence identity" is determined by comparing two optimally aligned sequences over a window of comparison of at least 20 positions, wherein the portion of the polynucleotide or polypeptide sequence in the comparison window may comprise additions or deletions (i.e. gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases or amino acid residue occurs in both sequences to yield the number of matched positions, dividing the number of matched positions by the total number of positions in the reference sequence (i.e. the window size) and multiplying the results by 100 to yield the percentage of sequence identity.

Variants may also, or alternatively, be substantially homologous to a native gene, or a portion or complement thereof. Such polynucleotide variants are capable of hybridizing under moderately stringent conditions to a naturally occurring DNA sequence encoding a native lung tumor protein (or a complementary sequence). Suitable moderately stringent conditions include prewashing in a solution of 5 X SSC, 0.5% SDS, 1.0 mM EDTA (pH 8.0); hybridizing at 50°C-65°C, 5 X SSC, overnight; followed by washing twice at 65°C for 20 minutes with each of 2X, 0.5X and 0.2X SSC containing 0.1% SDS.

It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention. Further, alleles of the genes comprising the polynucleotide sequences provided herein are within the scope of the present invention. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

Polynucleotides may be prepared using any of a variety of techniques. For example, a polynucleotide may be identified, as described in more detail below, by screening a microarray of cDNAs for tumor-associated expression (*i*.*e*., expression that is at least two fold greater in a lung tumor than in normal tissue, as determined using a representative assay provided herein). Such screens may be performed using a Synteni microarray (Palo Alto, CA) according to the manufacturer's instructions (and essentially as described by Schena et al., Proc. Natl. Acad. Sci. USA 93:10614-10619, 1996 and Heller et al., Proc. Natl. Acad. Sci. USA 94:2150-2155, 1997). Alternatively, polypeptides may be amplified from cDNA prepared from cells expressing the proteins described herein, such as lung tumor cells. Such polynucleotides may be amplified via polymerase chain reaction (PCR). For this approach, sequence-specific primers may be designed based on the sequences provided herein, and may be purchased or synthesized.

An amplified portion may be used to isolate a full length gene from a suitable library (e.g., a lung tumor cDNA library) using well known techniques. Within such techniques, a library (cDNA or genomic) is screened using one or more polynucleotide probes or primers suitable for amplification. Preferably, a library is size-selected to include larger molecules. Random primed libraries may also be preferred for identifying 5' and upstream regions of genes. Genomic libraries are preferred for obtaining introns and extending 5' sequences.

For hybridization techniques, a partial sequence may be labeled (e.g., by nick-translation or end-labeling with ³²P) using well known techniques. A bacterial or bacteriophage library is then screened by hybridizing filters containing denatured bacterial colonies (or lawns containing phage plaques) with the labeled probe *(see* Sambrook et al., Molecular Cloning: A Laboratory Manual, Cold Spring Harbor Laboratories, Cold Spring Harbor, NY, 1989). Hybridizing colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones. The resulting overlapping sequences are then assembled into a single contiguous sequence. A full length cDNA molecule can be generated by ligating suitable fragments, using well known techniques.

Alternatively, there are numerous amplification techniques for obtaining a full length coding sequence from a partial cDNA sequence. Within such techniques, amplification is generally performed via PCR. Any of a variety of commercially available kits may be used to perform the amplification step. Primers may be designed using, for example, software well known in the art. Primers are preferably 22-30 nucleotides in length, have a GC content of at least 50% and anneal to the target sequence at temperatures of about 68°C to 72°C. The amplified region may be sequenced as described above, and overlapping sequences assembled into a contiguous sequence.

One such amplification technique is inverse PCR (*see* Triglia et al., Nucl. Acids Res. 16:8186, 1988), which uses restriction enzymes to generate a fragment in the known region of the gene. The fragment is then circularized by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Another such technique is known as "rapid amplification of cDNA ends" or RACE. This technique involves the use of an internal primer and an external primer, which hybridizes to a polyA region or vector sequence, to identify sequences that are 5' and 3' of a known sequence. Additional techniques include capture PCR (Lagerstrom et al., PCR Methods Applic. 1:111-19, 1991) and walking PCR (Parker et al., Nucl. Acids. Res. 19:3055-60, 1991). Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (e.g., NCBI BLAST searches), and such ESTs may be used to generate a contiguous full length sequence. Full length DNA sequences may also be obtained by analysis of genomic fragments.

Certain nucleic acid sequences of cDNA molecules encoding portions of lung tumor proteins are provided in SEQ ID NO: 1-109, 111, 113, 115-151, 153, 154,157, 158, 160, 162-164, 167, 168, 171, 173, 175, 177-224, 255-337, 345, 347 and 349.

Polynucleotide variants may generally be prepared by any method known in the art, including chemical synthesis by, for example, solid phase phosphoramidite chemical synthesis. Modifications in a polynucleotide sequence may also be introduced using standard mutagenesis techniques, such as oligonucleotide-directed site-specific mutagenesis *(see* Adelman et al., DNA 2:183, 1983). Alternatively, RNA molecules may be generated by *in vitro* or *in vivo* transcription of DNA sequences encoding a lung tumor protein, or portion thereof, provided that the DNA is incorporated into a vector with a suitable RNA polymerase promoter (such as T7 or SP6). Certain portions may be used to prepare an encoded polypeptide, as described herein. In addition, or alternatively, a portion may be administered to a patient such that the encoded polypeptide is generated *in vivo* (*e.g.*, by transfecting antigen-presenting cells, such as dendritic cells, with a cDNA construct encoding a lung tumor polypeptide, and administering the transfected cells to the patient).

A portion of a sequence complementary to a coding sequence (*i.e.,* an antisense polynucleotide) may also be used as a probe or to modulate gene expression cDNA constructs that can be transcribed into antisense RNA may also be introduced into cells of tissues to facilitate the production of antisense RNA. An antisense polynucleotide may be used, as described herein, to inhibit expression of a tumor protein. Antisense technology can be used to control gene expression through triple-helix formation, which compromises the ability of the double helix to open sufficiently for the binding of polymerases, transcription factors or regulatory molecules *(see* Gee et al., In Huber and Carr, Molecular and Immunologic Approaches, Futura Publishing Co. (Mt. Kisco, NY; 1994)). Alternatively, an antisense molecule may be designed to hybridize with a control region of a gene (e.g., promoter, enhancer or transcription initiation site), and block transcription of the gene; or to block translation by inhibiting binding of a transcript to ribosomes.

A portion of a coding sequence, or of a complementary sequence, may also be designed as a probe or primer to detect gene expression. Probes may be labeled with a variety of reporter groups, such as radionuclides and enzymes, and are preferably at least 10 nucleotides in length, more preferably at least 20 nucleotides in length and still more preferably at least 30 nucleotides in length. Primers, as noted above, are preferably 22-30 nucleotides in length.

Any polynucleotide may be further modified to increase stability *in vivo*. Possible modifications include, but are not limited to, the addition of flanking sequences at the 5' and/or 3' ends; the use of phosphorothioate or 2' O-methyl rather than phosphodiesterase linkages in the backbone; and/or the inclusion of nontraditional bases such as inosine, queosine and wybutosine, as well as acetyl- methyl-, thio- and other modified forms of adenine, cytidine, guanine, thymine and uridine.

Nucleotide sequences as described herein may be joined to a variety of other nucleotide sequences using established recombinant DNA techniques. For example, a polynucleotide may be cloned into any of a variety of cloning vectors, including plasmids, phagemids, lambda phage derivatives and cosmids. Vectors of particular interest include expression vectors, replication vectors, probe generation vectors and sequencing vectors. In general, a vector will contain an origin of replication functional in at least one organism, convenient restriction endonuclease sites and one or more selectable markers. Other elements will depend upon the desired use, and will be apparent to those of ordinary skill in the art.

Within certain embodiments, polynucleotides may be formulated so as to permit entry into a cell of a mammal, and expression therein. Such formulations are particularly useful for therapeutic purposes, as described below. Those of ordinary skill in the art will appreciate that there are many ways to achieve expression of a polynucleotide in a target cell, and any suitable method may be employed. For example, a polynucleotide may be incorporated into a viral vector such as, but not limited to, adenovirus, adeno-associated virus, retrovirus, or vaccinia or other pox virus (e.g., avian pox virus). ). The polynucleotides may also be administered as naked plasmid vectors. Techniques for incorporating DNA into such vectors are well known to those of ordinary skill in the art. A retroviral vector may additionally transfer or incorporate a gene for a selectable marker (to aid in the identification or selection of transduced cells) and/or a targeting moiety, such as a gene that encodes a ligand for a receptor on a specific target cell, to render the vector target specific. Targeting may also be accomplished using an antibody, by methods known to those of ordinary skill in the art.

Other formulations for therapeutic purposes include colloidal dispersion systems, such as macromolecule complexes, nanocapsules, microspheres, beads, and lipid-based systems including oil-in-water emulsions, micelles, mixed micelles, and liposomes. A preferred colloidal system for use as a delivery vehicle *in vitro* and *in vivo* is a liposome (*i*.*e*., an artificial membrane vesicle). The preparation and use of such systems is well known in the art.

### LUNG TUMOR POLYPEPTIDES

Within the context of the present invention, polypeptides may comprise at least an immunogenic portion of a lung tumor protein or a variant thereof, as described herein. As noted above, a "lung tumor protein" is a protein that is expressed by lung tumor cells. Proteins that are lung tumor proteins also react detectably within an immunoassay (such as an ELISA) with antisera from a patient with lung cancer. Polypeptides as described herein may be of any length. Additional sequences derived from the native protein and/or heterologous sequences may be present, and such sequences may (but need not) possess further immunogenic or antigenic properties.

An "immunogenic portion," as used herein is a portion of a protein that is recognized (*i.e.,* specifically bound) by a B-cell and/or T-cell surface antigen receptor. Such immunogenic portions generally comprise at least 5 amino acid residues, more preferably at least 10, and still more preferably at least 20 amino acid residues of a lung tumor protein or a variant thereof. Certain preferred immunogenic portions include peptides in which an N-terminal leader sequence and/or transmembrane domain have been deleted. Other preferred immunogenic portions may contain a small N- and/or C-terminal deletion *(e.g.,* 1-30 amino acids, preferably 5-15 amino acids), relative to the mature protein.

Immunogenic portions may generally be identified using well known techniques, such as those summarized in Paul, Fundamental Immunology, 3rd ed., 243-247 (Raven Press, 1993) and references cited therein. Such techniques include screening polypeptides for the ability to react with antigen-specific antibodies, antisera and/or T-cell lines or clones. As used herein, antisera and antibodies are "antigen-specific" if they specifically bind to an antigen (*i.e.,* they react with the protein in an ELISA or other immunoassay, and do not react detectably with unrelated proteins). Such antisera and antibodies may be prepared as described herein, and using well known techniques. An immunogenic portion of a native lung tumor protein is a portion that reacts with such antisera and/or T-cells at a level that is not substantially less than the reactivity of the full length polypeptide (e.g., in an ELISA and/or T-cell reactivity assay). Such immunogenic portions may react within such assays at a level that is similar to or greater than the reactivity of the full length polypeptide. Such screens may generally be performed using methods well known to those of ordinary skill in the art, such as those described in Harlow and Lane, Antibodies: A Laboratory Manual, Cold Spring Harbor Laboratory, 1988. For example, a polypeptide may be immobilized on a solid support and contacted with patient sera to allow binding of antibodies within the sera to the immobilized polypeptide. Unbound sera may then be removed and bound antibodies detected using, for example, ¹²⁵I-labeled Protein A.

As noted above, a composition may comprise a variant of a native lung tumor protein. A polypeptide "variant," as used herein, is a polypeptide that differs from a native lung tumor protein in one or more substitutions, deletions, additions and/or insertions, such that the immunogenicity of the polypeptide is not substantially diminished. In other words, the ability of a variant to react with antigen-specific antisera may be enhanced or unchanged, relative to the native protein, or may be diminished by less than 50%, and preferably less than 20%, relative to the native protein. Such variants may generally be identified by modifying one of the above polypeptide sequences and evaluating the reactivity of the modified polypeptide with antigen-specific antibodies or antisera as described herein. Preferred variants include those in which one or more portions, such as an N-terminal leader sequence or transmembrane domain, have been removed. Other preferred variants include variants in which a small portion (e.g., 1-30 amino acids, preferably 5-15 amino acids) has been removed from the N- and/or C-terminal of the mature protein.

Polypeptide variants preferably exhibit at least about 70%, more preferably at least about 90% and most preferably at least about 95% identity (determined as described above) to the identified polypeptides.

Preferably, a variant contains conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. Amino acid substitutions may generally be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gIn, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide.

As noted above, polypeptides may comprise a signal (or leader) sequence at the N-terminal end of the protein which co-translationally or post-translationally directs transfer of the protein. The polypeptide may also be conjugated to a linker or other sequence for ease of synthesis, purification or identification of the polypeptide (*e.g.,* poly-His), or to enhance binding of the polypeptide to a solid support. For example, a polypeptide may be conjugated to an immunoglobulin Fc region.

Polypeptides may be prepared using any of a variety of well known techniques. Recombinant polypeptides encoded by DNA sequences as described above may be readily prepared from the DNA sequences using any of a variety of expression vectors known to those of ordinary skill in the art. Expression may be achieved in any appropriate host cell that has been transformed or transfected with an expression vector containing a DNA molecule that encodes a recombinant polypeptide. Suitable host cells include prokaryotes, yeast, higher eukaryotic and plant cells. Preferably, the host cells employed are *E. coli,* yeast or a mammalian cell line such as COS or CHO. Supernatants from suitable host/vector systems which secrete recombinant protein or polypeptide into culture media may be first concentrated using a commercially available filter. Following concentration, the concentrate may be applied to a suitable purification matrix such as an affinity matrix or an ion exchange resin. Finally, one or more reverse phase HPLC steps can be employed to further purify a recombinant polypeptide.

Portions and other variants having fewer than about 100 amino acids, and generally fewer than about 50 amino acids, may also be generated by synthetic means, using techniques well known to those of ordinary skill in the art. For example, such polypeptides may be synthesized using any of the commercially available solid-phase techniques, such as the Merrifield solid-phase synthesis method, where amino acids are sequentially added to a growing amino acid chain. *See* Merrifield, J. Am. Chem. Soc. 85:2149-2146, 1963. Equipment for automated synthesis of polypeptides is commercially available from suppliers such as Perkin Elmer/Applied BioSystems Division (Foster City, CA), and may be operated according to the manufacturer's instructions.

Within certain specific embodiments, a polypeptide may be a fusion protein that comprises multiple polypeptides as described herein, or that comprises at least one polypeptide as described herein and an unrelated sequence, such as a known tumor protein. A fusion partner may, for example, assist in providing T helper epitopes (an immunological fusion partner), preferably T helper epitopes recognized by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the protein or to enable the protein to be targeted to desired intracellular compartments. Still further fusion partners include affinity tags, which facilitate purification of the protein.

Fusion proteins may generally be prepared using standard techniques, including chemical conjugation. Preferably, a fusion protein is expressed as a recombinant protein, allowing the production of increased levels, relative to a non-fused protein, in an expression system. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion protein that retains the biological activity of both component polypeptides.

A peptide linker sequence may be employed to separate the first and the second polypeptide components by a distance sufficient to ensure that each polypeptide folds into its secondary and tertiary structures. Such a peptide linker sequence is incorporated into the fusion protein using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., Gene 40:39-46, 1985; Murphy et al., Proc. Natl. Acad. Sci. USA 83:8258-8262, 1986; U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

Fusion proteins are also provided that comprise a polypeptide of the present invention together with an unrelated immunogenic protein. Preferably the immunogenic protein is capable of eliciting a recall response. Examples of such proteins include tetanus, tuberculosis and hepatitis proteins (*see,* for example, Stoute et al. New Engl. J Med., 336:86-91, 1997).

Within preferred embodiments, an immunological fusion partner is derived from protein D, a surface protein of the gram-negative bacterium Haemophilus influenza B (WO 91/18926). Preferably, a protein D derivative comprises approximately the first third of the protein (e.g., the first N-terminal 100-110 amino acids), and a protein D derivative may be lipidated. Within certain preferred embodiments, the first 109 residues of a Lipoprotein D fusion partner is included on the N-terminus to provide the polypeptide with additional exogenous T-cell epitopes and to increase the expression level in *E. coli* (thus functioning as an expression enhancer). The lipid tail ensures optimal presentation of the antigen to antigen presenting cells. Other fusion partners include the non-structural protein from influenzae virus, NS1 (hemaglutinin). Typically, the N-terminal 81 amino acids are used, although different fragments that include T-heiper epitopes may be used.

In another embodiment, the immunological fusion partner is the protein known as LYTA, or a portion thereof (preferably a C-terminal portion). LYTA is derived from *Streptococcus pneumoniae,* which synthesizes an N-acetyl-L-alanine amidase known as amidase LYTA (encoded by the LytA gene; Gene 43:265-292, 1986). LYTA is an autolysin that specifically degrades certain bonds in the peptidoglycan backbone. The C-terminal domain of the LYTA protein is responsible for the affinity to the choline or to some choline analogues such as DEAE. This property has been exploited for the development of *E. coli* C-LYTA expressing plasmids useful for expression of fusion proteins. Purification of hybrid proteins containing the C-LYTA fragment at the amino terminus has been described *(see* Biotechnology 10:795-798, 1992). Within a preferred embodiment, a repeat portion of LYTA may be incorporated into a fusion protein. A repeat portion is found in the C-terminal region starting at residue 178. A particularly preferred repeat portion incorporates residues 188-305.

In general, polypeptides (including fusion proteins) and polynucleotides as described herein are isolated. An "isolated" polypeptide or polynucleotide is one that is removed from its original environment. For example, a naturally-occurring protein is isolated if it is separated from some or all of the coexisting materials in the natural system. Preferably, such polypeptides are at least about 90% pure, more preferably at least about 95% pure and most preferably at least about 99% pure. A polynucleotide is considered to be isolated if, for example, it is cloned into a vector that is not a part of the natural environment.

### BINDING AGENTS

The present invention further provides agents, such as antibodies and antigen-binding fragments thereof, that specifically bind to a lung tumor protein. As used herein, an antibody, or antigen-binding fragment thereof, is said to "specifically bind" to a lung tumor protein if it reacts at a detectable level (within, for example, an ELISA) with a lung tumor protein, and does not react detectably with unrelated proteins under similar conditions. As used herein, "binding" refers to a noncovalent association between two separate molecules such that a complex is formed. The ability to bind may be evaluated by, for example, determining a binding constant for the formation of the complex. The binding constant is the value obtained when the concentration of the complex is divided by the product of the component concentrations. In general, two compounds are said to "bind," in the context of the present invention, when the binding constant for complex formation exceeds about 10³ L/mol. The binding constant may be determined using methods well known in the art.

Binding agents may be further capable of differentiating between patients with and without a cancer, such as lung cancer, using the representative assays provided herein. In other words, antibodies or other binding agents that bind to a lung tumor protein will generate a signal indicating the presence of a cancer in at least about 20% of patients with the disease, and will generate a negative signal indicating the absence of the disease in at least about 90% of individuals without the cancer. To determine whether a binding agent satisfies this requirement, biological samples (e.g., blood, sera, sputum urine and/or tumor biopsies ) from patients with and without a cancer (as determined using standard clinical tests) may be assayed as described herein for the presence of polypeptides that bind to the binding agent. It will be apparent that a statistically significant number of samples with and without the disease should be assayed. Each binding agent should satisfy the above criteria; however, those of ordinary skill in the art will recognize that binding agents may be used in combination to improve sensitivity.

Any agent that satisfies the above requirements may be a binding agent. For example, a binding agent may be a ribosome, with or without a peptide component, an RNA molecule or a polypeptide. In a preferred embodiment, a binding agent is an antibody or an antigen-binding fragment thereof. Antibodies may be prepared by any of a variety of techniques known to those of ordinary skill in the art. *See, e.g.,* Harlow and Lane, Antibodies: A Laboratory Manual, Cold Spring Harbor Laboratory, 1988. In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising the polypeptide is initially injected into any of a wide variety of mammals (e.g., mice, rats, rabbits, sheep or goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically.

Polyclonal antibodies specific for the polypeptide may then be purified from such antisera by, for example, affinity chromatography using the polypeptide coupled to a suitable solid support.

Monoclonal antibodies specific for an antigenic polypeptide of interest may be prepared, for example, using the technique of Kohler and Milstein, Eur. J. Immunol. 6:511-519, 1976, and improvements thereto. Briefly, these methods involve the preparation of immortal cell lines capable of producing antibodies having the desired specificity (*i.e.,* reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

Within certain embodiments, the use of antigen-binding fragments of antibodies may be preferred. Such fragments include Fab fragments, which may be prepared using standard techniques. Briefly, immunoglobulins may be purified from rabbit serum by affinity chromatography on Protein A bead columns (Harlow and Lane, Antibodies: A Laboratory Manual, Cold Spring Harbor Laboratory, 1988) and digested by papain to yield Fab and Fc fragments. The Fab and Fc fragments may be separated by affinity chromatography on protein A bead columns.

Monoclonal antibodies of the present invention may be coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I, ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

A therapeutic agent may be coupled (e.g., covalently bonded) to a suitable monoclonal antibody either directly or indirectly (e.g., via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (*e.g*., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, *e.g.*, U.S. Patent No. 4,671,958, to Rodwell et al.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond (*e.g.,* U.S. Patent No. 4,489,710, to Spitler), by irradiation of a photolabile bond (*e*.*g*., U.S. Patent No. 4,625,014, to Senter et al.), by hydrolysis of derivatized amino acid side chains (*e*.*g*., U.S. Patent No. 4,638,045, to Kohn et al.), by serum complement-mediated hydrolysis (e.g., U.S. Patent No. 4,671,958, to Rodwell et al.), and acid-catalyzed hydrolysis (*e.g.,* U.S. Patent No. 4,569,789, to Blattler et al.).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers which provide multiple sites for attachment can be used. Alternatively, a carrier can be used.

A carrier may bear the agents in a variety of ways, including covalent bonding either directly or via a linker group. Suitable carriers include proteins such as albumins (e.g., U.S. Patent No. 4,507,234, to Kato et al.), peptides and polysaccharides such as aminodextran (e.g., U.S. Patent No. 4,699,784, to Shih et al.). A carrier may also bear an agent by noncovalent bonding or by encapsulation, such as within a liposome vesicle (*e.g*., U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al, discloses representative chelating compounds and their synthesis.

A variety of routes of administration for the antibodies and immunoconjugates may be used. Typically, administration will be intravenous, intramuscular, subcutaneous or in the bed of a resected tumor. It will be evident that the precise dose of the antibody/immunoconjugate will vary depending upon the antibody used, the antigen density on the tumor, and the rate of clearance of the antibody.

### T CELLS

Immunotherapeutic compositions may also, or alternatively, comprise T cells specific for a lung tumor protein. Such cells may generally be prepared *in vitro* or *ex vivo,* using standard procedures. For example, T cells may be isolated from bone marrow, peripheral blood, or a fraction of bone marrow or peripheral blood of a patient, using a commercially available cell separation system, such as the Isolex^{™} System, available from Nexell Therapeutics, Inc. Irvine, CA (see also U.S. Patent No. 5,240,856; U.S. Patent No. 5,215,926; WO 89/06280; WO 91/16116 and WO 92/07243). Alternatively, T cells may be derived from related or unrelated humans, non-human mammals, cell lines or cultures.

T cells may be stimulated with a lung tumor polypeptide, polynucleotide encoding a lung tumor polypeptide and/or an antigen presenting cell (APC) that expresses such a polypeptide. Such stimulation is performed under conditions and for a time sufficient to permit the generation of T cells that are specific for the polypeptide. Preferably, a lung tumor polypeptide or polynucleotide is present within a delivery vehicle, such as a microsphere, to facilitate the generation of specific T cells.

T cells are considered to be specific for a lung tumor polypeptide if the T cells specifically proliferate, secrete cytokines or kill target cells coated with the polypeptide or expressing a gene encoding the polypeptide. T cell specificity may be evaluated using any of a variety of standard techniques. For example, within a chromium release assay or proliferation assay, a stimulation index of more than two fold increase in lysis and/or proliferation, compared to negative controls, indicates T cell specificity. Such assays may be performed, for example, as described in Chen et al., Cancer Res. 54:1065-1070, 1994. Alternatively, detection of the proliferation of T cells may be accomplished by a variety of known techniques. For example, T cell proliferation can be detected by measuring an increased rate of DNA synthesis (*e.g.,* by pulse-labeling cultures of T cells with tritiated thymidine and measuring the amount of tritiated thymidine incorporated into DNA). Contact with a lung tumor polypeptide (100 ng/ml - 100 µg/ml, preferably 200 ng/ml - 25 µg/ml) for 3 - 7 days should result in at least a two fold increase in proliferation of the T cells. Contact as described above for 2-3 hours should result in activation of the T cells, as measured using standard cytokine assays in which a two fold increase in the level of cytokine release (e.g., TNF or IFN-γ) is indicative of T cell activation *(see* Coligan et al., Current Protocols in Immunology, vol. 1, Wiley Interscience (Greene 1998)). T cells that have been activated in response to a lung tumor polypeptide, polynucleotide or polypeptide-expressing APC may be CD4⁺ and/or CD8⁺. Lung tumor protein-specific T cells may be expanded using standard techniques. Within preferred embodiments, the T cells are derived from either a patient or a related, or unrelated, donor and are administered to the patient following stimulation and expansion.

For therapeutic purposes, CD4⁺ or CD8⁺ T cells that proliferate in response to a lung tumor polypeptide, polynucleotide or APC can be expanded in number either *in vitro* or *in vivo.* Proliferation of such T cells *in vitro* may be accomplished in a variety of ways. For example, the T cells can be re-exposed to a lung tumor polypeptide, or a short peptide corresponding to an immunogenic portion of such a polypeptide, with or without the addition of T cell growth factors, such as interleukin-2, and/or stimulator cells that synthesize a lung tumor polypeptide. Alternatively, one or more T cells that proliferate in the presence of a lung tumor protein can be expanded in number by cloning. Methods for cloning cells are well known in the art, and include limiting dilution.

### PHARMACEUTICAL COMPOSITIONS AND VACCINES

Within certain aspects, polypeptides, polynucleotides, T cells and/or binding agents disclosed herein may be incorporated into pharmaceutical compositions or immunogenic compositions *(i.e.,* vaccines). Pharmaceutical compositions comprise one or more such compounds and a physiologically acceptable carrier. Vaccines may comprise one or more such compounds and an immunostimulant. An immunostimulant may be any substance that enhances or potentiates an immune response to an exogenous antigen. Examples of immunostimulants include adjuvants, biodegradable microspheres (*e.g.,* polylactic galactide) and liposomes (into which the compound is incorporated; *see e.g.,* Fullerton, U.S. Patent No. 4,235,877). Vaccine preparation is generally described in, for example, M.F. Powell and M.J. Newman, eds., "Vaccine Design (the subunit and adjuvant approach)," Plenum Press (NY, 1995). Pharmaceutical compositions and vaccines within the scope of the present invention may also contain other compounds, which may be biologically active or inactive. For example, one or more immunogenic portions of other tumor antigens may be present, either incorporated into a fusion polypeptide or as a separate compound, within the composition or vaccine.

A pharmaceutical composition or vaccine may contain DNA encoding one or more of the polypeptides as described above, such that the polypeptide is generated *in situ.* As noted above, the DNA may be present within any of a variety of delivery systems known to those of ordinary skill in the art, including nucleic acid expression systems, bacteria and viral expression systems. Numerous gene delivery techniques are well known in the art, such as those described by Rolland, Crit. Rev. Therap. Drug Carrier Systems 15:143-198, 1998, and references cited therein. Appropriate nucleic acid expression systems contain the necessary DNA sequences for expression in the patient (such as a suitable promoter and terminating signal). Bacterial delivery systems involve the administration of a bacterium (such as *Bacillus-Cadmette-Guerrin)* that expresses an immunogenic portion of the polypeptide on its cell surface or secretes such an epitope. In a preferred embodiment, the DNA may be introduced using a viral expression system (e.g., vaccinia or other pox virus, retrovirus, or adenovirus), which may involve the use of a non-pathogenic (defective), replication competent virus. Suitable systems are disclosed, for example, in Fisher-Hoch et al., Proc. Natl. Acad. Sci. USA 86:317-321, 1989; Flexner et al., Ann. N.Y. Acad. Sci. 569:86-103, 1989; Flexner et al., Vaccine 8:17-21, 1990; U.S. Patent Nos. 4,603,112, 4,769,330, and 5,017,487; WO 89/01973; U.S. Patent No. 4,777,127; GB 2,200,651; EP 0,345,242; WO 91/02805; Berkner, Biotechniques 6:616-627, 1988; Rosenfeld et al., Science 252:431-434, 1991; Kolls et al., Proc. Natl. Acad. Sci. USA 91:215-219, 1994; Kass-Eisler et al., Proc. Natl. Acad. Sci. USA 90:11498-11502,1993; Guzman et al., Circulation 88:2838-2848, 1993; and Guzman et al., Cir. Res. 73:1202-1207, 1993. Techniques for incorporating DNA into such expression systems are well known to those of ordinary skill in the art. The DNA may also be "naked," as described, for example, in Ulmer et al., Science 259:1745-1749, 1993 and reviewed by Cohen, Science 259:1691-1692, 1993. The uptake of naked DNA may be increased by coating the DNA onto biodegradable beads, which are efficiently transported into the cells.

While any suitable carrier known to those of ordinary skill in the art may be employed in the pharmaceutical compositions of this invention, the type of carrier will vary depending on the mode of administration. Compositions of the present invention may be formulated for any appropriate manner of administration, including for example, topical, oral, nasal, intravenous, intracranial, intraperitoneal, subcutaneous or intramuscular administration. For parenteral administration, such as subcutaneous injection, the carrier preferably comprises water, saline, alcohol, a fat, a wax or a buffer. For oral administration, any of the above carriers or a solid carrier, such as mannitol, lactose, starch, magnesium stearate, sodium saccharine, talcum, cellulose, glucose, sucrose, and magnesium carbonate, may be employed. Biodegradable microspheres (*e.g.*, polylactate polyglycolate) may also be employed as carriers for the pharmaceutical compositions of this invention. Suitable biodegradable microspheres are disclosed, for example, in U.S. Patent Nos. 4,897,268 and 5,075,109.

Such compositions may also comprise buffers (e.g., neutral buffered saline or phosphate buffered saline), carbohydrates (e.g., glucose, mannose, sucrose or dextrans), mannitol, proteins, polypeptides or amino acids such as glycine, antioxidants, chelating agents such as EDTA or glutathione, adjuvants (e.g., aluminum hydroxide) and/or preservatives. Alternatively, compositions of the present invention may be formulated as a lyophilizate. Compounds may also be encapsulated within liposomes using well known technology.

Any of a variety of immunostimulants may be employed in the vaccines of this invention. For example, an adjuvant may be included. Most adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Suitable adjuvants are commercially available as, for example, Freund's Incomplete Adjuvant and Complete Adjuvant (Difco Laboratories, Detroit, MI); Merck Adjuvant 65 (Merck and Company, Inc., Rahway, NJ); AS-2 (SmithKline Beecham, Philadelphia, PA); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF or interleukin-2, -7, or -12, may also be used as adjuvants.

Within the vaccines provided herein, the adjuvant composition is preferably designed to induce an immune response predominantly of the Th1 type. High levels of Th1-type cytokines (e.g., IFN-γ, TNFα, IL-2 and IL-12) tend to favor the induction of cell mediated immune responses to an administered antigen. In contrast, high levels of Th2-type cytokines (e.g., IL-4, IL-5, IL-6 and IL-10) tend to favor the induction of humoral immune responses. Following application of a vaccine as provided herein, a patient will support an immune response that includes Th1- and Th2-type responses. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, Ann. Rev. Immunol. 7:145-173,1989.

Preferred adjuvants for use in eliciting a predominantly Th1-type response include, for example, a combination of monophosphoryl lipid A, preferably 3-de-O-acylated monophosphoryl lipid A (3D-MPL), together with an aluminum salt. MPL adjuvants are available from Ribi ImmunoChem Research Inc. (Hamilton, MT) *(see* US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555 and WO 99/33488. Immunostimulatory DNA sequences are also described, for example, by Sato et al., Science 273:352, 1996. Another preferred adjuvant is a saponin, preferably QS21 (Aquila Biopharmaceuticals Inc., Framingham, MA), which may be used alone or in combination with other adjuvants. For example, an enhanced system involves the combination of a monophosphoryl lipid A and saponin derivative, such as the combination of QS21 and 3D-MPL as described in WO 94/00153, or a less reactogenic composition where the QS21 is quenched with cholesterol, as described in WO 96/33739. Other preferred formulations comprises an oil-in-water emulsion and tocopherol. A particularly potent adjuvant formulation involving QS21, 3D-MPL and tocopherol in an oil-in-water emulsion is described in WO 95/17210.

Other preferred adjuvants include Montanide ISA 720 (Seppic, France), SAF (Chiron, California, United States), ISCOMS (CSL), MF-59 (Chiron), the SBAS series of adjuvants (e.g., SBAS-2 or SBAS-4, available from SmithKline Beecham, Rixensart, Belgium), Detox (Ribi ImmunoChem Research Inc., Hamilton, MT), RC-529 (Ribi ImmunoChem Research Inc., Hamilton, MT) and Aminoalkyl glucosaminide 4-phosphates (AGPs).

Any vaccine provided herein may be prepared using well known methods that result in a combination of antigen, immune response enhancer and a suitable carrier or excipient. The compositions described herein may be administered as part of a sustained release formulation (*i.e.*, a formulation such as a capsule, sponge or gel (composed of polysaccharides, for example) that effects a slow release of compound following administration). Such formulations may generally be prepared using well known technology (*see, e.g.* Coombes et al., Vaccine 14:1429-1438, 1996) and administered by, for example, oral, rectal or subcutaneous implantation, or by implantation at the desired target site. Sustained-release formulations may contain a polypeptide, polynucleotide or antibody dispersed in a carrier matrix and/or contained within a reservoir surrounded by a rate controlling membrane.

Carriers for use within such formulations are biocompatible, and may also be biodegradable; preferably the formulation provides a relatively constant level of active component release. Such carriers include microparticles of poly(lactide-co-glycolide), as well as polyacrylate, latex, starch, cellulose and dextran. Other delayed-release carriers include supramolecular biovectors, which comprise a non-liquid hydrophilic core (*e.g.,* a cross-linked polysaccharide or oligosaccharide) and, optionally, an external layer comprising an amphiphilic compound, such as a phospholipid (*see e.g.,* U.S. Patent No. 5,151,254 and PCT applications WO 94/20078, WO/94/23701 and WO 96/06638). The amount of active compound contained within a sustained release formulation depends upon the site of implantation, the rate and expected duration of release and the nature of the condition to be treated or prevented.

Any of a variety of delivery vehicles may be employed within pharmaceutical compositions and vaccines to facilitate production of an antigen-specific immune response that targets tumor cells. Delivery vehicles include antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-tumor effects *per se* and/or to be immunologically compatible with the receiver (*i*.*e*., matched HLA haplotype). APCs may generally be isolated from any of a variety of biological fluids and organs, including tumor and peritumoral tissues, and may be autologous, allogeneic, syngeneic or xenogeneic cells.

Certain preferred embodiments of the present invention use dendritic cells or progenitors thereof as antigen-presenting cells. Dendritic cells are highly potent APCs (Banchereau and Steinman, Nature 392:245-251, 1998) and have been shown to be effective as a physiological adjuvant for eliciting prophylactic or therapeutic antitumor immunity *(see* Timmerman and Levy, Ann. Rev. Med. 50:507-529, 1999). In general, dendritic cells may be identified based on their typical shape (stellate *in situ,* with marked cytoplasmic processes (dendrites) visible *in vitro*), their ability to take up, process and present antigens with high efficiency, and their ability to activate naïve T cell responses. Dendritic cells may, of course, be engineered to express specific cell-surface receptors or ligands that are not commonly found on dendritic cells *in vivo* or *ex vivo,* and such modified dendritic cells are contemplated by the present invention. As an alternative to dendritic cells, secreted vesicles antigen-loaded dendritic cells (called exosomes) may be used within a vaccine (*see* Zitvogel et al., Nature Med 4:594-600, 1998).

Dendritic cells and progenitors may be obtained from peripheral blood, bone marrow, tumor-infiltrating cells, peritumoral tissues-infiltrating cells, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNFα to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand and/or other compound(s) that induce differentiation, maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fcγ receptor and mannose receptor. The mature phenotype is typically characterized by a lower expression of these markers, but a high expression of cell surface molecules responsible for T cell activation such as class I and class II MHC, adhesion molecules (*e.g.,* CD54 and CD11) and costimulatory molecules (*e.g.,* CD40, CD80, CD86 and 4-1BB).

APCs may generally be transfected with a polynucleotide encoding a lung tumor protein (or portion or other variant thereof) such that the lung tumor polypeptide, or an immunogenic portion thereof, is expressed on the cell surface. Such transfection may take place *ex vivo,* and a composition or vaccine comprising such transfected cells may then be used for therapeutic purposes, as described herein. Alternatively, a gene delivery vehicle that targets a dendritic or other antigen presenting cell may be administered to a patient, resulting in transfection that occurs *in vivo. In vivo* and *ex vivo* transfection of dendritic cells, for example, may generally be performed using any methods known in the art, such as those described in WO 97/24447, or the gene gun approach described by Mahvi et al., Immunology and cell Biology 75:456-460, 1997. Antigen loading of dendritic cells may be achieved by incubating dendritic cells or progenitor cells with the lung tumor polypeptide, DNA (naked or within a plasmid vector) or RNA; or with antigen-expressing recombinant bacterium or viruses (e.g., vaccinia, fowlpox, adenovirus or lentivirus vectors). Prior to loading, the polypeptide may be covalently conjugated to an immunological partner that provides T cell help *(e.g.,* a carrier molecule). Alternatively, a dendritic cell may be pulsed with a non-conjugated immunological partner, separately or in the presence of the polypeptide.

Vaccines and pharmaceutical compositions may be presented in unit-dose or multi-dose containers, such as sealed ampoules or vials. Such containers are preferably hermetically sealed to preserve sterility of the formulation until use. In general, formulations may be stored as suspensions, solutions or emulsions in oily or aqueous vehicles. Alternatively, a vaccine or pharmaceutical composition may be stored in a freeze-dried condition requiring only the addition of a sterile liquid carrier immediately prior to use.

### CANCER THERAPY

In further aspects of the present invention, the compositions described herein may be used for immunotherapy of cancer, such as lung cancer. Within such methods, pharmaceutical compositions and vaccines are typically administered to a patient. As used herein, a "patient" refers to any warm-blooded animal, preferably a human. A patient may or may not be afflicted with cancer. Accordingly, the above pharmaceutical compositions and vaccines may be used to prevent the development of a cancer or to treat a patient afflicted with a cancer. A cancer may be diagnosed using criteria generally accepted in the art, including the presence of a malignant tumor. Pharmaceutical compositions and vaccines may be administered either prior to or following surgical removal of primary tumors and/or treatment such as administration of radiotherapy or conventional chemotherapeutic drugs.

Within certain embodiments, immunotherapy may be active immunotherapy, in which treatment relies on the *in vivo* stimulation of the endogenous host immune system to react against tumors with the administration of immune response-modifying agents (such as polypeptides and polynucleotides disclosed herein).

Within other embodiments, immunotherapy may be passive immunotherapy, in which treatment involves the delivery of agents with established tumor-immune reactivity (such as effector cells or antibodies) that can directly or indirectly mediate antitumor effects and does not necessarily depend on an intact host immune system. Examples of effector cells include T cells as discussed above, T lymphocytes (such as CD8⁺ cytotoxic T lymphocytes and CD4⁺ T-helper tumor-infiltrating lymphocytes), killer cells (such as Natural Killer cells and lymphokine-activated killer cells), B cells and antigen-presenting cells (such as dendritic cells and macrophages) expressing a polypeptide provided herein. T cell receptors and antibody receptors specific for the polypeptides recited herein may be cloned, expressed and transferred into other vectors or effector cells for adoptive immunotherapy. The polypeptides provided herein may also be used to generate antibodies or anti-idiotypic antibodies (as described above and in U.S. Patent No. 4,918,164) for passive immunotherapy.

Effector cells may generally be obtained in sufficient quantities for adoptive immunotherapy by growth *in vitro,* as described herein. Culture conditions for expanding single antigen-specific effector cells to several billion in number with retention of antigen recognition *in vivo* are well known in the art. Such *in vitro* culture conditions typically use intermittent stimulation with antigen, often in the presence of cytokines (such as IL-2) and non-dividing feeder cells. As noted above, immunoreactive polypeptides as provided herein may be used to rapidly expand antigen-specific T cell cultures in order to generate a sufficient number of cells for immunotherapy. In particular, antigen-presenting cells, such as dendritic, macrophage, monocyte, fibroblast and/or B cells, may be pulsed with immunoreactive polypeptides or transfected with one or more polynucleotides using standard techniques well known in the art. For example, antigen-presenting cells can be transfected with a polynucleotide having a promoter appropriate for increasing expression in a recombinant virus or other expression system. Cultured effector cells for use in therapy must be able to grow and distribute widely, and to survive long term *in vivo.* Studies have shown that cultured effector cells can be induced to grow in vivo and to survive long term in substantial numbers by repeated stimulation with antigen supplemented with IL-2 *(see,* for example, Cheever et al., Immunological Reviews 157:177, 1997).

Alternatively, a vector expressing a polypeptide recited herein may be introduced into antigen presenting cells taken from a patient and clonally propagated *ex vivo* for transplant back into the same patient. Transfected cells may be reintroduced into the patient using any means known in the art, preferably in sterile form by intravenous, intracavitary, intraperitoneal or intratumor administration.

Routes and frequency of administration of the therapeutic compositions disclosed herein, as well as dosage, will vary from individual to individual, and may be readily established using standard techniques. In general, the pharmaceutical compositions and vaccines may be administered by injection (*e*.*g*., intracutaneous, intramuscular, intravenous or subcutaneous), intranasally (e.g., by aspiration) or orally. Preferably, between 1 and 10 doses may be administered over a 52 week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response, and is at least 10-50% above the basal (*i*.*e*., untreated) level. Such response can be monitored by measuring the anti-tumor antibodies in a patient or by vaccine-dependent generation of cytolytic effector cells capable of killing the patient's tumor cells *in vitro.* Such vaccines should also be capable of causing an immune response that leads to an improved clinical outcome (e.g., more frequent remissions, complete or partial or longer disease-free survival) in vaccinated patients as compared to non-vaccinated patients. In general, for pharmaceutical compositions and vaccines comprising one or more polypeptides, the amount of each polypeptide present in a dose ranges from about 25 µg to 5 mg per kg of host. Suitable dose sizes will vary with the size of the patient, but will typically range from about 0.1 mL to about 5 mL.

In general, an appropriate dosage and treatment regimen provides the active compound(s) in an amount sufficient to provide therapeutic and/or prophylactic benefit. Such a response can be monitored by establishing an improved clinical outcome (*e.g.*, more frequent remissions, complete or partial, or longer disease-free survival) in treated patients as compared to non-treated patients. Increases in preexisting immune responses to a lung tumor protein generally correlate with an improved clinical outcome. Such immune responses may generally be evaluated using standard proliferation, cytotoxicity or cytokine assays, which may be performed using samples obtained from a patient before and after treatment.

### METHODS FOR DETECTING CANCER

In general, a cancer may be detected in a patient based on the presence of one or more lung tumor proteins and/or polynucleotides encoding such proteins in a biological sample (for example, blood, sera, sputum urine and/or tumor biopsies) obtained from the patient. In other words, such proteins may be used as markers to indicate the presence or absence of a cancer such as lung cancer. In addition, such proteins may be useful for the detection of other cancers. The binding agents provided herein generally permit detection of the level of antigen that binds to the agent in the biological sample. Polynucleotide primers and probes may be used to detect the level of mRNA encoding a tumor protein, which is also indicative of the presence or absence of a cancer. In general, a lung tumor sequence should be present at a level that is at least three fold higher in tumor tissue than in normal tissue

There are a variety of assay formats known to those of ordinary skill in the art for using a binding agent to detect polypeptide markers in a sample. *See, e.g.,* Harlow and Lane, Antibodies: A Laboratory Manual, Cold Spring Harbor Laboratory, 1988. In general, the presence or absence of a cancer in a patient may be determined by (a) contacting a biological sample obtained from a patient with a binding agent; (b) detecting in the sample a level of polypeptide that binds to the binding agent; and (c) comparing the level of polypeptide with a predetermined cut-off value.

In a preferred embodiment, the assay involves the use of binding agent immobilized on a solid support to bind to and remove the polypeptide from the remainder of the sample. The bound polypeptide may then be detected using a detection reagent that contains a reporter group and specifically binds to the binding agent/polypeptide complex. Such detection reagents may comprise, for example, a binding agent that specifically binds to the polypeptide or an antibody or other agent that specifically binds to the binding agent, such as an anti-immunoglobulin, protein G, protein A or a lectin. Alternatively, a competitive assay may be utilized, in which a polypeptide is labeled with a reporter group and allowed to bind to the immobilized binding agent after incubation of the binding agent with the sample. The extent to which components of the sample inhibit the binding of the labeled polypeptide to the binding agent is indicative of the reactivity of the sample with the immobilized binding agent. Suitable polypeptides for use within such assays include full length lung tumor proteins and portions thereof to which the binding agent binds, as described above.

The solid support may be any material known to those of ordinary skill in the art to which the tumor protein may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The binding agent may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the agent and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of binding agent ranging from about 10 ng to about 10 µg, and preferably about 100 ng to about 1 µg, is sufficient to immobilize an adequate amount of binding agent.

Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner (*see, e.g.,* Pierce Immunotechnology Catalog and Handbook, 1991, at A12-A13).

In certain embodiments, the assay is a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a detection reagent (preferably a second antibody capable of binding to a different site on the polypeptide) containing a reporter group is added. The amount of detection reagent that remains bound to the solid support is then determined using a method appropriate for the specific reporter group.

More specifically, once the antibody is immobilized on the support as described above, the remaining protein binding sites on the support are typically blocked. Any suitable blocking agent known to those of ordinary skill in the art, such as bovine serum albumin or Tween 20^{™} (Sigma Chemical Co., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (*i*.*e*., incubation time) is a period of time that is sufficient to detect the presence of polypeptide within a sample obtained from an individual with lung cancer. Preferably, the contact time is sufficient to achieve a level of binding that is at least about 95% of that achieved at equilibrium between bound and unbound polypeptide. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20^{™}. The second antibody, which contains a reporter group, may then be added to the solid support. Preferred reporter groups include those groups recited above.

The detection reagent is then incubated with the immobilized antibody-polypeptide complex for an amount of time sufficient to detect the bound polypeptide. An appropriate amount of time may generally be determined by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of a cancer, such as lung cancer, the signal detected from the reporter group that remains bound to the solid support is generally compared to a signal that corresponds to a predetermined cut-off value. In one preferred embodiment, the cut-off value for the detection of a cancer is the average mean signal obtained when the immobilized antibody is incubated with samples from patients without the cancer. In general, a sample generating a signal that is three standard deviations above the predetermined cut-off value is considered positive for the cancer. In an alternate preferred embodiment, the cut-off value is determined using a Receiver Operator Curve, according to the method of Sackett et al., Clinical Epidemiology: A Basic Science for Clinical Medicine, Little Brown and Co., 1985, p. 106-7. Briefly, in this embodiment, the cut-off value may be determined from a plot of pairs of true positive rates (*i.e.,* sensitivity) and false positive rates (100%-speciflcity) that correspond to each possible cut-off value for the diagnostic test result. The cut-off value on the plot that is the closest to the upper left-hand corner (*i*.*e*., the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for a cancer.

In a related embodiment, the assay is performed in a flow-through or strip test format, wherein the binding agent is immobilized on a membrane, such as nitrocellulose. In the flow-through test, polypeptides within the sample bind to the immobilized binding agent as the sample passes through the membrane. A second, labeled binding agent then binds to the binding agent-polypeptide complex as a solution containing the second binding agent flows through the membrane. The detection of bound second binding agent may then be performed as described above. In the strip test format, one end of the membrane to which binding agent is bound is immersed in a solution containing the sample. The sample migrates along the membrane through a region containing second binding agent and to the area of immobilized binding agent. Concentration of second binding agent at the area of immobilized antibody indicates the presence of a cancer. Typically, the concentration of second binding agent at that site generates a pattern, such as a line, that can be read visually. The absence of such a pattern indicates a negative result. In general, the amount of binding agent immobilized on the membrane is selected to generate a visually discernible pattern when the biological sample contains a level of polypeptide that would be sufficient to generate a positive signal in the two-antibody sandwich assay, in the format discussed above. Preferred binding agents for use in such assays are antibodies and antigen-binding fragments thereof. Preferably, the amount of antibody immobilized on the membrane ranges from about 25 ng to about 1µg, and more preferably from about 50 ng to about 500 ng. Such tests can typically be performed with a very small amount of biological sample.

Of course, numerous other assay protocols exist that are suitable for use with the tumor proteins or binding agents of the present invention. The above descriptions are intended to be exemplary only. For example, it will be apparent to those of ordinary skill in the art that the above protocols may be readily modified to use lung tumor polypeptides to detect antibodies that bind to such polypeptides in a biological sample. The detection of such lung tumor protein specific antibodies may correlate with the presence of a cancer.

A cancer may also, or alternatively, be detected based on the presence of T cells that specifically react with a lung tumor protein in a biological sample. Within certain methods, a biological sample comprising CD4⁺ and/or CD8⁺ T cells isolated from a patient is incubated with a lung tumor polypeptide, a polynucleotide encoding such a polypeptide and/or an APC that expresses at least an immunogenic portion of such a polypeptide, and the presence or absence of specific activation of the T cells is detected. Suitable biological samples include, but are not limited to, isolated T cells. For example, T cells may be isolated from a patient by routine techniques (such as by Ficoll/Hypaque density gradient centrifugation of peripheral blood lymphocytes). T cells may be incubated *in vitro* for 2-9 days (typically 4 days) at 37°C with polypeptide (*e.g.,* 5 - 25 µg/ml), It may be desirable to incubate another aliquot af a T cell sample in the absence of lung tumor polypeptide to serve as a control. For CD4⁺ T cells, activation is preferably detected by evaluating proliferation of the T cells. For CD8⁺ T cells, activation is preferably detected by evaluating cytolytic activity. A level of proliferation that is at least two fold greater and/or a level of cytolytic activity that is at least 20% greater than in disease-free patients indicates the presence of a cancer in the patient.

As noted above, a cancer may also, or alternatively, be detected based on the level of mRNA encoding a lung tumor protein in a biological sample. For example, at least two oligonucleotide primers may be employed in a polymerase chain reaction (PCR) based assay to amplify a portion of a lung tumor cDNA derived from a biological sample, wherein at least one of the oligonucleotide primers is specific for (*i.e.*, hybridizes to) a polynucleotide encoding the lung tumor protein. The amplified cDNA is then separated and detected using techniques well known in the art, such as gel electrophoresis. Similarly, oligonucleotide probes that specifically hybridize to a polynucleotide encoding a lung tumor protein may be used in a hybridization assay to detect the presence of polynucleotide encoding the tumor protein in a biological sample.

To permit hybridization under assay conditions, oligonucleotide primers and probes should comprise an oligonucleotide sequence that has at least about 60%, preferably at least about 75% and more preferably at least about 90%, identity to a portion of a polynucleotide encoding a lung tumor protein that is at least 10 nucleotides, and preferably at least 20 nucleotides, in length. Preferably, oligonucleotide primers and/or probes will hybridize to a polynucleotide encoding a polypeptide disclosed herein under moderately stringent conditions, as defined above. Oligonucleotide primers and/or probes which may be usefully employed in the diagnostic methods described herein preferably are at least 10-40 nucleotides in length. In a preferred embodiment, the oligonucleotide primers comprise at least 10 contiguous nucleotides, more preferably at least 15 contiguous nucleotides, of a DNA molecule having a sequence recited in SEQ ID NO: 1-109, 111, 113, 115-151, 153, 154, 157, 158, 160, 162-164, 167, 168, 171, 173, 175, 177-224, 255-337, 345, 347 and 349. Techniques for both PCR based assays and hybridization assays are well known in the art *(see,* for example, Mullis et al., Cold Spring Harbor Symp. Quant. Biol., 51:263, 1987; Erlich ed., PCR Technology, Stockton Press, NY, 1989).

One preferred assay employs RT-PCR, in which PCR is applied in conjunction with reverse transcription. Typically, RNA is extracted from a biological sample, such as biopsy tissue, and is reverse transcribed to produce cDNA molecules. PCR amplification using at least one specific primer generates a cDNA molecule, which may be separated and visualized using, for example, gel electrophoresis. Amplification may be performed on biological samples taken from a test patient and from an individual who is not afflicted with a cancer. The amplification reaction may be performed on several dilutions of cDNA spanning two orders of magnitude. A two-fold or greater increase in expression in several dilutions of the test patient sample as compared to the same dilutions of the non-cancerous sample is typically considered positive.

In another embodiment, the disclosed compositions may be used as markers for the progression of cancer. In this embodiment, assays as described above for the diagnosis of a cancer may be performed over time, and the change in the level of reactive polypeptide(s) or polynucleotide evaluated. For example, the assays may be performed every 24-72 hours for a period of 6 months to 1 year, and thereafter performed as needed. In general, a cancer is progressing in those patients in whom the level of polypeptide or polynucleotide detected increases over time. In contrast, the cancer is not progressing when the level of reactive polypeptide or polynucleotide either remains constant or decreases with time.

Certain *in vivo* diagnostic assays may be performed directly on a tumor. One such assay involves contacting tumor cells with a binding agent. The bound binding agent may then be detected directly or indirectly via a reporter group. Such binding agents may also be used in histological applications. Alternatively, polynucleotide probes may be used within such applications.

As noted above, to improve sensitivity, multiple lung tumor protein markers may be assayed within a given sample. It will be apparent that binding agents specific for different proteins provided herein may be combined within a single assay. Further, multiple primers or probes may be used concurrently. The selection of tumor protein markers may be based on routine experiments to determine combinations that results in optimal sensitivity. In addition, or alternatively, assays for tumor proteins provided herein may be combined with assays for other known tumor antigens.

### DIAGNOSTIC KITS

The present invention further provides kits for use within any of the above diagnostic methods. Such kits typically comprise two or more components necessary for performing a diagnostic assay. Components may be compounds, reagents, containers and/or equipment. For example, one container within a kit may contain a monoclonal antibody or fragment thereof that specifically binds to a lung tumor protein. Such antibodies or fragments may be provided attached to a support material, as described above. One or more additional containers may enclose elements, such as reagents or buffers, to be used in the assay. Such kits may also, or alternatively, contain a detection reagent as described above that contains a reporter group suitable for direct or indirect detection of antibody binding.

Alternatively, a kit may be designed to detect the level of mRNA encoding a lung tumor protein in a biological sample. Such kits generally comprise at least one oligonucleotide probe or primer, as described above, that hybridizes to a polynucleotide encoding a lung tumor protein. Such an oligonucleotide may be used, for example, within a PCR or hybridization assay. Additional components that may be present within such kits include a second oligonucleotide and/or a diagnostic reagent or container to facilitate the detection of a polynucleotide encoding a lung tumor protein.

The following Examples are offered by way of illustration and not by way of limitation.

### EXAMPLE 1

### ISOLATION AND CHARACTERIZATION OF cDNA SEQUENCES ENCODING LUNG TUMOR POLYPEPTIDES

This example illustrates the isolation of cDNA molecules encoding lung tumor-specific polypeptides from lung tumor cDNA libraries.

### A. ISOLATION OF cDNA SEQUENCES FROM A LUNG SQUAMOUS CELL CARCINOMA LIBRARY

A human lung squamous cell carcinoma cDNA expression library was constructed from poly A⁺ RNA from a pool of two patient tissues using a Superscript Plasmid System for cDNA Synthesis and Plasmid Cloning kit (BRL Life Technologies, Gaithersburg, MD) following the manufacturer's protocol. Specifically, lung carcinoma tissues were homogenized with polytron (Kinematica, Switzerland) and total RNA was extracted using Trizol reagent (BRL Life Technologies) as directed by the manufacturer. The poly A⁺ RNA was then purified using an oligo dT cellulose column as described in Sambrook et al., Molecular Cloning: A Laboratory Manual, Cold Spring Harbor Laboratories, Cold Spring Harbor, NY, 1989. First-strand cDNA was synthesized using the NotI/Oligo-dT18 primer. Double-stranded cDNA was synthesized, ligated with BstXI/EcoRI adaptors (Invitrogen, San Diego, CA) and digested with NotI. Following size fractionation with cDNA size fractionation columns (BRL Life Technologies), the cDNA was ligated into the BstXI/NotI site of pcDNA3.1 (Invitrogen) and transformed into ElectroMax *E. coli* DH10B cells (BRL Life Technologies) by electroporation.

Using the same procedure, a normal human lung cDNA expression library was prepared from a pool of four tissue specimens. The cDNA libraries were characterized by determining the number of independent colonies, the percentage of clones that carried insert, the average insert size and by sequence analysis. The lung squamous cell carcinoma library contained 2.7 x 10⁶ independent colonies, with 100% of clones having an insert and the average insert size being 2100 base pairs. The normal lung cDNA library contained 1.4 x 10⁶ independent colonies, with 90% of clones having inserts and the average insert size being 1800 base pairs. For both libraries, sequence analysis showed that the majority of clones had a full length cDNA sequence and were synthesized from mRNA

cDNA library subtraction was performed using the above lung squamous cell carcinoma and normal lung cDNA libraries, as described by Hara et al. (Blood, 84:189-199, 1994) with some modifications. Specifically, a lung squamous cell carcinoma-specific subtracted cDNA library was generated as follows. Normal tissue cDNA library (80 µg) was digested with BamHI and XhoI, followed by a filling-in reaction with DNA polymerase Klenow fragment. After phenol-chloroform extraction and ethanol precipitation, the DNA was dissolved in 133 µl of H₂O, heat-denatured and mixed with 133 µl (133 µg) of Photoprobe biotin (Vector Laboratories, Burlingame, CA). As recommended by the manufacturer, the resulting mixture was irradiated with a 270 W sunlamp on ice for 20 minutes. Additional Photoprobe biotin (67 µl) was added and the biotinylation reaction was repeated. After extraction with butanol five times, the DNA was ethanol-precipitated and dissolved in 23 µl H₂O to form the driver DNA.

To form the tracer DNA, 10 µg lung squamous cell carcinoma cDNA library was digested with NotI and SpeI, phenol chloroform extracted and passed through Chroma spin-400 columns (Clontech, Palo Alto, CA). Typically, 5 µg of cDNA was recovered after the sizing column. Following ethanol precipitation, the tracer DNA was dissolved in 5 µl H₂O. Tracer DNA was mixed with 15 µl driver DNA and 20 µl of 2 x hybridization buffer (1.5 M NaCl/10 mM EDTA/50 mM HEPES pH 7.5/0.2% sodium dodecyl sulfate), overlaid with mineral oil, and heat-denatured completely. The sample was immediately transferred into a 68 °C water bath and incubated for 20 hours (long hybridization [LH]). The reaction mixture was then subjected to a streptavidin treatment followed by phenol/chloroform extraction. This process was repeated three more times. Subtracted DNA was precipitated, dissolved in 12 µl H₂O, mixed with 8 µl driver DNA and 20 µl of 2 x hybridization buffer, and subjected to a hybridization at 68 °C for 2 hours (short hybridization [SH]). After removal of biotinylated double-stranded DNA, subtracted cDNA was ligated into NotI/Spel site of chloramphenicol resistant pBCSK⁺ (Stratagene, La Jolla, CA) and transformed into ElectroMax *E. coli* DH10B cells by electroporation to generate a lung squamous cell carcinoma specific subtracted cDNA library (herein after referred to as "lung subtraction I").

A second lung squamous cell carcinoma specific subtracted cDNA library (referred to as "lung subtraction II") was generated in a similar way to the lung subtraction library I, except that eight frequently recovered genes from lung subtraction I were included in the driver DNA, and 24,000 independent clones were recovered.

To analyze the subtracted cDNA libraries, plasmid DNA was prepared from 320 independent clones, randomly picked from the subtracted lung squamous cell carcinoma specific libraries. Representative cDNA clones were further characterized by DNA sequencing with a Perkin Elmer/Applied Biosystems Division Automated Sequencer Model 373A and/or Model 377 (Foster City, CA). The cDNA sequences for sixty isolated clones are provided in SEQ ID NO: 1-60. These sequences were compared to known sequences in the gene bank using the EMBL and GenBank databases (release 96). No significant homologies were found to the sequences provided in SEQ ID NO: 2, 3, 19, 38 and 46. The sequences of SEQ ID NO: 1, 6-8, 10-13, 15, 17, 18, 20-27, 29, 30, 32, 34-37, 39-45, 47-49, 51, 52, 54, 55 and 57-59 were found to show some homology to previously identified expressed sequence tags (ESTs). The sequences of SEQ ID NO: 9, 28, 31 and 33 were found to show some homology to previously identified non-human gene sequences and the sequences of SEQ ID NO: 4, 5, 14, 50, 53, 56 and 60 were found to show some homology to gene sequences previously identified in humans.

The subtraction procedure described above was repeated using the above lung squamous cell carcinoma cDNA library as the tracer DNA, and the above normal lung tissue cDNA library and a cDNA library from normal liver and heart (constructed from a pool of one sample of each tissue as described above), plus twenty other cDNA clones that were frequently recovered in lung subtractions I and II, as the driver DNA (lung subtraction III). The normal liver and heart cDNA library contained 1.76 x 10⁶ independent colonies, with 100% of clones having inserts and the average insert size being 1600 base pairs. Ten additional clones were isolated (SEQ ID NO: 61-70). Comparison of these cDNA sequences with those in the gene bank as described above, revealed no significant homologies to the sequences provided in SEQ ID NO: 62 and 67. The sequences of SEQ ID NO: 61, 63-66, 68 and 69 were found to show some homology to previously isolated ESTs and the sequence provided in SEQ ID NO: 70 was found to show some homology to a previously identified rat gene.

In further studies, the subtraction procedure described above was repeated using the above lung squamous cell carcinoma cDNA library as the tracer DNA, and a cDNA library from a pool of normal lung, kidney, colon, pancreas, brain, resting PBMC, heart, skin and esophagus as the driver DNA, with esophagus cDNAs making up one third of the driver material. Since esophagus is enriched in normal epithelial cells, including differentiated squamous cells, this procedure is likely to enrich genes that are tumor specific rather than tissues specific. The cDNA sequences of 48 clones determined in this subtraction are provided in SEQ ID NO: 177-224. The sequences of SEQ ID NO: 177, 178, 180, 181, 183, 187, 192, 195-197, 208, 211, 212, 215, 216, 218 and 219 showed some homology to previously identified genes. The sequences of SEQ ID NO: 179, 182, 184-186, 188-191, 193, 194, 198-207, 209 210, 213, 214, 217, 220 and 224 showed some homology to previously determined ESTs. The sequence of SEQ ID NO: 221-223 showed no homology to any previously determined sequence.

### B: ISOLATION OF cDNA SEQUENCES FROM A LUNG ADENOCARCINOMA LIBRARY

A human lung adenocarcinoma cDNA expression library was constructed as described above. The library contained 3.2 x 10⁶ independent colonies, with 100% of clones having an insert and the average insert size being 1500 base pairs. Library subtraction was performed as described above using the normal lung and normal liver and heart cDNA expression libraries described above as the driver DNA. Twenty-six hundred independent clones were recovered.

Initial cDNA sequence analysis from 100 independent clones revealed many ribosomal protein genes. The cDNA sequences for fifteen clones isolated in this subtraction are provided in SEQ ID NO: 71-86. Comparison of these sequences with those in the gene bank as described above revealed no significant homologies to the sequence provided in SEQ ID NO: 84. The sequences of SEQ ID NO: 71, 73, 74, 77, 78 and 80-82 were found to show some homology to previously isolated ESTs, and the sequences of SEQ ID NO: 72, 75, 76, 79, 83 and 85 were found to show some homology to previously identified human genes.

In further studies, a cDNA library (referred to as mets3616A) was constructed from a metastatic lung adenocarcinoma. The determined cDNA sequences of 25 clones sequenced at random from this library are provided in SEQ ID NO: 255-279. The anets3616A cDNA library was subtracted against a cDNA library prepared from a pool of normal lung, liver, pancreas, skin, kidney, brain and resting PBMC. To increase the specificity of the subtraction, the driver was spiked with genes that were determined to be most abundant in the mets3616A cDNA library, such as EF1-alpha, integrin-beta and anticoagulant protein PP4, as well as with cDNAs that were previously found to be differentially expressed in subtracted lung adenocarcinoma cDNA libraries. The determined cDNA sequences of 51 clones isolated from the subtracted library (referred to as mets3616A-S1) are provided in SEQ ID NO: 280-330.

Comparison of the sequences of SEQ ID NO: 255-330 with those in the public databases revealed no significant homologies to the sequences of SEQ ID NO: 255-258, 260, 262-264, 270, 272, 275, 276, 279, 281, 287, 291, 296, 300 and 310. The sequences of SEQ ID NO: 259, 261, 265-269, 271, 273, 274, 277, 278, 282-285, 288-290, 292, 294, 297-299, 301, 303-309, 313, 314, 316, 320-324 and 326-330 showed some homology to previously identified gene sequences, while the sequences of SEQ ID NO: 280,286,293, 302, 310, 312, 315, 317-319 and 325 showed some homology to previously isolated expressed sequence tags (ESTs).

### EXAMPLE 2

### DETERMINATION OF TISSUE SPECIFICITY OF LUNG TUMOR POLYPEPTIDES

Using gene specific primers, mRNA expression levels for seven representative lung tumor polypeptides described in Example 1 were examined in a variety of normal and tumor tissues using RT-PCR.

Briefly, total RNA was extracted from a variety of normal and tumor tissues using Trizol reagent as described above. First strand synthesis was carried out using 2 µg of total RNA with SuperScript II reverse transcriptase (BRL Life Technologies) at 42 °C for one hour. The cDNA was then amplified by PCR with gene-specific primers. To ensure the semi-quantitative nature of the RT-PCR, β-actin was used as an internal control for each of the tissues examined. 1 µl of 1:30 dilution of cDNA was employed to enable the linear range amplification of the β-actin template and was sensitive enough to reflect the differences in the initial copy numbers. Using these conditions, the β-actin levels were determined for each reverse transcription reaction from each tissue. DNA contamination was minimized by DNase treatment and by assuring a negative PCR result when using first strand cDNA that was prepared without adding reverse transcriptase.

mRNA Expression levels were examined in five different types of tumor tissue (lung squamous cell carcinoma from 3 patients, lung adenocarcinoma, colon tumor from 2 patients, breast tumor and prostate tumor), and thirteen different normal tissues (lung from 4 donors, prostate, brain, kidney, liver, ovary, skeletal muscle, skin, small intestine, stomach, myocardium, retina and testes). Using a 10-fold amount of cDNA, the antigen LST-S1-90 (SEQ ID NO: 3) was found to be expressed at high levels in lung squamous cell carcinoma and in breast tumor, and at low to undetectable levels in the other tissues examined.

The antigen LST-S2-68 (SEQ ID NO: 15) appears to be specific to lung and breast tumor, however, expression was also detected in normal kidney. Antigens LST-S1-169 (SEQ ID NO: 6) and LST-S1-133 (SEQ ID NO: 5) appear to be very abundant in lung tissues (both normal and tumor), with the expression of these two genes being decreased in most of the normal tissues tested. Both LST-S1-169 and LST-S1-133 were also expressed in breast and colon tumors. Antigens LST-S1-6 (SEQ ID NO: 7) and LST-S2-I2-5F (SEQ ID NO: 47) did not show tumor or tissue specific expression, with the expression of LST-S1-28 being rare and only detectable in a few tissues. The antigen LST-S3-7 (SEQ ID NO: 63) showed lung and breast tumor specific expression, with its message only being detected in normal testes when the PCR was performed for 30 cycles. Lower level expression was detected in some normal tissues when the cycle number was increased to 35. Antigen LST-S3-13 (SEQ ID NO: 66) was found to be expressed in 3 out of 4 lung tumors, one breast tumor and both colon tumor samples. Its expression in normal tissues was lower compared to tumors, and was only detected in 1 out of 4 normal lung tissues and in normal tissues from kidney, ovary and retina. Expression of antigens LST-S3-4 (SEQ ID NO: 62) and LST-S3-14 (SEQ ID NO: 67) was rare and did not show any tissue or tumor specificity. Consistent with Northern blot analyses, the RT-PCT results on antigen LAT-S1-A-10A (SEQ ID NO: 78) suggested that its expression is high in lung, colon, stomach and small intestine tissues, including lung and colon tumors, whereas its expression was low or undetectable in other tissues.

A total of 2002 cDNA fragments isolated in lung subtractions I, II and III, described above, were colony PCR amplified and their mRNA expression levels in lung tumor, normal lung, and various other normal and tumor tissues were determined using microarray technology (Synteni, Palo Alto, CA). Briefly, the PCR amplification products were dotted onto slides in an array format, with each product occupying a unique location in the array. mRNA was extracted from the tissue sample to be tested, reverse transcribed, and fluorescent-labeled cDNA probes were generated. The microarrays were probed with the labeled cDNA probes, the slides scanned and fluorescence intensity was measured. This intensity correlates with the hybridization intensity. Seventeen non-redundant cDNA clones showed over-expression in lung squamous tumors, with expression in normal tissues tested (lung, skin, lymph node, colon, liver, pancreas, breast, heart, bone marrow, large intestine, kidney, stomach, brain, small intestine, bladder and salivary gland) being either undetectable, or 10-fold less compared to lung squamous tumors. The determined partial cDNA sequences for the clone L513S are provided in SEQ ID NO: 87 and 88; those for L514S are provided in SEQ ID NO: 89 and 90; those for L516S in SEQ ID NO: 91 and 92; that for L517S in SEQ ID NO: 93; that for L519S in SEQ ID NO: 94; those for L520S in SEQ ID NO: 95 and 96; those for L521S in SEQ ID NO: 97 and 98; that for L522S in SEQ ID NO: 99; that for L523S in SEQ ID NO: 100; that for L524S in SEQ ID NO: 101; that for L525S in SEQ ID NO: 102; that for L526S in SEQ ID NO: 103; that for L527S in SEQ ID NO: 104; that for L528S in SEQ ID NO: 105; that for L529S in SEQ ID NO: 106; and those for L530S in SEQ ID NO: 107 and 108. Additionally, the full-length cDNA sequence for L530S is provided in SEQ ID NO: 151, with the corresponding predicted amino acid sequence being provided in SEQ ID NO: 152. L530S shows homology to a splice variant of a p53 tumor suppressor homologue, p63. The cDNA sequences of 7 known isoforms of p63 are provided in SEQ ID NO: 331-337, with the corresponding predicted amino acid sequences being provided in SEQ ID NO: 338-344, respectively.

Due to polymorphisms, the clone L531S appears to have two forms. A first determined full-length cDNA sequence for L531S is provided in SEQ ID NO: 109, with the corresponding predicted amino acid sequence being provided in SEQ ID NO: 110. A second determined full-length cDNA sequence for L531S is provided in SEQ ID NO: 111, with the corresponding predicted amino acid sequence being provided in SEQ ID NO: 112. The sequence of SEQ ID NO: 111 is identical to that of SEQ ID NO: 109, except that it contains a 27 bp insertion. Similarly, L514S also has two alternatively spliced forms; the first variant cDNA is listed as SEQ ID NO: 153, with the corresponding amino acid sequence being provided in SEQ ID NO: 155. The second variant form of L514S full-length cDNA is provided in SEQ ID NO: 154, with its corresponding amino acid sequence being provided in SEQ ID NO: 156.

Full length cloning for L524S (SEQ ID NO: 101) yielded two variants (SEQ ID NO: 163 and 164) with the corresponding predicted amino acid sequences of SEQ ID NO: 165 and 166, respectively. Both variants have been shown to encode parathyroid hormone-related peptide.

Attempts to isolate the full-length cDNA for L519S, resulted in the isolation of the extended cDNA sequence provided in SEQ ID NO: 173, which contains a potential open reading frame. The predicted amino acid sequence encoded by the sequence of SEQ ID NO: 173 is provided in SEQ ID NO: 174. Additionally, the full-length cDNA sequence for the clone of SEQ ID NO: 100 (known as L523S), a known gene, is provided in SEQ ID NO: 175, with the corresponding predicted amino acid sequence provided in SEQ ID NO: 176. In further studies, a full-length cDNA sequence for L523S was isolated from a L523S-positive tumor cDNA library by PCR amplification using gene specific primers designed from the sequence of SEQ ID NO: 175. The determined cDNA sequence is provided in SEQ ID NO: **. The amino acid sequence encoded by this sequence is provided in SEQ ID NO: **. This protein sequence differs from the previously published protein sequence at two amino acid positions, namely at positions 158 and 410.

Comparison of the sequences of L514S and L531S (SEQ ID NO: 87 and 88, 89 and 90, and 109, respectively) with those in the gene bank, as described above, revealed no significant homologies to known sequences. The sequences of L513S, L516S, L517S, L519S, L520S and L530S (SEQ ID NO: 87 and 88, 91 and 92, 93, 94, 95 and 96, 107 and 108, respectively) were found to show some homology to previously identified ESTs. The sequences of L521S, L522S, L523S, L524S, L525S, L526S, L527S, L528S and L529S (SEQ ID NO: 97 and 98, 99, 99, 101, 102, 103, 104, 105, and 106, respectively) were found to represent known genes. The determined full-length cDNA sequences for L520S is provided in SEQ ID NO: 113, with the corresponding predicted amino acid sequence being provided in SEQ ID NO: 114. Subsequent microarray analysis has shown L520S to be overexpressed in breast tumors in addition to lung squamous tumors.

Further analysis has demonstrated that L529S (SEQ ID NO: 106 and 115), L525S (SEQ ID NO: 102 and 120) and L527S (SEQ ID NO: 104) are cytoskeletal components and potentially squamous cell specific proteins. L529S is connexin 26, a gap junction protein. It is highly expressed in lung squamous tumor 9688T, and moderately over-expressed in two others. However, lower level expression of connexin 26 is also detectable in normal skin, colon, liver and stomach. The over-expression of connexin 26 in some breast tumors has been reported and a mutated form of L529S may result in over-expression in lung tumors. L525S is plakophilin 1, a desmosomal protein found in plaque-bearing adhering junctions of the skin. Expression levels for L525S mRNA is highly elevated in three out of four lung squamous tumors tested, and in normal skin. L527S has been identified as keratin 6 isoform, type II 58 Kd keratin, and cytokeratin 13 and shows over-expression in squamous tumors and low expression in normal skin, breast and colon tissues. Notably, keratin and keratin-related genes have been extensively documented as potential markers for lung cancer including CYFRA2.1 (Pastor, A., et al, Eur. Respir. J., 10:603-609, 1997). L513S (SEQ ID NO: 87 and 88) shows moderate over-expression in several tumor tissues tested, and encodes a protein that was first isolated as a pemphigus vulgaris antigen.

L520S (SEQ ID NO: 95 and 96) and L521S (SEQ ID NO: 97 and 98) are highly expressed in lung squamous tumors, and L520S is up-regulated in normal salivary gland and L521 S is over-expressed in normal skin. Both belong to a family of small proline rich proteins and represent markers for fully differentiated squamous cells. L521 S has been described as a specific marker for lung squamous tumor (Hu, R., et al, Lung Cancer, 20:25-30, 1998). L515S (SEQ ID NO: 162) encodes IGF-β2 and L516S is an aldose reductase homologue and both are moderately expressed in lung squamous tumors and in normal colon. Notably, L516S (SEQ ID NO: 91 and 92) is up-regulated in metastatic tumors but not primary lung adenocarcinoma, an indication of its potential role in metatasis and a potential prognostic marker. L522S (SEQ ID NO: 99) is moderately over-expressed in lung squamous tumors with minimum expression in normal tissues. L522S has been shown to belong to a class IV alcohol dehydrogenase, ADH7, and its expression profile suggests it is a squamous cell specific antigen. L523S (SEQ ID NO: 100) is moderately over-expressed in lung squamous tumor, human pancreatic cancer cell lines and pancreatic cancer tissues, suggesting this gene may be a shared antigen between pancreatic and lung squamous cell cancer.

L524S (SEQ ID NO: 101) is over-expressed in the majority of squamous tumors tested and is homologous with parathyroid hormone-related peptide (PTHrP), which is best known to cause humoral hypercalcaemia associated with malignant tumors such as leukemia, prostate and breast cancer. It is also believed that PTHrP is most commonly associated with squamous carcinoma of lung and rarely with lung adenocarcinoma (Davidson, L.A., et al, J. Pathol., 178: 398-401, 1996). L528S (SEQ ID NO: 105) is highly over-expressed in two lung squamous tumors with moderate expression in two other squamous tumors, one lung adenocarcinoma and some normal tissues, including skin, lymph nodes, heart, stomach and lung. It encodes the NMB gene that is similar to the precursor of melanocyte specific gene Pmel17, wfhich is reported to be preferentially expressed in low-metastatic potential melanoma cell lines.

This suggests that L528S may be a shared antigen in both melanoma and lung squamous cell carcinoma. L526S (SEQ ID NO: 103) is overexpressed in all lung squamous cell tumor tissues tested and has been shown to share homology with a gene (ATM) in which a mutation causes ataxia telangiectasia, a genetic disorder in humans causing a predisposition to cancer, among other symptoms. ATM encodes a protein that activates p53 mediated cell-cycle checkpoint through direct binding and phosphorylation of the p53 molecule. Approximately 40% of lung cancer is associated with p53 mutations, and it is speculated that over-expression of ATM is a result of compensation for loss of p53 function, but it is unknown whether over-expression is the cause of result of lung squamous cell carcinoma. Additionally, expression of L526S (ATM) is also detected in a metastatic but not lung adenocarcinoma, suggesting a role in metastasis.

Expression of L523S (SEQ ID NO: 175), was also examined by real time RT-PCR as described above. In a first study using a panel of lung squamous tumors, L523S was found to be expressed in 4/7 lung squamous tumors, 2/3 head and neck squamous tumors and 2/2 lung adenocarcinomas, with low level expression being observed in skeletal muscle, soft palate and tonsil. In a second study using a lung adenocarcinoma panel, expression of L523S was observed in 4/9 primary adenocarcinomas, 2/2 lung pleural effusions, 1/1 metastatic lung adenocarcinomas and 2/2 lung squamous tumors, with little expression being observed in normal tissues.

Expression of L523S in lung tumors and various normal tissues was also examined by Northern blot analysis, using standard techniques. In a first study, L523S was found to be expressed in a number of lung adenocarcinomas and squamous cell carcinomas, as well as normal tonsil. No expression was observed in normal lung. In a second study using a normal tissue blot (HB-12) from Clontech, no expression was observed in brain, skeletal muscle, colon, thymus, spleen, kidney, liver, small intestine, lung or PBMC, although there was strong expression in placenta.

### EXAMPLE 3

### ISOLATION AND CHARACTERIZATION OF LUNG TUMOR POLYPEPTIDES BY PCR-BASED SUBTRACTION

Eight hundred and fifty seven clones from a cDNA subtraction library, containing cDNA from a pool of two human lung squamous tumors subtracted against eight normal human tissue cDNAs including lung, PBMC, brain, heart, kidney, liver, pancreas, and skin, (Clontech, Palo Alto, CA) were derived and submitted to a first round of PCR amplification. This library was subjected to a second round of PCR amplification, following the manufacturer's protocol. The resulting cDNA fragments were subcloned into the vector P7- Adv vector (Clontech, Palo Alto, CA) and transformed into DH5α *E. coli* (Gibco, BRL). DNA was isolated from independent clones and sequenced using a Perkin Elmer/Applied Biosystems Division Automated Sequencer Model 373A.

One hundred and sixty two positive clones were sequenced. Comparison of the DNA sequences of these clones with those in the the EMBL and GenBank databases, as described above, revealed no significant homologies to 13 of these clones, hereinafter referred to as Contigs 13, 16, 17, 19, 22, 24, 29, 47, 49, 56-59. The determined cDNA sequences for these clones are provided in SEQ ID NO: 125, 127-129, 131-133, 142, 144, 148-150, and 157, respectively. Contigs 1, 3-5, 7-10, 12, 11, 15, 20, 31, 33, 38, 39, 41, 43, 44, 45, 48, 50, 53, 54 (SEQ ID NO: 115-124, 126, 130, 134-141, 143, 145-147, respectively) were found to show some degree of homology to previously identified DNA sequences. Contig 57 (SEQ ID NO: 149) was found to represent the clone L519S (SEQ ID NO: 94) disclosed in US. Patent Application No. 09/123,912, filed July 27, 1998. To the best of the inventors' knowledge, none of these sequences have been previously shown to be differentially over-expressed in lung tumors.

mRNA expression levels for representative clones in lung tumor tissues, normal lung tissues (n=4), resting PBMC, salivary gland, heart, stomach, lymph nodes, skeletal muscle, soft palate, small intestine, large intestine, bronchial, bladder, tonsil, kidney, esophagus, bone marrow, colon, adrenal gland, pancreas, and skin, (all derived from human) were determined by RT-PCR as described above. Expression levels using microarray technology, as described above, were examined in one sample of each tissue type unless otherwise indicated.

Contig 3 (SEQ ID NO: 116) was found to be highly expressed in all head and neck squamous cell tumors tested (17/17), and expressed in the majority (8/12) of lung squamous tumors, (high expression in 7/12, moderate in 2/12, and low in 2/12), while showing negative expression for 2/4 normal lung tissues and low expression in the remaining two samples. Contig 3 showed moderate expression in skin and soft palate, and lowered expression levels in resting PBMC, large intestine, salivary gland, tonsil, pancreas, esophagus, and colon. Contig 11 (SEQ ID NO: 124) was found to be expressed in all head and neck squamous cell tumors tested (17/17): highly expressed in 14/17, and moderately expressed in 3/17. Additionally, expression in lung squamous tumors showed high expression in 3/12 and moderate in 4/12. Contig 11 was negative for 3/4 normal lung samples, with the remaining sample having only low expression. Contig 11 showed low to moderate reactivity to salivary gland, soft palate, bladder, tonsil, skin, esophagus, and large intestine. Contig 13 (SEQ ID NO: 125) was found to be expressed in all head and neck squamous cell tumors tested (17/17): highly expressed in 12/17, and moderately expressed in 5/17. Contig 13 was expressed in 7/12 lung squamous tumors, with high expression in 4/12 and moderate expression in three samples. Analysis of normal lung samples showed negative expression for 2/4 and low to moderate expression in the remaining two samples. Contig 13 did show low to moderate reactivity to resting PBMC, salivary gland, bladder, pancreas, tonsil, skin, esophagus, and large intestine, as well as high expression in soft palate. Contig 16 (SEQ ID NO: 127) was found to be moderately expressed in some head and neck squamous cell tumors (6/17) and one lung squamous tumor; while showing no expression in any normal lung samples tested. Contig 16 did show low reactivity to resting PBMC, large intestine, skin, salivary gland, and soft palate. Contig 17 (SEQ ID NO: 128) was shown to be expressed in all head and neck squamous cell tumors tested (17/17): highly expressed in 5/17, and moderately expressed in 12/17. Expression levels in lung squamous tumors showed one tumor sample with high expression and 3/12 with moderate levels. Contig 17 was negative for 2/4 normal lung samples, with the remaining samples having only low expression. Additionally, low level expression was found in esophagus and soft palate. Contig 19 (SEQ ID NO: 129) was found to be expressed in most head and neck squamous cell tumors tested (11/17); with two samples having high levels, 6/17 showing moderate expression, and low expression being found in 3/17. Testing in lung squamous tumors revealed only moderate expression in 3/12 samples. Expression levels in 2/4 of normal lung samples were negative, the two other samples having only low expression. Contig 19 showed low expression levels in esophagus, resting PBMC, salivary gland, bladder, soft palate and pancreas.

Contig 22 (SEQ ID NO: 131), was shown to be expressed in most head and neck squamous cell tumors tested (13/17) with high expression in four of these samples, moderate expression in 6/17, and low expression in 3/17. Expression levels in lung squamous tumors were found to be moderate to high for 3/12 tissues tested, with negative expression in two normal lung samples and low expression in two other samples (n=4). Contig 22 showed low expression in skin, salivary gland and soft palate. Similarly, Contig 24 (SEQ ID NO: 132) was found to be expressed in most head and neck squamous cell tumors tested (13/17) with high expression in three of these samples, moderate expression in 6/17, and low expression in 4/17. Expression levels in lung squamous tumors were found to be moderate to high for 3/12 tissues tested, with negative expression for three normal lung samples and low expression in one sample (n=4). Contig 24 showed low expression in skin, salivary gland and soft palate, Contig 29 (SEQ ID NO: 133) was expressed in nearly all head and neck squamous cell tumors tested (16/17): highly expressed in 4/17, moderately expressed in 11/17, with low expression in one sample. Also, it was moderately expressed in 3/12 lung squamous tumors, while being negative for 2/4 normal lung samples. Contig 29 showed low to moderate expression in large intestine, skin, salivary gland, pancreas, tonsil, heart and soft palate. Contig 47 (SEQ ID NO: 142) was expressed in most head and neck squamous cell tumors tested (12/17): moderate expression in 10/17, and low expression in two samples. In lung squamous tumors, it was highly expressed in one sample and moderately expressed in two others (n=13). Contig 47 was negative for 2/4 normal lung samples, with the remaining two samples having moderate expression. Also, Contig 47 showed moderate expression in large intestine, and pancreas, and low expression in skin, salivary gland, soft palate, stomach, bladder, resting PBMC, and tonsil.

Contig 48 (SEQ ID NO: 143) was expressed in all head and neck squamous cell tumors tested (17/17): highly expressed in 8/17 and moderately expressed in 7/17, with low expression in two samples. Expression levels in lung squamous tumors were high to moderate in three samples (n=13). Contig 48 was negative for one out of four normal lung samples, the remaining showing low or moderate expression. Contig 48 showed moderate expression in soft palate, large intestine, pancreas, and bladder, and low expression in esophagus, salivary gland, resting PBMC, and heart. Contig 49 (SEQ ID NO: 144) was expressed at low to moderate levels in 6/17 head and neck squamous cell tumors tested. Expression levels in lung squamous tumors were moderate in three samples (n=13). Contig 49 was negative for 2/4 normal lung samples, the remaining samples showing low expression. Moderate expression levels in skin, salivary gland, large intestine, pancreas, bladder and resting PBMC were shown, as well as low expression in soft palate, lymph nodes, and tonsil. Contig 56 (SEQ ID NO: 148) was expressed in low to moderate levels in 3/17 head and neck squamous cell tumors tested, and in lung squamous tumors, showing low to moderate levels in three out of thirteen samples. Notably, low expression levels were detected in one adenocarcinoma lung tumor sample (n=2). Contig 56 was negative for 3/4 normal lung samples, and showed moderate expression levels in only large intestine, and low expression in salivary gland, soft palate, pancreas, bladder, and resting PBMC. Contig 58, also known as L769P, (SEQ ID NO: 150) was expressed at moderate levels in 11/17 head and neck squamous cell tumors tested and low expression in one additional sample. Expression in lung squamous tumors showed low to moderate levels in three out of thirteen samples. Contig 58 was negative for 3/4 normal lung samples, with one sample having low expression. Moderate expression levels in skin, large intestine, and resting PBMC were demonstrated, as well as low expression in salivary gland, soft palate, pancreas, and bladder. Contig 59 (SEQ ID NO; 157) was expressed in some head, neck, and lung squamous tumors. Low level expression of Contig 59 was also detected in salivary gland and large intestine.

The full-length cDNA sequence for Contig 22, also referred to as L763P, is provided in SEQ ID NO: 158, with the corresponding predicted amino acid sequence being provided in SEQ ID NO: 159. Real-time RT-PCR analysis of L763P revealed that it is highly expressed in 3/4 lung squamous tumors as well as 4/4 head and neck squamous tumors, with low level expression being observed in normal brain, skin, soft pallet and trachea. Subsequent database searches revealed that the sequence of SEQ ID NO: 158 contains a mutation, resulting in a frameshift in the corresponding protein sequence. A second cDNA sequence for L763P is provided in SEQ ID NO: 345, with the corresponding amino acid sequence being provided in SEQ ID NO: 346. The sequences of SEQ ID NO: 159 and 346 are identical with the exception of the C-terminal 33 amino acids of SEQ ID NO: 159.

The full-length cDNA sequence incorporating Contigs 17, 19, and 24, referred to as L762P, is provided in SEQ ID NO: 160, with the corresponding predicted amino acid sequence being provided in SEQ ID NO: 161. Further analysis of L762P has determined it to be a type I membrane protein and two additional variants have been sequenced. Variant 1 (SEQ ID NO: 167, with the corresponding amino acid sequence in SEQ ID NO: 169) is an alternatively spliced form of SEQ ID NO: 160 resulting in deletion of 503 nucleotides, as well as deletion of a short segment of the expressed protein. Variant 2 (SEQ ID NO: 168, with the corresponding amino acid sequence in SEQ ID NO: 170) has a two nucleotide deletion at the 3' coding region in comparison to SEQ ID NO: 160, resulting in a secreted form of the expressed protein. Real-time RT-PCR analysis of L762P revealed that is over-expressed in 3/4 lung squamous tumors and 4/4 head & neck tumors, with low level expression being observed in normal skin, soft pallet and trachea.

The full-length cDNA sequence for contig 56 (SEQ ID NO: 148), also referred to as L773P, is provided in SEQ ID NO: 171, with the predicted amino acid sequence in SEQ ID NO: 172. L773P was found to be identical to dihydroxyl dehydrogenase at the 3' portion of the gene, with divergent 5' sequence. As a result, the 69 N-terminal amino acids are unique, The cDNA sequence encoding the 69 N-terminal amino acids is provided in SEQ ID NO: 349, with the N-terminal amino acid sequence being provided in SEQ ID NO: 350. Real-time PCR revealed that L773P is highly expressed in lung squamous tumor and lung adenocarcinoma, with no detectable expression in normal tissues. Subsequent Northern blot analysis of L773P demonstrated that this transcript is differentially over-expressed in squamous tumors and detected at approximately 1.6 Kb in primary lung tumor tissue and approximately 1.3 Kb in primary head and neck tumor tissue.

Subsequent microarray analysis has shown Contig 58, also referred to as L769S (SEQ ID NO: 150), to be overexpressed in breast tumors in addition to lung squamous tumors.

### EXAMPLE 4

### SYNTHESIS OF POLYPEPTIDES

Polypeptides may be synthesized on a Perkin Elmer/Applied Biosystems Division 430A peptide synthesizer using FMOC chemistry with HPTU (O-Benzotriazole-N,N,N',N'-tetramethyluronium hexafluorophosphate) activation. A Gly-Cys-Gly sequence may be attached to the amino terminus of the peptide to provide a method of conjugation, binding to an immobilized surface, or labeling of the peptide. Cleavage of the peptides from the solid support may be carried out using the following cleavage mixture: trifluoroacetic acid:ethanedithiol:thioanisole:water:phenol (40:1:2:2:3). After cleaving for 2 hours, the peptides may be precipitated in cold methyl-t-butyl-ether. The peptide pellets may then be dissolved in water containing 0.1% trifluoroacetic acid (TFA) and lyophilized prior to purification by C18 reverse phase HPLC. A gradient of 0%-60% acetonitrile (containing 0.1% TFA) in water (containing 0.1% TFA) may be used to elute the peptides. Following lyophilization of the pure fractions, the peptides may be characterized using electrospray or other types of mass spectrometry and by amino acid analysis.

### EXAMPLE 5

### PREPARATION OF ANTIBODIES AGAINST LUNG CANCER ANTIGENS

Polyclonal antibodies against the lung cancer antigens L514S, L528S and L53 1 S (SEQ ID NO: 155, 225 and 112, respectively) were prepared as follows.

Rabbits were immunized with recombinant protein expressed in and purified from *E*. *coli* as described above. For the initial immunization, 400 µg of antigen combined with muramyl dipeptide (MDP) was injected subcutaneously (S.C.). Animals were boosted S.C. 4 weeks later with 200 µg of antigen mixed with incomplete Freund's Adjuvant (IFA). Subsequent boosts of 100 µg of antigen mixed with IFA were injected S.C. as necessary to induce high antibody titer responses. Serum bleeds from immunized rabbits were tested for antigen-specific reactivity using ELISA assays with purified protein. Polyclonal antibodies against L514S, L528S and L531S were affinity purified from high titer polyclonal sera using purified protein attached to a solid support.

Immunohistochemical analysis using polyclonal antibodies against L514S was performed on a panel of 5 lung tumor samples, 5 normal lung tissue samples and normal colon, kidney, liver, brain and bone marrow. Specifically, tissue samples were fixed in formalin solution for 24 hours and embedded in paraffin before being sliced into 10 micron sections. Tissue sections were permeabilized and incubated with antibody for 1 hr. HRP-labeled anti-mouse followed by incubation with DAB chromogen was used to visualize L514S immunoreactivity. L514S was found to be highly expressed in lung tumor tissue with little or no expression being observed in normal lung, brain or bone marrow. Light staining was observed in colon and kidney. Staining was seen in normal liver but no mRNA has been detected in this tissue making this result suspect.

### EXAMPLE 6

### PEPTIDE PRIMING OF MICE AND PROPAGATION OF CTL LINES

Immunogenic peptides from the lung cancer antigen L762P (SEQ ID NO: 161) for HLA-A2/K⁶-restricted CD8+ T cells were identified as follows.

The location of HLA-A2 binding peptides within the lung cancer antigen L762P (SEQ ID NO: 161) was predicted using a computer program which predicts peptides sequences likely to being to HLA-A*0201 by fitting to the known peptide binding motif for HLA-A*0201 (Rupert et al. (1993) Cell 74:929; Rammensee et al. (1995) Immunogenetics 41:178-228). A series of 19 synthetic peptides corresponding to a selected subset of the predicted HLA-A*0201 binding peptides was prepared as described above.

Mice expressing the transgene for human HLA A2/k^{b} (provided by Dr L. Sherman, The Scripps Research Institute, La Jolla, CA) were immunized with the synthetic peptides, as described by Theobald et al., Proc. Natl. Acad. Sci. USA 92:11993-11997, 1995 with the following modifications. Mice were immunized with 50µg of L726P peptide and 120µg of an I-A^{b} binding peptide derived from hepatitis B Virus protein emulsified in incomplete Freund's adjuvant. Three weeks later these mice were sacrificed and single cell suspensions prepared. Cells were then resuspended at 7 x 10⁶ cells/ml in complete media (RPMI-1640; Gibco BRL, Gaithersburg, MD) containing 10% FCS, 2mM Glutamine (Gibco BRL), sodium pyruvate (Gibco BRL), non-essential amino acids (Gibco BRL), 2 x 10⁻⁵ M 2-mercaptoethanol, 50U/ml penicillin and streptomycin, and cultured in the presence of irradiated (3000 rads) L762P peptide- (5µg/ml) and 10mg/ml B₂-microglobulin- (3 µg/ml) LPS blasts (A2 transgenic spleens cells cultured in the presence of 7µg/ml dextran sulfate and 25µg/ml LPS for 3 days). After six days, cells (5 x 10⁵/ml) were restimulated with 2.5 x 10⁶/ml peptide pulsed irradiated (20,000 rads) EL4A2Kb cells (Sherman et al, Science 258:815-818, 1992) and 5 x 10⁶/ml irradiated (3000 rads) A2/K^{b}-transgenic spleen feeder cells. Cells were cultured in the presence of 10U/ml IL-2. Cells were restimulated on a weekly basis as described, in preparation for cloning the line.

Peptide-specific cell lines were cloned by limiting dilution analysis with irradiated (20,000 rads) L762P peptide-pulsed EL4 A2Kb tumor cells (1 x 10⁴ cells/well) as stimulators and irradiated (3000 rads) A2/K^{b}-transgenic spleen cells as feeders (5 x 10⁵ cells/ well) grown in the presence of 10U/ml IL-2. On day 7, cells were restimulated as before. On day 14, clones that were growing were isolated and maintained in culture.

Cell lines specific for L762P-87 (SEQ ID NO: 226; corresponding to amino acids 87-95 of SEQ ID NO: 161), L726P-145 (SEQ ID NO: 227; corresponding to amino acids 145-153 of SEQ ID NO: 161), L726P-585 (SEQ ID NO: 228; corresponding to amino acids 585-593 of SEQ ID NO: 161), L762P-425 (SEQ ID NO: 229; corresponding to amino acids 425-433 of SEQ ID NO: 161), L762P(10)-424 (SEQ ID NO: 230; corresponding to amino acids 424-433 of SEQ ID NO: 161) and L762P(10)-458 (SEQ ID NO: 231; corresponding to amino acids 458-467 of SEQ ID NO: 161) demonstrated significantly higher reactivity (as measured by percent specific lysis) against L762P peptide-pulsed EL4-A2/K^{b} tumor target cells than control peptide-pulsed EL4-A2/K^{b} tumor target cells.

### EXAMPLE 7

### IDENTIFICATION OF CD4 IMMUNOGENIC T CELL EPITOPES DERIVED FROM THE LUNG CANCER ANTIGEN L762P

CD4 T cell lines specific for the antigen L762P (SEQ ID NO: 161) were generated as follows.

A series of 28 overlapping peptides were synthesized that spanned approximately 50% of the L762P sequence. For priming, peptides were combined into pools of 4-5 peptides, pulsed at 20 micrograms/ml into dendritic cells for 24 hours. The dendritic cells were then washed and mixed with positively selected CD4+ T cells in 96 well U-bottomed plates. Forty cultures were generated for each peptide pool. Cultures were restimulated weekly with fresh dendritic cells loaded with peptide pools. Following a total of 3 stimulation cycles, cells were rested for an additional week and tested for specificity to antigen presenting cells (APC) pulsed with peptide pools using interferon-gamma ELISA and proliferation assays. For these assays, adherent monocytes loaded with either the relevant peptide pool or an irrelevant peptide were used as APC. T cell lines that appeared to specifically recognize L762P peptide pools both by cytokine release and proliferation were identified for each pool. Emphasis was placed on identifying T cells with proliferative responses. T cell lines that demonstrated either both L762P-specific cytokine secretion and proliferation, or strong proliferation alone were further expanded to be tested for recognition of individual peptides from the pools, as well as for recognition of recombinant L762P. The source of recombinant L762P was *E. coli,* and the material was partially purified and endotoxin positive. These studies employed 10 micrograms of individual peptides, 10 or 2 micrograms of an irrelevant peptide, and 2 or 0.5 micrograms of either L762P protein or an irrelevant, equally impure, *E. coli* generated recombinant protein. Significant interferon-gamma production and CD4 T cell proliferation was induced by a number of L762P-derived peptides in each pool. The amino acid sequences for these peptides are provided in SEQ ID NO: 232-251. These peptides correspond to amino acids 661-680, 676-696, 526-545, 874-893, 811-830, 871-891, 856-875, 826-845, 795-815, 736-755, 706-725, 706-725, 691-710, 601-620, 571-590, 556-575, 616-635, 646-665, 631-650, 541-560 and 586-605, respectively, of SEQ ID NO: 161.

CD4 T cell lines that demonstrated specificity for individual L762P-derived peptides were further expanded by stimulation with the relevant peptide at 10 micrograms/ml. Two weeks post-stimulation, T cell lines were tested using both proliferation and IFN-gamma ELISA assays for recognition of the specific peptide. A number of previously identified T cells continued to demonstrate L762P-peptide specific activity. Each of these lines was further expanded on the relevant peptide and, following two weeks of expansion, tested for specific recognition of the L762P-peptide in titration experiments, as well as for recognition of recombinant *E. coli*-derived L762P protein. For these experiments, autologous adherent monocytes were pulsed with either the relevant L762P-derived peptide, an irrelevant mammaglobin-derived peptide, recombinant *E. coli*-derived L762P (approx. 50% pure), or an irrelevant *E. coli*-derived protein. The majority of T cell lines were found to show low affinity for the relevant peptide, since specific proliferation and IFN-gamma ratios dramatically decreased as L762P peptide was diluted. However, four lines were identified that demonstrated significant activity even at 0.1 micrograms/ml peptide. Each of these lines (referred to as A/D5, D/F5, E/A7 and E/B6) also appeared to specifically proliferate in response to the *E. coli*-derived L762P protein preparation, but not in response to the irrelevant protein preparation. The amino acid sequences of the L762P-derived peptides recognized by these lines are provided in SEQ ID NO: 234, 249, 236 and 245, respectively. No protein specific IFN-gamma was detected for any of the lines. Lines A/D5, E/A7 and E/B6 were cloned on autologous adherent monocytes pulsed with the relevant peptide at 0.1 (A/D5 and E/A7) or 1 (D/F5) microgram/ml. Following growth, clones were tested for specificity for the relevant peptide. Numerous clones specific for the relevant peptide were identified for lines A/D5 and E/A7.

### EXAMPLE 8

### PROTEIN EXPRESSION OF LUNG TUMOR-SPECIFIC ANTIGENS

### a) Expression of L514S in E. coli

The lung tumor antigen L514S (SEQ ID NO: 89) was subcloned into the expression vector pE32b at NcoI and NotI sites, and transformed into *E. coli* using standard techniques. The protein was expressed from residues 3-153 of SEQ ID NO: 89. The expressed amino acid sequence and the corresponding DNA sequence are provided in SEQ ID NO: 252 and 253, respectively.

### b) Expression of L762P

Amino acids 32-944 of the lung tumor antigen L762P (SEQ ID NO: 161), with a 6X His Tag, were subcloned into a modified pET28 expression vector, using kanamycin resistance, and transformed into BL21 CodonPlus using standard techniques. Low to moderate levels of expression were observed. The determined DNA sequence of the L762P expression construct is provided in SEQ ID NO: 254.

From the foregoing it will be appreciated that, although specific embodiments of the invention have been described herein for purposes of illustration, various modifications may be made without deviating from the spirit and scope of the invention. Accordingly, the invention is not limited except as by the appended claims.

The present invention will now be described by way of reference to the following clauses.
1. An isolated polypeptide, comprising at least an immunogenic portion of a lung tumor protein, or a variant thereof, wherein the tumor protein comprises an amino acid sequence that is encoded by a polynucleotide sequence selected from the group consisting of:
   (a) sequences recited in SEQ ID NO: 1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 173, 175, 179, 182, 184-186, 188-191, 193, 194,198-207, 209, 210, 213, 214, 217, 220-224, 253, 254-258, 260, 262-264, 270, 272, 275, 276, 279-281, 286, 287, 291, 293, 295, 296, 300, 302, 308-310, 313, 315-317, 323, 345, 347 and 349;
   (b) sequences that hybridize to a sequence recited in any one of SEQ ID NO: 1-3, 6-8,10-13, 15-27, 29, 30, 32, 34-49, 51, 52, 54, 55, 57-59, 61-69, 71, 73, 74, 77, 78, 80-82, 84, 86-96, 107-109, 111,113,125,127,128,129,131-133,142,144,148-151,153,154, 157, 158, 160, 167, 168, 171, 173, 175, 179, 182, 184-186, 188-191, 193, 194, 198-207, 209, 210, 213, 214, 217, 220-224, 253, 254-258, 260, 262-264, 270, 272, 275, 276, 279-281, 286, 287, 291, 293, 295, 296, 300, 302, 308-310, 313, 315-317, 323, 345, 347 and 349 under moderately stringent conditions; and
   (c) complements of sequences of (a) or (b).
2. An isolated polypeptide according to clause 1, wherein the polypeptide comprises an amino acid sequence that is encoded by a polynucleotide sequence recited in any one of SEQ ID NO: 1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 173, 175, 179, 182, 184-186, 188-191, 193, 194, 198-207, 209, 210, 213, 214, 217, 220-224, 253, 254-258, 260, 262-264, 270, 272, 275, 276, 279-281, 286, 287, 291, 293, 295, 296, 300, 302, 308-310, 313, 315-317, 323, 345, 347 and 349 or a complement of any of the foregoing polynucleotide sequences.
3. An isolated polypeptide comprising a sequence recited in any one of SEQ ID NO: 110, 112, 114, 152, 155, 156, 159, 161, 165, 166, 169, 170, 172, 174, 176, 226-252, 346, 348 and 350.
4. An isolated polynucleotide encoding at least 15 amino acid residues of a lung tumor protein, or a variant thereof that differs in one or more substitutions, deletions, additions and/or insertions such that the ability of the variant to react with antigen-specific antisera is not substantially diminished, wherein the tumor protein comprises an amino acid sequence that is encoded by a polynucleotide comprising a sequence recited in any one of SEQ ID NO: 1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 173, 175, 179, 182, 184-186, 188-191, 193, 194, 198-207, 209, 210, 213, 214, 217, 220-224, 253, 254-258, 260, 262-264, 270, 272, 275, 276, 279-281, 286, 287, 291, 293, 295, 296, 300, 302, 309-310, 313, 315-317, 323, 345, 347 and 349 or a complement of any of the foregoing sequences.
5. An isolated polynucleotide encoding a lung tumor protein, or a variant thereof, wherein the tumor protein comprises an amino acid sequence that is encoded by a polynucleotide comprising a sequence recited in any one of SEQ ID NO: 1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 173, 175, 179, 182, 184-186, 188-191, 193, 194, 198-207, 209, 210, 213, 214, 217, 220-224, 253, 254-258, 260, 262-264, 270, 272, 275, 276, 279-281, 286, 287, 291, 293, 295, 296, 300, 302, 308-310, 313, 315-317, 323, 345, 347 and 349 or a complement of any of the foregoing sequences.
6. An isolated polynucleotide, comprising a sequence recited in any one of SEQ ID NO: 1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160, 167, 168, 171, 173, 175, 179, 182, 184-186, 188-191, 193, 194, 198-207, 209, 210, 213, 214, 217, 220-224, 253, 254-258, 260, 262-264, 270, 272, 275, 276, 279-281, 286, 287, 291, 293, 295, 296, 300, 302, 308-310, 313, 315-317, 323, 345, 347 and 349.
7. An isolated polynucleotide, comprising a sequence that hybridizes to a sequence recited in any one of SEQ ID NO: 1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158, 160,167, 168,171,173,175,179, 182,184-186, 188-191,193,194, 198-207, 209, 210, 213, 214, 217, 220-224, 253, 254-258, 260, 262-264, 270, 272, 275, 276, 279-281, 286, 287, 291, 293, 295, 296, 300, 302, 308-310, 313, 315-317, 323, 345, 347 and 349_ under moderately stringent conditions.
8. An isolated polynucleotide complementary to a polynucleotide according to any one of clauses 4-7.
9. An expression vector, comprising a polynucleotide according to any one of clauses 4-8.
10. A host cell transformed or transfected with an expression vector according to clause9.
11. An isolated antibody, or antigen-binding fragment thereof, that specifically binds to a lung tumor protein that comprises an amino acid sequence that is encoded by a polynucleotide sequence recited in any one of SEQ ID NO: 1-3, 6-8, 10-13, 15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151, 153, 154, 157, 158,160,167, 168, 171, 173, 175, 179, 182,184-186,188-191,193, 194, 198-207, 209, 210, 213, 214, 217, 220-224, 253,254-258,260,262-264, 270, 272, 275, 276, 279-281, 286, 287, 291, 293, 295, 296, 300, 302, 308-310, 313, 315-317, 323, 345, 347 and 349_or a complement of any of the foregoing polynucleotide sequences.
12. A fusion protein, comprising at least one polypeptide according to clause 1.
13. A fusion protein according to clause 12, wherein the fusion protein comprises an expression enhancer that increases expression of the fusion protein in a host cell transfected with a polynucleotide encoding the fusion protein.
14. A fusion protein according to clause 12, wherein the fusion protein comprises a T helper epitope that is not present within the polypeptide of claim 1.
15. A fusion protein according to clause 12, wherein the fusion protein comprises an affinity tag.
16. An isolated polynucleotide encoding a fusion protein according to clause 12.
17. A pharmaceutical composition, comprising a physiologically acceptable carrier and at least one component selected from the group consisting of:
   (a) a polypeptide according to clause 1;
   (b) a polynucleotide according to clause 4;
   (c) an antibody according to clause 11;
   (d) a fusion protein according to clause 12; and
   (e) a polynucleotide according to clause 16.
18. A vaccine comprising an immunostimulant and at least one component selected from the group consisting of:
   (a) a polypeptide according to clause 1;
   (b) a polynucleotide according to clause 4;
   (c) an antibody according to clause 11;
   (d) a fusion protein according to clause 12; and
   (e) a polynucleotide according to clause 16.
19. A vaccine according to clause 18, wherein the immunostimulant is an adjuvant.
20. A vaccine according to any clause 18, wherein the immunostimulant induces a predominantly Type I response.
21. A method for inhibiting the development of a cancer in a patient, comprising administering to a patient an effective amount of a pharmaceutical composition according to clause 17.
22. A method for inhibiting the development of a cancer in a patient, comprising administering to a patient an effective amount of a vaccine according to clause 18.
23. A pharmaceutical composition comprising an antigen-presenting cell that expresses a polypeptide according to clause 1, in combination with a pharmaceutically acceptable carrier or excipient.
24. A pharmaceutical composition according to clause 23, wherein the antigen presenting cell is a dendritic cell or a macrophage.
25. A vaccine comprising an antigen-presenting cell that expresses a polypeptide comprising at least an immunogenic portion of a lung tumor protein, or a variant thereof, wherein the tumor protein comprises an amino acid sequence that is encoded by a polynucleotide sequence selected from the group consisting of:
   (a) sequences recited in SEQ ID NO: 1-109, 111, 113, 115-151, 153, 154, 157, 158, 160, 162-164, 167, 168, 171, 173, 175, 177-224, 255-337, 345, 347 and 349;
   (b) sequences that hybridize to a sequence recited in any one of SEQ ID NO: 1-109, 111, 113, 115-151, 153, 154, 157, 158, 160, 162-164, 167, 168, 171, 173, 175, 177-224, 255-337, 345, 347 and 349 under moderately stringent conditions; and
   (c) complements of sequences of (i) or (ii);
   in combination with an immunostimulant.
26. A vaccine according to clause 25, wherein the immunostimulant is an adjuvant.
27. A vaccine according to clause 25, wherein the immunostimulant induces a predominantly Type I response.
28. A vaccine according to clause 25, wherein the antigen-presenting cell is a dendritic cell.
29. A method for inhibiting the development of a cancer in a patient, comprising administering to a patient an effective amount of an antigen-presenting cell that expresses a polypeptide comprising at least an immunogenic portion of a lung tumor protein, or a variant thereof, wherein the tumor protein comprises an amino acid sequence that is encoded by a polynucleotide sequence selected from the group consisting of:
   (a) sequences recited in SEQ ID NO: 1-109, 111, 113, 115-151, 153, 154, 157, 158, 160, 162-164, 167, 168, 171, 173, 175, 177-224, 255-337, 345, 347 and 349;
   (b) sequences that hybridize to a sequence recited in any one of SEQ ID NO: 1-109, 111, 113, 115-151, 153, 154, 157, 158, 160, 162-164, 167, 168, 171, 173, 175, 177-224, 255-337, 345, 347 and 349 under moderately stringent conditions; and
   (c) complements of sequences of (i) or (ii)encoded by a polynucleotide recited in any one of SEQ ID NO: 1-109, 111, 113, 115-151, 153, 154, 157, 158, 160, 162-164, 167, 168,171,173, 175, 177-224, 255-337, 345, 347 and 349;
   and thereby inhibiting the development of a cancer in the patient.
30. A method according to clause 29, wherein the antigen-presenting cell is a dendritic cell.
31. A method according to any one of clauses 21, 22 and 29, wherein the cancer is lung cancer.
32. A method for removing tumor cells from a biological sample, comprising contacting a biological sample with T cells that specifically react with a lung tumor protein, wherein the tumor protein comprises an amino acid sequence that is encoded by a polynucleotide sequence selected from the group consisting of:
   (i) polynucleotides recited in any one of SEQ ID NO: 1-109, 111, 113, 115-151, 153, 154, 157, 158, 160, 162-164, 167, 168, 171, 173, 175, 177-224, 255-337, 345, 347 and 349; and
   (ii) complements of the foregoing polynucleotides;
   wherein the step of contacting is performed under conditions and for a time sufficient to permit the removal of cells expressing the antigen from the sample.
33. A method according to clause 32, wherein the biological sample is blood or a fraction thereof.
34. A method for inhibiting the development of a cancer in a patient, comprising administering to a patient a biological sample treated according to the method of clause 32.
35. A method for stimulating and/or expanding T cells specific for a lung tumor protein, comprising contacting T cells with at least one component selected from the group consisting of:
   (a) polypeptides comprising at least an immunogenic portion of a lung tumor protein, or a variant thereof, wherein the tumor protein comprises an amino acid sequence that is encoded by a polynucleotide sequence selected from the group consisting of:
      (i) sequences recited in SEQ ID NO: 1-109,111, 113,115-151, 153, 154, 157, 158, 160, 162-164, 167, 168, 171, 173, 175, 177-224, 255-337, 345, 347 and 349;
      (ii) sequences that hybridize to a sequence recited in any one of SEQ ID NO: 1-109, 111, 113, 115-151, 153, 154, 157, 158, 160, 162-164, 167, 168, 171, 173, 175, 177-224, 255-337, 345, 347 and 349 under moderately stringent conditions; and
      (iii) complements of sequences of (i) or (ii);
   (b) polynucleotides encoding a polypeptide of (a); and
   (c) antigen presenting cells that express a polypeptide of (a);
   under conditions and for a time sufficient to permit the stimulation and/or expansion of T cells.
36. An isolated T cell population, comprising T cells prepared according to the method of clause 35.
37. A method for inhibiting the development of a cancer in a patient, comprising administering to a patient an effective amount of a T cell population according to clause 36.
38. A method for inhibiting the development of a cancer in a patient, comprising the steps of:
   (a) incubating CD4⁺ and/or CD8+ T cells isolated from a patient with at least one component selected from the group consisting of:
      (i) polypeptides comprising at least an immunogenic portion of a lung tumor protein, or a variant thereof, wherein the tumor protein comprises an amino acid sequence that is encoded by a polynucleotide sequence selected from the group consisting of:
         (1) sequences recited in SEQ ID NO: 1-109, 111, 113, 115-151, 153, 154, 157, 158, 160, 162-164, 167, 168, 171, 173, 175, 177-224,255-337, 345, 347 and 349;
         (2) sequences that hybridize to a sequence recited in any one of SEQ ID NO:_1-109, 111, 113, 115-151, 153, 154, 157, 158, 160, 162-164, 167, 168, 171, 173, 175, 177-224, 255-337, 345, 347 and 349 under moderately stringent conditions; and
         (3) complements of sequences of (1) or (2);
      (ii) polynucleotides encoding a polypeptide of (i); and
      (iii) antigen presenting cells that expresses a polypeptide of (i);
      such that T cells proliferate; and
   (b) administering to the patient an effective amount of the proliferated T cells, and thereby inhibiting the development of a cancer in the patient.
39. A method for inhibiting the development of a cancer in a patient, comprising the steps of:
   (a) incubating CD4⁺ and/or CD8+ T cells isolated from a patient with at least one component selected from the group consisting of:
      (i) polypeptides comprising at least an immunogenic portion of a lung tumor protein, or a variant thereof, wherein the tumor protein comprises an amino acid sequence that is encoded by a polynucleotide sequence selected from the group consisting of:
         (1) sequences recited in SEQ ID NO: 1-109,111,113, 115-151, 153, 154, 157, 158, 160, 162-164, 167, 168, 171, 173, 175, 177-224,255-337, 345, 347 and 349;
         (2) sequences that hybridize to a sequence recited in any one of SEQ ID NO: 1-109, 111, 113, 115-151, 153, 154, 157, 158, 160, 162-164, 167, 168, 171, 173, 175, 177-224, 255-337, 345, 347 and 349 under moderately stringent conditions; and
         (3) complements of sequences of (1) or (2);
      (ii) polynucleotides encoding a polypeptide of (i); and
      (iii) antigen presenting cells that express a polypeptide of (i);
      such that T cells proliferate;
   (b) cloning at least one proliferated cell to provide cloned T cells; and
   (c) administering to the patient an effective amount of the cloned T cells, and thereby inhibiting the development of a cancer in the patient.
40. A method for determining the presence or absence of a cancer in a patient, comprising the steps of:
   (a) contacting a biological sample obtained from a patient with a binding agent that binds to a lung tumor protein, wherein the tumor protein comprises an amino acid sequence that is encoded by a polynucleotide sequence recited in any one of SEQ ID NO: 1-109, 111, 113, 115-151, 153, 154, 157, 158, 160, 162-164, 167, 168, 171, 173, 175, 177-224, 255-337, 345, 347 and 349 or a complement of any of the foregoing polynucleotide sequences;
   (b) detecting in the sample an amount of polypeptide that binds to the binding agent; and
   (c) comparing the amount of polypeptide to a predetermined cut-off value, and therefrom determining the presence or absence of a cancer in the patient.
41. A method according to clause 40, wherein the binding agent is an antibody.
42. A method according to clause 43, wherein the antibody is a monoclonal antibody.
43. A method according to clause 40, wherein the cancer is lung cancer.
44. A method for monitoring the progression of a cancer in a patient, comprising the steps of:
   (a) contacting a biological sample obtained from a patient at a first point in time with a binding agent that binds to a lung tumor protein, wherein the tumor protein comprises an amino acid sequence that is encoded by a polynucleotide sequence recited in any one of SEQ ID NO: 1-109, 111, 113, 115-151, 153, 154, 157, 158, 160, 162-164, 167, 168, 171, 173; 175, 177-224, 255-337, 345, 347 and 349 or a complement of any of the foregoing polynucleotide sequences;
   (b) detecting in the sample an amount of polypeptide that binds to the binding agent;
   (c) repeating steps (a) and (b) using a biological sample obtained from the patient at a subsequent point in time; and
   (d) comparing the amount of polypeptide detected in step (c) to the amount detected in step (b) and therefrom monitoring the progression of the cancer in the patient.
45. A method according to clause 44, wherein the binding agent is an antibody.
46. A method according to clause 45, wherein the antibody is a monoclonal antibody.
47. A method according to clause 44, wherein the cancer is a lung cancer.
48. A method for determining the presence or absence of a cancer in a patient, comprising the steps of:
   (a) contacting a biological sample obtained from a patient with an oligonucleotide that hybridizes to a polynucleotide that encodes a lung tumor protein, wherein the tumor protein comprises an amino acid sequence that is encoded by a polynucleotide sequence recited in any one of SEQ ID NO: 1-109, 111, 113, 115-151, 153, 154,157,158, 160, 162-164,167, 168,171, 173, 175, 177-224, 255-337, 345, 347 and 349 or a complement of any of the foregoing polynucleotide sequences;
   (b) detecting in the sample an amount of a polynucleotide that hybridizes to the oligonucleotide; and
   (c) comparing the amount of polynucleotide that hybridizes to the oligonucleotide to a predetermined cut-off value, and therefrom determining the presence or absence of a cancer in the patient.
49. A method according to clause 48, wherein the amount of polynucleotide that hybridizes to the oligonucleotide is determined using a polymerase chain reaction.
50. A method according to clause 48, wherein the amount of polynucleotide that hybridizes to the oligonucleotide is determined using a hybridization assay.
51. A method for monitoring the progression of a cancer in a patient, comprising the steps of:
   (a) contacting a biological sample obtained from a patient with an oligonucleotide that hybridizes to a polynucleotide that encodes a lung tumor protein, wherein the tumor protein comprises an amino acid sequence that is encoded by a polynucleotide sequence recited in any one of SEQ ID NO: 1-109, 111, 113, 115-151, 153, 154, 157, 158, 160, 162-164,167,168,171, 173, 175, 177-224, 255-337, 345, 347 and 349 or a complement of any of the foregoing polynucleotide sequences;
   (b) detecting in the sample an amount of a polynucleotide that hybridizes to the oligonucleotide;
   (c) repeating steps (a) and (b) using a biological sample obtained from the patient at a subsequent point in time; and
   (d) comparing the amount of polynucleotide detected in step (c) to the amount detected in step (b) and therefrom monitoring the progression of the cancer in the patient.
52. A method according to clause 51, wherein the amount of polynucleotide that hybridizes to the oligonucleotide is determined using a polymerase chain reaction.
53. A method according to clause 51, wherein the amount of polynucleotide that hybridizes to the oligonucleotide is determined using a hybridization assay.
54. A diagnostic kit, comprising:
   (a) one or more antibodies according to claim 11; and
   (b) a detection reagent comprising a reporter group.
55. A kit according to clause 54, wherein the antibodies are immobilized on a solid support.
56. A kit according to clause 54, wherein the detection reagent comprises an anti-immunoglobulin, protein G, protein A or lectin.
57. A kit according to clause 54, wherein the reporter group is selected from the group consisting of radioisotopes, fluorescent groups, luminescent groups, enzymes, biotin and dye particles.
58. An oligonucleotide comprising 10 to 40 contiguous nucleotides that hybridize under moderately stringent conditions to a polynucleotide that encodes a lung tumor protein, wherein the tumor protein comprises an amino acid sequence that is encoded by a polynucleotide sequence recited in any one of SEQ ID NO: 1-3, 6-8, 10-13, 15-27, 29, 30, 32, 34-49, 51, 52, 54, 55, 57-59, 61-69, 71, 73, 74, 77, 78, 80-82,84, 86-96, 107-109, 111, 113, 125, 127, 128, 129, 131-133, 142, 144, 148-151, 153, 154, 157,158, 160, 167, 168, 171, 173, 175, 179, 182, 184-186, 188-191, 193, 194, 198-207, 209, 210, 213, 214, 217, 220-224, 253, 254-258, 260, 262-264, 270, 272, 275, 276, 279-281, 286, 287, 291, 293, 295, 296, 300, 302, 308-310, 313, 315-317, 323, 345, 347 and 349 or a complement of any of the foregoing polynucleotides.
59. A oligonucleotide according to clause. 58, wherein the oligonucleotide comprises 10-40 contiguous nucleotides recited in any one of SEQ ID NO: 1-3, 6-8, 10-13,15-27, 29, 30, 32, 34-49, 51, 52, 54, 55, 57-59, 61-69, 71, 73, 74, 77, 78, 80-82, 84, 86-96, 107-109, 111, 113, 125, 127, 128, 129, 131-133, 142, 144, 148-151,153,154,157,158,160,167,168,171,173, 175,179,182,184-186, 188-191, 193,194,198-207,209,210,213,214,217,220-224, 253,254-258, 260, 262-264, 270, 272, 275, 276, 279-281, 286, 287, 291, 293, 295, 296, 300, 302, 308-310, 313, 315-317, 323, 345, 347 and 349.
60. A diagnostic kit, comprising:
   (a) an oligonucleotide according to clause 59; and
   (b) a diagnostic reagent for use in a polymerase chain reaction or hybridization assay.

## Claims

1. The use of the polypeptide of SEQ ID NO:176, or an immunogenic portion thereof, or a composition comprising the polypeptide of SEQ ID NO:176, or an immunogenic portion thereof, for the manufacture of a medicament for the treatment of lung cancer.

2. The use of a fusion protein comprising the polypeptide of SEQ ID NO:176, or an immunogenic portion thereof, or a composition comprising the fusion protein for the manufacture of a medicament for the treatment of lung cancer.

3. The use of the polypeptide of SEQ ID NO:176, or an immunogenic portion thereof, or a composition comprising the polypeptide of SEQ ID NO:176, or an immunogenic portion thereof, for the manufacture of a medicament for inducing an immune response in a lung cancer patient.

4. The use of a fusion protein comprising the polypeptide of SEQ ID NO:176, or an immunogenic portion thereof, or a composition comprising the fusion protein for the manufacture of a medicament for inducing an immune response in a lung cancer patient.

5. The use of the polynucleotide of SEQ ID No. 175 or a composition comprising the polynucleotide of SEQ ID NO:175 for the manufacture of a medicament for the treatment of lung cancer.

6. The use of the polynucleotide of SEQ ID No. 175 or a composition comprising the polynucleotide of SEQ ID NO:175 for the manufacture of a medicament for inducing an immune response in a lung cancer patient.

7. The use of any one of claims 1-6, wherein the composition comprises an immunostimulant.

8. The use of claim 7 wherein the immunostimulant induces an immune response predominantly of the Th1 type.

9. The use of claim 7 wherein the immunostimulant is selected from the group consisting of monophosphoryl lipid A, 3d-MPL, RC-529, AGP's, Seppic Montanide ISA 720, a cytokine, a microsphere, AS-2, QS21, saponin based adjuvants, and immune stimulating complex (iscom) based adjuvants.
